# EUROPEAN PATENT APPLICATION

(11) **EP 1 529 843 A2**
(43) Date of publication of application: **11.05.2005**
(21) Application number: 04105707.6
(22) Date of filing: 30.07.2001
(51) Int. Cl.: C12N 15/12, C12N 15/62, C12N 5/10, C12N 1/21, C07K 14/47, C07K 14/475, C07K 16/18, A61K 39/395, A61K 31/713, A61K 38/18

(54) **Novel secreted proteins and their uses**

(30) Priority: 11.08.2000 US 224642 P; 19.10.2000 US 241779 P
(62) Divisional of application: 01958861.5
(71) Applicant: Eli Lilly & Company, Indianapolis, IN 46285 (US)
(72) Inventor: Edmonds, Brian Taylor, Carmel, IN 46032 (US); Micanovic, Radmila, Indianapolis, IN 46236 (US); Ou, Weijia, Fishers, IN 46038 (US); Su, Eric Wen, Carmel, IN 46032 (US); Tschang, Sheng-Hung Rainbow, Carmel, IN 46033 (US); Wang, He, Carmel, IN 46033 (US)
(74) Representative: Ingham, Stephen H.

(57) **Abstract**

The present invention provides nucleic acid sequences encoding novel human proteins. These novel nucleic acids are useful for constructing the claimed DNA vectors and host cells of the invention and for preparing the claimed nucleic acids, recombinant proteins and antibodies that are useful in the claimed methods and medical uses.

## Description

### FIELD OF THE INVENTION

This application claims priority of Provisional Applications Serial No. 60/224,642 filed August 11, 2000, and Serial No. 60/241,779 filed October 19, 2000.

The present invention relates to the identification and isolation of novel DNA, therapeutic and drug discovery uses, and the recombinant production of novel secreted polypeptides, designated herein as LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), and LP223(b). The present invention also relates to vectors, host cells, and antibodies directed to these polypeptides.

### BACKGROUND OF THE INVENTION

Extracellular proteins play an important role in the formation, differentiation and maintenance of multicellular organisms. The fate of many individual cells, e.g., proliferation, migration, differentiation, or interaction with other cells, is typically governed by information received from other cells and/or the immediate environment. This information is often transmitted by secreted polypeptides (for instance, mitogenic factors, survival factors, cytotoxic factors, differentiation factors, neuropeptides, and hormones) which are, in turn, received and interpreted by diverse cell receptors or membrane-bound proteins. These secreted polypeptides or signaling molecules normally pass through the cellular secretory pathway to reach their site of action in the extracellular environment.

Secreted proteins have various industrial applications, including pharmaceuticals, diagnostics, biosensors and bioreactors. Most protein drugs available at present such as thrombolytic agents, interferons, interleukins, erythropoietins, colony stimulating factors, and various other cytokines are secretory proteins. Their receptors, which are membrane proteins, also have potential as therapeutic or diagnostic agents.

The present invention describes the cloning and characterization of novel proteins, termed LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), and LP223(b), as well as active variants and/or fragments thereof.

### SUMMARY OF THE INVENTION

The present invention provides isolated LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), and LP223(b) polypeptide encoding nucleic acids and the polypeptides encoded thereby, including fragments and/or specified variants thereof. Contemplated by the present invention are LP probes, primers, recombinant vectors, host cells, transgenic animals, chimeric antibodies and constructs, LP polypeptide antibodies, as well as methods of making and using them diagnostically and therapeutically as described and enabled herein.

The present invention includes isolated nucleic acid molecules comprising polynucleotides that encode LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), and LP223(b) polypeptides as defined herein, as well as fragments and/or specified variants thereof, or isolated nucleic acid molecules that are complementary to polynucleotides that encode such LP polypeptides, or fragments and/or specified variants thereof as defined herein.

A polypeptide of the present invention includes an isolated LP polypeptide comprising at least one fragment, domain, or specified variant of at least 90-100% of the contiguous amino acids of at least one portion of SEQ ID NO:2, 4, 6, 8, 10, 12, 14, 16, 18, 20, 22, 24, 26, 28, 30, 32, 34, 36, or 38.

The present invention also provides an isolated LP polypeptide as described herein, wherein the polypeptide further comprises at least one specified substitution, insertion, or deletion corresponding to portions or specific residues of SEQ ID NO:2, 4, 6, 8, 10, 12, 14, 16, 18, 20, 22, 24, 26, 28, 30, 32, 34, 36, or 38.

The present invention also provides an isolated nucleic acid probe, primer, or fragment, as described herein, wherein the nucleic acid comprises a polynucleotide of at least 10 nucleotides, corresponding or complementary to at least 10 nucleotides of SEQ ID NO:1, 3, 5, 7, 9, 11, 13, 15, 17, 19, 21, 23, 25, 27, 29, 31, 33, 35, or 37.

The present invention also provides compositions, including pharmaceutical compositions, comprising an LP polypeptide, an LP polypeptide-encoding polynucleotide, an LP polynucleotide, and/or an LP polypeptide antibody, wherein the composition has a measurable effect on an activity associated with a particular LP polypeptide as disclosed herein. A method of treatment or prophylaxis based on an LP polypeptide associated activity as disclosed herein can be effected by administration of one or more of the polypeptides, nucleic acids, antibodies, vectors, host cells, transgenic cells, and/or compositions described herein to a mammal in need of such treatment or prophylactic. Accordingly, the present invention also includes methods for the prophylaxis or treatment of a patho-physiological condition in which at least one cell type involved in said condition is sensitive or responsive to an LP polypeptide, LP polypeptide-encoding polynucleotide, LP nucleic acid, LP polypeptide antibody, host cell, transgenic cell, and/or composition of the present invention.

The present invention also provides an article of manufacture comprising a container, holding a composition effective for treating a condition disclosed herein, and a label.

The present invention also provides a method for identifying compounds that bind an LP polypeptide, comprising:
a) admixing at least one isolated LP polypeptide as described herein with a test compound or composition; and
b) detecting at least one binding interaction between the polypeptide and the compound or composition, optionally further comprising detecting a change in biological activity, such as a reduction or increase.

### DETAILED DESCRIPTION OF THE PREFERRED EMBODIMENTS

Applicants have identified cDNA clones comprising polynucleotides that encode novel polypeptides or novel variants of known polypeptides:

### 1) LP105

LP105 polypeptides comprising the amino acid sequence of the open reading frame encoded by the polynucleotide sequence as shown in SEQ ID NO:1 are contemplated as one embodiment of the present invention. Specifically, polypeptides of the present invention comprise the amino acid sequence as shown in SEQ ID NO:2, as well as fragments, variants, and derivatives thereof. Accordingly, LP105 polynucleotides encoding the LP105 polypeptides of the present invention are also contemplated by the present invention. LP105 polynucleotides are predominantly expressed in prostate and kidney tissues.

The LP105 polypeptide as shown in SEQ ID NO:2 shares sequence similarity with a recently reported human lefty protein (WO 99/09198). Lefty proteins are members of the TGF-beta family and two LEFTY genes, LEFTY A and B, have been localized by FISH to 1q42, a region syntenic to the location to which the mouse Lefty genes have been mapped at 1H5 [Kosaki, *et al.*, *Am. J. Hum. Genet*. 64(3):712-21 (1999); Meno, *et al*., *Genes Cells* 2(8):513-24 (1997)]. LEFTY A is identical to EBAF [Kothapalli, *et al*., *J*. *Clin*. *Invest*. 99(10):2342-50 (1997)]. Analysis of 126 human cases of L-R axis malformation showed a single nonsense and a single missense mutation in the LEFTY A gene. Both mutations lay in the cysteine-knot region of the LEFTY A protein and the phenotype of affected individuals was very similar to that typically seen in Lefty-1 -/- mice with L-R axis malformations. Furthermore, the LP105 polypeptide as shown in SEQ ID NO:2 shares sequence similarity with human bone morphogenic protein 18 (WO 99/29718). Compositions comprising LP105 polypeptides, polynucleotides, and/or antibodies are useful for the treatment of defects in or wounds to tissues including, but not limited to, epidermis, nerve, muscle, cardiac muscle, and organs including, but not limited to liver, lung, epithelium, brain, spleen, cardiac, pancreas and kidney. Furthermore, compositions comprising LP105 polypeptides, polynucleotides, and/or antibodies can be useful for modulating sexual development, pituitary hormone production, hematopoiesis, wound healing, tissue repair, and the formation of bone and cartilage. Compositions comprising LP105 polypeptides, polynucleotides, and/or antibodies can also be used to treat such conditions as cancer including, but not limited to prostate and kidney cancer, interstitial lung disease, infectious diseases, autoimmune diseases, arthritis, leukemia, lymphomas, immunosuppression, immunity, humoral immunity, inflammatory bowel disease, myelosuppression, periodontal disease, osteoarthritis, osteoporosis, and other abnormalities of bone, cartilage, muscle, tendon, ligament, meniscus, and/or other connective tissues as well as dysfunctional growth and differentiation patterns of cells.

### 2) LP061

In another embodiment, polypeptides comprising the amino acid sequence of the open reading frame encoded by the polynucleotide sequence as shown in SEQ ID NO:3 are contemplated by the present invention. Specifically, polypeptides of the present invention comprise the amino acid sequence as shown in SEQ ID NO:4, as well as fragments, variants, and derivatives thereof. Accordingly, LP061 polynucleotides encoding LP061 polypeptides of the present invention are also contemplated by the present invention. LP061 polynucleotides are predominantly expressed in diseased thyroid. The present invention includes a human nucleotide sequence (Incyte clone 2719035H1) as shown in SEQ ID NO:3 which appears to be a shortened splice-variant of the published human fukutin nucleotide sequence (GenBank AB008226).

Fukuyama-type congenital muscular dystrophy (FCMD), one of the most common autosomal recessive disorders in Japan (incidence is 0.7 to 1.2 per 10,000 births), is characterized by congenital muscular dystrophy, in conjunction with brain malformation (micropolygria). The FCMD gene was mapped to a region of less than 100 kilobases which included the marker locus D9S2107 on human chromosome 9q31. The mutation responsible for FCMD is a retrotransposal insertion of tandemly repeated sequences within this candidate-gene in all FCMD chromosomes carrying the founder haplotype (87%). The inserted sequence is about 3 kilobases long and is located in the 3' untranslated region of a gene encoding a new 461 amino acid protein. This novel gene, termed fukutin, is expressed in heart, brain, skeletal muscle, pancreas and lymphoblasts in normal individuals, but not in FCMD patients who carry the insertion. Two independent point mutations confirm that mutation of this gene is responsible for FCMD. The predicted fukutin protein contains an amino-terminal signal sequence and one glycosylation site, which together with results from transfection experiments suggests that fukutin is a secreted protein. Abnormalities in basal lamina in FCMD muscle and brain have been seen by electron microscopy [Nakano, *et al*., *Acta Neuropathol.* 91(3):313-21 (1996); Ishii, *et al., Neuromuscul*. *Disord*. 7(3):191-7 (1997)]. Therefore it has been suggested that secreted fukutin may be associated with the extracellular matrix surrounding expressing cells where it forms a complex with other extracellular components to stabilize a microenvironment supportive for normal cellular/tissue function. In muscle, fukutin complexes might stabilize the function of muscle fibers/sarcomeres; in brain, fukutin may assist the migration of neuronal precursors during cortical development [Fukuyama, *et al.*, *Brain Dev.* 3 (1) :1-29 (1981).

Accordingly, compositions comprising LP061 polypeptides, polynucleotides, and/or antibodies are useful for diagnosis, treatment and intervention of diseases, disorders, and/or conditions including, but not limited to, brain malformation (micropolygria), Alzheimer's Disease, Parkinson's Disease, Huntington's Disease, Tourette Syndrome, ALS, muscular pathologies including, but not limited to, muscular dystrophies including, but not limited to, congenital muscular dystrophies such as fukuyama-type congenital muscular dystrophy, meningitis, encephalitis, demyelinating diseases, peripheral neuropathies, neoplasia, trauma, spinal cord injuries, ischemia and infarction, aneurysms, hemorrhages, schizophrenia, mania, dementia, paranoia, obsessive compulsive disorder, panic disorder learning disabilities, psychoses, autism, and altered behaviors, including disorders in feeding, sleep patterns, balance, and perception.

### 3) LP224

In another embodiment, polypeptides comprising the amino acid sequence of the open reading frame encoded by the polynucleotide sequence as shown in SEQ ID NO:5 are contemplated by the present invention. Specifically, polypeptides of the present invention comprise the amino acid sequence as shown in SEQ ID NO:6, as well as fragments, variants, and derivatives thereof. Accordingly, LP224 polynucleotides encoding LP224 polypeptides of the present invention are also contemplated by the present invention.

The gene encoding the LP224 polypeptide has been localized to chromosome 4p16 (GenBank hit g7022852) and the LP224 polypeptide as shown in SEQ ID NO:6 shares sequence similarity with a protein encoded by the leucine-rich, glioma inactivated 1 tumor suppressor gene. LGI1 has the highest homology with a number of transmembrane and extracellular proteins which function as receptors and adhesion proteins. LGI1 is predominantly expressed in neural tissues, especially in brain; its expression is reduced in low grade brain tumors and it is significantly reduced or absent in malignant gliomas. Its localization to the 10q24 region, and rearrangements or inactivation in malignant brain tumors, suggest that LGI1 is a candidate tumor suppressor gene involved in progression of glial tumors [Chernova, *et al*., *Oncogene* 17(22):2873-81 (1998)]. Accordingly, compositions comprising LP224 polypeptides, polynucleotides, and/or antibodies are useful for diagnosis, treatment and intervention of bipolar affective disorder [Ewald, *et al*., *Mol*. *Psychiatry* 3(5):442-8 (1998)], hearing defects [Van Camp, *et al*., *J*. *Med*. *Genet*. 36(7):532-6, (1999)], cherubism [Mangion, *et al*., *Am*. *J*. *Hum*. *Genet*. 65(1):151-7 (1999)], Wolf-Hirschhorn (WH) syndrome [Zollino, *et al.*, *Am. J. Med. Genet.* 82(5):371-5 (1999); *Ann. Genet.* 41(2):73-6 (1998)], Ellis-van Creveld syndrome [EVC; Polymeropoulos, *et al*., *Genomics* 35(1):1-5 (1996)], autosomal dominant postaxial polydactyly, nail dystrophy, and dental abnormalities [Howard, *et al*., *Am*. *J*. *Hum*. *Genet*. 61(6):1405-12 (1997)], Pitt-Rogers-Danks (PRD) syndrome and overgrowth syndrome [Partington, *et al*., *J*. *Med*. *Genet*. 34(9):719-28 (1997)], bladder cancer and malignant brain tumors [Bell, *et al.*, *Genes Chromosomes Cancer* 17(2):108-17 (1996)].

### 4) LP240

In another embodiment, polypeptides comprising the amino acid sequence of the open reading frame encoded by the polynucleotide sequence as shown in SEQ ID NO:7 are contemplated by the present invention. Specifically, polypeptides of the present invention comprise the amino acid sequence as shown in SEQ ID NO:8, as well as fragments, variants, and derivatives thereof. Accordingly, LP240 polynucleotides encoding LP240 polypeptides of the present invention are also contemplated by the present invention. The gene encoding the LP240 polypeptide as shown in SEQ ID NO:8 has been localized to chromosome 19.

LP240 polynucleotides, polypeptides, and antibodies corresponding to LP240 are useful for diagnosis, treatment and intervention of diseases, disorders, and/or conditions including, but not limited to, stroke, Alzheimer's disease, Parkinson's disease, Huntington's disease, neurodegenerative disorders, brain cancer, cardiovascular disease, atherosclerosis, myocardial infarction, inflammation, and rheumatoid arthritis.

### 5) LP239(a) and LP239(b)

In another embodiment, polypeptides comprising the amino acid sequences of the open reading frames encoded by the polynucleotide sequences as shown in SEQ ID NO:9 and SEQ ID NO:23 are contemplated by the present invention. Specifically, LP239(a) and LP239(b) polypeptides of the present invention comprise the amino acid sequences as shown in SEQ ID NO:10 and SEQ ID NO:24, respectively, as well as fragments, variants, and derivatives thereof. Accordingly, LP239(a) and LP239(b) polynucleotides comprising polynucleotides as identified in SEQ ID NO:9 and SEQ ID NO:23, respectively, are also contemplated by the present invention.

LP239 polypeptide-encoding polynucleotide sequences are primarily expressed in digestive, hemic, immune, and nervous system tissues. The LP239(a) and LP239(b) polypeptides as shown in SEQ ID NO:10 and SEQ ID NO:24 share sequence similarity with a thyroxine-binding globulin [see AF204929; Mori, *et al.*, *Endocr. J.* 46(4):613-9 (1999)]. LP239 polypeptides therefore may serve as hormone binding proteins in serum, protease inhibitors, or hormones when administered to patients suffering from a deficiency of endogenous LP239 polypeptides. LP239 polynucleotides, polypeptides, and antibodies corresponding to LP239 are useful for diagnosis, treatment and intervention of diseases, disorders, and/or conditions including, but not limited to, hypothyroidism, anemia, sepsis, gram negative bacteremia, allergic responses, allergic autoimmune diseases, type 1 diabetes, Th1-dependent insulitis, inflammation, multiple sclerosis, rheumatoid arthritis, inflammatory bowel disease, liver failure, ARDS, cancers, leukemia, and immunodeficiency.

### 6) LP243 (a) and LP243 (b)

In another embodiment, polypeptides comprising the amino acid sequences of the open reading frames encoded by the polynucleotide sequences as shown in SEQ ID NO:11 and SEQ ID NO:13 are contemplated by the present invention. Specifically, LP243(a) and LP243(b) polypeptides of the present invention comprise the amino acid sequences as shown in SEQ ID NO:12 and SEQ ID NO:14, respectively, as well as fragments, variants, and derivatives thereof. Accordingly, LP243(a) and LP243(b) polynucleotides comprising polynucleotides as identified in SEQ ID NO:11 and SEQ ID NO:13 are also contemplated by the present invention.

The gene encoding the disclosed LP243 polypeptides have been localized to chromosome 19p13.3 (GenBank hit g2894631) and is mainly expressed in nervous system and digestive system. The LP243 polypeptides as shown in SEQ ID NO:12 and SEQ ID NO:14 share sequence similarity with the amino acid sequence of protein PRO227 disclosed in WO 99/14328, the amino acid sequence of the human Tango-79 protein disclosed in WO 99/06427, the glioma amplified on chromosome 1 (GAC1) protein (AF030435), and the *Rattus norvegicus* (AF133730) SLIT protein. GAC1 is a member of the leucine-rich repeat superfamily on chromosome band 1q32.1. GAC1 is amplified and overexpressed in malignant gliomas [Almeida, *et al., Oncogene* 16(23):2997 (1998)]. In *Drosophila*, at least, SLIT proteins are believed to be involved in axon pathway development. The two predicted proteins LP243(a) and LP243(b) as shown in SEQ ID NO:12 and 14, respectively, have different N-terminals but are identical starting from amino acid 27 through amino acid 557 as shown in SEQ ID NO:12. The present invention provides isolated LP243(a) and LP243(b) polypeptides as described herein, wherein the polypeptide has at least one activity, such as, but not limited to, inducing cellular proliferation, tumorigenesis, synapse formation, neurotransmission, learning, cognition, homeostasis, neuronal outgrowth, differentiation or survival, or tissue regeneration including but not limited to neural tissue regeneration. An LP243 polynucleotide, polypeptide, and/or antibody can thus be screened for a corresponding activity according to known methods.

The present invention also provides a composition comprising an isolated LP243 nucleic acid, polypeptide, and/or antibody as described herein and a carrier or diluent. The carrier or diluent can optionally be pharmaceutically acceptable, according to known methods. LP243 polynucleotides are expressed in nervous tissues and the polypeptides encoded thereby appear to play a role in tumorigenesis, neurodegenerative diseases, behavioral disorders, and/or inflammatory conditions. Accordingly, compositions comprising LP243 polypeptides, polynucleotides, and/or antibodies are useful for diagnosis, treatment and intervention of diseases, disorders, and/or conditions including, but not limited to, cancer, including, but not limited to sporadic ovarian tumors [Wang, *et al*., *Br*. *J*. *Cancer* 80(1-2):70-2 (1999)], Peutz-Jeghers syndrome [Nakagawa, *et al.*, *Hum*. *Genet*. 102(2):203-6 (1998); Olschwang, *et al.*, *J*. *Med*. *Genet*. 35(1):42-4 (1998)], adenoma malignum of the uterine cervix [Lee, *et al*., *Cancer Res.* 15;58(6):1140-3 (1998)], pancreatic carcinomas [Hoglund, *et al., Genes Chromosomes Cancer* 21(1):8-16 (1998)], multiple myeloma and plasma cell leukemia [Taniwaki, *et al*., *Blood* 84(7):2283-90 (1994)], Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder, learning disabilities, ALS, psychoses, autism, and altered behaviors, including disorders in feeding, sleep patterns, balance, and perception.

### 7) LP253

Cytokines are secreted regulatory peptides that mediate a wide range of biological activities by binding to specific cell surface receptors on target cells. Cytokine actions include control of cell proliferation and differentiation, regulation of hematopoiesis, and immune inflammatory responses. Cytokines are also major orchestrators of host defense processes and, as such, are involved in responses to exogenous as well as endogenous insults and in repair or restoration of tissue integrity. In order for a cytokine to exert its effect on cells, it is now accepted by those skilled in the art that the molecule must interact with molecules, located on cell membranes, referred to as receptors. Patents which exemplify disclosures of interleukin receptors include Honjo, *et al.*, U.S. Patent 4,816,565; Urdal, *et al*., U.S. Patent 4,578,335; Dower, *et al.*, U.S. Patent 5,180,812; and Taniguchi, *et al.*, U.S. Patent 5,198,359; and WO 2000/15759, the disclosures of which are incorporated by reference.

In another embodiment of the present invention, we provide polypeptides comprising the amino acid sequence of the open reading frame encoded by the polynucleotide sequence as shown in SEQ ID NO:15. Specifically, LP253 polypeptides of the present invention comprise the amino acid sequence as shown in SEQ ID NO:16, as well as fragments, variants, and derivatives thereof. Accordingly, LP253 polynucleotides comprising polynucleotides as identified in SEQ ID NO:15 are also contemplated by the present invention. LP253 encoding polynucleotide sequences are primarily expressed in digestive, hemic, immune, and nervous system tissues. The LP253 polypeptide as shown in SEQ ID NO:16 shares structural similarity with other interleukin receptors.

The effects of IL-1 *in vivo* can be regulated via the administration of a soluble form of its receptor [Fanslow, *et al*., *Science* 248:739-41 (1990)]. Systemic administration of a soluble, extracellular portion of the receptor for IL-1 (soluble IL-1R) had profound inhibitory effects on the development of *in vivo* alloreactivity. Survival of heterotopic heart allografts was prolonged from twelve days in controls to seventeen days in mice treated with soluble IL-1R. Lymph node hyperplasia in response to localized injection of allogeneic cells was completely blocked by soluble IL-1R treatment.

The availability of the purified LP253 polypeptide, in soluble form, presents therapeutic possibilities as well. The results that Fanslow report demonstrate the ability of a soluble cytokine receptor to modulate biological activity upon exogeneous administration *in vivo,* presumably by acting as a neutralizing agent for the endogeneously produced, corresponding ligand, and provides evidence of the therapeutic potential of soluble cytokine receptors in a variety of clinical disorders.

Administration of a soluble form of LP253 would interfere with the effect of its endogenous ligand on the cells, since the ligand would not bind to the endogenous membrane bound LP253 as freely. Hence, an aspect of the present invention is the treatment of pathological conditions caused by excess expression and/or activity of LP253 polypeptides by adding an amount of soluble LP253 polypeptides sufficient to inhibit binding of a cytokine to the aforementioned cells. This methodology can also be modified, and the soluble receptor can also be used as a screening agent for pharmaceuticals. Briefly, a pharmaceutical which works as an LP253 antagonist can do so by blocking the binding of endogenous ligand to the LP253. Prior to determining whether a material would be effective *in vivo*, one may use the purified LP253 polypeptide in connection with a potential pharmaceutical to determine if there is binding. If there is in fact binding, further testing may be indicated.

Expression of recombinant polypeptides in high levels and its use as an antigen allows production of additional neutralizing monoclonal and polyclonal antibodies. Such neutralizing antibodies can be used in *in vivo* model settings to elucidate the role that LP253 and its ligand play in normal as well as pathologic immune responses (i.e., disease states that are aggravated by activated T- and NK-cells like auto-immune diseases, graft versus host disease and rheumatoid arthritis). Thus, purified LP253 polypeptides, polynucleotides, and/or antibodies compositions will be useful in diagnostic assays for LP253 and its ligand, and also in raising antibodies to LP253 for use in diagnosis or therapy.

LP253 polypeptides, polynucleotides, and/or antibodies can be administered, for example, for the purpose of suppressing immune responses in a human. A variety of diseases or conditions are caused by an immune response to alloantigen, including allograft rejection and graft-versus-host reaction. In alloantigen-induced immune responses, soluble LP253 polypeptides may suppress lymphoproliferation and inflammation which result upon activation of T cells. Soluble LP253 polypeptides may therefore be used to effectively suppress alloantigen-induced immune responses in the clinical treatment of, for example, rejection of allografts (such as skin, kidney, and heart transplants), and graft-versus-host reactions in patients who have received bone marrow transplants.

LP253 polypeptides, polynucleotides, and/or antibodies may also be used in clinical treatment of autoimmune dysfunctions, such a rheumatoid arthritis, diabetes and multiple sclerosis, which are dependent upon the activation of T cells against antigens not recognized as being indigenous to the host. LP253 polypeptides, polynucleotides, and/or antibodies may also be useful in treatment of septic shock in which interferon gamma produced in response to various interleukins plays a central role in causing morbidity and mortality [Doherty, *et al.*, *J. Immunol.* 149:1666 (1992)]. In addition, compositions comprising soluble LP253 polypeptides may be used directly in therapy to bind or scavenge endogenous LP253 ligands, thereby providing a means for regulating the immune or inflammatory activities. In its use to prevent or reverse pathologic immune responses, soluble LP253 polypeptides can be combined with other cytokine antagonists such as antibodies to the other known interleukin receptors, soluble interleukin receptors, soluble TNF (tumor necrosis factor) receptors, and/or interleukin receptor antagonists, and the like.

The dose ranges for the administration of the LP253 polypeptides, polynucleotides, and/or antibodies and fragments thereof may be determined by those of ordinary skill in the art without undue experimentation. In general, appropriate dosages are those which are large enough to produce the desired effect, for example, blocking the binding of endogenous ligands of LP253 to endogenous LP253.

### 8) LP218

In another embodiment, polypeptides comprising the amino acid sequence of the open reading frame encoded by the polynucleotide sequence as shown in SEQ ID NO:17 are contemplated by the present invention. Specifically, LP218 polypeptides of the present invention comprise the amino acid sequence as shown in SEQ ID NO:18, as well as fragments, variants, and derivatives thereof. Accordingly, LP218 polynucleotides comprising polynucleotides as identified in SEQ ID NO:17 are also contemplated by the present invention.

The gene encoding the LP218 polypeptide as shown in SEQ ID NO:18 has been localized to chromosome 22q11 and shares sequence similarity with acetyl LDL receptor. LP218 polynucleotides are expressed in hemic, immune, reproductive, and urinary tract tissues. Accordingly, compositions comprising LP218 polypeptides, polynucleotides, and/or antibodies are useful for diagnosis, treatment and intervention of schizophrenia [Li, *Mol*. *Psychiatry* 5(1):77-84 (2000)], hypocalcemia [Garabedian, *et al*., *Genet Couns*. 10(4):389-94 (1999)], rhabdoid tumor [Zhou, *et al.*, *Gene* 241(1):133-41 (2000)], DiGeorge/velo-cardio-facial syndrome [Amati, *et al*., *Eur*. *J*. *Hum*. *Genet*. 7(8):903-9 (1999)], congenital heart disease [Borgmann, *et al.*, *Eur*. *J*. *Pediatr*. 158(12):958-63 (1999)], and abdominal lymphatic dysplasia [Mansir, *et al*., *Genet*. *Couns.* 10(1):67-70 (1999)], oesophageal atresia [Digilio, *et al*., *J*. *Med*. *Genet*. 36 (2) :137-9 (1999)].

### 9) LP251(a) and LP251(b)

In another embodiment, polypeptides comprising the amino acid sequence of the open reading frames encoded by the polynucleotide sequences as shown in SEQ ID NO:19 and SEQ ID NO:33 are contemplated by the present invention. Specifically, LP251(a) and LP251(b) polypeptides of the present invention comprise the amino acid sequence as shown in SEQ ID NO:20 and SEQ ID NO:34, respectively, as well as fragments, variants, and derivatives thereof. Accordingly, LP251(a) and LP251(b) polynucleotides comprising polynucleotides as identified in SEQ ID NO:19 or SEQ ID NO:33 are also contemplated by the present invention.

The LP251 polypeptides as shown in SEQ ID NO:20 and SEQ ID NO:34 share sequence similarity with aqualysin I precursor, a subtilisin-type serine protease which is secreted into the culture medium by *Thermus aquaticus* YT-1, an extremely thermophilic gram-negative bacterium [Terada, *et al.*, *J. Biol. Chem.* 265(12):6576-81 (1990)].

The gene encoding the disclosed polypeptides has been localized to chromosome 1p33-p34.3. Accordingly, compositions comprising LP251 polypeptides, polynucleotides, and/or antibodies are useful for diagnosis, treatment and intervention of breast cancer [Emi, *et al*., *Genes Chromosomes Cancer* 26(2):134-41 (1999)], myelodysplastic syndromes including but not limited to thrombocytemia and abnormal megakaryopoiesis [Jondeau, *et al*., *Leukemia* 10(11):1692-5 (1996)], Schnyder's crystalline corneal dystrophy [Shearman, *et al*., *Hum*. *Mol*. *Genet*. 5(10):1667-72 1996), tumorigenesis including, but not limited to, colorectal cancer [Praml, *et al*., *Oncogene* 11(7):1357-62 (1995)], Schwartz-Jampel syndrome [Nicole, *Hum*. *Mol*. *Genet*. 4(9):1633-6 (1995), and autosomal dominant hypercholesterolemia [Varret, *et al.*, *Am. J. Hum. Genet.* 64 (5) :1378-87 (1999)].

Furthermore, LP251 polynucleotides are mainly expressed in skin and liver. Accordingly, compositions comprising LP251 polypeptides, polynucleotides, and/or antibodies are generally useful for the treatment of defects in or wounds to tissues including, but not limited to skin and liver.

### 10) LP252

In another embodiment, polypeptides comprising the amino acid sequence of the open reading frame encoded by the polynucleotide sequence as shown in SEQ ID NO:21 are contemplated by the present invention. Specifically, LP252 polypeptides of the present invention comprise the amino acid sequence as shown in SEQ ID NO:22, as well as fragments, variants, and derivatives thereof. Accordingly, LP252 polynucleotides comprising polynucleotides as identified in SEQ ID NO:21 are also contemplated by the present invention.

The LP252 polypeptide as shown in SEQ ID NO:22 shares sequence similarity with thyroid hormone-induced protein B precursor. Furthermore, the gene encoding the disclosed polypeptide has been localized to chromosome 9p12-13. Accordingly, compositions comprising LP252 polypeptides, polynucleotides, and/or antibodies are useful for diagnosis, treatment and intervention of ectrodactyly, ectodermal dysplasia and cleft lip/palate syndrome [Fukushima, *et al*., *Clin*. *Genet.* 44(1):50 (1993); Hasegawa, *et al*., *Clin*. *Genet*. 40(3):202-6 (1991)]; and cancer, including, but not limited to, kidney cancer and carcinoma of the respiratory tract [Schraml, *et al*., *J*. *Pathol.* 190(4):457-61 (2000); Higashi, *et al.*, *Genes Chromosomes Cancer* 3(1):21-3 (1991)].

### 11) LP223(a) and (b)

In another embodiment, polypeptides comprising the amino acid sequences of the open reading frames encoded by the polynucleotide sequences as shown in SEQ ID NO:25 and SEQ ID NO:37 are contemplated by the present invention. Specifically, LP223(a) and LP223(b) polypeptides of the present invention comprise the amino acid sequences as shown in SEQ ID NO:26 and SEQ ID NO:38, respectively, as well as fragments, variants, and derivatives thereof. Accordingly, LP223(a) and LP223(b) polynucleotides comprising polynucleotides as identified in SEQ ID NO:25 and SEQ ID NO:37 are also contemplated by the present invention. The LP223(a) and LP223(b) polypeptides as shown in SEQ ID NO:26 and SEQ ID NO:38 share sequence similarity with a human secreted protein sequence disclosed in WO 2000/04140.

The present invention also provides isolated LP223 polypeptides as described herein, wherein the polypeptides have at least one activity, such as, but not limited to, inducing cellular proliferation, synapse formation, neurotransmission, learning, cognition, homeostasis, neuronal outgrowth, differentiation or survival, or tissue regeneration including but not limited to neural tissue regeneration. An LP223 polynucleotide, polypeptide, and/or antibody can thus be screened for a corresponding activity according to known methods.

The present invention also provides a composition comprising an isolated LP223 nucleic acid, polypeptide, and/or antibody as described herein and a carrier or diluent. The carrier or diluent can optionally be pharmaceutically acceptable, according to known methods. LP223 polynucleotides and polypeptides are expressed in nervous tissues and appear to play a role in neurodegenerative diseases, behavioral disorders, and/or inflammatory conditions. Accordingly, compositions comprising LP223 polypeptides, polynucleotides, and/or antibodies are useful for diagnosis, treatment and intervention of diseases, disorders, and/or conditions including, but not limited to, Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder learning disabilities, ALS, psychoses, autism, and altered behaviors, including disorders in feeding, sleep patterns, balance, and perception. In addition, the gene or gene product may also play a role in the treatment and/or detection of developmental disorders associated with the developing embryo, sexually-linked disorders, or disorders of the cardiovascular system.

### 12) LP255(a) and LP255(b)

In another embodiment, polypeptides comprising the amino acid sequences of the open reading frames encoded by the polynucleotide sequences as shown in SEQ ID NO:27 and SEQ ID NO:35 are contemplated by the present invention. Specifically, LP255(a) and LP255(b) polypeptides of the present invention comprise the amino acid sequences as shown in SEQ ID NO:28 and SEQ ID NO:36, respectively, as well as fragments, variants, and derivatives thereof. Accordingly, LP255(a) and LP255(b) polynucleotides comprising polynucleotides as identified in SEQ ID NO:27 and SEQ ID NO:35 are also contemplated by the present invention. The LP255(a) and LP255(b) polypeptides as shown in SEQ ID NO:28 and SEQ ID NO:36 share sequence similarity with a human secreted protein sequence disclosed in WO 99/14328 as PR0332.

The present invention also provides isolated LP255 polypeptides as described herein, wherein the polypeptides have at least one activity, such as, but not limited to, promoting cell growth. An LP255 polynucleotide, polypeptide, and/or antibody can thus be screened for a corresponding activity according to known methods.

The present invention also provides a composition comprising an isolated LP255 nucleic acid, polypeptide, and/or antibody as described herein and a carrier or diluent. The carrier or diluent can optionally be pharmaceutically acceptable, according to known methods. LP255 polynucleotides and polypeptides are expressed in nervous tissues and appear to play a role in neurological disorders. Accordingly, compositions comprising LP255 polypeptides, polynucleotides, and/or antibodies are useful for diagnosis, treatment and intervention of diseases, disorders, and/or conditions arising from Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, panic disorder learning disabilities, ALS, psychoses, autism, and altered behaviors, including disorders in feeding, sleep patterns, balance, and perception.

### 13) LP244

In another embodiment, polypeptides comprising the amino acid sequence of the open reading frame encoded by the polynucleotide sequence as shown in SEQ ID NO:29 are contemplated by the present invention. Specifically, LP244 polypeptides of the present invention comprise the amino acid sequence as shown in SEQ ID NO:30, as well as fragments, variants, and derivatives thereof. Accordingly, LP244 polynucleotides comprising polynucleotides as identified in SEQ ID NO:29 are also contemplated by the present invention.

The gene encoding the LP244 polypeptide has been localized to chromosome 19 and is predominantly expressed in embryonic structures. The LP244 polypeptide as shown in SEQ ID NO:30 appears to be a novel shortened splice-variant of the human Epstein-Barr virus induced gene 3 (EBI3; GenBank accession no. NP_005746) wherein the first 67 amino acids of the LP244 polypeptide are identical to those of EBI3. EBI3 is reported to be a hematopoietin receptor family member related to the p40 subunit of interleukin-12 and to the ciliary neurotrophic factor receptor, whose expression is induced in B lymphocytes by Epstein-Barr virus (EBV) infection [Devergne, *et al*., *J*. *Virology* 70 (2) : 1143-53 (1996)]. The gene encodes a 34-kDa glycoprotein which lacks a membrane-anchoring motif and is secreted. Despite the absence of a membrane-anchoring motif and of cysteines likely to mediate covalent linkage to an integral membrane protein, EBI3 is also present on the plasma membrane of EBV-transformed B lymphocytes and of transfected cells. Most newly synthesized EBI3 is retained in the endoplasmic reticulum in an endoglycosidase H-sensitive form associated with the molecular chaperone calnexin and with a novel 60-kDa protein. EBI3 is expressed *in vivo* by scattered cells in interfollicular zones of tonsil tissue, by cells associated with sinusoids in perifollicular areas of spleen tissue, and at very high levels by placental syncytiotrophoblasts. EBI3 expression *in vitro* is induced in EBV-negative cell lines by expression of the EBV latent infection membrane protein-1 and in peripheral blood mononuclear cells by pokeweed mitogen stimulation. EBI3 maps to chromosome 19p13.2/3, near genes encoding the erythropoietin receptor and the cytokine receptor-associated kinase, Tyk2. EBI3 synthesis by trophoblasts and by EBV-transformed cells and similarities to interleukin-12 p40 are compatible with a role for EBI3 in regulating cell-mediated immune responses. LP244 therefore may function as a modulator of EBI3 activity. Administration of a soluble form of LP244 would interfere with the effect of its endogenous ligand on the cells, since the ligand would not bind to the endogenous membrane bound LP244 as freely. Hence, an aspect of the present invention is the treatment of pathological conditions caused by excessive expression of LP244 polypeptides by adding an amount of soluble LP244 polypeptides sufficient to inhibit binding of a cytokine to the aforementioned cells. This methodology can also be modified, and the soluble receptor can also be used as a screening agent for pharmaceuticals. Briefly, a pharmaceutical which works as an LP244 antagonist can do so by blocking the binding of endogenous ligand to the LP244. Prior to determining whether a material would be effective *in vivo*, one may use the purified LP244 polypeptide in connection with a potential pharmaceutical to determine if there is binding. If there is in fact binding, further testing may be indicated.

Expression of recombinant polypeptides in high levels and its use as an antigen allows production of additional neutralizing monoclonal and polyclonal antibodies. Such neutralizing antibodies can be used in *in vivo* model settings to elucidate the role that LP244 and its ligand play in normal as well as pathologic immune responses (i.e., disease states that are aggravated by activated T- and NK-cells like autoimmune diseases, graft versus host disease, and rheumatoid arthritis). Thus, purified LP244 polypeptides, polynucleotides, and/or antibodies compositions will be useful in diagnostic assays for LP244 and its ligand, and also in raising antibodies to LP244 for use in diagnosis or therapy. More specifically, compositions comprising LP244 polypeptides, polynucleotides, and/or antibodies are useful for diagnosis, treatment and intervention of diseases, disorders, and/or conditions including, but not limited to, infectious diseases, hypothyroidism, anemia, sepsis, gram negative bacteremia, allergic responses, allergic autoimmune diseases, type 1 diabetes, Th1-dependent insulitis, inflammation, multiple sclerosis, rheumatoid arthritis, inflammatory bowel disease, liver failure, ARDS, cancers, leukemia, and immuno-deficiency, arthritis, leukemia, lymphomas, immuno-suppression, immunity, humoral immunity, myelosuppression, periodontal disease, and osteoarthritis.

LP244 polypeptides, polynucleotides, and/or antibodies can be administered, for example, for the purpose of suppressing immune responses in a human. A variety of diseases or conditions are caused by an immune response to alloantigen, including allograft rejection and graft-versus-host reaction. In alloantigen-induced immune responses, soluble LP244 polypeptides may suppress lymphoproliferation and inflammation which result upon activation of T cells. Soluble LP244 polypeptides may therefore be used to effectively suppress alloantigen-induced immune responses in the clinical treatment of, for example, rejection of allografts (such as skin, kidney, and heart transplants), and graft-versus-host reactions in patients who have received bone marrow transplants.

LP244 polypeptides, polynucleotides, and/or antibodies may also be used in clinical treatment of autoimmune dysfunctions, such a rheumatoid arthritis, diabetes and multiple sclerosis, which are dependent upon the activation of T cells against antigens not recognized as being indigenous to the host. LP244 polypeptides, polynucleotides, and/or antibodies may also be useful in treatment of septic shock in which interferon gamma produced in response to various interleukins plays a central role in causing morbidity and mortality [Doherty, *et al.*, *J. Immunol.* 149:1666 (1992)]. In addition, compositions comprising soluble LP244 compositions may be used directly in therapy to bind or scavenge endogenous LP244 ligands, thereby providing a means for regulating the immune or inflammatory activities. In its use to prevent or reverse pathologic immune responses, soluble LP244 polypeptides can be combined with other cytokine antagonists such as antibodies to the other known interleukin receptors, soluble interleukin receptors, soluble TNF (tumor necrosis factor) receptors, and/or interleukin receptor antagonists, and the like.

### 14) LP186

In another embodiment, polypeptides comprising the amino acid sequence of the open reading frame encoded by the polynucleotide sequence as shown in SEQ ID NO:31 are contemplated by the present invention. Specifically, LP186 polypeptides of the present invention comprise the amino acid sequence as shown in SEQ ID NO:32, as well as fragments, variants, and derivatives thereof. Accordingly, LP186 polynucleotides comprising polynucleotides as identified in SEQ ID NO:31 are also contemplated by the present invention.

The LP186 polypeptide as shown in SEQ ID NO:32 shares sequence similarity with the human delta homologue, DLL3 [Bulman, *et al.*, *Nature Genetics* 24(4):438-41 (2000)]. Mutations in the human delta homologue, DLL3, cause axial skeletal defects in spondylocostal dysostosis. Two of the mutations predict truncations within conserved extracellular domains. The third is a missense mutation in a highly conserved glycine residue of the fifth epidermal growth factor (EGF) repeat, which has revealed an important functional role for this domain. Spondylocostal dysostosis (SD) is a group of vertebral malsegmentation syndromes with reduced stature resulting from axial skeletal defects. SD is characterized by multiple hemivertebrae, rib fusions and deletions with a non-progressive kyphoscoliosis. D113 is mutated in the X-ray-induced mouse mutant pudgy (pu), causing a variety of vertebrocostal defects similar to SD phenotypes [Kusumi, *et al*., *Nature Genetics* 19(3):274-8 (1998)]. These mutations highlight the critical role of the Notch signalling pathway and its components in patterning the mammalian axial.

LP186 polypeptide-encoding polynucleotide sequences are primarily expressed in the nervous system. Thus, polynucleotides, polypeptides, and antibodies corresponding to this gene are useful for diagnosis, treatment and intervention of diseases, disorders, and conditions of the nervous system. Thus, the present sequence represents a polypeptide which suppresses proliferation and differentiation of undifferentiated cells such as neurons and blood cells. The polypeptide may be used for the prevention and control of disorders involving undifferentiated cells, such as leukaemia and malignant tumours, and improvement of blood formation, e.g., after immunosuppression.

The polynucleotides and polypeptides of the present invention are designated herein as "LP polynucleotides" or "LP polypeptide-encoding polynucleotides" and "LP polypeptides." When immediately followed by a numerical designation (i.e., LP105), the term "LP" refers to a specific group of molecules as defined herein. A complete designation wherein the term "LP" is immediately followed by a numerical designation and a molecule type (i.e., LP105 polypeptide) refers to a specific type of molecule within the designated group of molecules as designated herein.

The terms "LP polypeptide-encoding polynucleotides" or "LP polynucleotides" and "LP polypeptides" wherein the term "LP" is followed by an actual numerical designation as used herein encompass novel polynucleotides and polypeptides, respectively, which are further defined herein. The LP molecules described herein may be isolated from a variety of sources including, but not limited to human tissue types, or prepared by recombinant or synthetic methods.

One aspect of the present invention provides an isolated nucleic acid molecule comprising a polynucleotide which encodes an LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), or LP223(b) polypeptide as defined herein. In a preferred embodiment of the present invention, the isolated nucleic acid comprises 1) a polynucleotide encoding an LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), or LP223(b) polypeptide having an amino acid sequence as shown in SEQ ID NO:2, 4, 6, 8, 10, 12, 14, 16, 18, 20, 22, 24, 26, 28, 30, 32, 34, 36, or 38, respectively; 2) a polynucleotide complementary to such encoding nucleic acid sequences, and which remain stably bound to them under at least moderate, and optionally, high stringency conditions; or 3) any fragment and/or variant of 1) or 2).

The term "LP polypeptide" specifically encompasses truncated or secreted forms of an LP polypeptide, (e.g., soluble forms containing, for instance, an extracellular domain sequence), variant forms (e.g., alternatively spliced forms) and allelic variants of an LP polypeptide.

In one embodiment of the invention, the native sequence LP polypeptide is a full-length or mature LP polypeptide comprising amino acids as shown in SEQ ID NO:2, 4, 6, 8, 10, 12, 14, 16, 18, 20, 22, 24, 26, 28, 30, 32, 34, 36, or 38. The predicted signal peptides are indicated in the sequence listing of the present application by negative integers below the amino acids disclosed for a particular polypeptide. Also, while the LP polypeptides disclosed herein are shown to begin with a methionine residue designated as amino acid position 1, it is conceivable and possible that another methionine residue located either upstream or downstream from amino acid position 1 may be employed as the starting amino acid residue.

A "portion" of an LP polypeptide sequence is at least about 20, 25, 30, 35, 40, 45, 50, 55, 60, 65, 70, 75, 80, 85, 90, 95, or 100 contiguous amino acid residues in length.

"LP polypeptide variant" is intended to refer to an "active" LP polypeptide, wherein activity is as defined herein, having at least about 90% amino acid sequence identity with an LP polypeptide having a deduced amino acid sequences as shown above. Such LP polypeptide variants include, for instance, LP polypeptides, wherein one or more amino acid residues are added, substituted or deleted, at the N- or C-terminus or within the sequences shown. Ordinarily, an LP polypeptide variant will have at least about 90% amino acid sequence identity, preferably at least about 91% sequence identity, yet more preferably at least about 92% sequence identity, yet more preferably at least about 93% sequence identity, yet more preferably at least about 94% sequence identity, yet more preferably at least about 95% sequence identity, yet more preferably at least about 96% sequence identity, yet more preferably at least about 97% sequence identity, yet more preferably at least about 98% sequence identity, yet more preferably at least about 99% amino acid sequence identity with the amino acid sequence described, with or without the signal peptide.

"Percent (%) amino acid sequence identity" with respect to the LP amino acid sequences identified herein is defined as the percentage of amino acid residues in a candidate sequence that are identical with the amino acid residues in an LP polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as ALIGN, ALIGN-2, Megalign (DNASTAR) or BLAST (e.g., Blast, Blast-2, WU-Blast-2) software. Those skilled in the art can determine appropriate parameters for measuring alignment, including any algorithms needed to achieve maximal alignment over the full length of the sequences being compared. For example, the percent identity values used herein are generated using WU-BLAST-2 [Altschul, *et al*., *Methods in Enzymology* 266:460-80 (1996)]. Most of the WU-BLAST-2 search parameters are set to the default values. Those not set to default values, i.e., the adjustable parameters, are set with the following values: overlap span = 1; overlap fraction = 0.125; word threshold (T) = 11; and scoring matrix = BLOSUM 62. For purposes herein, a percent amino acid sequence identity value is determined by dividing (a) the number of matching identical amino acid residues between the amino acid sequence of the LP polypeptide of interest and the comparison amino acid sequence of interest (i.e., the sequence against which the LP polypeptide of interest is being compared) as determined by WU-BLAST-2, by (b) the total number of amino acid residues of the LP polypeptide of interest, respectively.

An "LP variant polynucleotide," "LP polynucleotide variant," or "LP variant nucleic acid sequence" are intended to refer to an nucleic acid molecule as defined below having at least about 75% nucleic acid sequence identity with the polynucleotide sequence as shown in SEQ ID NO: 1, 3, 5, 7, 9, 11, 13, 15, 17, 19, 21, 23, 25, 27, 29, 31, 33, 35, or 37. Ordinarily, an LP polynucleotide variant will have at least about 75% nucleic acid sequence identity, more preferably at least about 80% nucleic acid sequence identity, yet more preferably at least about 81% nucleic acid sequence identity, yet more preferably at least about 82% nucleic acid sequence identity, yet more preferably at least about 83% nucleic acid sequence identity, yet more preferably at least about 84% nucleic acid sequence identity, yet more preferably at least about 85% nucleic acid sequence identity, yet more preferably at least about 86% nucleic acid sequence identity, yet more preferably at least about 87% nucleic acid sequence identity, yet more preferably at least about 88% nucleic acid sequence identity, yet more preferably at least about 89% nucleic acid sequence identity, yet more preferably at least about 90% nucleic acid sequence identity, yet more preferably at least about 91% nucleic acid sequence identity, yet more preferably at least about 92% nucleic acid sequence identity, yet more preferably at least about 93% nucleic acid sequence identity, yet more preferably at least about 94% nucleic acid sequence identity, yet more preferably at least about 95% nucleic acid sequence identity, yet more preferably at least about 96% nucleic acid sequence identity, yet more preferably at least about 97% nucleic acid sequence identity, yet more preferably at least about 98% nucleic acid sequence identity, yet more preferably at least about 99% nucleic acid sequence identity with the nucleic acid sequences shown above. Variants specifically exclude or do not encompass the native nucleotide sequence, as well as those prior art sequences that share 100% identity with the nucleotide sequences of the invention.

"Percent (%) nucleic acid sequence identity" with respect to the LP polynucleotide sequences identified herein is defined as the percentage of nucleotides in a candidate sequence that are identical with the nucleotides in the LP polynucleotide sequence after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as ALIGN, Align-2, Megalign (DNASTAR), or BLAST (e.g., Blast, Blast-2) software. Those skilled in the art can determine appropriate parameters for measuring alignment, including any algorithms needed to achieve maximal alignment over the full length of the sequences being compared. For purposes herein, however, % nucleic acid identity values are generated using the WU-BLAST-2 (BlastN module) program [Altschul, *et al.*, *Methods in Enzymology* 266:460-80 (1996)]. Most of the WU-BLAST-2 search parameters are set to the default values. Those not set default values, i.e., the adjustable parameters, are set with the following values: overlap span = 1; overlap fraction = 0.125; word threshold (T) = 11; and scoring matrix = BLOSUM62. For purposes herein, a percent nucleic acid sequence identity value is determined by dividing (a) the number of matching identical nucleotides between the nucleic acid sequence of the LP polypeptide-encoding nucleic acid molecule of interest and the comparison nucleic acid molecule of interest (i.e., the sequence against which the LP polypeptide-encoding nucleic acid molecule of interest is being compared) as determined by WU-BLAST-2, by (b) the total number of nucleotides of the LP polypeptide-encoding nucleic acid molecule of interest.

In other embodiments, the LP variant polypeptides are encoded by nucleic acid molecules which are capable of hybridizing, preferably under stringent hybridization and wash conditions, to nucleotide sequences encoding the full-length LP polypeptide as shown in SEQ ID NO:2, 4, 6, 8, 10, 12, 14, 16, 18, 20, 22, 24, 26, 28, 30, 32, 34, 36, or 38. This scope of variant polynucleotides specifically excludes those sequences that are known as of the filing and/or priority dates of the present application.

The term "mature protein" or "mature polypeptide" as used herein refers to the form(s) of the protein produced by expression in a mammalian cell. It is generally hypothesized that once export of a growing protein chain across the rough endoplasmic reticulum has been initiated, proteins secreted by mammalian cells have a signal peptide (SP) sequence which is cleaved from the complete polypeptide to produce a "mature" form of the protein. Oftentimes, cleavage of a secreted protein is not uniform and may result in more than one species of mature protein. The cleavage site of a secreted protein is determined by the primary amino acid sequence of the complete protein and generally cannot be predicted with complete accuracy. Methods for predicting whether a protein has an SP sequence, as well as the cleavage point for that sequence, are available. A cleavage point may exist within the N-terminal domain between amino acid 10 and amino acid 35. More specifically the cleavage point is likely to exist after amino acid 15 but before amino acid 30, more likely after amino acid 27. As one of ordinary skill would appreciate, however, cleavage sites sometimes vary from organism to organism and cannot be predicted with absolute certainty. Optimally, cleavage sites for a secreted protein are determined experimentally by amino-terminal sequencing of the one or more species of mature proteins found within a purified preparation of the protein.

The term "positives," in the context of sequence comparison performed as described above, includes residues in the sequences compared that are not identical but have similar properties (e.g., as a result of conservative substitutions). The percent identity value of positives is determined by the fraction of residues scoring a positive value in the BLOSUM 62 matrix. This value is determined by dividing (a) the number of amino acid residues scoring a positive value in the BLOSUM62 matrix of WU-BLAST-2 between the LP polypeptide amino acid sequence of interest and the comparison amino acid sequence (i.e., the amino acid sequence against which the LP polypeptide sequence is being compared) as determined by WU-BLAST-2, by (b) the total number of amino acid residues of the LP polypeptide of interest.

"Isolated," when used to describe the various polypeptides disclosed herein, means a polypeptide that has been identified and separated and/or recovered from a component of its natural environment. Preferably, the isolated polypeptide is free of association with all components with which it is naturally associated. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the LP polypeptide natural environment will not be present. Ordinarily, however, isolated polypeptide will be prepared by at least one purification step.

An "isolated LP polypeptide-encoding nucleic acid" or "isolated LP nucleic acid" is a nucleic acid molecule that is identified and separated from at least one contaminant nucleic acid molecule with which it is ordinarily associated in the natural source of the nucleic acid. Such an isolated nucleic acid molecule is other than in the form or setting in which it is found in nature. Isolated nucleic acid molecules therefore are distinguished from the nucleic acid molecule as it exists in natural cells. However, an isolated LP polypeptide-encoding nucleic acid molecule includes LP polypeptide-encoding nucleic acid molecules contained in cells that ordinarily express LP polypeptide where, for example, the nucleic acid molecule is in a chromosomal location different from that of natural cells.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adapters or linkers are used in accordance with conventional practice.

The term "amino acid" is used herein in its broadest sense, and includes naturally occurring amino acids as well as non-naturally occurring amino acids, including amino acid analogs and derivatives. The latter includes molecules containing an amino acid moiety. One skilled in the art will recognize, in view of this broad definition, that reference herein to an amino acid includes, for example, naturally occurring proteogenic L-amino acids; D-amino acids; chemically modified amino acids such as amino acid analogs and derivatives; naturally-occurring non-proteogenic amino acids such as norleucine, beta-alanine, ornithine, etc.; and chemically synthesized compounds having properties known in the art to be characteristic of amino acids. As used herein, the term "proteogenic" indicates that the amino acid can be incorporated into a peptide, polypeptide, or protein in a cell through a metabolic pathway.

The incorporation of non-natural amino acids, including synthetic non-native amino acids, substituted amino acids, or one or more D-amino acids into the LP peptides, polypeptides, or proteins of the present invention ("D-LP polypeptides") is advantageous in a number of different ways. D-amino acid-containing peptides, polypeptides, or proteins exhibit increased stability *in vitro* or *in vivo* compared to L-amino acid-containing counterparts. Thus, the construction of peptides, polypeptides, or proteins incorporating D-amino acids can be particularly useful when greater intracellular stability is desired or required. More specifically, D-peptides, polypeptides, or proteins are resistant to endogenous peptidases and proteases, thereby providing improved bioavailability of the molecule and prolonged lifetimes *in vivo* when such properties are desirable. When it is desirable to allow the peptide, polypeptide, or protein to remain active for only a short period of time, the use of L-amino acids therein will permit endogenous peptidases, proteases, etc., in a cell to digest the molecule *in vivo*, thereby limiting the cell's exposure to the molecule. Additionally, D-peptides, polypeptides, or proteins cannot be processed efficiently for major histocompatibility complex class II-restricted presentation to T helper cells, and are therefore less likely to induce humoral immune responses in the whole organism.

In addition to using D-amino acids, those of ordinary skill in the art are aware that modifications in the amino acid sequence of a peptide, polypeptide, or protein can result in equivalent, or possibly improved, second generation peptides, polypeptides, or proteins, that display equivalent or superior functional characteristics when compared to the original amino acid sequences. Alterations in the LP peptides, polypeptides, or proteins of the present invention can include one or more amino acid insertions, deletions, substitutions, truncations, fusions, shuffling of subunit sequences, and the like, either from natural mutations or human manipulation, provided that the sequences produced by such modifications have substantially the same (or improved or reduced, as may be desirable) activity(ies) as the naturally-occurring counterpart sequences disclosed herein.

One factor that can be considered in making such changes is the hydropathic index of amino acids. The importance of the hydropathic amino acid index in conferring interactive biological function on a protein has been discussed by Kyte and Doolittle [*J*. *Mol*. *Biol.* 157:105-32 (1982)]. It is accepted that the relative hydropathic character of amino acids contributes to the secondary structure of the resultant protein. This, in turn, affects the interaction of the protein with molecules such as enzymes, substrates, receptors, ligands, DNA, antibodies, antigens, etc. Based on its hydrophobicity and charge characteristics, each amino acid has been assigned a hydropathic index as follows: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate/glutamine/aspartate/asparagine (-3.5); lysine (-3.9); and arginine (-4.5).

As is known in the art, certain amino acids in a peptide, polypeptide, or protein can be substituted for other amino acids having a similar hydropathic index or score and produce a resultant peptide, polypeptide, or protein having similar biological activity, i.e., which still retains biological functionality. In making such changes, it is preferable that amino acids having hydropathic indices within ±2 are substituted for one another. More preferred substitutions are those wherein the amino acids have hydropathic indices within ±1. Most preferred substitutions are those wherein the amino acids have hydropathic indices within ±0.5.

Like amino acids can also be substituted on the basis of hydrophilicity. U.S. Patent 4,554,101 discloses that the greatest local average hydrophilicity of a protein, as governed by the hydrophilicity of its adjacent amino acids, correlates with a biological property of the protein. The following hydrophilicity values have been assigned to amino acids: arginine/lysine (+3.0); aspartate/glutamate (+3.0±1); serine (+0.3); asparagine/glutamine (+0.2); glycine (0); threonine (-0.4); proline (-0.5±1); alanine/histidine (-0.5); cysteine (-1.0); methionine (-1.3); valine (-1.5); leucine/isoleucine (-1.8); tyrosine (-2.3); phenylalanine (-2.5); and tryptophan (-3.4). Thus, one amino acid in a peptide, polypeptide, or protein can be substituted by another amino acid having a similar hydrophilicity score and still produce a resultant peptide, polypeptide, or protein having similar biological activity, i.e., still retaining correct biological function. In making such changes, amino acids having hydropathic indices within ± 2 are preferably substituted for one another, those within ± 1 are more preferred, and those within ± 0.5 are most preferred.

As outlined above, amino acid substitutions in the LP polypeptides of the present invention can be based on the relative similarity of the amino acid side-chain substituents, for example, their hydrophobicity, hydrophilicity, charge, size, etc. Exemplary substitutions that take various of the foregoing characteristics into consideration in order to produce conservative amino acid changes resulting in silent changes within the present peptides, polypeptides, or proteins can be selected from other members of the class to which the naturally occurring amino acid belongs. Amino acids can be divided into the following four groups: (1) acidic amino acids; (2) basic amino acids; (3) neutral polar amino acids; and (4) neutral non-polar amino acids. Representative amino acids within these various groups include, but are not limited to: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine, cystine, tyrosine, asparagine, and glutamine; and (4) neutral non-polar amino acids such as alanine, leucine, isoleucine, valine, proline, phenylalanine, tryptophan, and methionine.

It should be noted that changes which are not expected to be advantageous can also be useful if these result in the production of functional sequences. Since small peptides polypeptides, and some proteins can be easily produced by conventional solid phase synthetic techniques, the present invention includes peptides, polypeptides, or proteins such as those discussed herein, containing the amino acid modifications discussed above, alone or in various combinations. To the extent that such modifications can be made while substantially retaining the activity of the peptide, polypeptide, or protein, they are included within the scope of the present invention. The utility of such modified peptides, polypeptides, or proteins can be determined without undue experimentation by, for example, the methods described herein.

While biologically functional equivalents of the present LP polypeptides can have any number of conservative or non-conservative amino acid changes that do not significantly affect their activity(ies), or that increase or decrease activity as desired, 40, 30, 20, 10, 5, or 3 changes, such as 1 to 30 changes or any range or value therein, may be preferred. In particular, ten or fewer amino acid changes may be preferred. More preferably, seven or fewer amino acid changes may be preferred; most preferably, five or fewer amino acid changes may be preferred. The encoding nucleotide sequences (gene, plasmid DNA, cDNA, synthetic DNA, or mRNA, for example) will thus have corresponding base substitutions, permitting them to code on expression for the biologically functional equivalent forms of the LP polypeptides. In any case, the LP peptides, polypeptides, or proteins exhibit the same or similar biological or immunological activity(ies) as that(those) of the LP polypeptides specifically disclosed herein, or increased or reduced activity, if desired. The activity(ies) of the variant LP polypeptides can be determined by the methods described herein. Variant LP polypeptides biologically functionally equivalent to those specifically disclosed herein have activity(ies) differing from those of the presently disclosed molecules by about ±50% or less, preferably by about ±40% or less, more preferably by about ±30% or less, more preferably by about ±20% or less, and even more preferably by about ±10% or less, when assayed by the methods disclosed herein.

Amino acids in an LP polypeptide of the present invention that are essential for activity can be identified by methods known in the art, such as site-directed mutagenesis or alanine-scanning mutagenesis [Cunningham and Wells, *Science* 244:1081-5 (1989)]. The latter procedure introduces single alanine mutations at every residue in the molecule. The resulting mutant molecules are then tested for biological activity. Sites that are critical for ligand-protein binding can also be identified by structural analysis such as crystallization, nuclear magnetic resonance, or photoaffinity labeling [Smith, *et al*., *J*. *Mol*. *Biol.* 224:899-904 (1992); de Vos, *et al.*, *Science* 255:306-12 (1992)].

"Stringency" of hybridization reactions is readily determinable by one of ordinary skill in the art, and generally is an empirical calculation dependent upon probe length, washing temperature, and salt concentration. In general, longer nucleic acid probes required higher temperatures for proper annealing, while shorter nucleic acid probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reactions more stringent, while lower temperatures less so. For additional details and explanation of stringency of hybridization reactions, see Ausubel, *et al*., *Current Protocols in Molecular Biology*, Wiley Interscience Publishers (1995).

"Stringent conditions" or "high stringency conditions," as defined herein, may be identified by those that (1) employ low ionic strength and high temperature for washing, for example, 15 mM sodium chloride/1.5 mM sodium citrate/0.1% sodium dodecyl sulfate at 50 degrees C; (2) employ during hybridization a denaturing agent, such as formamide, for example, 50% (v/v) formamide with 0.1% bovine serum albumin/0.1% ficoll/0.1% polyvinylpyrrolidone/50 mM sodium phosphate buffer at pH 6.5 with 750 mM sodium chloride/75 mM sodium citrate at 42 degrees C; or (3) employ 50% formamide, 5X SSC (750 mM sodium chloride, 75 mM sodium citrate), 50 mM sodium phosphate (pH 6.8), 0.1% sodium pyrophosphate, 5X Denhardt's solution, sonicated salmon sperm DNA (50 µg/mL), 0.1% SDS, and 10% dextran sulfate at 42 degrees C with washes at 42 degrees C in 0.2X SSC (30 mM sodium chloride/3 mM sodium citrate) and 50% formamide at 55 degrees C, followed by a high-stringency wash consisting of 0.1X SSC containing EDTA at 55 degrees C.

"Moderately stringent conditions" may be identified as described by Sambrook, *et al.* [*Molecular Cloning: A Laboratory Manual*, New York: Cold Spring Harbor Press, (1989)], and include the use of washing solution and hybridization conditions (e.g., temperature, ionic strength and %SDS) less stringent than those described above. An example of moderately stringent conditions is overnight incubation at 37 degrees C in a solution comprising: 20% formamide, 5X SSC (750 mM sodium chloride, 75 mM sodium citrate), 50 mM sodium phosphate at pH 7.6, 5X Denhardt's solution, 10% dextran sulfate, and 20 mg/mL denatured sheared salmon sperm DNA, followed by washing the filters in 1X SSC at about 37 to 50 degrees C. The skilled artisan will recognize how to adjust the temperature, ionic strength, etc., as necessary to accommodate factors such as probe length and the like.

The term "epitope tagged" where used herein refers to a chimeric polypeptide comprising an LP polypeptide, or domain sequence thereof, fused to a "tag polypeptide." The tag polypeptide has enough residues to provide an epitope against which an antibody may be made, or which can be identified by some other agent, yet is short enough such that it does not interfere with the activity of the LP polypeptide. The tag polypeptide preferably is also fairly unique so that the antibody does not substantially cross-react with other epitopes. Suitable tag polypeptides generally have at least six amino acid residues and usually between about eight to about fifty amino acid residues (preferably, between about ten to about twenty residues).

As used herein, the term "immunoadhesin," sometimes referred to as an Fc fusion, designates antibody-like molecules that combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3 or IgG-4 subtypes, IgA (including IgA-1 and IgA-2), IgE, IgD or IgM.

"Active" or "activity" for the purposes herein refers to form(s) of LP polypeptide which retain all or a portion of the biologic and/or immunologic activities of native or naturally-occurring LP polypeptide. Elaborating further, "biological" activity refers to a biological function (either inhibitory or stimulatory) caused by a native or naturally-occurring LP polypeptide other than the ability to induce the production of an antibody against an antigenic epitope possessed by a native or naturally-occurring LP polypeptide. An "immunological" activity refers only to the ability to induce the production of an antibody against an antigenic epitope possessed by a native or naturally-occurring LP polypeptide.

The term "antagonist" is used in the broadest sense and includes any molecule that partially or fully blocks, inhibits, or neutralizes a biological activity of a native LP polypeptide disclosed herein. In a similar manner, the term "agonist" is used in the broadest sense and includes any molecule that mimics a biological activity of a native LP polypeptide disclosed herein. Suitable agonist or antagonist molecules specifically include agonist or antagonist antibodies or antibody fragments, fragments or amino acid sequence variants of native LP polypeptides, peptides, ribozymes, anti-sense nucleic acids, small organic molecules, etc. Methods for identifying agonists or antagonists of an LP polypeptide may comprise contacting an LP polypeptide with a candidate agonist or antagonist molecule and measuring a detectable change in one or more biological activities normally associated with the LP polypeptide. "Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (e.g., bispecific antibodies) formed from at least two intact antibodies, and antibody fragments so long as they exhibit the desired biological activity.

The terms "treating," "treatment," and "therapy" as used herein refer to curative therapy, prophylactic therapy, and preventive therapy. An example of "preventive therapy" is the prevention or lessened targeted pathological condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption but, rather, is cyclic in nature.

Administration "in combination with" one or more further therapeutic agents includes simultaneous (concurrent) and consecutive administration in any order.

A "therapeutically-effective amount" is the minimal amount of active agent (e.g., an LP polypeptide, antagonist or agonist thereof) which is necessary to impart therapeutic benefit to a mammal. For example, a "therapeutically-effective amount" to a mammal suffering or prone to suffering or to prevent it from suffering is such an amount which induces, ameliorates, or otherwise causes an improvement in the pathological symptoms, disease progression, physiological conditions associated with or resistance to succumbing to the aforedescribed disorder.

"Carriers" as used herein include pharmaceutically-acceptable carriers, excipients, or stabilizers which are nontoxic to the cell or mammal being exposed thereto at the dosages and concentrations employed. Often the physiologically-acceptable carrier is an aqueous pH buffered solution. Examples of physiologically acceptable carriers include buffers such as phosphate, citrate, and other organic acids; antioxidants including ascorbic acid; low molecule weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN®, polyethylene glycol (PEG), and PLURONIC®.

"Antibody fragments" comprise a portion of an intact antibody, preferably the antigen binding or variable region of the intact antibody. Examples of antibody fragments include Fab, Fab', F(ab')₂ and Fv fragments; diabodies; linear antibodies [Zapata, *et al*., *Protein Engin.* 8 (10):1057-62 (1995)]; single-chain antibody molecules; and multispecific antibodies formed from antibody fragments.

"Fv" is the minimum antibody fragment which contains a complete antigen-recognition and binding site. This region consists of a dimer of one heavy- and one light-chain variable domain in tight, non-covalent association. It is in this configuration that the three CDRs of each variable domain interact to define an antigen-binding site on the surface of the V_{H}V_{L} dimer. Collectively, the six CDRs confer antigen-binding specificity to the antibody. However, even a single variable domain (or half of an Fv comprising only three CDR specific for an antigen) has the ability to recognize and bind antigen, although at a lower affinity than the entire binding site.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domain, which enables the sFv to form the desired structure for antigen binding. For a review of sFv, see Pluckthun, *The Pharmacology of Monoclonal Antibodies*, vol. 113, Rosenburg and Moore, eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404 097; WO 93/11161; and Hollinger, *et al.*, *Proc. Natl. Acad. Sci. USA* 90:6444-8 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or non-proteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue, or preferably, silver stain. Isolated antibody includes the antibody *in situ* within recombinant cells since at least one component of the antibody's natural environment will not be present. Ordinarily, however, isolated antibody will be prepared by at least one purification step.

An "LP polypeptide antibody" or "LP antibody" refers to an antibody as defined herein that recognizes and binds at least one epitope of an LP polypeptide of the present invention. The term "LP polypeptide antibody" or "LP antibody" wherein the term "LP" is followed by a numerical designation refers to an antibody that recognizes and binds to at least one epitope of that particular LP polypeptide as disclosed herein.

A "liposome" is a small vesicle composed of various types of lipids, phospholipids and/or surfactant which is useful for delivery of a drug (such as an LP polypeptide or antibody thereto) to a mammal. The components of the liposome are commonly arranged in a bilayer formation, similar to the lipid arrangement of biological membranes.

A "small molecule" is defined herein to have a molecular weight below about 500 daltons.

The term "modulate" means to affect (e.g., either upregulate, downregulate or otherwise control) the level of a signaling pathway. Cellular processes under the control of signal transduction include, but are not limited to, transcription of specific genes, normal cellular functions, such as metabolism, proliferation, differentiation, adhesion, apoptosis and survival, as well as abnormal processes, such as transformation, blocking of differentiation and metastasis.

An LP polynucleotide can be composed of any polyribonucleotide or polydeoxyribonucleotide, which may be unmodified RNA or DNA or modified RNA or DNA. For example, the LP polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, LP polynucleotides can be composed of triple-stranded regions comprising RNA or DNA or both RNA and DNA. LP polynucleotides may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

LP polypeptides can be composed of amino acids joined to each other by peptide bonds or modified peptide bonds, i.e., peptide isosteres, and may contain amino acids other than the gene-encoded amino acids. The LP polypeptides may be modified by either natural processes, such as post-translational processing, or by chemical modification techniques which are well known in the art. Such modifications are well described in basic texts and in more detailed monographs, as well as in a voluminous research literature. Modifications can occur anywhere in the LP polypeptides, including the peptide backbone, the amino acid side-chains and the amino or carboxyl termini. It will be appreciated that the same type of modification may be present in the same or varying degrees at several sites in a given LP polypeptide. Also, a given LP polypeptide may contain many types of modifications. LP polypeptides may be branched, for example, as a result of ubiquitination, and they may be cyclic, with or without branching. Cyclic, branched, and branched cyclic LP polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. See, for instance, Creighton, *Proteins* - *Structure and Molecular Properties*, *2nd Ed*., W. H. Freeman and Company, New York (1993); Johnson, *Post-translational Covalent Modification of Proteins*, Academic Press, New York, pp. 1-12 (1983); Seifter, *et al.*, *Meth. Enzymol.* 182:626-46 (1990); Rattan, *et al.*, *Ann. NY Acad. Sci.* 663:48-62 (1992).

Variations in the full-length sequence LP polypeptide or in various domains of the LP polypeptide described herein can be made, for example, using any of the techniques and guidelines for conservative and non-conservative mutations set forth, for instance, in U.S. Patent 5,364,934. Variations may be a substitution, deletion or insertion of one or more codons encoding LP polypeptide that results in a change in the amino acid sequence of the LP polypeptide as compared with the native sequence LP polypeptide or an LP polypeptide as disclosed herein. Optionally the variation is by substitution of at least one amino acid with any other amino acid in one or more of the domains of the LP polypeptide. Guidance in determining which amino acid residue may be inserted, substituted or deleted without adversely affecting the desired activity may be found by comparing the sequence of the LP polypeptide with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of one to five amino acids. The variation allowed may be determined by systematically making insertions, deletions or substitutions of amino acids in the sequence and testing the resulting variants for activity (such as in any of the *in vitro* assays described herein) for activity exhibited by the full-length or mature polypeptide sequence.

LP polypeptide fragments are also provided herein. Such fragments may be truncated at the N-terminus or C-terminus, or may lack internal residues, for example, when compared with a full length or native protein. Certain fragments contemplated by the present invention may lack amino acid residues that are not essential for a desired biological activity of the LP polypeptide.

LP polypeptide fragments may be prepared by any of a number of conventional techniques. Desired peptide fragments may be chemically synthesized. An alternative approach involves generating LP fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment by polymerase chain reaction (PCR). Oligonucleotides that define the desired termini of the DNA fragment are employed at the 5' and 3' primers in the PCR. Preferably, LP polypeptide fragments share at least one biological and/or immunological activity with at least one of the LP polypeptides as shown in SEQ ID NO:2, 4, 6, 8, 10, 12, 14, 16, 18, 20, 22, 24, 26, 28, 30, 32, 34, 36, or 38.

Covalent modifications of LP polypeptides are included within the scope of this invention. One type of covalent modification includes reacting targeted amino acid residues of an LP polypeptide with an organic derivatizing agent that is capable of reacting with selected side chains or the N- or C-terminal residues of an LP polypeptide. Derivatization with bifunctional agents is useful, for instance, for crosslinking LP polypeptide to a water-insoluble support matrix or surface for use in the method for purifying anti-LP polypeptide antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylproprionate), bifunctional maleimides such as bis-N-maleimido-1,8-octane and agents such as methyl-3-[(p-azidophenyl)dithiolproprioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the alpha-amino groups of lysine, arginine, and histidine side chains [Creighton, *Proteins*: *Structure and Molecular Properties*, W.H. Freeman & Co., San Francisco, pp. 79-86 (1983)], acetylation of the N-terminal amine, and amidation of any C-terminal carboxyl group.

Another type of covalent modification of the LP polypeptides included within the scope of this invention comprises altering the native glycosylation pattern of the polypeptide. "Altering the native glycosylation pattern" is intended for purposes herein to mean deleting one or more carbohydrate moieties found in native sequence LP polypeptide and/or adding one or more glycosylation sites that are not present in the native sequences of LP polypeptides. Additionally, the phrase includes qualitative changes in the glycosylation of the native proteins, involving a change in the nature and proportions of the various carbohydrate moieties present.

Addition of glycosylation sites to LP polypeptides may be accomplished by altering the amino acid sequence thereof. The alteration may be made, for example, by the addition of, or substitution by, one or more serine or threonine residues to the native sequences of LP polypeptides (for O-linked glycosylation sites). The LP amino acid sequences may optionally be altered through changes at the DNA level, particularly by mutating the DNA encoding the LP polypeptides at preselected bases such that codons are generated that will translate into the desired amino acids.

Another means of increasing the number of carbohydrate moieties on the LP polypeptides is by chemical or enzymatic coupling of glycosides to the polypeptide. Such methods are described in the art, e.g., in WO 87/05330, and in Aplin and Wriston, *CRC Crit*. *Rev*. *Biochem*., pp. 259-306 (1981).

Removal of carbohydrate moieties present on the LP polypeptide may be accomplished chemically or enzymatically or by mutational substitution of codons encoding for amino acid residues that serve as targets for glycosylation. Chemical deglycosylation techniques are known in the art and described, for instance, by Sojar, *et al*., *Arch. Biochem. Biophys.* 259:52-7 (1987), and by Edge, *et al.*, *Anal. Biochem.* 118:131-7 (1981). Enzymatic cleavage of carbohydrate moieties on polypeptides can be achieved by the use of a variety of endo- and exo-glycosidases as described by Thotakura, *et al.*, *Meth. Enzymol.* 138:350-9 (1987).

Another type of covalent modification of LP comprises linking any one of the LP polypeptides to one of a variety of non-proteinaceous polymers (e.g., polyethylene glycol, polypropylene glycol, or polyoxyalkylenes) in the manner set forth in U.S. Patent Nos. 4,640,835; 4,496,689; 4,301,144; 4,670,417; 4,791,192, or 4,179,337.

LP polypeptides of the present invention may also be modified in a way to form chimeric molecules comprising an LP polypeptide fused to another heterologous polypeptide or amino acid sequence. In one embodiment, such a chimeric molecule comprises a fusion of an LP polypeptide with a tag polypeptide which provides an epitope to which an anti-tag antibody can selectively bind. The epitope tag is generally placed at the amino- or carboxyl- terminus of LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), or LP223(b) polypeptide. The presence of such epitope-tagged forms of an LP polypeptide can be detected using an antibody against the tag polypeptide. Also, provision of the epitope tag enables an LP polypeptide to be readily purified by affinity purification using an anti-tag antibody or another type of affinity matrix that binds to the epitope tag.

In an alternative embodiment, the chimeric molecule may comprise a fusion of an LP polypeptide with an immunoglobulin or a particular region of an immunoglobulin. For a bivalent form of the chimeric molecule, such a fusion could be to the Fc region of an IgG molecule. The Ig fusions preferably include the substitution of a soluble transmembrane domain deleted or inactivated form of an LP polypeptide in place of at least one variable region within an Ig molecule. In a particularly preferred embodiment, the immunoglobulin fusion includes the hinge, CH2 and CH3 or the hinge, CH1, CH2 and CH3 regions of an IgG1 molecule. For the production of immunoglobulin fusions, see also U.S. Patent 5,428,130.

In yet a further embodiment, the LP polypeptides of the present invention may also be modified in a way to form a chimeric molecule comprising an LP polypeptide fused to a leucine zipper. Various leucine zipper polypeptides have been described in the art. See, e.g., Landschulz, *et al., Science* 240(4860):1759-64 (1988); WO 94/10308; Hoppe, *et al*., *FEBS Letters* 344(2-3):191-5 (1994); Abel, *et al*., *Nature* 341(6237):24-5 (1989). It is believed that use of a leucine zipper fused to an LP polypeptide may be desirable to assist in dimerizing or trimerizing soluble LP polypeptide in solution. Those skilled in the art will appreciate that the zipper may be fused at either the N- or C-terminal end of an LP polypeptide.

The description below relates primarily to production of LP polypeptides by culturing cells transformed or transfected with a vector containing an LP polypeptide-encoding nucleic acid. It is, of course, contemplated that alternative methods, which are well known in the art, may be employed to prepare LP polypeptides. For instance, the LP polypeptide sequence, or portions thereof, may be produced by direct peptide synthesis using solid-phase techniques [see, e.g., Stewart, *et al*., *Solid-Phase Peptide Synthesis*, W.H. Freeman & Co., San Francisco, CA (1969); Merrifield, *J*. *Am*. *Chem*. *Soc*. 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of an LP polypeptide may be chemically synthesized separately and combined using chemical or enzymatic methods to produce a full-length LP polypeptide.

DNA encoding an LP polypeptide may be obtained from a cDNA library prepared from tissue believed to possess the LP polypeptide-encoding mRNA and to express it at a detectable level. Libraries can be screened with probes (such as antibodies to an LP polypeptide or oligonucleotides of at least about 20 to 80 bases) designed to identify the gene of interest or the protein encoded by it. Screening the cDNA or genomic library with the selected probe may be conducted using standard procedures, such as described in Sambrook, *et al*., *Molecular Cloning*: *A Laboratory Manual*, Cold Spring Harbor Laboratory Press, NY (1989). An alternative means to isolate the gene encoding an LP polypeptide is to use PCR methodology [Sambrook, *et al.*, supra; Dieffenbach, *et al*., *PCR Primer*: *A Laboratory Manual*, Cold Spring Harbor Laboratory Press, NY (1995)].

Nucleic acids having protein coding sequence may be obtained by screening selected cDNA or genomic libraries using the deduced amino acid sequence disclosed herein for the first time and, if necessary, using conventional primer extension procedures as described in Sambrook, *et al.*, supra, to detect precursors and processing intermediates of mRNA that may not have been reverse-transcribed into cDNA.

Host cells are transfected or transformed with expression or cloning vectors described herein for LP polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in *Mammalian Cell Biotechnology*: *A Practical Approach*, Butler, ed. (IRL Press, 1991) and Sambrook, *et al.*, supra. Methods of transfection are known to the ordinarily skilled artisan, for example, calcium phosphate and electroporation. General aspects of mammalian cell host system transformations have been described in U.S. Patent 4,399,216. Transformations into yeast are typically carried out according to the method of van Solingen, *et al*., *J Bact*. 130(2):946-7 (1977) and Hsiao, *et al.*, *Proc. Natl. Acad. Sci. USA* 76(8):3829-33 (1979). However, other methods for introducing DNA into cells, such as by nuclear microinjection, electroporation, bacterial protoplast fusion with intact cells, or polycations, e.g., polybrene or polyornithine, may also be used. For various techniques for transforming mammalian cells, see Keown, *et al.*, *Methods in Enzymology* 185:527-37 (1990) and Mansour, *et al.*, *Nature* 336 (6197) :348-52 (1988).

Suitable host cells for cloning or expressing the nucleic acid (e.g., DNA) in the vectors herein include prokaryote, yeast, or higher eukaryote cells. Suitable prokaryotes include but are not limited to eubacteria, such as Gram-negative or Gram-positive organisms, for example, Enterobacteriacea such as *E. coli*. Various *E. coli* strains are publicly available, such as *E. coli* K12 strain MM294 (ATCC 31,446); *E*. *coli* strain X1776 (ATCC 31,537); *E*. *coli* strain W3110 (ATCC 27,325) and K5 772 (ATCC 53,635). Other suitable prokaryotic host cells include Enterobacteriaceae such as Escherichia, e.g., *E. coli,* Enterobacter, Erwinia, Klebsiella, Proteus, Salmonella, e.g., Salmonella typhimurium, Serratia, e.g., Serratia marcescans, and Shigella, as well as Bacilli such as B. subtilis and B. licheniformis (e.g., B. licheniformis 41P disclosed in DD 266,710, published 12 April 1989), Pseudomonas such as P. aeruginosa, and Streptomyces. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3 110 may be modified to effect a genetic mutation in a gene encoding proteins endogenous to the host, with examples of such hosts including *E. coli* W3110 strain 1A2, which has the complete genotype tonAD; *E. coli* W3110 strain 9E4, which has the complete genotype tonAD ptr3; *E. coli* W3110 strain 27C7 (ATCC 55,244), which has the complete genotype tonAD ptr3 phoADE15 D(argF-lac)169 ompTD degP41kan^{R'}; *E. coli* W3110 strain 37D6, which has the complete genotype tonAD ptr3 phoADE15 D(argF-Iac)169 ompTD degP41kan^{R} rbs7D ilvG; *E. coli* W3110 strain 40B4, which is strain 37D6 with a non-kanamycin resistant degP deletion mutation; and an *E. coli* strain having mutant periplasmic protease as disclosed in U.S. Patent 4,946,783 issued 7 August 1990. Alternatively, *in vivo* methods of cloning, e.g., PCR or other nucleic acid polymerase reactions, are suitable.

In addition to prokaryotes, eukaryotic microbes such as filamentous fungi or yeast are suitable cloning or expression hosts for LP vectors. Saccharomyces cerevisiae is a commonly used lower eukaryotic host microorganism. Others include Schizosaccharomyces pombe [Beach and Nurse, *Nature* 290:140-3 (1981); EP 139,383 published 2 May 1995]; Muyveromyces hosts [U.S. Patent 4,943,529 Fleer, *et al.*, *Bio*/*Technology* 9(10):968-75 (1991)] such as, e.g., K lactis (MW98-8C, CBS683, CBS4574) [de Louvencourt, *et al*., *J*. *Bacteriol.* 154(2):737-42 (1983)]; K. fiagilis (ATCC 12,424), K. bulgaricus (ATCC 16,045), K wickeramii (ATCC 24,178), K waltii (ATCC 56,500), K. drosophilarum (ATCC 36.906) [Van den Berg, *et al.*, *Bio*/*Technology* 8(2):135-9 (1990)]; K. thermotolerans, and K. marxianus; yarrowia (EP 402,226); Pichia pastoris (EP 183,070) [Sreekrishna, *et al.*, *J. Basic Microbiol.* 28(4):265-78 (1988)]; Candida; Trichoderma reesia (EP 244,234); Neurospora crassa [Case, *et al*., *Proc*. *Natl*. *Acad Sci*. *USA* 76(10):5259-63 (1979)]; Schwanniomyces such as Schwanniomyces occidentulis (EP 394,538 published 31 October 1990); and filamentous fungi such as, e.g., Neurospora, Penicillium, Tolypocladium (WO 91/00357 published 10 January 1991), and Aspergillus hosts such as A. nidulans [Ballance, *et al.*, *Biochem. Biophys. Res. Comm.* 112(1):284-9 (1983); Tilburn, *et al.*, *Gene* 26(2-3):205-21 (1983); Yelton, *et al*., *Proc*. *Natl*. *Acad*. *Sci*. *USA* 81(5):1470-4 (1984)] and A. niger [Kelly and Hynes, *EMBO J*. 4(2):475-9 (1985)]. Methylotropic yeasts are selected from the genera consisting of Hansenula, Candida, Kloeckera, Pichia, Saccharomyces, Torulopsis, and Rhodotoruia. A list of specific species that are exemplary of this class of yeast may be found in Antony, *The Biochemistry of Methylotrophs* 269 (1982).

Suitable host cells for the expression of glycosylated LP polypeptides are derived from multicellular organisms. Examples of invertebrate cells include insect cells such as Drosophila S2 and Spodoptera Sp, Spodoptera high5 as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line [293 or 293 cells subcloned for growth in suspension culture, Graham, *et al.*, *J. Gen Virol.,* 36(1):59-74 (1977)]; Chinese hamster ovary cells/-DHFR [CHO, Urlaub and Chasin, *Proc. Natl. Acad. Sci. USA*, 77(7):4216-20 (1980)]; mouse sertoli cells [TM4, Mather, *Biol. Reprod.* 23(1):243-52 (1980)]; human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

LP polypeptides may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the LP polypeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including Saccharomyces and Kluyveromyces cc-factor leaders, the latter described in U.S. Patent 5,010,182), or acid phosphatase leader, the C. albicans glucoamylase leader (EP 362,179), or the signal described in WO 90/13646. In mammalian cell expression, mammalian signal sequences may be used to direct secretion of the protein, such as signal sequences from secreted polypeptides of the same or related species as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for Bacilli.

An example of suitable selectable markers for mammalian cells are those that enable the identification of cells competent to take up the LP polypeptide-encoding nucleic acid, such as DHFR or thymidine kinase. An appropriate host cell when wild-type DHFR is employed is the CHO cell line deficient in DHFR activity, prepared and propagated as described Urlaub and Chasin, *Proc. Natl*. *Acad. Sci. USA*, 77(7):4216-20 (1980). A suitable selection gene for use in yeast is the trpl gene present in the yeast plasmid YRp7 [Stinchcomb, *et al.*, *Nature* 282(5734):39-43 (1979); Kingsman, *et al.*, *Gene* 7(2):141-52 (1979); Tschumper, *et al*., *Gene* 10(2):157-66 (1980)]. The trpl gene provides a selection marker for a mutant strain of yeast lacking the ability to grow in tryptophan, for example, ATCC No. 44076 or PEPC1 [Jones, *Genetics* 85:23-33 (1977)].

Expression and cloning vectors usually contain a promoter operably linked to the LP polypeptide-encoding nucleic acid sequence to direct mRNA synthesis. Promoters recognized by a variety of potential host cells are well known. Promoters suitable for use with prokaryotic hosts include the P-lactamase and lactose promoter systems [Chang, et *al*., *Nature* 275(5681):617-24 (1978); Goeddel, *et al*., *Nature* 281(5732):544-8 (1979)], alkaline phosphatase, a tryptophan (up) promoter system [Goeddel, *Nucleic Acids Res*. 8(18):4057-74 (1980); EP 36,776 published 30 September 1981], and hybrid promoters such as the tat promoter [deBoer, *et al.*, *Proc. Natl*. *Acad. Sci. USA* 80(1):21-5 (1983)]. Promoters for use in bacterial systems also will contain a Shine-Dalgarno (S.D.) sequence operably linked to the DNA encoding LP polypeptide.

Examples of suitable promoting sequences for use with yeast hosts include the promoters for 3-phosphoglycerate kinase [Hitzeman, *et al*., *J*. *Biol. Chem.* 255(24):12073-80 (1980)] or other glycolytic enzymes [Hess, *et al*., *J*. *Adv*. *Enzyme Reg*. 7:149 (1968); Holland, *Biochemistry* 17(23):4900-7 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657. LP transcription from vectors in mammalian host cells is controlled, for example, by promoters obtained from the genomes of viruses such as polyoma virus, fowlpox virus, adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a polynucleotide encoding an LP polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, alpha-ketoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the LP polypeptide coding sequence but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and occasionally 3' untranslated regions of eukaryotic or viral DNAs or cDNAs. These regions contain nucleotide segments transcribed as polyadenylated fragments in the untranslated portion of the mRNA encoding LP.

Gene amplification and/or expression may be measured in a sample directly, for example, by conventional Southern blotting, Northern blotting to quantitate the transcription of mRNA [Thomas, *Proc. Natl. Acad. Sci. USA* 77(9):5201-5 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal and may be prepared in any mammal. Conveniently, the antibodies may be prepared against a native sequence provided herein or against exogenous sequence fused to an LP polypeptide-encoding DNA and encoding a specific antibody epitope.

Various forms of an LP polypeptide may be recovered from culture medium or from host cell lysates. If membrane-bound, it can be released from the membrane using a suitable detergent solution (e.g., Triton X-100™) or by enzymatic cleavage. Cells employed in expression of an LP polypeptide can be disrupted by various physical or chemical means, such as freeze-thaw cycling, sonication, mechanical disruption, or cell lysing agents.

It may be desireable to purify LP polypeptides from recombinant cell proteins or polypeptides. The following procedures are exemplary of suitable purification procedures: by fractionation on an ion-exchange column; ethanol precipitation; reversed-phase HPLC; chromatography on silica or on a cation-exchange resin such as DEAE; chromatofocusing; SDS-PAGE; ammonium sulfate precipitation; gel filtration using, for example, Sephadex® G-75; protein A Sepharose® columns to remove contaminants such as IgG; and metal chelating columns to bind epitope-tagged forms of an LP polypeptide. Various methods of protein purification may be employed and such methods are known in the art and described, for example, in Deutscher, *Methods in Enzymology* 182:83-9 (1990) and Scopes, *Protein Purification: Principles and Practice*, Springer-Verlag, NY (1982). The purification step(s) selected will depend, for example, on the nature of the production process used and the particular LP polypeptide produced.

Nucleotide sequences (or their complement) encoding LP polypeptides have various applications in the art of molecular biology, including uses as hybridization probes, in chromosome and gene mapping and in the generation of anti-sense RNA and DNA. LP polypeptide-encoding nucleic acids will also be useful for the preparation of LP polypeptides by the recombinant techniques described herein.

The full-length LP polypeptide-encoding nucleotide sequence (e.g., SEQ ID NO:1, 3, 5, 7, 9, 11, 13, 15, 17, 19, 21, 23, 25, 27, 29, 31, 33, 35, or 37), or portions thereof, may be useful as hybridization probes for probing a cDNA or genomic library to isolate the full-length LP polypeptide-encoding cDNA or genomic sequences including promoters, enhancer elements and introns of native sequence LP polypeptide-encoding DNA or to isolate still other genes (for instance, those encoding naturally-occurring variants of LP polypeptides or the same from other species) which have a desired sequence identity to the LP polypeptide-encoding nucleotide sequence disclosed in SEQ ID NO:1, 3, 5, 7, 9, 11, 13, 15, 17, 19, 21, 23, 25, 27, 29, 31, 33, 35, or 37. Hybridization techniques are well known in the art and some of which are described in further detail in the Examples below.

Other useful fragments of the LP polypeptide-encoding nucleic acids include anti-sense or sense oligonucleotides comprising a single-stranded nucleic acid sequence (either RNA or DNA) capable of binding to target LP polypeptide-encoding mRNA (sense) of LP polypeptide-encoding DNA (anti-sense) sequences. Anti-sense or sense oligonucleotides, according to the present invention, comprise a fragment of the coding region of LP polypeptide-encoding DNA. Such a fragment generally comprises at least about 14 nucleotides, preferably from about 14 to 30 nucleotides. The ability to derive an anti-sense or a sense oligonucleotide, based upon a cDNA sequence encoding a given protein is described in, for example, Stein and Cohen, *Cancer Res.* 48(10):2659-68 (1988) and van der Krol, *et al.*, *Bio*/*Techniques* 6(10):958-76 (1988).

Binding of anti-sense or sense oligonucleotides to target nucleic acid sequences results in the formation of duplexes that block transcription or translation of the target sequence by one of several means, including enhanced degradation of the duplexes, premature termination of transcription or translation, or by other means. The anti-sense oligonucleotides thus may be used to block expression of LP mRNA and therefore any LP polypeptide encoded thereby. Anti-sense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Other examples of sense or anti-sense oligonucleotides include those oligonucleotides which are covalently linked to organic moieties, such as those described in WO 90/10448, and other moieties that increase affinity of the oligonucleotide for a target nucleic acid sequence, such poly-L-lysine. Further still, intercalating agents, such as ellipticine, and alkylating agents or metal complexes may be attached to sense or anti-sense oligonucleotides to modify binding specificities of the anti-sense or sense oligonucleotide for the target nucleotide sequence.

Anti-sense or sense oligonucleotides may be introduced into a cell containing the target nucleic acid sequence by any gene transfer method, including, for example, calcium phosphate-mediated DNA transfection, electroporation, or by using gene transfer vectors such as Epstein-Barr virus. In a preferred procedure, an anti-sense or sense oligonucleotide is inserted into a suitable retroviral vector. A cell containing the target nucleic acid sequence is contacted with the recombinant retroviral vector, either *in vivo* or *ex vivo*. Suitable retroviral vectors include, but are not limited to, those derived from the murine retrovirus M-MSV, N2 (a retrovirus derived from M-MuLV), or the double copy vectors designated CDTSA, CTSB and DCTSC (see WO 90/13641).

Alternatively, a sense or an anti-sense oligonucleotide may be introduced into a cell containing the target nucleic acid sequence by formation of an oligonucleotide-lipid complex, as described in WO 90/10448. The sense or anti-sense oligonucleotide-lipid complex is preferably dissociated within the cell by an endogenous lipase.

When the amino acid sequence for an LP polypeptide encodes a protein which binds to another protein (for example, where the LP polypeptide functions as a receptor), the LP polypeptide can be used in assays to identify the other proteins or molecules involved in the binding interaction. By such methods, inhibitors of the receptor/ligand binding interaction can be identified. Proteins involved in such binding interactions can also be used to screen for peptide or small molecule inhibitors or agonists of the binding interaction. Also, the receptor LP polypeptide can be used to isolate correlative ligand(s). Screening assays can be designed to find lead compounds that mimic the biological activity of the LP polypeptides disclosed herein or a receptor for such LP polypeptides. Typical screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode an LP polypeptide of the present invention or any of its modified forms can also be used to generate either transgenic animals or "knockout" animals which, in turn, are useful in the development and screening of therapeutically useful reagents. Methods for generating transgenic animals, particularly animals such as mice or rats, have become conventional in the art and are described, for example, in U.S. Patents 4,736,866 and 4,870,009. Typically, particular cells would be targeted for an LP transgene incorporation with tissue-specific enhancers. Transgenic animals that include a copy of a transgene introduced into the germ line of the animal at an embryonic stage can be used to examine the effect of increased expression of DNA encoding an LP polypeptide. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologs of LP polynucleotides can be used to construct a "knockout" animal which has a defective or altered gene encoding a particular LP polypeptide as a result of homologous recombination between the endogenous gene encoding the LP polypeptide and the altered genomic DNA introduced into an embryonic cell of the animal. For example, cDNA encoding an LP polypeptide can be used to clone genomic DNA encoding that LP polypeptide in accordance with established techniques. A portion of the genomic DNA encoding an LP polypeptide can be deleted or replaced with another gene, such as a gene encoding a selectable marker which can be used to monitor integration. Typically, several kilobases of unaltered flanking DNA (both at the 5' and 3' ends) are included in the vector [see, e.g., Thomas and Capecchi, *Cell* 51(3):503-12 (1987) for a description of homologous recombination vectors]. The vector is introduced into an embryonic stem cell line (e.g., by electroporation), and cells in which the introduced DNA has homologously recombined with the endogenous DNA are selected [see, e.g., Li, *et al*., *Cell* 69(6):915-26 (1992)]. The selected cells are then injected into a blastocyst of an animal (e.g., a mouse or rat) to form aggregation chimeras [see, e.g., Bradley, *Teratocarcinomas and Embryonic Stem Cells*: *A Practical Approach*, E. J. Robertson, ed., pp. 113-152 (IRL, Oxford, 1987)]. A chimeric embryo can then be implanted into a suitable pseudopregnant female foster animal and the embryo brought to term to create a "knockout" animal. Progeny harboring the homologously recombined DNA in their germ cells can be identified by standard techniques and used to breed animals in which all cells of the animal contain the homologously recombined DNA. Knockout animals can be characterized, for instance, for their ability to defend against certain pathological conditions and for their development of pathological conditions due to absence of the native LP polypeptide.

Transgenic non-human mammals are useful as an animal models in both basic research and drug development endeavors. Transgenic animals expressing at least one LP polypeptide or nucleic acid can be used to test compounds or other treatment modalities which may prevent, suppress, or cure a pathology or disease associated with at least one of the above mentioned activities. Such transgenic animals can also serve as a model for the testing of diagnostic methods for those same diseases. Furthermore, tissues derived from such transgenic non-human mammals are useful as a source of cells for cell culture in efforts to develop *in vitro* bioassays to identify compounds that modulate LP polypeptide activity or LP polypeptide dependent signaling. Accordingly, another aspect of the present invention contemplates a method of identifying compounds efficacious in the treatment of at least one previously described disease or pathology associated with an LP polypeptide associated activity. A non-limiting example of such a method comprises:
a) generating a transgenic non-human animal which expresses an LP polypeptide of the present invention and which is, as compared to a wild-type animal, pathologically distinct in some detectable or measurable manner from wild-type version of said non-human mammal;
b) exposing said transgenic animal to a compound, and;
c) determining the progression of the pathology in the treated transgenic animal, wherein an arrest, delay, or reversal in disease progression in transgenic animal treated with said compound as compared to the progression of the pathology in an untreated control animals is indicative that the compound is useful for the treatment of said pathology.

Another embodiment of the present invention provides a method of identifying compounds capable of inhibiting LP polypeptide activity *in vivo* and/or *in vitro* wherein said method comprises:
a) administering an experimental compound to an LP polypeptide expressing transgenic non-human animal, or tissues derived therefrom, exhibiting one or more physiological or pathological conditions attributable to the expression of an LP transgene; and
b) observing or assaying said animal and/or animal tissues to detect changes in said physiological or pathological condition or conditions.

Another embodiment of the invention provides a method for identifying compounds capable of overcoming deficiencies in LP polypeptide activity *in vivo* or *in vitro* wherein said method comprises:
a) administering an experimental compound to an LP polypeptide expressing transgenic non-human animal, or tissues derived therefrom, exhibiting one or more physiological or pathological conditions attributable to the disruption of the endogenous LP polypeptide-encoding gene; and
b) observing or assaying said animal and/or animal tissues to detect changes in said physiological or pathological condition or conditions.

Various means for determining a compound's ability to modulate the activity of an LP polypeptide in the body of the transgenic animal are consistent with the invention. Observing the reversal of a pathological condition in the LP polypeptide expressing transgenic animal after administering a compound is one such means. Another more preferred means is to assay for markers of LP activity in the blood of a transgenic animal before and after administering an experimental compound to the animal. The level of skill of an artisan in the relevant arts readily provides the practitioner with numerous methods for assaying physiological changes related to therapeutic modulation of LP activity.

"Gene therapy" includes both conventional gene therapy, where a lasting effect is achieved by a single treatment, and the administration of gene therapeutic agents, which involves the one time or repeated administration of a therapeutically effective DNA or mRNA. Anti-sense RNAs and DNAs can be used as therapeutic agents for blocking the expression of certain genes *in vivo*. It has already been shown that short anti-sense oligonucleotides can be imported into cells where they act as inhibitors, despite their low intracellular concentrations caused by their restricted uptake by the cell membrane [Zamecnik, *et al*., *Proc*. *Natl*. *Acad Sci*. *USA* 83(12):4143-6 (1986)]. The oligonucleotides can be modified to enhance their uptake, e.g., by substituting their negatively charged phosphodiester groups with uncharged groups.

There are a variety of techniques available for introducing nucleic acids into viable cells. The techniques vary depending upon whether the nucleic acid is transferred into cultured cell *in vitro* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically, retroviral) vectors and viral coat protein-liposome mediated transfection [Dzau, *et al*., *Trends in Biotechnology* 11(5):205-10 (1993)]. In some situations it is desirable to provide the nucleic acid source with an agent that targets the target cells, such as an antibody specific for a cell surface membrane protein or the target cell, a ligand for a receptor on the target cells, etc. Where liposomes are employed, proteins which bind to a cell surface membrane protein associated with endocytosis may by used for targeting and/or to facilitate uptake, e.g., capsid proteins or fragments thereof trophic for a particular cell type, antibodies for proteins which undergo internalization in cycling, proteins that target intracellular localization and enhance intracellular half-life. The technique of receptor-mediated endocytosis is described, for example, by Wu, *et al.*, *J. Biol*. *Chem.* 262(10):4429-32 (1987); and Wagner, *et al*., *Proc. Natl. Acad. Sci. USA* 87(9):3410-4 (1990). For a review of gene marking and gene therapy protocols, see Anderson, *Science* 256 (5058) :808-13 (1992).

The nucleic acid molecules encoding LP polypeptides or fragments thereof described herein are useful for chromosome identification. In this regard, there exists an ongoing need to identity new chromosome markers, since relatively few chromosome marking reagents, based upon actual sequence data, are presently available. Each LP polypeptide-encoding nucleic acid molecule of the present invention can be used as a chromosome marker. An LP polypeptide-encoding nucleic acid or fragments thereof can also be used for chromosomal localization of the gene encoding that LP polypeptide.

The present invention further provides anti-LP polypeptide antibodies. Exemplary antibodies include polyclonal, monoclonal, humanized, bispecific, and heteroconjugate antibodies.

The anti-LP polypeptide antibodies of the present invention may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the LP polypeptide or a fusion protein thereof. It may be useful to conjugate the immunizing agent to a protein known to be immunogenic in the mammal being immunized. Examples of such immunogenic proteins include, but are not limited to, keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

The anti-LP polypeptide antibodies may, alternatively, be monoclonal antibodies. Monoclonal antibodies may be prepared using hybridoma methods, such as those described by Kohler and Milstein, *Nature* 256(5517):495-7 (1975). In a hybridoma method, a mouse, hamster, or other appropriate host animal is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include an LP polypeptide or a fusion protein thereof. Generally, either peripheral blood lymphocytes ("PBLs") are used, if cells of human origin are desired, or spleen cells or lymph node cells are used, if non-human mammalian sources are desired. The lymphocytes are then fused with an immortalized cell line using a suitable fusing agent, such as polyethylene glycol, to form a hybridoma cell [Goding, *Monoclonal Antibodies*: *Principles and Practice*, Academic Press, pp. 59-103 (1986)]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which prevents the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, CA, and the American Type Culture Collection (ATCC), Rockville, MD. Human myeloma and mouse-human heteromyeloma cell lines also have been described for the production of human monoclonal antibodies [Kozbor, *J. Immunol.* 133(6):3001-5 (1984); Brodeur, *et al.*, *Monoclonal Antibody Production Techniques and Applications*, Marcel Dekker, Inc., NY, pp. 51-63 (1987)].

The culture medium in which the hybridoma cells are cultured can then be assayed for the presence of monoclonal antibodies directed against an LP polypeptide. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Rodbard, *Anal. Biochem.* 107(1):220-39 ( 1980).

After the desired hybridoma cells are identified, the clones may be subcloned by limiting dilution procedures and grown by standard methods [Goding, *Monoclonal Antibodies*: *Principles and Practice*, Academic Press, pp. 59-103 (1986)]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies may also be made by recombinant DNA methods, such as those described in U.S. Patent 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent 4,816,567; Morrison, *et al*., *Proc*. *Natl*. *Acad*. *Sci*. *USA* 81(21):6851-5 (1984)] or by covalently joining to the immunoglobulin coding sequence all or part of the coding sequence for a non-immunoglobulin polypeptide. Such a non-immunoglobulin polypeptide can be substituted for the constant domains of an antibody of the invention or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly Fab fragments, can be accomplished using routine techniques known in the art.

The anti-LP polypeptide antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary-determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin, and all or substantially all of the FR regions are those of a human immunoglobulin consensus sequence. The humanized antibody optimally also will comprise at least a portion of an immunoglobulin constant region (Fc), typically that of a human immunoglobulin [Jones, *et al*., *Nature* 321(6069):522-5 (1986); Riechmann, *et al*., *Nature* 332(6162):323-7 (1988); and Presta, *Curr*. *Op*. *Struct*. *Biol*. 2:593-6 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones, *et al*., *Nature* 321(6069):522-5 (1986); Riechmann, *et al., Nature* 332(6162):323-7 (1988); Verhoeyen, *et al*., *Science* 239(4847):1534-6 (1988)], by substituting rodent CDRs or CDR sequences for the corresponding sequences of a human antibody. Accordingly, such "humanized" antibodies are chimeric antibodies (U.S. Patent 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, *J. Mol. Biol*. 227(2):381-8(1992); Marks, *et al.*, *J. Mol. Biol.* 222(3):581-97 (1991)]. The techniques of Cole, *et al.*, and Boerner, *et al.*, are also available for the preparation of human monoclonal antibodies (Cole, *et al*., *Monoclonal Antibodies and Cancer Therapy*, Alan R. Liss, p. 77 (1985), and Boerner, *et al.*, *J. Immunol*. 147(1):86-95 (1991)]. Similarly, human antibodies can be made by introducing human immunoglobulin loci into transgenic animals, e.g., mice in which the endogenous immunoglobulin genes have been partially or complete inactivated. Upon challenge, human antibody production is observed, which closely resembles that seen in humans in all respects, including gene rearrangement, assembly and antibody repertoire. This approach is described, for example, in U.S. Patents 5,545,807; 5,545,806; 5,569,825; 5,625,126; 5,633,425; 5,661,016, and in the following scientific publications: Marks, *et al.*, *Biotechnology* 10(7):779-83 (1992); Lonberg, *et al.*, *Nature* 368(6474) :856-9 (1994); Morrison, *Nature* 368(6474):812-3 (1994); Fishwild, *et al.*, *Nature Biotechnology* 14(7):845-51 (1996); Neuberger, *Nature Biotechnology* 14(7):826 (1996); Lonberg and Huszar, *Int. Rev. Immunol*. 13(1):65-93 (1995).

Bispecific antibodies are monoclonal, preferably human or humanized, antibodies that have binding specificities for at least two different antigens. In the present case, one of the binding specificities is for an LP polypeptide, the other one is for any other antigen, and preferably for a cell-surface protein or receptor or receptor subunit. Methods for making bispecific antibodies are known in the art. Antibodies with more than two valencies are contemplated. For example, trispecific antibodies can be prepared [Tutt, *et al*., *J Immunol.* 147 (1) :60-9 (1991)].

Heteroconjugate antibodies are also within the scope of the present invention. Heteroconjugate antibodies are composed of two covalently joined antibodies. Such antibodies have, for example, been proposed to target immune system cells to unwanted cells [U.S. Patent 4,676,980], and for treatment of HIV infection [WO 91/00360; WO 92/20373]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent 4,676,980.

The invention also pertains to immunoconjugates comprising an antibody conjugated to a cytotoxic agent such as a chemotherapeutic agent, toxin (e.g., an enzymatically active toxin of bacterial, fungal, plant or animal origin, or fragments thereof, or a small molecule toxin), or a radioactive isotope (i.e., a radioconjugate).

Conjugates of the antibody and cytotoxic agent are made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutaraldehyde), bis-azido compounds, bis-diazonium derivatives (such as bis-2-diazoniumbenzoyl)ethylenediamine) diisocyanates (such as tolylene-2,6-diisocyanate), and bioactive fluorine compounds. For example, a ricin immunotoxin can be prepared as described in Vitetta, *et al.*, *Science* 238 (4830) :1098-104 (1987). Carbon-14-labeled 1-isothiocyanatobenzyl-3-methyldiethylene triaminepentaacetic acid (MX-DTPA) is an exemplary chelating agent for conjugation of radionucleotide to the antibody.

In another embodiment, the antibody may be conjugated to a "receptor" (such as streptavidin) for utilization in tumor pretargeting wherein the antibody-receptor conjugate is administered to the patient, followed by removal of unbound conjugate from the circulation using a clearing agent, and then administration of a "ligand" (e.g., avidin) which is conjugated to a cytotoxic agent (e.g., a radionucleotide).

The antibodies disclosed herein may also be formulated as immunoliposomes. Liposomes containing the antibody are prepared by methods known in the art, such as described in Eppstein, *et al*., *Proc*. *Natl*. *Acad*. *Sci*. *USA* 82:3688-92 (1985); Hwang, *et al*., *Proc*. *Natl*. *Acad*. *Sci*. *USA* 77(7):4030-4 (1980); and U.S. Patents 4,485,045 and 4,544,545. Liposomes with enhanced circulation time are disclosed in U.S. Patent 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin, *et al*., *J*. *Biol*. *Chem.* 257(1):286-8 (1982) via a disulfide interchange reaction. A chemotherapeutic agent (such as Doxorubicin) is optionally contained within the liposome. See Gabizon, *et al.*, *J. National Cancer Inst.* 81(19):484-8 ( 1989).

Antibodies specifically binding an LP polypeptide identified herein, as well as other molecules identified by the screening assays disclosed hereinbefore, can be administered for the treatment of various disorders in the form of pharmaceutical compositions.

If an LP polypeptide is intracellular and whole antibodies are used as inhibitors, internalizing antibodies are preferred. However, lipofections or liposomes can also be used to deliver the antibody or an antibody fragment into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, e.g., Marasco, *et al*., *Proc. Natl*. *Acad. Sci. USA* 90 (16) :7889-93 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokines, chemotherapeutic agent, or growth-inhibitory agent. Such molecules are suitable present in combination in amounts that are effective for the purpose intended. The active ingredients may also be entrapped in microcapsules prepared, for example, by coacervation techniques or by interfacial polymerization, for example, hydroxymethylcellulose or gelatin-microcapsules and poly-(methylmethacrylate) microcapsules, respectively, in colloidal drug delivery systems (for example, liposomes, albumin microspheres, microemulsions, nano-particles, and nanocapsules) or in macroemulsions. Such techniques are disclosed in *Remington's Pharmaceutical Sciences* 16th edition (1980).

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol), polylactides (U.S. Patent 3,773,919), copolymers of L-glutamic acid gamma-ethyl-L-glutamate, non-degradable ethylene-vinylacetate, degradable lactic acid-glycolic acid copolymers such as the LUPRON DEPOT™ (injectable microspheres composed of lactic acid-glycolic acid copolymer and leuprolide acetate), and poly-D-(-)3-hydroxylbutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 degrees C, resulting in a loss of biological activity and possible changes in immunogenicity. Rational strategies can be devised for stabilization depending on the mechanisms involved. For example, if the aggregation mechanism is discovered to be intermolecular S-S bond formation through thiosulfide interchange, stabilization may be achieved by modifying sulfhydryl residues, lyophilizing from acidic solutions, controlling moisture content, using appropriate additives, and developing specific polymer matrix compositions.

The anti-LP polypeptide antibodies of the present invention have various utilities. For example, such antibodies may be used in diagnostic assays for LP polypeptide expression, e.g., detecting expression in specific cells, tissues, or serum. Various diagnostic assay techniques known in the art may be used, such as competitive binding assays, direct or indirect sandwich assays and immunoprecipitation assays conducted in either heterogeneous or homogeneous phases [Zola, *Monoclonal Antibodies*:*A Manual of Techniques,* CRC Press, Inc., pp. 147-158 (1987)]. The antibodies used in the assays can be labeled with a detectable moiety. The detectable moiety should be capable of producing, either directly or indirectly, a detectable signal. For example, the detectable moiety may be a radioisotope, such as ³H, ¹⁴C, ³²P, ³⁵S, or ¹²⁵I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the detectable moiety may be employed, including those methods described by Hunter, *et al*., *Nature* 144:945 (1962); David, *et al*., *Biochemistry* 13(5):1014-21 ( 1974); Pain, *et al*., *J Immunol*. *Meth*., 40(2) : 219-30 (1981); and Nygren, *J. Histochem*. *Cytochem*. 30(5):407-12 (1982).

Anti-LP polypeptide antibodies also are useful for affinity purification from recombinant cell culture or natural sources. In this process, the antibodies are immobilized on a suitable support, such a Sephadex® resin or filter paper, using methods well known in the art. The immobilized antibody is then contacted with a sample containing the LP polypeptide to be purified, and thereafter the support is washed with a suitable solvent that will remove substantially all the material in the sample except the LP polypeptide, which is bound to the immobilized antibody. Finally, the support is washed with another suitable solvent that will release the desired polypeptide from the antibody.

This invention encompasses methods of screening compounds to identify those that mimic the activity of the LP polypeptide (agonists) disclosed herein or prevent the effects of the LP polypeptide (antagonists). Screening assays for antagonist drug candidates are designed to identity compounds that bind or complex with an LP polypeptide encoded by the genes identified herein or otherwise interfere with the interaction of LP polypeptides with other cellular proteins. Such screening assays will include assays amenable to high-throughput screening of chemical libraries, making them particularly suitable for identifying small molecule drug candidates.

The assays can be performed in a variety of formats. In binding assays, the interaction is binding, and the complex formed can be isolated or detected in the reaction mixture. In a particular embodiment, an LP polypeptide encoded by a gene identified herein or the drug candidate is immobilized on a solid phase, e.g., on a microtiter plate, by covalent or non-covalent attachments. Noncovalent attachment generally is accomplished by coating the solid surface with a solution comprising LP polypeptide and drying. Alternatively, an immobilized antibody, e.g., a monoclonal antibody, specific for the polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to an LP polypeptide, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored through gradients or chromatographic columns. In addition, protein-protein interactions can be monitored by using a yeast-based genetic system described by Fields and co-workers [Fields and Song, *Nature* 340(6230):245-6 (1989); Chien, *et al*., *Proc*. *Natl*. *Acad*. *Sci*. *USA* 88(21):9578-82 (1991); Chevray and Nathans, *Proc. Natl. Acad. Sci. USA* 89(13):5789-93 (1992)]. Many transcriptional activators, such as yeast GAL4, consist of two physically discrete modular domains, one acting as the DNA-binding domain, while the other functions as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another in which candidate activating proteins are fused to the activation domain. The expression of GAL1-lacZ reporter gene under control of a GAL4-activated promoter depends on reconstitution of GAL4 activity via protein-protein interaction. Colonies containing interacting polypeptides are detected with chromogenic substrate for beta-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of an LP polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture to serve as a positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

Antagonists may be detected by combining at least one LP polypeptide and a potential antagonist with a membrane-bound or recombinant receptor for that LP polypeptide under appropriate conditions for a competitive inhibition assay. The LP polypeptide can be labeled, such as by radioactivity, such that the number of LP polypeptide molecules bound to the receptor can be used to determine the effectiveness of the potential antagonist. The gene encoding the receptor for an LP polypeptide can be identified by numerous methods known to those of skill in the art, for example, ligand panning and FACS sorting. See Coligan, *et al.*, *Current Protocols in Immunology* 1(2) : Ch. 5 (1991). Preferably, expression cloning is employed such that polyadenylated mRNA is prepared from a cell responsive to the secreted form of a particular LP polypeptide, and a cDNA library created from this mRNA is divided into pools and used to transfect COS cells or other cells that are not responsive to the secreted LP polypeptide. Transfected cells that are grown on glass slides are exposed to the labeled LP polypeptide. The LP polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

As an alternative approach for receptor identification, a labeled LP polypeptide can be photoaffinity-linked with cell membrane or extract preparations that express the receptor molecule. Cross-linked material is resolved by PAGE and exposed to X-ray film. The labeled complex containing the receptor can be excised, resolved into peptide fragments, and subjected to protein micro-sequencing. The amino acid sequence obtained from micro-sequencing would be used to design a set of degenerate oligonucleotide probes to screen a cDNA library to identify the gene encoding the putative receptor.

In another assay for antagonists, mammalian cells or a membrane preparation expressing the receptor would be incubated with a labeled LP polypeptide in the presence of the candidate compound. The ability of the compound to enhance or block this interaction could then be removed.

Alternatively, a potential antagonist may be a closely related protein, for example, a mutated form of the LP polypeptide that recognizes the receptor but imparts no effect, thereby competitively inhibiting the action of the polypeptide.

Another potential LP antagonist is an anti-sense RNA or DNA construct prepared using anti-sense technology, where, e.g., an anti-sense RNA or DNA molecule acts to block directly the translation of mRNA by hybridizing to targeted mRNA and prevent its translation into protein. Anti-sense technology can be used to control gene expression through triple-helix formation or anti-sense DNA or RNA, both of which methods are based on binding of a polynucleotide to DNA or RNA. For example, the 5' coding portion of the polynucleotide sequence, which encodes the mature form of an LP polypeptide can be used to design an anti-sense RNA oligonucleotide sequence of about 10 to 40 base pairs in length. A DNA oligonucleotide is designed to be complementary to a region of the gene involved in transcription [triple helix; see Lee, *et al*., *Nucl. Acids Res* 6(9):3073-91 (1979); Cooney, *et al*., *Science* 241(4864):456-9 (1988); Beal and Dervan, *Science* 251(4999):1360-3 (1991)], thereby preventing transcription and production of the LP polypeptide. The anti-sense RNA oligonucleotide hybridizes to the mRNA *in vivo* and blocks translation of the mRNA molecules [anti-sense; see Okano, *J*. *Neurochem*. 56(2):560-7 (1991); *Oligodeoxynucleotides as Anti-sense Inhibitors of Gene Expression* (CRC Press: Boca Raton, FL 1988)]. The oligonucleotides described above can also be delivered to cells such that the anti-sense RNA or DNA may be expressed *in vivo* to inhibit production of the LP polypeptide. When anti-sense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the LP polypeptide, thereby blocking the normal biological activity of the LP polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details, see, e.g., Rossi, *Current Biology* 4(5):469-71 (1994) and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, supra.

Another use of the compounds of the invention (e.g., LP polypeptides, fragments and variants thereof and LP antibodies directed thereto) described herein is to help diagnose whether a disorder is driven to some extent by the modulation of signaling by an LP polypeptide

A diagnostic assay to determine whether a particular disorder is driven by LP polypeptide dependent signaling can be carried out using the following steps:
a) culturing test cells or tissues expressing an LP polypeptide;
b) administering a compound which can inhibit LP polypeptide dependent signaling; and
c) measuring LP polypeptide mediated phenotypic effects in the test cells.

The steps can be carried out using standard techniques in light of the present disclosure. Appropriate controls take into account the possible cytotoxic effect of a compound, such as treating cells not associated with a cell proliferative disorder (e.g., control cells) with a test compound and can also be used as part of the diagnostic assay. The diagnostic methods of the invention involve the screening for agents that modulate the effects of LP polypeptide-associated disorders.

The LP polypeptides or antibodies thereto as well as LP polypeptide antagonists or agonists can be employed as therapeutic agents. Such therapeutic agents are formulated according to known methods to prepare pharmaceutically useful compositions, whereby the LP polypeptide or antagonist or agonist thereof is combined in a mixture with a pharmaceutically acceptable carrier.

In the case of LP polypeptide antagonistic or agonistic antibodies, if the LP polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology [see, e.g., Marasco, *et al.*, *Proc. Natl. Acad. Sci. USA* 90(16):7889-93 (1993)].

Therapeutic formulations are prepared for storage by mixing the active ingredient having the desired degree of purity with optional pharmaceutically acceptable carriers, excipients or stabilizers [*Remington's Pharmaceutical Sciences* 16th edition (1980)], in the form of lyophilized formulations or aqueous solutions.

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly(methylmethacrylate) microcapsules, respectively, in colloidal drug delivery systems (for example, liposomes, albumin microspheres, microemulsions, nano-particles and nanocapsules) or in macroemulsions. Such techniques are disclosed in *Remington's Pharmaceutical Sciences, supra.*

The formulations to be used for *in vivo* administration must be sterile. This is readily accomplished by filtration through sterile filtration membranes.

Therapeutic compositions herein generally are placed into a container having a sterile access port, for example, and intravenous solution bag or vial having a stopper pierceable by a hypodermic injection needle.

Sustained-release preparations may be prepared. Suitable examples of sustained-release preparations include semipermeable matrices of solid hydrophobic polymers containing the therapeutic agent(s), which matrices are in the form of shaped articles, e.g., films, or microcapsules. Examples of sustained-release matrices include polyesters, hydrogels [for example, poly(2-hydroxyethyl-methacrylate), or poly(vinylalcohol)], polylactides, copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. Microencapsulation of recombinant proteins for sustained release has been successfully performed with human growth hormone (rhGH), interferon, and interleukin-2. Johnson, *et al*., *Nat*. *Med*. 2(7):795-9 (1996); Yasuda, *et al*., *Biomed*. *Ther*. 27:1221-3 (1993); Hora, *et al*., *Bio*/*Technology* 8(8):755-8 (1990); Cleland, "Design and Production of Single Immunization Vaccines Using Polylactide Polyglycolide Microsphere Systems" in *Vaccine Design*: *The Subunit and Adjuvant Approach*, Powell and Newman, Eds., Plenum Press, NY, 1995, pp. 439-462 WO 97/03692; WO 96/40072; WO 96/07399; and U.S. Patent 5,654,010.

The sustained-release formulations of these proteins may be developed using polylactic-coglycolic acid (PLGA) polymer due to its biocompatibility and wide range of biodegradable properties. The degradation products of PLGA, lactic and glycolic acids, can be cleared quickly within the human body. Moreover, the degradability of this polymer can be adjusted from months to years depending on its molecular weight and composition. See Lewis, "Controlled release of bioactive agents from lactide/glycolide polymer" in *Biodegradable Polymers as Drug Delivery Systems* (Marcel Dekker; New York, 1990), M. Chasin and R. Langer (Eds.) pp. 1-41.

While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 degrees C, resulting in a loss of biological activity and possible changes in immunogenicity.

It is contemplated that the compounds, including, but not limited to, antibodies, small organic and inorganic molecules, peptides, anti-sense molecules, ribozymes, etc., of the present invention may be used to treat various conditions including those characterized by overexpression and/or activation of the disease-associated genes identified herein. The active agents of the present invention (e.g., antibodies, polypeptides, nucleic acids, ribozymes, small organic or inorganic molecules) are administered to a mammal, preferably a human, in accord with known methods, such as intravenous administration as a bolus or by continuous infusion over a period of time, by intramuscular, intraperitoneal, intracerebral, intracerobrospinal, subcutaneous, intra-articular, intrasynovial, intrathecal, intraoccular, intralesional, oral, topical, inhalation, pulmonary, and/or through sustained release.

Other therapeutic regimens may be combined with the administration of LP polypeptide antagonists or antagonists, anti-cancer agents, e.g., antibodies of the instant invention.

For the prevention or treatment of disease, the appropriate dosage of an active agent, (e.g., an antibody, polypeptide, nucleic acid, ribozyme, or small organic or inorganic molecule) will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

Dosages and desired drug concentration of pharmaceutical compositions of the present invention may vary depending on the particular use envisioned. The determination of the appropriate dosage or route of administration is well within the skill of an ordinary artisan. Animal experiments provide reliable guidance for the determination of effective does for human therapy. Interspecies scaling of effective doses can be performed following the principles laid down by Mordenti and Chappell, "The Use of Interspecies Scaling in Toxicokinetics," in *Toxicokinetics and New Drug Development*, Yacobi, *et al*., Eds., Pergamon Press, NY, p.4246 (1989).

When *in vivo* administration of a composition comprising an LP polypeptide, an LP polypeptide antibody, an LP polypeptide-encoding nucleic acid, ribozyme, or small organic or inorganic molecule is employed, normal dosage amounts may vary from about 1 ng/kg up to 100 mg/kg of mammal body weight or more per day, preferably about 1 pg/kg/day up to 100 mg/kg of mammal body weight or more per day, depending upon the route of administration. Guidance as to particular dosages and methods of delivery is provided in the literature; see, for example, U.S. Patents 4,657,760, 5,206,344 or 5,225,212. It is within the scope of the invention that different formulations will be effective for different treatment compounds and different disorders, that administration targeting one organ or tissue, for example, may necessitate delivery in a manner different from that to another organ or tissue. Moreover, dosages may be administered by one or more separate administrations or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays.

In another embodiment of the invention, an article of manufacture containing materials useful for the diagnosis or treatment of the disorders described above is provided. The article of manufacture comprises a container and a label. Suitable containers include, for example, bottles, vials, syringes, and test tubes. The containers may be formed from a variety of materials such as glass or plastic. The container holds a composition which is effective for diagnosing or treating the condition and may have a sterile access port (for example, the container may be an intravenous solution bag or a vial having a stopper pierceable by a hypodermic injection needle). The active agent in the composition is typically an LP polypeptide, antagonist or agonist thereof. The label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

In another embodiment of the invention, therapeutic utility of the LP polypeptide is determined by measuring phosphorylation of tyrosine residues on specific cell lines. The early cellular response of cells stimulated with the majority of proteins is protein phosphorylation of the tyrosine residues. This response includes autophosphorylation of corresponding receptors, which thereby leads to the activation of catalytic properties and the initiation of intracellular pathways specific to the cell type. Moreover, signaling downstream of receptors requires phosphorylation of specific kinases inside the cell and other intracellular enzymes of different origin as well as the phosphorylation of multiple adapter/scaffold, structural proteins and transcriptional factors.
Therefore, diverse protein-induced cell responses can be visualized by monitoring the state of protein phosphorylation after cell stimulation.

Immunodetection is used to detect the protein phosphorylation of the stimulated cell. Several antibodies that are directed against specific phosphorylated epitopes in signaling molecules are readily available. Two specific antibodies are used: phosphospecific anti-MAPK and anti-AKT antibodies. Additionally, non-specific anti-phosphotyrosine antibodies, which recognize tyrosine-phosphorylated proteins, are used. While anti-phosphotyrosine antibodies allow detection of diverse tyrosine phosphorylated proteins without directly addressing the nature of their identity, the phosphospecific anti-MAPK and anti-AKT antibodies recognize only the corresponding proteins in their phosphorylated form.

Another assay to determine utility of LP polypeptides involves transfection of cell lines with reporter plasmids followed by cell stimulation with an LP polypeptide. Each reporter consists of a defined element, responsive to specific intracellular signaling pathways, upstream of a sequence involving a reporter protein such as luciferase. Upon stimulation of the element, reporter transcription and translation ensues, and the resulting protein levels can be detected using an assay such as a luminescence assay. The cell stimulation period depends on the reporter plasmid used.

For each reporter used, positive controls are designed in the form of agonist cocktails which include approximately maximal stimulatory doses of several ligands known to stimulate the represented signaling pathway. Using this design, the chances of finding a positive stimulus for each cell line is maximized. The caveat, however, is that some cell line / reporter combinations will not be stimulated by the specific agonist cocktail, due to lack of an appropriate ligand in the cocktail. Alternately, the lack of signal induction by an agonist cocktail may be the lack of all signaling components within a particular cell line to activate the transcriptional element. Cell line / reporter combinations with no exogenous stimulus added make up the negative controls.

Another assay to determine utility of LP polypeptides involves transfection of cell lines with reporter plasmids followed by cell stimulation with an LP polypeptide. Each reporter consists of a defined element, responsive to specific intracellular signaling pathways, upstream of a sequence involving a reporter protein such as luciferase. Upon stimulation of the element, reporter transcription and translation ensues, and the resulting protein levels can be detected using an assay such as a luminescence assay. The cell stimulation period depends on the reporter plasmid used.

For each reporter used, positive controls are designed in the form of agonist cocktails which include approximately maximal stimulatory doses of several ligands known to stimulate the represented signaling pathway. Using this design, the chances of finding a positive stimulus for each cell line is maximized. The caveat, however, is that some cell line/reporter combinations will not be stimulated by the specific agonist cocktail, due to lack of an appropriate ligand in the cocktail.
Alternately, the lack of signal induction by an agonist cocktail may be the lack of all signaling components within a particular cell line to activate the transcriptional element. Cell line / reporter combinations with no exogenous stimulus added make up the negative controls.

In another assay, utility of LP polypeptide is determined by proliferation of cells. In this assay, gross changes in the number of cells remaining in a culture are monitored after exposure to an LP polypeptide for three days. The cells are incubated in an appropriate assay medium to produce a sub-optimal growth rate. For example, usually a 1:10 or 1:20 dilution of normal culture medium results in a 40 to 60% reduction in cell number compared to the undiluted culture medium. This broad growth zone is chosen so that if an LP polypeptide is a stimulator of growth, the cells have room to expand, and conversely, if the LP polypeptide is deleterious, a reduction in cell density can be detected. After a period of exposure, the assay media is replaced with media containing a detection agent such as Calcein AM, a membrane-permeant fluorescent dye, allowing quantification of the cell number.

For use in another assay, a FLAG-HIS (FLIS)-tagged version of the LP polypeptide is expressed using mammalian cells such as HEK-293EBNA, COS-7, or HEK293T. The coding region of the cDNA is amplified by PCR of a vector containing a fragment encoding the LP polypeptide. The PCR-generated fragment is cleaved with restriction enzymes and gel-purified. The fragment is then ligated into a mammalian expression vector containing the FLIS epitope tag fused to the C-terminus. Protein expressed by this plasmid construct includes both the FLAG tag (Asp-Tyr-Lys-Asp-Asp-Asp-Asp-Lys) and the 6X His tag at the COOH -terminus of the protein. This tag provides epitopes for commercially available tag-specific antibodies, enabling detection of the protein.

To determine expression of the LP polypeptide in tissues, a protein-binding assay is performed. The fixed tissue sample is exposed to the FLIS-tagged LP polypeptide, followed by exposure to a primary antibody and a secondary antibody containing a fluorescent dye. Tagged LP polypeptide that binds to the antigens in the tissue will fluoresce. Binding of the protein to an antigen in the tissue suggests that the protein is expressed in that tissue. Thus, protein expression can be determined by measuring which tissues fluoresce.

Having generally described the invention, the same will be more readily understood by reference to the following examples, which are provided by way of illustration and are not intended as limiting.

### EXAMPLES

### Example 1

### Expression and Purification of LP polypeptides in E. coli

The bacterial expression vector pQE60 is used for bacterial expression in this example. (QIAGEN, Inc., Chatsworth, CA). pQE60 encodes ampicillin antibiotic resistance ("Ampr") and contains a bacterial origin of replication ("ori"), an IPTG inducible promoter, a ribosome binding site ("RBS"), six codons encoding histidine residues that allow affinity purification using nickel-nitrilotriacetic acid ("Ni-NTA") affinity resin sold by QIAGEN, Inc., and suitable single restriction enzyme cleavage sites. These elements are arranged such that a DNA fragment encoding a polypeptide can be inserted in such a way as to produce that polypeptide with the six His residues (i.e., a "6 X His tag") covalently linked to the carboxyl terminus of that polypeptide. However, a polypeptide coding sequence can optionally be inserted such that translation of the six His codons is prevented and, therefore, a polypeptide is produced with no 6 X His tag.

The nucleic acid sequence encoding the desired portion of an LP polypeptide lacking the hydrophobic leader sequence is amplified from a cDNA clone using PCR oligonucleotide primers (based on the sequences presented, e.g., in SEQ ID NO : 1, 3, 5, 7, 9, 11, 13, 15, 17, 19, 21, 23, 25, 27, 29, 31, 33, 35, or 37) which anneal to the amino terminal encoding DNA sequences of the desired portion of the LP polypeptide-encoding nucleic acid and to sequences in the construct 3' to the cDNA coding sequence. Additional nucleotides containing restriction sites to facilitate cloning in the pQE60 vector are added to the 5' and 3' sequences, respectively.

For cloning, the 5' and 3' primers have nucleotides corresponding or complementary to a portion of the coding sequence of the LP polypeptide-encoding nucleic acid, e.g., as presented in SEQ ID NO:1, 3, 5, 7, 9, 11, 13, 15, 17, 19, 21, 23, 25, 27, 29, 31, 33, 35, or 37, according to known method steps. One of ordinary skill in the art would appreciate, of course, that the point in a polynucleotide sequence where the 5' primer begins can be varied to amplify a desired portion of the complete polypeptide-encoding polynucleotide shorter or longer than the polynucleotide which encodes the mature form of the polypeptide.

The amplified nucleic acid fragments and the vector pQE60 are digested with appropriate restriction enzymes, and the digested DNAs are then ligated together. Insertion of the LP polypeptide-encoding DNA into the restricted pQE60 vector places the LP105, LP061, LP224, LP240, LP239(a), LP243(a), LP243(b), LP253, LP218, LP251(a), LP252, LP239(b), LP223(a), LP255(a), LP244, LP186, LP251(b), LP255(b), or LP223(b) polypeptide coding region including its associated stop codon downstream from the IPTG-inducible promoter and in-frame with an initiating AUG codon. The associated stop codon prevents translation of the six histidine codons downstream of the insertion point.

The ligation mixture is transformed into competent *E. coli* cells using standard procedures such as those described in Sambrook, *et al.*, 1989; Ausubel, 1987-1998. *E. coli* strain M15/rep4, containing multiple copies of the plasmid pREP4, which expresses the lac repressor and confers kanamycin resistance ("Kanr"), is used in carrying out the illustrative example described herein. This strain, which is only one of many that are suitable for expressing LP polypeptides, is available commercially from QIAGEN, Inc. Transformants are identified by their ability to grow on LB plates in the presence of ampicillin and kanamycin. Plasmid DNA is isolated from resistant colonies and the identity of the cloned DNA confirmed by restriction analysis, PCR and DNA sequencing.

Clones containing the desired constructs are grown overnight ("O/N") in liquid culture in LB media supplemented with both ampicillin (100 µg/mL) and kanamycin (25 µg/mL). The O/N culture is used to inoculate a large culture, at a dilution of approximately 1:25 to 1:250. The cells are grown to an optical density at 600 nm ("OD600") of between 0.4 and 0.6. Isopropyl-beta-D-thiogalactopyranoside ("IPTG") is then added to a final concentration of 1 mM to induce transcription from the lac repressor sensitive promoter, by inactivating the lacI repressor. Cells subsequently are incubated further for three to four hours. Cells then are harvested by centrifugation.

The cells are then stirred for three to four hours at 4 degrees C in 6 M guanidine hydrochloride, pH 8. The cell debris is removed by centrifugation, and the supernatant containing the LP polypeptide is dialyzed against 50 mM sodium acetate buffer, pH 6, supplemented with 200 mM sodium chloride. Alternatively, a polypeptide can be successfully refolded by dialyzing it against 500 mM sodium chloride, 20% glycerol, 25 mM Tris hydrochloride, pH 7.4, containing protease inhibitors.

If insoluble protein is generated, the protein is made soluble according to known method steps. After renaturation, the polypeptide is purified by ion exchange, hydrophobic interaction, and size exclusion chromatography. Alternatively, an affinity chromatography step such as an antibody column is used to obtain pure LP polypeptide. The purified polypeptide is stored at 4 degrees C or frozen at negative 40 degrees C to negative 120 degrees C.

### Example 2

### Cloning and Expression of LP polypeptides in a Baculovirus Expression System

In this example, the plasmid shuttle vector pA2 GP is used to insert the cloned DNA encoding the mature LP polypeptide into a baculovirus, using a baculovirus leader and standard methods as described in Summers, *et al*., *A Manual of Methods for Baculovirus Vectors and Insect Cell Culture Procedures*, Texas Agricultural Experimental Station Bulletin No. 1555 (1987). This expression vector contains the strong polyhedrin promoter of the *Autographa californica* nuclear polyhedrosis virus (AcMNPV) followed by the secretory signal peptide (leader) of the baculovirus gp67 polypeptide and convenient restriction sites such as BamHI, Xba I, and Asp718. The polyadenylation site of the simian virus 40 ("SV40") is used for efficient polyadenylation. For easy selection of recombinant virus, the plasmid contains the beta-galactosidase gene from *E. coli* under control of a weak *Drosophila* promoter in the same orientation, followed by the polyadenylation signal of the polyhedrin gene. The inserted genes are flanked on both sides by viral sequences for cell-mediated homologous recombination with wild-type viral DNA to generate viable virus that expresses the cloned polynucleotide.

Other baculovirus vectors are used in place of the vector above, such as pAc373, pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow, *et al.*, *Virology* 170:31-39.

The cDNA sequence encoding the mature LP polypeptide in a clone, lacking the AUG initiation codon and the naturally associated nucleotide binding site, is amplified using PCR oligonucleotide primers corresponding to the 5' and 3' sequences of the gene. Non-limiting examples include 5' and 3' primers having nucleotides corresponding or complementary to a portion of the coding sequence of an LP polypeptide-encoding polynucleotide, e.g., as presented in SEQ ID NO:1, 3, 5, 7, 9, 11, 13, 15, 17, 19, 21, 23, 25, 27, 29, 31, 33, 35, or 37 according to known method steps.

The amplified fragment is isolated from a 1% agarose gel using a commercially available kit (e.g., "Geneclean," BIO 101 Inc., La Jolla, CA). The fragment is then digested with the appropriate restriction enzyme and again is purified on a 1% agarose gel. This fragment is designated herein "F1."

The plasmid is digested with the corresponding restriction enzymes and optionally, can be dephosphorylated using calf intestinal phosphatase, using routine procedures known in the art. The DNA is then isolated from a 1% agarose gel using a commercially available kit ("Geneclean" BIO 101 Inc., La Jolla, CA). This vector DNA is designated herein "V1."

Fragment F1 and the dephosphorylated plasmid V1 are ligated together with T4 DNA ligase. *E*. *coli* HB101 or other suitable *E. coli* hosts such as XL-1 Blue (Stratagene Cloning Systems, La Jolla, CA) cells are transformed with the ligation mixture and spread on culture plates. Bacteria are identified that contain the plasmid bearing a human LP polypeptide-encoding polynucleotide using the PCR method, in which one of the primers that is used to amplify the gene and the second primer is from well within the vector so that only those bacterial colonies containing an LP polypeptide-encoding polynucleotide will show amplification of the DNA. The sequence of the cloned fragment is confirmed by DNA sequencing. The resulting plasmid is designated herein as pBacLP.

Five µg of the plasmid pBacLP plasmid construct is cotransfected with 1.0 µg of a commercially available linearized baculovirus DNA ("BaculoGold® baculovirus DNA", PharMingen, San Diego, CA), using the lipofection method described by Felgner, *et al.*, *Proc*. *Natl*. *Acad*. *Sci*. *USA* 84: 7413-7 (1987). 1 µg of BaculoGold® virus DNA and 5 µg of the plasmid pBacLP are mixed in a sterile well of a microtiter plate containing 50 µL of serum-free Grace's medium (Life Technologies, Inc., Rockville, MD). Afterwards, 10 µL Lipofectin plus 90 µL Grace's medium are added, mixed and incubated for fifteen minutes at room temperature. Then, the transfection mixture is added dropwise to Sf9 insect cells (ATCC CRL 1711) seeded in a 35 mm tissue culture plate with 1 mL Grace's medium without serum. The plate is rocked back and forth to mix the newly added solution. The plate is then incubated for five hours at 27 degrees C. After five hours, the transfection solution is removed from the plate and 1 mL of Grace's insect medium supplemented with 10% fetal calf serum is added. The plate is put back into an incubator and cultivation is continued at 27 degrees C for four days.

After four days, the supernatant is collected, and a plaque assay is performed. An agarose gel with "Blue Gal" (Life Technologies, Inc., Rockville, MD) is used to allow easy identification and isolation of gal-expressing clones, which produce blue-stained plaques. (A detailed description of a "plaque assay" of this type can also be found in the user's guide for insect cell culture and baculovirology distributed by Life Technologies, Inc., Rockville, MD, pp. 9-10). After appropriate incubation, blue stained plaques are picked with a micropipettor tip (e.g., Eppendorf). The agar containing the recombinant viruses is then resuspended in a microcentrifuge tube containing 200 µL of Grace's medium and the suspension containing the recombinant baculovirus is used to infect Sf9 cells seeded in 35 mm dishes. Four days later, the supernatants of these culture dishes are harvested and then stored at 4 degrees C.

To verify the expression of the LP polypeptide, Sf9 cells are grown in Grace's medium supplemented with 10% heat-inactivated FBS. The cells are infected with the recombinant baculovirus at a multiplicity of infection ("MOI") of about two. Six hours later, the medium is removed and replaced with SF900 II medium minus methionine and cysteine (available, e.g., from Life Technologies, Inc., Rockville, MD). If radiolabeled polypeptides are desired, 42 hours later, 5 mCi of ³⁵S-methionine and 5 mCi ³⁵S-cysteine (available from Amersham, Piscataway, NJ) are added. The cells are further incubated for sixteen hours and then harvested by centrifugation. The polypeptides in the supernatant as well as the intracellular polypeptides are analyzed by SDS-PAGE, followed by autoradiography (if radiolabeled). Microsequencing of the amino acid sequence of the amino terminus of purified polypeptide can be used to determine the amino terminal sequence of the mature polypeptide and, thus, the cleavage point and length of the secretory signal peptide.

### Example 3

### Cloning and Expression of an LP Polypeptide in Mammalian Cells

A typical mammalian expression vector contains at least one promoter element, which mediates the initiation of transcription of mRNA, the polypeptide coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as pIRES1neo, pRetro-Off, pRetro-On, PLXSN, or pLNCX (Clontech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109). Other suitable mammalian host cells include human Hela 293, H9, Jurkat cells, mouse NIH3T3, C127 cells, Cos 1, Cos 7 and CV 1, quail QC1-3 cells, mouse L cells, and Chinese hamster ovary (CHO) cells.

Alternatively, the gene is expressed in stable cell lines that contain the gene integrated into a chromosome. The co-transfection with a selectable marker such as DHRF (dihydrofolate reductase), GPT neomycin, or hygromycin allows the identification and isolation of the transfected cells.

The transfected gene can also be amplified to express large amounts of the encoded polypeptide. The DHFR marker is useful to develop cell lines that carry several hundred or even several thousand copies of the gene of interest. Another useful selection marker is the enzyme glutamine synthase (GS) [Murphy, *et al*., *Biochem*. *J*. 227:277-9 (1991); Bebbington, *et al*., *Bio*/*Technology* 10:169-75 (1992)]. Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of polypeptides.

The expression vectors pC1 and pC4 contain the strong promoter (LTR) of the Rous Sarcoma Virus [Cullen, *et al*., *Mol*. *Cell*. *Biol*. 5:438-47 (1985)] plus a fragment of the CMV-enhancer [Boshart, *et al*., *Cell* 41:521-30 (1985)]. Multiple cloning sites, e.g., with the restriction enzyme cleavage sites BamHI, XbaI, and Asp718, facilitate the cloning of the gene of interest. The vectors contain in addition the 3' intron, the polyadenylation and termination signal of the rat preproinsulin gene.

### Example 3 (a)

### Cloning and Expression in COS Cells

The expression plasmid, pLP HA, is made by cloning a cDNA encoding LP polypeptide into the expression vector pcDNAI/Amp or pcDNAIII (which can be obtained from Invitrogen, Inc.).

The expression vector pcDNAI/amp contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) or HIS tag (see, e.g., Ausubel, *supra*) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin polypeptide described by Wilson, *et al*., *Cell* 37:767-8 (1984). The fusion of the HA tag to the target polypeptide allows easy detection and recovery of the recombinant polypeptide with an antibody that recognizes the HA epitope. pcDNAIII contains, in addition, the selectable neomycin marker.

A DNA fragment encoding the LP polypeptide is cloned into the polylinker region of the vector so that recombinant polypeptide expression is directed by the CMV promoter. The plasmid construction strategy is as follows. The LP polypeptide-encoding cDNA of a clone is amplified using primers that contain convenient restriction sites, much as described above for construction of vectors for expression of LP polypeptides in *E. coli.* Non-limiting examples of suitable primers include those based on the coding sequences presented in SEQ ID NO:1, 3, 5, 7, 9, 11, 13, 15, 17, 19, 21, 23, 25, 27, 29, 31, 33, 35, and 37.

The PCR amplified DNA fragment and the vector, pcDNAI/Amp, are digested with suitable restriction enzyme(s) and then ligated. The ligation mixture is transformed into *E. coli* strain SURE (available from Stratagene Cloning Systems, La Jolla, CA), and the transformed culture is plated on ampicillin media plates which then are incubated to allow growth of ampicillin resistant colonies. Plasmid DNA is isolated from resistant colonies and examined by restriction analysis or other means for the presence of the LP polypeptide-encoding fragment.

For expression of recombinant LP polypeptide, COS cells are transfected with an expression vector, as described above, using DEAE-DEXTRAN, as described, for instance, in Sambrook, *et al.*, *Molecular Cloning: a Laboratory Manual*, Cold Spring Laboratory Press, Cold Spring Harbor, New York (1989). Cells are incubated under conditions for expression of the LP polypeptide-encoding polynucleotide by the vector.

Expression of the LP polypeptide-HA fusion is detected by radiolabeling and immunoprecipitation, using methods described in, for example Harlow, *et al*., *Antibodies*: A Laboratory Manual, 2nd Ed., Cold Spring Harbor Laboratory Press, Cold Spring Harbor, New York (1988). To this end, two days after transfection, the cells are labeled by incubation in media containing ³⁵S-cysteine for eight hours. The cells and the media are collected, and the cells are washed and lysed with detergent-containing RIPA buffer: 150 mM sodium chloride, 1% NP-40, 0.1% SDS, 0.5% DOC, 50 mM TRIS, pH 7.5, as described by Wilson, *et al.*, cited above. Proteins are precipitated from the cell lysate and from the culture media using an HA-specific monoclonal antibody. The precipitated polypeptides then are analyzed by SDS-PAGE and autoradiography. An expression product of the expected size is seen in the cell lysate, which is not seen in negative controls.

### Example 3(b)

### Cloning and Expression in CHO Cells

The vector pC4 is used for the expression of the LP polypeptide. Plasmid pC4 is a derivative of the plasmid pSV2-dhfr (ATCC Accession No. 37146). The plasmid contains the mouse DHFR gene under control of the SV40 early promoter. Chinese hamster ovary cells or other cells lacking dihydrofolate activity that are transfected with these plasmids can be selected by growing the cells in a selective medium (alpha minus MEM, Life Technologies) supplemented with methotrexate. The amplification of the DHFR genes in cells resistant to methotrexate (MTX) has been well documented [see, e.g., Alt, *et al.*, *J. Biol*. *Chem.* 253:1357-70 (1978); Hamlin and Ma, *Biochem*. *et Biophys. Acta* 1097:107-43 (1990); and Page and Sydenham, *Biotechnology* 9:64-8 (1991)]. Cells grown in increasing concentrations of MTX develop resistance to the drug by overproducing the target enzyme, DHFR, as a result of amplification of the DHFR gene. If a second gene is linked to the DHFR gene, it is usually co-amplified and overexpressed. It is known in the art that this approach can be used to develop cell lines carrying more than 1,000 copies of the amplified gene(s). Subsequently, when the methotrexate is withdrawn, cell lines are obtained which contain the amplified gene integrated into one or more chromosome(s) of the host cell.

Plasmid pC4 contains for expressing the gene of interest the strong promoter of the long terminal repeat (LTR) of the Rous Sarcoma Virus [Cullen, et *al*., *Mol*. *Cell*. *Biol.* 5: 438-47 (1985)] plus a fragment isolated from the enhancer of the immediate early gene of human cytomegalovirus (CMV) [Boshart, *et al*., *Cell* 41: 521-30 (1985)]. Downstream of the promoter are BamHI, XbaI, and Asp718 restriction enzyme cleavage sites that allow integration of the genes. Behind these cloning sites, the plasmid contains the 3' intron and polyadenylation site of the rat preproinsulin gene. Other high efficiency promoters can also be used for the expression, e.g., the human beta-actin promoter, the SV40 early or late promoters or the long terminal repeats from other retroviruses, e.g., HIV and HTLVI. Clontech's Tet-Off and Tet-On gene expression systems and similar systems can be used to express the LP polypeptide in a regulated way in mammalian cells [Gossen, and Bujard, *Proc. Natl. Acad. Sci. USA* 89:5547-51 (1992)]. For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with restriction enzymes and then dephosphorylated using calf intestinal phosphatase by procedures known in the art. The vector is then isolated from a 1% agarose gel.

The DNA sequence encoding the complete the LP polypeptide is amplified using PCR oligonucleotide primers corresponding to the 5' and 3' sequences of the gene. Non-limiting examples include 5' and 3' primers having nucleotides corresponding or complementary to a portion of the coding sequences of an LP polypeptide-encoding polynucleotide, e.g., as presented in SEQ ID NO:1, 3, 5, 7, 9, 11, 13, 15, 17, 19, 21, 23, 25, 27, 29, 31, 33, 35, or 37, according to known method steps.

The amplified fragment is digested with suitable endonucleases and then purified again on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC4 using, for instance, restriction enzyme analysis.

Chinese hamster ovary (CHO) cells lacking an active DHFR gene are used for transfection. Five µg of the expression plasmid pC4 is cotransfected with 0.5 µg of the plasmid pSV2-neo using lipofectin. The plasmid pSV2-neo contains a dominant selectable marker, the neo gene from Tn5 encoding an enzyme that confers resistance to a group of antibiotics including G418. The cells are seeded in alpha minus MEM supplemented with 1 µg/mL G418. After two days, the cells are trypsinized and seeded in hybridoma cloning plates (Greiner, Germany) in alpha minus MEM supplemented with 10, 25, or 50 ng/mL of methotrexate plus 1 µg/mL G418. After about ten to fourteen days, single clones are trypsinized and then seeded in six-well petri dishes or 10 mL flasks using different concentrations of methotrexate (50 nM, 100 nM, 200 nM, 400 nM, 800 nM). Clones growing at the highest concentrations of methotrexate are then transferred to new six-well plates containing even higher concentrations of methotrexate (1 mM, 2 mM, 5 mM, 10 mM, 20 mM). The same procedure is repeated until clones are obtained which grow at a concentration of 100 to 200 mM. Expression of the desired gene product is analyzed, for instance, by SDS-PAGE and Western blot or by reversed phase HPLC analysis.

### Example 4

### Tissue Distribution of LP polypeptide-encoding mRNA

Northern blot analysis is carried out to examine expression of LP-polypeptide mRNA in human tissues, using methods described by, among others, Sambrook, *et al.*, cited above. A cDNA preferably probe encoding the entire LP polypeptide is labeled with ³²P using the Rediprime™ DNA labeling system (Amersham Life Science), according to the manufacturer's instructions. After labeling, the probe is purified using a CHROMA SPIN-100™ column (Clontech Laboratories, Inc.) according to the manufacturer's protocol number PT1200-1. The purified and labeled probe is used to examine various human tissues for LP polypeptide mRNA.

Multiple Tissue Northern (MTN) blots containing various human tissues (H) or human immune system tissues (IM) are obtained from Clontech and are examined with the labeled probe using ExpressHyb™ hybridization solution (Clontech) according to manufacturer's protocol number PT1190-1. Following hybridization and washing, the blots are mounted, exposed to film at negative 70 degrees C overnight, and developed according to standard procedures.

### Example 5

### Protein Phosphorylation on Tyrosine Residues

Protein-induced cell responses are determined by monitoring tyrosine phosphorylation upon stimulation of cells by addition of LP polypeptides. This is accomplished in two steps: cell manipulation and immunodetection.

Protein phosphorylation is measured using the SK-N-MC neuroblastoma cell line (ATCC HTB-10). On day one, the cells are plated into poly-D-lysine-coated, 96 well plates containing cell propagation medium [DMEM:F12 (3:1), 20 mM HEPES at pH 7.5, 5% FBS, and 50 µg/mL Gentamicin]. The cells are seeded at a concentration of 20,000 cells per well in 100 µL medium. On day two, the propagation medium in each well is replaced with 100 µL starvation medium containing DMEM:F12 (3:1), 20 mM HEPES at pH 7.5, 0.5% FBS, and 50 µg/mL Gentamicin. The cells are incubated overnight.

On day three, pervanadate solution is made ten minutes before cell lysis; pervanadate is prepared by mixing 100 µL of sodium orthovanadate (100 mM) and 3.4 µL of hydrogen peroxide (producing 100X stock pervanadate solution). The lysis buffer is then prepared: 50 mM HEPES at pH 7.5, 150 mM sodium chloride, 10% glycerol, 1% TRITON-X100™, 1 mM EDTA, 1 mM pervanadate, and BM protease inhibitors. The cells are stimulated by adding 10 µL of the LP polypeptide solution to the cells, and incubating for ten minutes. Next, the medium is aspirated, and 75 µL lysis buffer are added to each well. The cells are lysed at 4 degrees C for fifteen minutes, then 25 µL of 4X loading buffer are added to the cell lysates. The resultant solution is mixed then heated to 95 degrees C.

Detection of tyrosine phosphorylation is accomplished by Western immunoblotting. Twenty microliters of each cell sample are loaded onto SDS-PAGE eight to sixteen percent amino acid-ready gels from Bio-Rad, and the gels are run. The proteins are electrotransferred in transfer buffer (25 mM Tris base at pH 8.3, 0.2 M glycine, 20% methanol) from the gel to a nitrocellulose membrane using 250 mA per gel over a one hour period. The membrane is incubated for one hour at ambient conditions in blocking buffer consisting of TBST (20 mM Tris hydrochloride at pH 7.5, 150 mM sodium chloride, 0.1% TWEEN®-20) with 1% BSA.

Next, the antibodies are added to the membrane. The membrane is incubated overnight at 4 degrees C with gentle rocking in primary antibody solution consisting of the antibody, TBST, and 1% BSA. The next day, the membrane is washed three times, five minutes per wash, with TBST. The membrane is then incubated in the secondary antibody solution consisting of the antibody, TBST, and 1% BSA for one hour at ambient conditions with gentle rocking. After the incubation, the membrane is washed four times with TBST, ten minutes per wash.

Detection is accomplished by incubating the membrane with 10 to 30 mL of SuperSignal Solution for one minute at ambient conditions. After one minute, excess developing solution is removed, and the membrane is wrapped in plastic wrap. The membrane is exposed to X-ray film for twenty second, one minute, and two minute exposures (or longer if needed). The number and intensity of immunostained protein bands are compared to bands for the negative control-stimulated cells (basal level of phosphorylation) by visual comparison.

LP251(a) stimulates phosphorylation in the SK-N-MC cell line and activates the AKT pathway.

### Example 6

### Cell Stimulation with Detection Utilizing Reporters

Protein-induced cell responses are measured using reporters. The SK-N-MC cell line (neuroblastoma; ATCC HTB-10) / NFκB reporter combination is used.

For the reporter used, positive controls are designed in the form of agonist cocktails. These cocktails include approximate maximal stimulatory doses of several ligands known to stimulate the regulated signal pathway. For the NFκB reporter, the NFκB / PkC pathway is stimulated by an agonist cocktail containing LPS and TNF-alpha as positive controls. Cell lines and reporters with no exogenous stimulus added are used as negative controls.

At time zero, the cells are transiently transfected with the reporter plasmids in tissue culture flasks using a standard optimized protocol for all cell lines (see Example 1). After 24 hours, the cells are trypsinized and seeded into 96-well poly-D-lysine coated assay plates at a rate of 20,000 cells per well in growth medium. After four to five hours, the medium is replaced with serum-free growth medium. At that time, stimulants for those reporters which required a 24-hour stimulation period are added. After 48 hours, stimulants for the reporters which required a five-hour stimulation period are added. Five hours later, all conditions are lysed using a lysis/luciferin cocktail, and the fluorescence of the samples is determined using a Micro Beta reader.

Each assay plate is plated to contain four positive control wells, sixteen negative control wells, and sixty-four test sample wells (two replicates of thirty-two test samples). The threshold value for a positive "hit" is a fluorescence signal equal to the mean plus two standard deviations of the negative control wells. Any test sample that, in both replicates, generates a signal above that threshold is defined as a "hit."

LP240 stimulates the NFκB pathway of the neuroblastoma cell line SK-N-MC.

### Example 7

### Cell Proliferation and Cytotoxicity Determination Utilizing Fluorescence Detection

This assay is designed to monitor gross changes in the number of cells remaining in culture after exposure to LP polypeptides for a period of three days. The following cells are used in this assay:
U373MG (astrocytoma cell line, ATCC HTB-22)
T1165 (plastocytoma cell line)
ECV304 endothelial cell line)

Prior to assay, cells are incubated in an appropriate assay medium to produce a sub-optimal growth rate, e.g., a 1:10 or 1:20 dilution of normal culture medium. Cells are grown in T-150 flasks, then harvested by trypsin digestion and replated at 40 to 50% confluence into poly-D-lysine-treated 96-well plates. Cells are only plated into the inner thirty-two wells to prevent edge artifacts due to medium evaporation; the outer wells are filled with buffer alone. Following incubation overnight to stabilize cell recovery, LP polypeptides are added to the appropriate wells. Each polypeptide is assayed in triplicate at two different concentrations, 1X and 0.1X dilution in assay medium. Two controls are also included on each assay plate: assay medium and normal growth medium.

After approximately 72 hours of exposure, the plates are processed to determine the number of viable cells. Plates are spun to increase the attachment of cells to the plate. The medium is then discarded, and 50 µL of detection buffer is added to each well. The detection buffer consisted on MEM medium containing no phenol red (Gibco) with calcein AM (Molecular Probes) and PLURONIC® F-127 (Molecular Probes), each at a 1:2000 dilution. After incubating the plates in the dark at room temperature for thirty minutes, the fluorescence intensity of each well is measured using a Cytofluor 4000-plate reader (PerSeptive Biosystems). For a given cell type, the larger the fluorescence intensity, the greater the number of cells in the well. To determine the effects on cell growth from each plate, the intensity of each well containing cells stimulated with an LP polypeptide is subtracted from the intensity of the wells containing assay medium only (controls). Thus, a positive number indicated stimulation of cell growth; a negative number indicated a reduction in growth. Additionally, confidence limits at 95 and 90% are calculated from the mean results. Results lying outside the 95% confidence limit are scored as "definite hits." Results lying between the 95 and 90% confidence limits are scored as "maybes." The distinction between definite hits and maybes varied due to intraplate variability; thus, subjective scoring is used as a final determination for "hits."

LP251(a) stimulates the growth of T1165 cells and suppresses the growth of U373MG cells. LP240 stimulates the proliferation of the ECV304 endothelial cell line.

## Claims

1. Isolated nucleic acid comprising DNA having at least 90% sequence identity to a polynucleotide selected from the group consisting of:
(a) a polynucleotide having a nucleotide sequence as shown in SEQ ID NO: 15;
(b) a polynucleotide encoding a polypeptide having the amino acid sequence as shown in SEQ ID NO:16;
(c) a polynucleotide encoding the mature form of a polypeptide having the amino acid sequence as shown in SEQ ID NO: 16;
(d) a polynucleotide fragment of a polynucleotide as in (a), (b), or (c); and
(e) a polynucleotide having a nucleotide sequence which is complementary to the nucleotide sequence of a polynucleotide as in (a), (b), (c), or (d).

2. An isolated nucleic acid molecule encoding an polypeptide comprising DNA that hybridizes to the complement of the nucleic acid sequence that encodes LP253, or any fragment or variant thereof.

3. The isolated nucleic acid molecule of claim 2, wherein hybridization occurs under stringent hybridization and wash conditions.

4. A vector comprising the nucleic acid molecule of any of Claims 1 to 3.

5. The vector of Claim 4, wherein said nucleic acid molecule is operably linked to control sequences recognized by a host cell transformed with the vector.

6. A host cell comprising the vector of Claim 5.

7. A process for producing an LP polypeptide comprising culturing the host cell of Claim 6 under conditions suitable for expression of said LP polypeptide and recovering said LP polypeptide from the cell culture.

8. An isolated polypeptide comprising an amino acid sequence comprising about 90% sequence identity to a sequence of amino acid residues comprising LP253, as shown in SEQ ID NO: 16.

9. An isolated polypeptide comprising a sequence of amino acid residues selected from the group consisting of:
(a) SEQ ID NO: 16;
(b) fragments of (a) sufficient to provide a binding site for an LP polypeptide antibody; and
(c) variants of (a) or (b).

10. An isolated polypeptide produced by the method of Claim 7.

11. A chimeric molecule comprising an LP polypeptide according to Claim 8 fused to a heterologous amino acid sequence.

12. The chimeric molecule of Claim 11, wherein said heterologous amino acid sequence is an epitope tag sequence.

13. The chimeric molecule of Claim 12, wherein said heterologous amino acid sequence is an Fc region of an immunoglobulin.

14. An antibody which specifically binds to an LP polypeptide according to Claim 8.

15. The antibody of Claim 14, where said antibody is a monoclonal antibody.

16. The antibody of Claim 15, wherein said antibody is selected from the group consisting of a humanized antibody and a human antibody.

17. A composition comprising a therapeutically effective amount of an active agent selected from the group consisting of:
(a) an LP polypeptide according to either Claim 8 or Claim 9;
(b) an agonist to an LP polypeptide according to either Claim 8 or Claim 9;
(c) an antagonist to an LP polypeptide according to Claim 8 or Claim 9;
(d) an LP polypeptide antibody according to Claim 14;
(e) an anti-LP253 polypeptide-encoding mRNA specific ribozyme; and
(f) a polynucleotide as in Claim 1, in combination with a pharmaceutically acceptable carrier.

18. An isolated polypeptide according to either Claim 8 or Claim 9 for use in the treatment of a disease, condition or disorder associated with aberrant levels of said polypeptide.

19. Use of an isolated polypeptide according to either Claim 8 or Claim 9 in the preparation of a composition for the suppression of alloantigen induced immune responses in a human.

20. Use of an isolated polypeptide according to either Claim 8 or Claim 9 in the preparation of a composition for the treatment or rejection of allografts or graft-versus-host reactions in patients who have received bone marrow transplants.

21. Use of an isolated polypeptide according to either Claim 8 or Claim 9 in the preparation of a composition for the treatment of an autoimmune dysfunction in a human.

22. Use of an isolated polypeptide according to Claim 8 or Claim 9 in the preparation of a composition for the treatment of a disease selected from the group consisting of rheumatoid arthritis, diabetes, multiple sclerosis and septic shock in a human.

23. An article of manufacture comprising a container, label and therapeutically effective amount of the composition of Claim 17.


SEQUENCE LISTING

<110>  Eli Lilly & Company

<120>  Novel secreted proteins and their uses

<130>  X-14001

<160>  38

<170>  PatentIn version 3.1

<210>  1

<211>  1438

<212>  DNA

<213>  Homo sapiens

<220>

<221>  CDS

<222>  (69)..(1196)

<223>


<220>

<221>  mat_peptide

<222>  (132)..()

<223>


<400>  1; LP105
ctgagaccct ccttcaacct ccctagagga cagccccact ctgcctcctg ctcccccagg      60

gcagcacc atg tgg ccc ccg tgg ctc tgc tgg gca ctc tgg gtg ctg ccc      110
         Met Trp Pro Pro Trp Leu Cys Trp Ala Leu Trp Val Leu Pro
             -20                 -15                 -10

ctg gct ggc ccc ggg gcg gcc ctg acc gag gag cag ctc ctg ggc agc      158
Leu Ala Gly Pro Gly Ala Ala Leu Thr Glu Glu Gln Leu Leu Gly Ser
        -5              -1  1               5

ctg ctg cgg cag ctg cag ctc agc gag gtg ccc gta ctg gac agg gcc      206
Leu Leu Arg Gln Leu Gln Leu Ser Glu Val Pro Val Leu Asp Arg Ala
10                  15                  20                  25

gac atg gag aag ctg gtc atc ccc gcc cac gtg agg gcc cag tat gta      254
Asp Met Glu Lys Leu Val Ile Pro Ala His Val Arg Ala Gln Tyr Val
                30                  35                  40

gtc ctg ctg cgg cgc agc cac ggg gac cgc tcc cgc gga aag agg ttc      302

Val Leu Leu Arg Arg Ser His Gly Asp Arg Ser Arg Gly Lys Arg Phe
            45                  50                  55

agc cag agc ttc cga gag gtg gcc ggc agg ttc ctg gcg tcg gag gcc     350
Ser Gln Ser Phe Arg Glu Val Ala Gly Arg Phe Leu Ala Ser Glu Ala
        60                  65                  70

agc aca cac ctg ctg gtg ttc ggc atg gag cag cgg ctg ccg ccc aac     398
Ser Thr His Leu Leu Val Phe Gly Met Glu Gln Arg Leu Pro Pro Asn
    75                  80                  85

agc gag ctg gtg cag gcc gtg ctg cgg ctc ttc cag gag ccg gtc ccc     446
Ser Glu Leu Val Gln Ala Val Leu Arg Leu Phe Gln Glu Pro Val Pro
90                  95                  100                 105

aag gcc gcg ctg cac agg cac ggg cgg ctg tcc ccg cgc agc gcc cag     494
Lys Ala Ala Leu His Arg His Gly Arg Leu Ser Pro Arg Ser Ala Gln
                110                 115                 120

gcc cgg gtg gct gtc gag tgg ctg cgc gtc cgc gac gac ggc tcc aac     542
Ala Arg Val Ala Val Glu Trp Leu Arg Val Arg Asp Asp Gly Ser Asn
            125                 130                 135

cgc acc tcc ctc atc gac tcc agg ctg gtg tcc gtc cac gag agc ggc     590
Arg Thr Ser Leu Ile Asp Ser Arg Leu Val Ser Val His Glu Ser Gly
        140                 145                 150

tgg aag gcc ttc gac gtg acc gag gcc gtg aac ttc tgg cag cag ctg     638
Trp Lys Ala Phe Asp Val Thr Glu Ala Val Asn Phe Trp Gln Gln Leu
    155                 160                 165

agc cgg ccc cgg cag ccg ctg ctg cta cag gtg tcg gtg cag agg gag     686
Ser Arg Pro Arg Gln Pro Leu Leu Leu Gln Val Ser Val Gln Arg Glu
170                 175                 180                 185

cat ctg ggc ccg ctg gcg tcc ggc gcc cac aag ctg gtc cgc ttt gcc     734
His Leu Gly Pro Leu Ala Ser Gly Ala His Lys Leu Val Arg Phe Ala
                190                 195                 200

tcg cag ggg gcg cca gcc ggg ctt ggg gag ccc cag ctg gag ctg cac     782
Ser Gln Gly Ala Pro Ala Gly Leu Gly Glu Pro Gln Leu Glu Leu His
            205                 210                 215

acc ctg gac ctc agg gac tat gga gct cag ggc gac tgt gac cct gaa     830
Thr Leu Asp Leu Arg Asp Tyr Gly Ala Gln Gly Asp Cys Asp Pro Glu
        220                 225                 230

gca cca gtg acc gag ggc acc tgc tgc tgc cac cag gag atg tac act     878
Ala Pro Val Thr Glu Gly Thr Cys Cys Cys His Gln Glu Met Tyr Thr
    235                 240                 245

gac ctg cag ggg atg aag tgg gcc aag aac tgg atg ccg gag ccc ctg     926
Asp Leu Gln Gly Met Lys Trp Ala Lys Asn Trp Met Pro Glu Pro Leu
250                 255                 260                 265

ggc ttc ctg gct tac aag tgt gtg ggc acc tgc cag cag ccc ctg gag     974
Gly Phe Leu Ala Tyr Lys Cys Val Gly Thr Cys Gln Gln Pro Leu Glu
                270                 275                 280

gcc ctg gcc ttc aat tgg cca ttt ctg ggg ccg cga cac tgc atc gcc     1022
Ala Leu Ala Phe Asn Trp Pro Phe Leu Gly Pro Arg His Cys Ile Ala
            285                 290                 295

tca gag act gcc tcg ctg ccc atg atc gtc agc atc aag gag gga ggc     1070
Ser Glu Thr Ala Ser Leu Pro Met Ile Val Ser Ile Lys Glu Gly Gly
        300                 305                 310

agg acc agg ccc cag gtg gtc agc ctg cct aac atg agg gtc cac cac     1118
Arg Thr Arg Pro Gln Val Val Ser Leu Pro Asn Met Arg Val His His
    315                 320                 325

tac gcc ccg cta atg ttt gtg att tta gta gag atg agg ttt cac cat     1166
Tyr Ala Pro Leu Met Phe Val Ile Leu Val Glu Met Arg Phe His His
330                 335                 340                 345

gtt ggc cag gct ggt ctc aaa ctc ctg acc tgaggtgggc ggatcacaag       1216
Val Gly Gln Ala Gly Leu Lys Leu Leu Thr
                350                 355

gtcaggagat cgagaccatc ctggctaaca aggtgaaacc ccgtctctac taaaaataca   1276

aaaaattagc tgggtgtcgt ggcgggtgcc tgtagtctca ggtactcggg aggctgaggc   1336

aggagaatgg catgaacccg ggaggcggag gttgcagcga gccaagatca cgccactgca   1396

cttcagcctg ggcaacagag caagactcca tctcaaaaaa aa                      1438

<210>  2

<211>  376

<212>  PRT

<213>  Homo sapiens

<400>  2; LP105

Met Trp Pro Pro Trp Leu Cys Trp Ala Leu Trp Val Leu Pro Leu Ala
    -20                 -15                 -10

Gly Pro Gly Ala Ala Leu Thr Glu Glu Gln Leu Leu Gly Ser Leu Leu
-5              -1  1               5                   10

Arg Gln Leu Gln Leu Ser Glu Val Pro Val Leu Asp Arg Ala Asp Met
            15                  20                  25

Glu Lys Leu Val Ile Pro Ala His Val Arg Ala Gln Tyr Val Val Leu
        30                  35                  40

Leu Arg Arg Ser His Gly Asp Arg Ser Arg Gly Lys Arg Phe Ser Gln
    45                  50                  55

Ser Phe Arg Glu Val Ala Gly Arg Phe Leu Ala Ser Glu Ala Ser Thr
60                  65                  70                  75

His Leu Leu Val Phe Gly Met Glu Gln Arg Leu Pro Pro Asn Ser Glu
                80                  85                  90

Leu Val Gln Ala Val Leu Arg Leu Phe Gln Glu Pro Val Pro Lys Ala
            95                  100                 105

Ala Leu His Arg His Gly Arg Leu Ser Pro Arg Ser Ala Gln Ala Arg
        110                 115                 120

Val Ala Val Glu Trp Leu Arg Val Arg Asp Asp Gly Ser Asn Arg Thr
    125                 130                 135

Ser Leu Ile Asp Ser Arg Leu Val Ser Val His Glu Ser Gly Trp Lys
140                 145                 150                 155

Ala Phe Asp Val Thr Glu Ala Val Asn Phe Trp Gln Gln Leu Ser Arg
                160                 165                 170

Pro Arg Gln Pro Leu Leu Leu Gln Val Ser Val Gln Arg Glu His Leu
            175                 180                 185

Gly Pro Leu Ala Ser Gly Ala His Lys Leu Val Arg Phe Ala Ser Gln
        190                 195                 200

Gly Ala Pro Ala Gly Leu Gly Glu Pro Gln Leu Glu Leu His Thr Leu
    205                 210                 215

Asp Leu Arg Asp Tyr Gly Ala Gln Gly Asp Cys Asp Pro Glu Ala Pro
220                 225                 230                 235

Val Thr Glu Gly Thr Cys Cys Cys His Gln Glu Met Tyr Thr Asp Leu
                240                 245                 250

Gln Gly Met Lys Trp Ala Lys Asn Trp Met Pro Glu Pro Leu Gly Phe
            255                 260                 265

Leu Ala Tyr Lys Cys Val Gly Thr Cys Gln Gln Pro Leu Glu Ala Leu
        270                 275                 280

Ala Phe Asn Trp Pro Phe Leu Gly Pro Arg His Cys Ile Ala Ser Glu
    285                 290                 295

Thr Ala Ser Leu Pro Met Ile Val Ser Ile Lys Glu Gly Gly Arg Thr
300                 305                 310                 315

Arg Pro Gln Val Val Ser Leu Pro Asn Met Arg Val His His Tyr Ala
                320                 325                 330

Pro Leu Met Phe Val Ile Leu Val Glu Met Arg Phe His His Val Gly
            335                 340                 345

Gln Ala Gly Leu Lys Leu Leu Thr
        350                 355

<210>  3

<211>  1041

<212>  DNA

<213>  Homo sapiens

<220>

<221>  CDS

<222>  (66)..(878)

<223>

<220>

<221>  mat_peptide

<222>  (129)..()

<223>

<400>  3; LP061
gcggccgcca gtgtgatgga tatctgcaga attcgccctt acaaccaagt gagcagcaca      60

gacta atg agt aga atc aat aag aac gtg gtt ttg gcc ctt tta acg ctg     110
      Met Ser Arg Ile Asn Lys Asn Val Val Leu Ala Leu Leu Thr Leu
          -20                 -15                 -10

aca agt tct gca ttt ctg ctg ttt cag ttg tac tac tac aag cac tat 158
Thr Ser Ser Ala Phe Leu Leu Phe Gln Leu Tyr Tyr Tyr Lys His Tyr
-5 -1 1 5 10

tta tca aca aag aat gga gct ggt ttg tca aaa tcc aaa gga agc cga 206
Leu Ser Thr Lys Asn Gly Ala Gly Leu Ser Lys Ser Lys Gly Ser Arg
15 20 25

att gga ttt gat agc aca cag tgg cgt gca gtt aaa aaa ttt att atg 254
Ile Gly Phe Asp Ser Thr Gln Trp Arg Ala Val Lys Lys Phe Ile Met
30 35 40

tta aca tcc aac caa aat gta cca gtg ttt ctt att gat cct ttg ata 302
Leu Thr Ser Asn Gln Asn Val Pro Val Phe Leu Ile Asp Pro Leu Ile
45 50 55

ctg gaa ttg att aat aag aac ttt gaa caa gtc aaa aat act tct cat 350
Leu Glu Leu Ile Asn Lys Asn Phe Glu Gln Val Lys Asn Thr Ser His
60 65 70

ggc tct act tca caa tgc aag ttt ttc tgt gtt cca aga gac ttt act 398
Gly Ser Thr Ser Gln Cys Lys Phe Phe Cys Val Pro Arg Asp Phe Thr
75 80 85 90

gca ttt gca ctg cag tat cac cta tgg aag aat gag gaa ggc tgg ttt 446
Ala Phe Ala Leu Gln Tyr His Leu Trp Lys Asn Glu Glu Gly Trp Phe
95 100 105

cgg ata gct gag aat atg gga ttt cag tgc cta aag att gag agt aaa 494
Arg Ile Ala Glu Asn Met Gly Phe Gln Cys Leu Lys Ile Glu Ser Lys
110 115 120

gat ccc cgg cta gac ggg ata gac tca ctc tct gga act gaa atc ccc 542
Asp Pro Arg Leu Asp Gly Ile Asp Ser Leu Ser Gly Thr Glu Ile Pro
125 130 135

ctg cac tat atc tgc aaa ctg gcc act cat gcg atc cac ttg gta gtc 590
Leu His Tyr Ile Cys Lys Leu Ala Thr His Ala Ile His Leu Val Val
140 145 150

ttt cat gag agg agt ggc aac tac ctc tgg cac ggc cac ttg aga ctt 638
Phe His Glu Arg Ser Gly Asn Tyr Leu Trp His Gly His Leu Arg Leu
155 160 165 170

aaa gaa cac att gac agg aaa ttt gtt ccc ttc caa aag tta cag ttt 686
Lys Glu His Ile Asp Arg Lys Phe Val Pro Phe Gln Lys Leu Gln Phe
175 180 185

ggt cgt tat cca gga gct ttt gac agg cca gag tta cag caa gtt act 734
Gly Arg Tyr Pro Gly Ala Phe Asp Arg Pro Glu Leu Gln Gln Val Thr
190 195 200

gtt gat gga ctg gaa gtt ctc att cca aag gat cca atg cac ttt gta 782
Val Asp Gly Leu Glu Val Leu Ile Pro Lys Asp Pro Met His Phe Val
205 210 215

gaa gaa gta cca cac tct agg ttt att gag tgt agg tat aaa gaa gct 830

Glu Glu Val Pro His Ser Arg Phe Ile Glu Cys Arg Tyr Lys Glu Ala
    220                 225                 230

cga gca ttc ttt cag ata cct gtt tcc gaa gtt tac act gtg ctg gac       878
Arg Ala Phe Phe Gln Ile Pro Val Ser Glu Val Tyr Thr Val Leu Asp
235                 240                 245                 250

tgagtttgta gacatgaagg tccatgtacc ctgtgaaacc ctcgaataca ttgaagccaa     938

ctatggtaag acctggaaga ttcctgtaaa gacgtgggac tggaagcgct ctcctcccaa     998

tgtgcaaccc aatggaatct ggcctatttc tgagtgggat gag                      1041

<210>  4

<211>  271

<212>  PRT

<213>  Homo sapiens

<400>  4; LP061

Met Ser Arg Ile Asn Lys Asn Val Val Leu Ala Leu Leu Thr Leu Thr
    -20                 -15                 -10

Ser Ser Ala Phe Leu Leu Phe Gln Leu Tyr Tyr Tyr Lys His Tyr Leu
-5                  -1  1               5                   10

Ser Thr Lys Asn Gly Ala Gly Leu Ser Lys Ser Lys Gly Ser Arg Ile
            15                  20                  25

Gly Phe Asp Ser Thr Gln Trp Arg Ala Val Lys Lys Phe Ile Met Leu
        30                  35                  40

Thr Ser Asn Gln Asn Val Pro Val Phe Leu Ile Asp Pro Leu Ile Leu
    45                  50                  55

Glu Leu Ile Asn Lys Asn Phe Glu Gln Val Lys Asn Thr Ser His Gly
60                  65                  70                  75

Ser Thr Ser Gln Cys Lys Phe Phe Cys Val Pro Arg Asp Phe Thr Ala
                80                  85                  90

Phe Ala Leu Gln Tyr His Leu Trp Lys Asn Glu Glu Gly Trp Phe Arg
            95                  100                 105

Ile Ala Glu Asn Met Gly Phe Gln Cys Leu Lys Ile Glu Ser Lys Asp
        110                 115                 120

Pro Arg Leu Asp Gly Ile Asp Ser Leu Ser Gly Thr Glu Ile Pro Leu
    125                 130                 135

His Tyr Ile Cys Lys Leu Ala Thr His Ala Ile His Leu Val Val Phe
140                 145                 150                 155

His Glu Arg Ser Gly Asn Tyr Leu Trp His Gly His Leu Arg Leu Lys
                160                 165                 170

Glu His Ile Asp Arg Lys Phe Val Pro Phe Gln Lys Leu Gln Phe Gly
            175                 180                 185

Arg Tyr Pro Gly Ala Phe Asp Arg Pro Glu Leu Gln Gln Val Thr Val
        190                 195                 200

Asp Gly Leu Glu Val Leu Ile Pro Lys Asp Pro Met His Phe Val Glu
    205                 210                 215

Glu Val Pro His Ser Arg Phe Ile Glu Cys Arg Tyr Lys Glu Ala Arg
220                 225                 230                 235

Ala Phe Phe Gln Ile Pro Val Ser Glu Val Tyr Thr Val Leu Asp
                240                 245                 250

<210>  5

<211>  3664

<212>  DNA

<213>  Homo sapiens

<220>

<221>  CDS

<222>  (347)..(1981)

<223>

<220>

<221>  mutation

<222>  (383)..(385)

<223>  variant wherein nucleotides 383-385 are deleted

<220>

<221>  mat_peptide

<222>  (416)..()

<223>

<220>

<221>  sig_peptide

<222>  (1)..(23)

<223>  wherein Leu at position -11 may be deleted

<400>  5; LP224
gccggcgctg gccccctccc ccgcggggcg ggccccgcca tgcattattc attggccaga      60

gccgggctgc gggcgcgagt gctgatgtca ggcaggcgcg agtgcggcgg gcgcagcggc     120

ggcggccgcc cagcagcccg ggctcgggtg ggggggcgcc gagtgggggt ggcggccagc     180

atgctgctcg gctgcggctc ggcctcccac acccgcggct ccctagtcct cgccagcagc     240

ggcggcggca gagcagcgag cggcgcccgc gtctgcagcg gcgccgggtc cgagcgcgcg     300

gcgcggcggt gggggtcggg gcccgggcgg ggagcgggga ccgggc atg gcg ctg        355
                                                   Met Ala Leu

cgg aga ggc ggc tgc gga gcg ctc ggg ctg ctg ctg ctg ctg ctg ggc       403
Arg Arg Gly Gly Cys Gly Ala Leu Gly Leu Leu Leu Leu Leu Leu Gly
-20                 -15                 -10                 -5

gcc gcg tgc ctg ata ccg cgg agc gcg cag gtg agg cgg ctg gcg cgc       451
Ala Ala Cys Leu Ile Pro Arg Ser Ala Gln Val Arg Arg Leu Ala Arg
            -1  1               5                   10

tgc ccc gcc act tgc agc tgt acc aag gag tct atc atc tgc gtg ggc       499
Cys Pro Ala Thr Cys Ser Cys Thr Lys Glu Ser Ile Ile Cys Val Gly
        15                  20                  25

tct tcc tgg gtg ccc agg atc gtg ccg ggc gac atc agc tcc ctg agc       547
Ser Ser Trp Val Pro Arg Ile Val Pro Gly Asp Ile Ser Ser Leu Ser
    30                  35                  40

ctg gta aat ggg acg ttt tca gaa atc aag gac cga atg ttt tcc cat 595
Leu Val Asn Gly Thr Phe Ser Glu Ile Lys Asp Arg Met Phe Ser His
45 50 55 60

ctg cct tct ctg cag ctg cta ttg ctg aat tct aac tca ttc acg atc 643
Leu Pro Ser Leu Gln Leu Leu Leu Leu Asn Ser Asn Ser Phe Thr Ile
65 70 75

atc cgg gat gat gct ttt gct gga ctt ttt cat ctt gaa tac ctg ttc 691
Ile Arg Asp Asp Ala Phe Ala Gly Leu Phe His Leu Glu Tyr Leu Phe
80 85 90

att gaa ggg aac aaa ata gaa acc att tca aga aat gcc ttt cgt ggc 739
Ile Glu Gly Asn Lys Ile Glu Thr Ile Ser Arg Asn Ala Phe Arg Gly
95 100 105

ctc cgt gac ctg act cac ctt tct ttg gcc aat aac cac ata aaa gca 787
Leu Arg Asp Leu Thr His Leu Ser Leu Ala Asn Asn His Ile Lys Ala
110 115 120

cta cca agg gat gtc ttc agt gat tta gac tct ctg att gaa cta gat 835
Leu Pro Arg Asp Val Phe Ser Asp Leu Asp Ser Leu Ile Glu Leu Asp
125 130 135 140

ttg agg ggt aat aaa ttt gaa tgt gac tgc aaa gcc aag tgg cta tac 883
Leu Arg Gly Asn Lys Phe Glu Cys Asp Cys Lys Ala Lys Trp Leu Tyr
145 150 155

ctg tgg ttg aag atg aca aat tcc acc gtt tct gat gtg ctg tgt att 931
Leu Trp Leu Lys Met Thr Asn Ser Thr Val Ser Asp Val Leu Cys Ile
160 165 170

ggt cca cca gag tat cag gaa aag aag cta aat gac gtg acc agc ttt 979
Gly Pro Pro Glu Tyr Gln Glu Lys Lys Leu Asn Asp Val Thr Ser Phe
175 180 185

gac tat gaa tgc aca act aca gat ttt gtt gtt cat cag act tta ccc 1027
Asp Tyr Glu Cys Thr Thr Thr Asp Phe Val Val His Gln Thr Leu Pro
190 195 200

tac cag tcg gtt tca gtg gat acg ttc aac tcc aag aac gat gtg tac 1075
Tyr Gln Ser Val Ser Val Asp Thr Phe Asn Ser Lys Asn Asp Val Tyr
205 210 215 220

gtg gcc atc gcg cag ccc agc atg gag aac tgc atg gtg ctg gag tgg 1123
Val Ala Ile Ala Gln Pro Ser Met Glu Asn Cys Met Val Leu Glu Trp
225 230 235

gac cac att gaa atg aat ttc cgg agc tat gac aac att aca ggt cag 1171
Asp His Ile Glu Met Asn Phe Arg Ser Tyr Asp Asn Ile Thr Gly Gln
240 245 250

tcc atc gtg ggc tgt aag gcc att ctc atc gat gat cag gtc ttt gtg 1219
Ser Ile Val Gly Cys Lys Ala Ile Leu Ile Asp Asp Gln Val Phe Val
255 260 265

gtg gta gcc cag ctc ttc ggt ggc tct cac att tac aaa tac gac gag 1267

Val Val Ala Gln Leu Phe Gly Gly Ser His Ile Tyr Lys Tyr Asp Glu
    270                 275                 280

agt tgg acc aaa ttt gtc aaa ttc caa gac ata gag gtc tct cgc att      1315
Ser Trp Thr Lys Phe Val Lys Phe Gln Asp Ile Glu Val Ser Arg Ile
285                 290                 295                 300

tcc aag ccc aat gac atc gag ctg ttt cag atc gac gac gag acg ttc      1363
Ser Lys Pro Asn Asp Ile Glu Leu Phe Gln Ile Asp Asp Glu Thr Phe
                305                 310                 315

ttt gtc atc gca gac agc tca aag gct ggt ctg tcc aca gtt tat aaa      1411
Phe Val Ile Ala Asp Ser Ser Lys Ala Gly Leu Ser Thr Val Tyr Lys
            320                 325                 330

tgg aac agc aaa gga ttc tat tct tac cag tca ctg cac gag tgg ttc      1459
Trp Asn Ser Lys Gly Phe Tyr Ser Tyr Gln Ser Leu His Glu Trp Phe
        335                 340                 345

agg gac acg gat gcg gag ttt gtt gat atc gat gga aaa tcg cat ctc      1507
Arg Asp Thr Asp Ala Glu Phe Val Asp Ile Asp Gly Lys Ser His Leu
    350                 355                 360

atc ctg tcc agc cgc tcc cag gtc ccc atc atc ctc cag tgg aat aaa      1555
Ile Leu Ser Ser Arg Ser Gln Val Pro Ile Ile Leu Gln Trp Asn Lys
365                 370                 375                 380

agc tct aag aag ttt gtc ccc cat ggt gac atc ccc aac atg gag gac      1603
Ser Ser Lys Lys Phe Val Pro His Gly Asp Ile Pro Asn Met Glu Asp
                385                 390                 395

gta ctg gct gtg aag agc ttc cga atg caa aat acc ctc tac ctt tcc      1651
Val Leu Ala Val Lys Ser Phe Arg Met Gln Asn Thr Leu Tyr Leu Ser
            400                 405                 410

ctt acc cgc ttc atc ggg gac tcc cgg gtc atg agg tgg aac agt aag      1699
Leu Thr Arg Phe Ile Gly Asp Ser Arg Val Met Arg Trp Asn Ser Lys
        415                 420                 425

cag ttt gtg gag atc caa gct ctt cca tcc cgg ggg gcc atg acc ctg      1747
Gln Phe Val Glu Ile Gln Ala Leu Pro Ser Arg Gly Ala Met Thr Leu
    430                 435                 440

cag ccc ttt tct ttt aaa gat aat cac tac ctg gcc ctg ggg agt gac      1795
Gln Pro Phe Ser Phe Lys Asp Asn His Tyr Leu Ala Leu Gly Ser Asp
445                 450                 455                 460

tat aca ttc tct cag ata tac cag tgg gat aaa gag aag cag cta ttc      1843
Tyr Thr Phe Ser Gln Ile Tyr Gln Trp Asp Lys Glu Lys Gln Leu Phe
                465                 470                 475

aaa aag ttt aag gag att tac gtg cag gcg cct cgt tca ttc aca gct      1891
Lys Lys Phe Lys Glu Ile Tyr Val Gln Ala Pro Arg Ser Phe Thr Ala
            480                 485                 490

gtc tcc acc gac agg aga gat ttc ttt ttt gca tcc agt ttc aaa ggg      1939
Val Ser Thr Asp Arg Arg Asp Phe Phe Phe Ala Ser Ser Phe Lys Gly
        495                 500                 505

aaa aca aag att ttt gaa cat ata att gtt gac tta agt ttg          1981
Lys Thr Lys Ile Phe Glu His Ile Ile Val Asp Leu Ser Leu
    510                 515                 520

tgaaggtgtg gtgggtgaaa ctaagagaaa tgtagcatta gctctcacaa aagaggacca   2041

agaaaaatca acaaacaaat caaagccagg ctcagagctc tgaaattaaa aagcactgaa   2101

atagttagat gttttcaaac ttttagaact cacattttaa tcagggattg catttattgg   2161

ctaactgcat gacatgccca ttctaccatt ttaaaaaaaa tcttaaagcc tgtaatttct   2221

gagaaaagag tacagcattt actcttatca tctagaaatg taatatgctt cccccccgct   2281

ttttgatgag gaagaagaca attggataag atgggacagc acttataatg aaataaaaaa   2341

aaactttgag cccctctcat tccactttag caatcttttt ggtaagaact cttaaagcca   2401

aaagtctgct gaaaagattt gctgattatt agtttaaaaa tcttgtaaca ctcagcagtg   2461

ctattttgag tcatcccagt ttcctgaaag taatgcccag tcttcctgaa tcctccttaa   2521

tagcagaacc ttggtgattt tgttggctca tatgaatgct tgtcatggat atgttaacaa   2581

tttagtgttt gacattgctt cctctgccac aaagacaata ctctggtgac acatgtctag   2641

acccagcaca ggctgtaggc ccaggagtga ctcaaaggag tttttccctc tttcttacgg   2701

ttcaaaggtg accctggtgg tggccagagc agtaatgctt gtttgatgct cttcatggct   2761

catctgcttc tcagaaccca cccgttgagt ttgtgggtaa ccagcaggca ggccaaagac   2821

tggtgctttt catttcatcc tttagaggga tgaaacagtt atttccgtct gatgagcatt   2881

cggtagaatt tttgaagtga gattttatga agtcaaaggg gactttacac agatctcgac   2941

ctgctttgaa acctagaggt ggccctttga tttgtgcgtg tccttgccct ctggacaact   3001

taatatttca agtaatcgaa taccaacttc cctgccagcc cacctgcctt ccgccccgct   3061

tgtgtaacag tcctgttttg ttgagttgct gctattgcac tgccagtgca gcccacacca   3121

aatcacaacc caagatactc agataggaag actccttcct ctcccagtac tttaccaaag   3181

gaacccccgc caggacccac atggggccac gtgttggcag tggaatcagc ctgtgcaggc   3241

tggggatctc aggctgatca gtaggggcca gctttggagc cagccaagct gaatcccaca   3301

ctccaggtct gtgctcaaga gaccagatgg tgtatttcca aatgggcctc tctggtatgg   3361

gcaataggca agctcctggg gtctggttat gtggaagatt cttagtggat gttccgcctg   3421

gttagctggt tctcttcaga gaatataaag tgaatgcctt taggggtagc tctgaaagag   3481

aaacccaaca acttcattcc tagccatgaa agtagcacga tcatattgta ctgtattgtt   3541

attgtaaaat gactatttgc catgtcatga gtaggtagat gttttgccac aaatatgaat   3601

gtgtttgttg tttcctgact ttaagcaatg aagattgaga caataaatag cactcagaga   3661

atg                                                                 3664

<210>  6

<211>  545

<212>  PRT

<213>  Homo sapiens

<400>  6; LP224

Met Ala Leu Arg Arg Gly Gly Cys Gly Ala Leu Gly Leu Leu Leu Leu
            -20                 -15                 -10

Leu Leu Gly Ala Ala Cys Leu Ile Pro Arg Ser Ala Gln Val Arg Arg
        -5              -1  1               5

Leu Ala Arg Cys Pro Ala Thr Cys Ser Cys Thr Lys Glu Ser Ile Ile
10                  15                  20                  25

Cys Val Gly Ser Ser Trp Val Pro Arg Ile Val Pro Gly Asp Ile Ser
                30                  35                  40

Ser Leu Ser Leu Val Asn Gly Thr Phe Ser Glu Ile Lys Asp Arg Met
            45                  50                  55

Phe Ser His Leu Pro Ser Leu Gln Leu Leu Leu Leu Asn Ser Asn Ser
        60                  65                  70

Phe Thr Ile Ile Arg Asp Asp Ala Phe Ala Gly Leu Phe His Leu Glu
    75                  80                  85

Tyr Leu Phe Ile Glu Gly Asn Lys Ile Glu Thr Ile Ser Arg Asn Ala
90                  95                  100                 105

Phe Arg Gly Leu Arg Asp Leu Thr His Leu Ser Leu Ala Asn Asn His
                110                 115                 120

Ile Lys Ala Leu Pro Arg Asp Val Phe Ser Asp Leu Asp Ser Leu Ile
            125                 130                 135

Glu Leu Asp Leu Arg Gly Asn Lys Phe Glu Cys Asp Cys Lys Ala Lys
        140                 145                 150

Trp Leu Tyr Leu Trp Leu Lys Met Thr Asn Ser Thr Val Ser Asp Val
    155                 160                 165

Leu Cys Ile Gly Pro Pro Glu Tyr Gln Glu Lys Lys Leu Asn Asp Val
170                 175                 180                 185

Thr Ser Phe Asp Tyr Glu Cys Thr Thr Thr Asp Phe Val Val His Gln
                190                 195                 200

Thr Leu Pro Tyr Gln Ser Val Ser Val Asp Thr Phe Asn Ser Lys Asn
            205                 210                 215

Asp Val Tyr Val Ala Ile Ala Gln Pro Ser Met Glu Asn Cys Met Val
        220                 225                 230

Leu Glu Trp Asp His Ile Glu Met Asn Phe Arg Ser Tyr Asp Asn Ile
    235                 240                 245

Thr Gly Gln Ser Ile Val Gly Cys Lys Ala Ile Leu Ile Asp Asp Gln
250                 255                 260                 265

Val Phe Val Val Val Ala Gln Leu Phe Gly Gly Ser His Ile Tyr Lys
                270                 275                 280

Tyr Asp Glu Ser Trp Thr Lys Phe Val Lys Phe Gln Asp Ile Glu Val
            285                 290                 295

Ser Arg Ile Ser Lys Pro Asn Asp Ile Glu Leu Phe Gln Ile Asp Asp
        300                 305                 310

Glu Thr Phe Phe Val Ile Ala Asp Ser Ser Lys Ala Gly Leu Ser Thr
    315                 320                 325

Val Tyr Lys Trp Asn Ser Lys Gly Phe Tyr Ser Tyr Gln Ser Leu His
330                 335                 340                 345

Glu Trp Phe Arg Asp Thr Asp Ala Glu Phe Val Asp Ile Asp Gly Lys
                350                 355                 360

Ser His Leu Ile Leu Ser Ser Arg Ser Gln Val Pro Ile Ile Leu Gln
            365                 370                 375

Trp Asn Lys Ser Ser Lys Lys Phe Val Pro His Gly Asp Ile Pro Asn
        380                 385                 390

Met Glu Asp Val Leu Ala Val Lys Ser Phe Arg Met Gln Asn Thr Leu
    395                 400                 405

Tyr Leu Ser Leu Thr Arg Phe Ile Gly Asp Ser Arg Val Met Arg Trp
410                 415                 420                 425

Asn Ser Lys Gln Phe Val Glu Ile Gln Ala Leu Pro Ser Arg Gly Ala
                430                 435                 440

Met Thr Leu Gln Pro Phe Ser Phe Lys Asp Asn His Tyr Leu Ala Leu
            445                 450                 455

Gly Ser Asp Tyr Thr Phe Ser Gln Ile Tyr Gln Trp Asp Lys Glu Lys
        460                 465                 470

Gln Leu Phe Lys Lys Phe Lys Glu Ile Tyr Val Gln Ala Pro Arg Ser
    475                 480                 485

Phe Thr Ala Val Ser Thr Asp Arg Arg Asp Phe Phe Phe Ala Ser Ser
490                 495                 500                 505

Phe Lys Gly Lys Thr Lys Ile Phe Glu His Ile Ile Val Asp Leu Ser
                510                 515                 520

Leu

<210>  7

<211>  898

<212>  DNA

<213>  Homo sapiens

<220>

<221>  CDS

<222>  (19)..(642)

<223>

<220>

<221>  mat_peptide

<222>  (64)..()

<223>

<400>  7; LP240
cggcgggccg ggacgggc atg gcc ctg ctg ctg tgc ctg gtg tgc ctg acg        51
                    Met Ala Leu Leu Leu Cys Leu Val Cys Leu Thr
                    -15                 -10                 -5

gcg gcg ctg gcc cac ggc tgt ctg cac tgc cac agc aac ttc tcc aag        99
Ala Ala Leu Ala His Gly Cys Leu His Cys His Ser Asn Phe Ser Lys
            -1  1               5                   10

aag ttc tcc ttc tac cgc cac cat gtg aac ttc aag tcc tgg tgg gtg        147
Lys Phe Ser Phe Tyr Arg His His Val Asn Phe Lys Ser Trp Trp Val
        15                  20                  25

ggc gac atc ccc gtg tca ggg gcg ctg ctc acc gac tgg agc gac gac        195
Gly Asp Ile Pro Val Ser Gly Ala Leu Leu Thr Asp Trp Ser Asp Asp
    30                  35                  40

acg atg aag gag ctg cac ctg gcc atc ccc gcc aag atc acc cgg gag        243
Thr Met Lys Glu Leu His Leu Ala Ile Pro Ala Lys Ile Thr Arg Glu
45                  50                  55                  60

aag ctg gac caa gtg gcg aca gca gtg tac cag atg atg gat cag ctg        291
Lys Leu Asp Gln Val Ala Thr Ala Val Tyr Gln Met Met Asp Gln Leu
                65                  70                  75

tac cag ggg aag atg tac ttc ccc ggg tat ttc ccc aac gag ctg cga        339
Tyr Gln Gly Lys Met Tyr Phe Pro Gly Tyr Phe Pro Asn Glu Leu Arg
            80                  85                  90

aac atc ttc cgg gag cag gtg cac ctc atc cag aac gcc atc atc gaa        387
Asn Ile Phe Arg Glu Gln Val His Leu Ile Gln Asn Ala Ile Ile Glu
        95                  100                 105

agc cgc atc gac tgt cag cac cgc tgt ggc atc ttc cag tac gag acc        435
Ser Arg Ile Asp Cys Gln His Arg Cys Gly Ile Phe Gln Tyr Glu Thr
    110                 115                 120

atc tcc tgc aac aac tgc aca gac tcg cac gtc gcc tgc ttt ggc tat        483
Ile Ser Cys Asn Asn Cys Thr Asp Ser His Val Ala Cys Phe Gly Tyr
125                 130                 135                 140

aac tgc gag tcc tcg gcg cag tgg aag tca gct gtc cag ggc ctc ctg        531
Asn Cys Glu Ser Ser Ala Gln Trp Lys Ser Ala Val Gln Gly Leu Leu
                145                 150                 155

aac tac ata aat aac tgg cac aac ctg gta tcg cca gcc tta agg tgt 579
Asn Tyr Ile Asn Asn Trp His Asn Leu Val Ser Pro Ala Leu Arg Cys
160 165 170

ctg gag ccc cca cac ttg gcc aac ctg acc ttg gaa aat gct gct gag 627
Leu Glu Pro Pro His Leu Ala Asn Leu Thr Leu Glu Asn Ala Ala Glu
175 180 185

tgt ctc aag cag cac tgacagcagc tgggcctgcc ccagggcaac gtgggggcgg 682
Cys Leu Lys Gln His
190

agactcagct ggacagcccc tgcctgtcac tctggagctg ggctgctgct gcctcaggac 742

cccctctccg accccggaca gagctgagct ggccagggcc aggagggcgg gagggaggga 802

atgggggtgg gctgtgcgca gcatcagcgc ctgggcaggt ccgcagagct gcgggatgtg 862

attaaagtcc ctgatgtttc tcaaaaaaaa aaaaaa 898

<210> 8

<211> 208

<212> PRT

<213> Homo sapiens

<400> 8; LP240

Met Ala Leu Leu Leu Cys Leu Val Cys Leu Thr Ala Ala Leu Ala His
-15 -10 -5 -1 1

Gly Cys Leu His Cys His Ser Asn Phe Ser Lys Lys Phe Ser Phe Tyr
                5                   10                  15

Arg His His Val Asn Phe Lys Ser Trp Trp Val Gly Asp Ile Pro Val
            20                  25                  30

Ser Gly Ala Leu Leu Thr Asp Trp Ser Asp Asp Thr Met Lys Glu Leu
        35                  40                  45

His Leu Ala Ile Pro Ala Lys Ile Thr Arg Glu Lys Leu Asp Gln Val
50 55 60 65

Ala Thr Ala Val Tyr Gln Met Met Asp Gln Leu Tyr Gln Gly Lys Met
70 75 80

Tyr Phe Pro Gly Tyr Phe Pro Asn Glu Leu Arg Asn Ile Phe Arg Glu
                85                  90                  95

Gln Val His Leu Ile Gln Asn Ala Ile Ile Glu Ser Arg Ile Asp Cys
            100                 105                 110

Gln His Arg Cys Gly Ile Phe Gln Tyr Glu Thr Ile Ser Cys Asn Asn
        115                 120                 125

Cys Thr Asp Ser His Val Ala Cys Phe Gly Tyr Asn Cys Glu Ser Ser
130                 135                 140                 145

Ala Gln Trp Lys Ser Ala Val Gln Gly Leu Leu Asn Tyr Ile Asn Asn
                150                 155                 160

Trp His Asn Leu Val Ser Pro Ala Leu Arg Cys Leu Glu Pro Pro His
                165                 170                 175

Leu Ala Asn Leu Thr Leu Glu Asn Ala Ala Glu Cys Leu Lys Gln His
            180                 185                 190

<210>  9

<211>  1580

<212>  DNA

<213>  Homo sapiens


<220>

<221>  CDS

<222>  (136)..(1386)

<223>


<220>

<221>  mat_peptide

<222>  (196)..()

<223>

<400>  9; LP239(a)
ctcaattctt aaaaaataag tccatgaagc agaaacatca aaaagcatgg gatttggaat      60

ttgagacctg actttgaagc ccacacagaa cacagacaag tcaaccatgc tatttgggac     120

atattttgtt ccaaa atg gca tct tac ctt tat gga gta ctc ttt gct gtt      171
                 Met Ala Ser Tyr Leu Tyr Gly Val Leu Phe Ala Val
                 -20                 -15                 -10

ggc ctc tgt gct cca atc tac tgt gtg tcc ccg gcc aat gcc ccc agt      219
Gly Leu Cys Ala Pro Ile Tyr Cys Val Ser Pro Ala Asn Ala Pro Ser
            -5                  -1  1               5

gca tac ccc cgc cct tcc tcc aca aag agc acc cct gcc tca cag gtg      267
Ala Tyr Pro Arg Pro Ser Ser Thr Lys Ser Thr Pro Ala Ser Gln Val
    10                  15                  20

tat tcc ctc aac acc gac ttt gcc ttc cgc cta tac cgc agg ctg gtt      315
Tyr Ser Leu Asn Thr Asp Phe Ala Phe Arg Leu Tyr Arg Arg Leu Val
25                  30                  35                  40

ttg gag acc ccg agt cag aac atc ttc ttc tcc cct gtg agt gtc tcc      363
Leu Glu Thr Pro Ser Gln Asn Ile Phe Phe Ser Pro Val Ser Val Ser
                45                  50                  55

act tcc ctg gcc atg ctc tcc ctt ggg gcc cac tca gtc acc aag acc      411
Thr Ser Leu Ala Met Leu Ser Leu Gly Ala His Ser Val Thr Lys Thr
            60                  65                  70

cag att ctc cag ggc ctg ggc ttc aac ctc aca cac aca cca gag tct      459
Gln Ile Leu Gln Gly Leu Gly Phe Asn Leu Thr His Thr Pro Glu Ser
        75                  80                  85

gcc atc cac cag ggc ttc cag cac ctg gtt cac tca ctg act gtt ccc      507
Ala Ile His Gln Gly Phe Gln His Leu Val His Ser Leu Thr Val Pro
    90                  95                  100

agc aaa gac ctg acc ttg aag atg gga agt gcc ctc ttc gtc aag aag      555
Ser Lys Asp Leu Thr Leu Lys Met Gly Ser Ala Leu Phe Val Lys Lys
105                 110                 115                 120

gag ctg cag ctg cag gca aat ttc ttg ggc aat gtc aag agg ctg tat      603
Glu Leu Gln Leu Gln Ala Asn Phe Leu Gly Asn Val Lys Arg Leu Tyr
                125                 130                 135

gaa gca gaa gtc ttt tct aca gat ttc tcc aac ccc tcc att gcc cag      651
Glu Ala Glu Val Phe Ser Thr Asp Phe Ser Asn Pro Ser Ile Ala Gln
            140                 145                 150

gcg agg atc aac agc cat gtg aaa aag aag acc caa ggg aag gtt gta      699
Ala Arg Ile Asn Ser His Val Lys Lys Lys Thr Gln Gly Lys Val Val
        155                 160                 165

gac ata atc caa ggc ctt gac ctt ctg acg gcc atg gtt ctg gtg aac      747
Asp Ile Ile Gln Gly Leu Asp Leu Leu Thr Ala Met Val Leu Val Asn
    170                 175                 180

cac att ttc ttt aaa gcc aag tgg gag aag ccc ttt cac cct gaa tat      795

His Ile Phe Phe Lys Ala Lys Trp Glu Lys Pro Phe His Pro Glu Tyr
185                 190                 195                 200

aca aga aag aac ttc cca ttc ctg gtg ggc gag cag gtc act gtg cat      843
Thr Arg Lys Asn Phe Pro Phe Leu Val Gly Glu Gln Val Thr Val His
                205                 210                 215

gtc ccc atg atg cac cag aaa gag cag ttc gct ttt ggg gtg gat aca      891
Val Pro Met Met His Gln Lys Glu Gln Phe Ala Phe Gly Val Asp Thr
            220                 225                 230

gag ctg aac tgc ttt gtg ctg cag atg gat tac aag gga gat gcc gtg      939
Glu Leu Asn Cys Phe Val Leu Gln Met Asp Tyr Lys Gly Asp Ala Val
        235                 240                 245

gcc ttc ttt gtc ctc cct agc aag ggc aag atg agg caa ctg gaa cag      987
Ala Phe Phe Val Leu Pro Ser Lys Gly Lys Met Arg Gln Leu Glu Gln
    250                 255                 260

gcc ttg tca gcc aga aca ctg aga aag tgg agc cac tca ctc cag aaa     1035
Ala Leu Ser Ala Arg Thr Leu Arg Lys Trp Ser His Ser Leu Gln Lys
265                 270                 275                 280

agg tgg ata gag gtg ttc atc ccc aga ttt tcc att tct gcc tcc tac     1083
Arg Trp Ile Glu Val Phe Ile Pro Arg Phe Ser Ile Ser Ala Ser Tyr
                285                 290                 295

aat ctg gaa acc atc ctc ccg aag atg ggc atc caa aat gtc ttt gac     1131
Asn Leu Glu Thr Ile Leu Pro Lys Met Gly Ile Gln Asn Val Phe Asp
            300                 305                 310

aaa aat gct gat ttt tct gga att gca aag aga gac tcc ctg cag gtt     1179
Lys Asn Ala Asp Phe Ser Gly Ile Ala Lys Arg Asp Ser Leu Gln Val
        315                 320                 325

tct aaa gca acc cac aag gct gtg ctg gat gtc agt gaa gag ggc act     1227
Ser Lys Ala Thr His Lys Ala Val Leu Asp Val Ser Glu Glu Gly Thr
    330                 335                 340

gag gcc aca gca gct acc acc acc aag ttc ata gtc cga tcg aag gat     1275
Glu Ala Thr Ala Ala Thr Thr Thr Lys Phe Ile Val Arg Ser Lys Asp
345                 350                 355                 360

ggc ccc tct tac ttc act gtc tcc ttc aat agg acc ttc ctg atg atg     1323
Gly Pro Ser Tyr Phe Thr Val Ser Phe Asn Arg Thr Phe Leu Met Met
                365                 370                 375

att aca aat aaa gcc aca gac ggt att ctc ttt cta ggg aaa gtg gaa     1371
Ile Thr Asn Lys Ala Thr Asp Gly Ile Leu Phe Leu Gly Lys Val Glu
            380                 385                 390

aat ccc act aaa tcc taggtgggaa atggcctgtt aactgatggc acattgctaa     1426
Asn Pro Thr Lys Ser
        395

tgcacaagaa ataacaaacc acatccctct ttctgttctg agggtgcatt tgaccccagt     1486

ggagctggat tcgctggcag ggatgccact tccaaggctc aatcaccaaa ccatcaacag     1546

ggaccccagt cacaagccaa cacccattaa cccc                                    1580

<210>  10

<211>  417

<212>  PRT

<213>  Homo sapiens

<400>  10; LP239(a)

Met Ala Ser Tyr Leu Tyr Gly Val Leu Phe Ala Val Gly Leu Cys Ala
-20                 -15                 -10                 -5

Pro Ile Tyr Cys Val Ser Pro Ala Asn Ala Pro Ser Ala Tyr Pro Arg
            -1  1               5                   10

Pro Ser Ser Thr Lys Ser Thr Pro Ala Ser Gln Val Tyr Ser Leu Asn
        15                  20                  25

Thr Asp Phe Ala Phe Arg Leu Tyr Arg Arg Leu Val Leu Glu Thr Pro
    30                  35                  40

Ser Gln Asn Ile Phe Phe Ser Pro Val Ser Val Ser Thr Ser Leu Ala
45                  50                  55                  60

Met Leu Ser Leu Gly Ala His Ser Val Thr Lys Thr Gln Ile Leu Gln
                65                  70                  75

Gly Leu Gly Phe Asn Leu Thr His Thr Pro Glu Ser Ala Ile His Gln
            80                  85                  90

Gly Phe Gln His Leu Val His Ser Leu Thr Val Pro Ser Lys Asp Leu
        95                  100                 105

Thr Leu Lys Met Gly Ser Ala Leu Phe Val Lys Lys Glu Leu Gln Leu
    110                 115                 120

Gln Ala Asn Phe Leu Gly Asn Val Lys Arg Leu Tyr Glu Ala Glu Val
125                 130                 135                 140

Phe Ser Thr Asp Phe Ser Asn Pro Ser Ile Ala Gln Ala Arg Ile Asn
                145                 150                 155

Ser His Val Lys Lys Lys Thr Gln Gly Lys Val Val Asp Ile Ile Gln
            160                 165                 170

Gly Leu Asp Leu Leu Thr Ala Met Val Leu Val Asn His Ile Phe Phe
        175                 180                 185

Lys Ala Lys Trp Glu Lys Pro Phe His Pro Glu Tyr Thr Arg Lys Asn
    190                 195                 200

Phe Pro Phe Leu Val Gly Glu Gln Val Thr Val His Val Pro Met Met
205                 210                 215                 220

His Gln Lys Glu Gln Phe Ala Phe Gly Val Asp Thr Glu Leu Asn Cys
                225                 230                 235

Phe Val Leu Gln Met Asp Tyr Lys Gly Asp Ala Val Ala Phe Phe Val
            240                 245                 250

Leu Pro Ser Lys Gly Lys Met Arg Gln Leu Glu Gln Ala Leu Ser Ala
        255                 260                 265

Arg Thr Leu Arg Lys Trp Ser His Ser Leu Gln Lys Arg Trp Ile Glu
    270                 275                 280

Val Phe Ile Pro Arg Phe Ser Ile Ser Ala Ser Tyr Asn Leu Glu Thr
285                 290                 295                 300

Ile Leu Pro Lys Met Gly Ile Gln Asn Val Phe Asp Lys Asn Ala Asp
                305                 310                 315

Phe Ser Gly Ile Ala Lys Arg Asp Ser Leu Gln Val Ser Lys Ala Thr
            320                 325                 330

His Lys Ala Val Leu Asp Val Ser Glu Glu Gly Thr Glu Ala Thr Ala
        335                 340                 345

Ala Thr Thr Thr Lys Phe Ile Val Arg Ser Lys Asp Gly Pro Ser Tyr
    350                 355                 360

Phe Thr Val Ser Phe Asn Arg Thr Phe Leu Met Met Ile Thr Asn Lys
365                 370                 375                 380

Ala Thr Asp Gly Ile Leu Phe Leu Gly Lys Val Glu Asn Pro Thr Lys
                385                 390                 395

Ser

<210>  11

<211>  2080

<212>  DNA

<213>  Homo sapiens

<220>

<221>  CDS

<222>  (12)..(1682)

<223>

<220>

<221>  mat_peptide

<222>  (96)..()

<223>

<220>

<221>  misc_feature

<222>  (82)..(82)

<223>  wherein n is A, G, C, or T

<400>  11; LP243(a)
tcaccaccat c atg gtg tcc acc gcc atg ggc tgc atc acc ttc ctg ggc       50
             Met Val Ser Thr Ala Met Gly Cys Ile Thr Phe Leu Gly
                         -25                 -20

gtg gtc ctc ttg ctg ctt tcg tgc tgc tgt tnc gtg tgg agc cgc aac       98
Val Val Leu Leu Leu Leu Ser Cys Cys Cys Xaa Val Trp Ser Arg Asn
-15                 -10                 -5                  -1  1

cgc atc cgc tgc ctg aac ccg ggc gac ctg gcc gcg ctg ccc gcg ctg       146

Arg Ile Arg Cys Leu Asn Pro Gly Asp Leu Ala Ala Leu Pro Ala Leu
            5                   10                  15

gag gag ctg gac ctg agc gag aac gcc atc gcg cac gtg gag ccc ggc      194
Glu Glu Leu Asp Leu Ser Glu Asn Ala Ile Ala His Val Glu Pro Gly
        20                  25                  30

gcc ttc gcc aac ctg ccg cgc ctg cgc gtc ctg cgt ctc cgt ggc aac      242
Ala Phe Ala Asn Leu Pro Arg Leu Arg Val Leu Arg Leu Arg Gly Asn
    35                  40                  45

cag ctg aag ctc atc ccg ccc ggg gtc ttc acg cgc ctg gac aac ctc      290
Gln Leu Lys Leu Ile Pro Pro Gly Val Phe Thr Arg Leu Asp Asn Leu
50                  55                  60                  65

acg ctg ctg gac ctg agc gag aac aag ctg gta atc ctg ctg gac tac      338
Thr Leu Leu Asp Leu Ser Glu Asn Lys Leu Val Ile Leu Leu Asp Tyr
                70                  75                  80

act ttc cag gac ctg cac agc ctg cgc cgg ctg gaa gtg ggc gac aac      386
Thr Phe Gln Asp Leu His Ser Leu Arg Arg Leu Glu Val Gly Asp Asn
            85                  90                  95

gac ctg gta ttc gtc tcg cgc cgc gcc ttc gcg ggg ctg ctg gcc ctg      434
Asp Leu Val Phe Val Ser Arg Arg Ala Phe Ala Gly Leu Leu Ala Leu
        100                 105                 110

gag gag ctg acc ctg gag cgc tgc aac ctc acg gct ctg tcc ggg gag      482
Glu Glu Leu Thr Leu Glu Arg Cys Asn Leu Thr Ala Leu Ser Gly Glu
    115                 120                 125

tcg ctg ggc cat ctg cgc agc ctg ggc gcc ctg cgg ctg cgc cac ctg      530
Ser Leu Gly His Leu Arg Ser Leu Gly Ala Leu Arg Leu Arg His Leu
130                 135                 140                 145

gcc atc gcc tcc ctg gag gac cag aac ttc cgc agg ctg ccc ggg ctg      578
Ala Ile Ala Ser Leu Glu Asp Gln Asn Phe Arg Arg Leu Pro Gly Leu
                150                 155                 160

ctg cac ctg gag att gac aac tgg ccg ctg ctg gag gag gtg gcg gcg      626
Leu His Leu Glu Ile Asp Asn Trp Pro Leu Leu Glu Glu Val Ala Ala
            165                 170                 175

ggc agc ctg cgg ggc ctg aac ctg acc tcg ctg tcg gtc acc cac acc      674
Gly Ser Leu Arg Gly Leu Asn Leu Thr Ser Leu Ser Val Thr His Thr
        180                 185                 190

aac atc acc gcc gtg ccg gcc gcc gcg ctg cgg cac cag gcg cac ctc      722
Asn Ile Thr Ala Val Pro Ala Ala Ala Leu Arg His Gln Ala His Leu
    195                 200                 205

acc tgc ctc aat ctg tcg cac aac ccc atc agc acg gtg ccg cgg ggg      770
Thr Cys Leu Asn Leu Ser His Asn Pro Ile Ser Thr Val Pro Arg Gly
210                 215                 220                 225

tcg ttc cgg gac ctg gtc cgc ctg cgc gag ctg cac ctg gcc ggg gcc      818
Ser Phe Arg Asp Leu Val Arg Leu Arg Glu Leu His Leu Ala Gly Ala
                230                 235                 240

ctg ctg gct gtg gtg gag ccg cag gcc ttc ctg ggc ctg cgc cag atc 866
Leu Leu Ala Val Val Glu Pro Gln Ala Phe Leu Gly Leu Arg Gln Ile
                245                 250                 255

cgc ctg ctc aac ctc tcc aac aac ctg ctc tcc acg ttg gag gag agc 914
Arg Leu Leu Asn Leu Ser Asn Asn Leu Leu Ser Thr Leu Glu Glu Ser
            260                 265                 270

acc ttc cac tcg gtg aac acg cta gag acg ctg cgc gtg gac ggg aac 962
Thr Phe His Ser Val Asn Thr Leu Glu Thr Leu Arg Val Asp Gly Asn
        275                 280                 285

ccg ctg gcc tgc gac tgt cgc ctg ctg tgg atc gtg cag cgt cgc aag 1010
Pro Leu Ala Cys Asp Cys Arg Leu Leu Trp Ile Val Gln Arg Arg Lys
290 295 300 305

acc ctc aac ttc gac ggg cgg ctg ccg gcc tgc gcc acc ccg gcc gag 1058
Thr Leu Asn Phe Asp Gly Arg Leu Pro Ala Cys Ala Thr Pro Ala Glu
310 315 320

gtg cgc ggc gac gcg ctg cga aac ctg ccg gac tcc gtg ctg ttc gag 1106
Val Arg Gly Asp Ala Leu Arg Asn Leu Pro Asp Ser Val Leu Phe Glu
                325                 330                 335

tac ttc gtg tgc cgc aaa ccc aag atc cgg gag cgg cgg ctg cag cgc 1154
Tyr Phe Val Cys Arg Lys Pro Lys Ile Arg Glu Arg Arg Leu Gln Arg
            340                 345                 350

gtc acg gcc acc gcg ggc gaa gac gtc cgc ttc ctc tgc cgc gcc gag 1202
Val Thr Ala Thr Ala Gly Glu Asp Val Arg Phe Leu Cys Arg Ala Glu
        355                 360                 365

ggc gag ccg gcg ccc acc gtg gcc tgg gtg acc ccc cag cac cgg ccg 1250
Gly Glu Pro Ala Pro Thr Val Ala Trp Val Thr Pro Gln His Arg Pro
370 375 380 385

gtg acg gcc acc agc gcg ggc cgg gcg cgc gtg ctc ccc ggg ggg acg 1298
Val Thr Ala Thr Ser Ala Gly Arg Ala Arg Val Leu Pro Gly Gly Thr
390 395 400

ctg gag atc cag gac gcg cgg ccg cag gac agc ggc acc tac acg tgc 1346
Leu Glu Ile Gln Asp Ala Arg Pro Gln Asp Ser Gly Thr Tyr Thr Cys
                405                 410                 415

gtg gcc agc aac gcg ggc ggc aac gac acc tac ttc gcc acg ctg acc 1394
Val Ala Ser Asn Ala Gly Gly Asn Asp Thr Tyr Phe Ala Thr Leu Thr
            420                 425                 430

gtg cgc ccc gag ccg gcc gcc aac cgg acc ccg ggc gag gcc cac aac 1442
Val Arg Pro Glu Pro Ala Ala Asn Arg Thr Pro Gly Glu Ala His Asn
        435                 440                 445

gag acg ctg gcg gcc ctg cgc gcg ccg ctc gac ctc acc acc atc ctg 1490
Glu Thr Leu Ala Ala Leu Arg Ala Pro Leu Asp Leu Thr Thr Ile Leu
450 455 460 465

gtg tcc acc gcc atg ggc tgc atc acc ttc ctg ggc gtg gtc ctc ttc 1538

Val Ser Thr Ala Met Gly Cys Ile Thr Phe Leu Gly Val Val Leu Phe
                470                 475                 480

tgc ttc gtg ctg ctg ttc gtg tgg agc cgc ggc cgc ggg cag cac aaa      1586
Cys Phe Val Leu Leu Phe Val Trp Ser Arg Gly Arg Gly Gln His Lys
            485                 490                 495

aac aac ttc tcg gtg gag tac tcc ttc cgc aag gtg gat ggg ccg gcc      1634
Asn Asn Phe Ser Val Glu Tyr Ser Phe Arg Lys Val Asp Gly Pro Ala
        500                 505                 510

gcc gcg gcg ggc cag gga ggc gcg cgc aag ttc aac atg aag atg atc      1682
Ala Ala Ala Gly Gln Gly Gly Ala Arg Lys Phe Asn Met Lys Met Ile
    515                 520                 525

tgaggggtcc ccagggcgga ccctcccctc ccctcccccg cgggccggcc gctcgcgtgt    1742

ccacctatgc atttcccgga ggggaagggg acggctgcac ggcgctcccc aggcagaact    1802

tccccttttt ttgtagacgc ccaaccgcag gactgttttt catcagcatg cgtctttttt    1862

gcagtttctc aagcgttttc taaagatttc aacccgtctt cctgtccctg ctatggtggc    1922

tgagctgggg ggtgggggtg cgggctgcaa ggatgggggg atgggctccc gtctaccaac    1982

ccggggtggc tgggggcgct cagagacccc aggatcctca gggcccgcaa ttcacaccca    2042

ggaggcacaa gactgctgcc cccaagccct gggcttcc                            2080

<210>  12

<211>  557

<212>  PRT

<213>  Homo sapiens



<220>

<221>  misc_feature

<222>  (-5)..(-5)

<223>  The 'Xaa' at location -5 stands for Tyr, Cys, Ser, or Phe.

<220>

<221>  misc_feature

<222>  (82)..(82)

<223>  wherein n is A, G, C, or T

<400>  12; LP243(a)

Met Val Ser Thr Ala Met Gly Cys Ile Thr Phe Leu Gly Val Val Leu
-25 -20 -15

Leu Leu Leu Ser Cys Cys Cys Xaa Val Trp Ser Arg Asn Arg Ile Arg
-10 -5 -1 1

Cys Leu Asn Pro Gly Asp Leu Ala Ala Leu Pro Ala Leu Glu Glu Leu
5 10 15 20

Asp Leu Ser Glu Asn Ala Ile Ala His Val Glu Pro Gly Ala Phe Ala
25 30 35

Asn Leu Pro Arg Leu Arg Val Leu Arg Leu Arg Gly Asn Gln Leu Lys
40 45 50

Leu Ile Pro Pro Gly Val Phe Thr Arg Leu Asp Asn Leu Thr Leu Leu
55 60 65

Asp Leu Ser Glu Asn Lys Leu Val Ile Leu Leu Asp Tyr Thr Phe Gln
70 75 80

Asp Leu His Ser Leu Arg Arg Leu Glu Val Gly Asp Asn Asp Leu Val
85 90 95 100

Phe Val Ser Arg Arg Ala Phe Ala Gly Leu Leu Ala Leu Glu Glu Leu
105 110 115

Thr Leu Glu Arg Cys Asn Leu Thr Ala Leu Ser Gly Glu Ser Leu Gly
120 125 130

His Leu Arg Ser Leu Gly Ala Leu Arg Leu Arg His Leu Ala Ile Ala
135 140 145

Ser Leu Glu Asp Gln Asn Phe Arg Arg Leu Pro Gly Leu Leu His Leu
150 155 160

Glu Ile Asp Asn Trp Pro Leu Leu Glu Glu Val Ala Ala Gly Ser Leu
165 170 175 180

Arg Gly Leu Asn Leu Thr Ser Leu Ser Val Thr His Thr Asn Ile Thr
185 190 195

Ala Val Pro Ala Ala Ala Leu Arg His Gln Ala His Leu Thr Cys Leu
200 205 210

Asn Leu Ser His Asn Pro Ile Ser Thr Val Pro Arg Gly Ser Phe Arg
        215                 220                 225

Asp Leu Val Arg Leu Arg Glu Leu His Leu Ala Gly Ala Leu Leu Ala
    230                 235                 240

Val Val Glu Pro Gln Ala Phe Leu Gly Leu Arg Gln Ile Arg Leu Leu
245                 250                 255                 260

Asn Leu Ser Asn Asn Leu Leu Ser Thr Leu Glu Glu Ser Thr Phe His
                265                 270                 275

Ser Val Asn Thr Leu Glu Thr Leu Arg Val Asp Gly Asn Pro Leu Ala
            280                 285                 290

Cys Asp Cys Arg Leu Leu Trp Ile Val Gln Arg Arg Lys Thr Leu Asn
        295                 300                 305

Phe Asp Gly Arg Leu Pro Ala Cys Ala Thr Pro Ala Glu Val Arg Gly
    310                 315                 320

Asp Ala Leu Arg Asn Leu Pro Asp Ser Val Leu Phe Glu Tyr Phe Val
325                 330                 335                 340

Cys Arg Lys Pro Lys Ile Arg Glu Arg Arg Leu Gln Arg Val Thr Ala
                345                 350                 355

Thr Ala Gly Glu Asp Val Arg Phe Leu Cys Arg Ala Glu Gly Glu Pro
            360                 365                 370

Ala Pro Thr Val Ala Trp Val Thr Pro Gln His Arg Pro Val Thr Ala
        375                 380                 385

Thr Ser Ala Gly Arg Ala Arg Val Leu Pro Gly Gly Thr Leu Glu Ile
    390                 395                 400

Gln Asp Ala Arg Pro Gln Asp Ser Gly Thr Tyr Thr Cys Val Ala Ser
405                 410                 415                 420

Asn Ala Gly Gly Asn Asp Thr Tyr Phe Ala Thr Leu Thr Val Arg Pro
                425                 430                 435

Glu Pro Ala Ala Asn Arg Thr Pro Gly Glu Ala His Asn Glu Thr Leu
            440                 445                 450

Ala Ala Leu Arg Ala Pro Leu Asp Leu Thr Thr Ile Leu Val Ser Thr
        455                 460                 465

Ala Met Gly Cys Ile Thr Phe Leu Gly Val Val Leu Phe Cys Phe Val
    470                 475                 480

Leu Leu Phe Val Trp Ser Arg Gly Arg Gly Gln His Lys Asn Asn Phe
485                 490                 495                 500

Ser Val Glu Tyr Ser Phe Arg Lys Val Asp Gly Pro Ala Ala Ala Ala
                505                 510                 515

Gly Gln Gly Gly Ala Arg Lys Phe Asn Met Lys Met Ile
            520                 525

<210>  13

<211>  2222

<212>  DNA

<213>  Homo sapiens


<220>

<221>  CDS

<222>  (49)..(1824)

<223>


<220>

<221>  mat_peptide

<222>  (121)..()

<223>


<400>  13; LP243(b)
aggtgcgcag gaggatggtg gcgcggccct aggcccacgc tccgcacc atg acc tgc       57
                                                     Met Thr Cys

tgg ctg tgc gtc ctg agc ctg ccc ctg ctc ctg ctg ccc gcg gcg ccg 105
Trp Leu Cys Val Leu Ser Leu Pro Leu Leu Leu Leu Pro Ala Ala Pro
-20 -15 -10

ccc ccg gct gga ggc tgc ccg gcc cgc tgc gag tgc acc gtg cag acc 153
Pro Pro Ala Gly Gly Cys Pro Ala Arg Cys Glu Cys Thr Val Gln Thr
-5 -1 1 5 10

cgc gcg gtg gcc tgc acg cgc cgc cgc ctg acc gcc gtg ccc gac ggc 201
Arg Ala Val Ala Cys Thr Arg Arg Arg Leu Thr Ala Val Pro Asp Gly
15 20 25

atc ccg gcc gag acc cgc ctg ctg gag ctc agc cgc aac cgc atc cgc 249
Ile Pro Ala Glu Thr Arg Leu Leu Glu Leu Ser Arg Asn Arg Ile Arg
30 35 40

tgc ctg aac ccg ggc gac ctg gcc gcg ctg ccc gcg ctg gag gag ctg 297
Cys Leu Asn Pro Gly Asp Leu Ala Ala Leu Pro Ala Leu Glu Glu Leu
45 50 55

gac ctg agc gag aac gcc atc gcg cac gtg gag ccc ggc gcc ttc gcc 345
Asp Leu Ser Glu Asn Ala Ile Ala His Val Glu Pro Gly Ala Phe Ala
60 65 70 75

aac ctg ccg cgc ctg cgc gtc ctg cgt ctc cgt ggc aac cag ctg aag 393
Asn Leu Pro Arg Leu Arg Val Leu Arg Leu Arg Gly Asn Gln Leu Lys
80 85 90

ctc atc ccg ccc ggg gtc ttc acg cgc ctg gac aac ctc acg ctg ctg 441
Leu Ile Pro Pro Gly Val Phe Thr Arg Leu Asp Asn Leu Thr Leu Leu
95 100 105

gac ctg agc gag aac aag ctg gta atc ctg ctg gac tac act ttc cag 489
Asp Leu Ser Glu Asn Lys Leu Val Ile Leu Leu Asp Tyr Thr Phe Gln
110 115 120

gac ctg cac agc ctg cgc cgg ctg gaa gtg ggc gac aac gac ctg gta 537
Asp Leu His Ser Leu Arg Arg Leu Glu Val Gly Asp Asn Asp Leu Val
125 130 135

ttc gtc tcg cgc cgc gcc ttc gcg ggg ctg ctg gcc ctg gag gag ctg 585
Phe Val Ser Arg Arg Ala Phe Ala Gly Leu Leu Ala Leu Glu Glu Leu
140 145 150 155

acc ctg gag cgc tgc aac ctc acg gct ctg tcc ggg gag tcg ctg ggc 633
Thr Leu Glu Arg Cys Asn Leu Thr Ala Leu Ser Gly Glu Ser Leu Gly
160 165 170

cat ctg cgc agc ctg ggc gcc ctg cgg ctg cgc cac ctg gcc atc gcc 681
His Leu Arg Ser Leu Gly Ala Leu Arg Leu Arg His Leu Ala Ile Ala
175 180 185

tcc ctg gag gac cag aac ttc cgc agg ctg ccc ggg ctg ctg cac ctg 729
Ser Leu Glu Asp Gln Asn Phe Arg Arg Leu Pro Gly Leu Leu His Leu
190 195 200

gag att gac aac tgg ccg ctg ctg gag gag gtg gcg gcg ggc agc ctg 777

Glu Ile Asp Asn Trp Pro Leu Leu Glu Glu Val Ala Ala Gly Ser Leu
    205                 210                 215

cgg ggc ctg aac ctg acc tcg ctg tcg gtc acc cac acc aac atc acc     825
Arg Gly Leu Asn Leu Thr Ser Leu Ser Val Thr His Thr Asn Ile Thr
220                 225                 230                 235

gcc gtg ccg gcc gcc gcg ctg cgg cac cag gcg cac ctc acc tgc ctc     873
Ala Val Pro Ala Ala Ala Leu Arg His Gln Ala His Leu Thr Cys Leu
                240                 245                 250

aat ctg tcg cac aac ccc atc agc acg gtg ccg cgg ggg tcg ttc cgg     921
Asn Leu Ser His Asn Pro Ile Ser Thr Val Pro Arg Gly Ser Phe Arg
            255                 260                 265

gac ctg gtc cgc ctg cgc gag ctg cac ctg gcc ggg gcc ctg ctg gct     969
Asp Leu Val Arg Leu Arg Glu Leu His Leu Ala Gly Ala Leu Leu Ala
        270                 275                 280

gtg gtg gag ccg cag gcc ttc ctg ggc ctg cgc cag atc cgc ctg ctc     1017
Val Val Glu Pro Gln Ala Phe Leu Gly Leu Arg Gln Ile Arg Leu Leu
    285                 290                 295

aac ctc tcc aac aac ctg ctc tcc acg ttg gag gag agc acc ttc cac     1065
Asn Leu Ser Asn Asn Leu Leu Ser Thr Leu Glu Glu Ser Thr Phe His
300                 305                 310                 315

tcg gtg aac acg cta gag acg ctg cgc gtg gac ggg aac ccg ctg gcc     1113
Ser Val Asn Thr Leu Glu Thr Leu Arg Val Asp Gly Asn Pro Leu Ala
                320                 325                 330

tgc gac tgt cgc ctg ctg tgg atc gtg cag cgt cgc aag acc ctc aac     1161
Cys Asp Cys Arg Leu Leu Trp Ile Val Gln Arg Arg Lys Thr Leu Asn
            335                 340                 345

ttc gac ggg cgg ctg ccg gcc tgc gcc acc ccg gcc gag gtg cgc ggc     1209
Phe Asp Gly Arg Leu Pro Ala Cys Ala Thr Pro Ala Glu Val Arg Gly
        350                 355                 360

gac gcg ctg cga aac ctg ccg gac tcc gtg ctg ttc gag tac ttc gtg     1257
Asp Ala Leu Arg Asn Leu Pro Asp Ser Val Leu Phe Glu Tyr Phe Val
    365                 370                 375

tgc cgc aaa ccc aag atc cgg gag cgg cgg ctg cag cgc gtc acg gcc     1305
Cys Arg Lys Pro Lys Ile Arg Glu Arg Arg Leu Gln Arg Val Thr Ala
380                 385                 390                 395

acc gcg ggc gaa gac gtc cgc ttc ctc tgc cgc gcc gag ggc gag ccg     1353
Thr Ala Gly Glu Asp Val Arg Phe Leu Cys Arg Ala Glu Gly Glu Pro
                400                 405                 410

gcg ccc acc gtg gcc tgg gtg acc ccc cag cac cgg ccg gtg acg gcc     1401
Ala Pro Thr Val Ala Trp Val Thr Pro Gln His Arg Pro Val Thr Ala
            415                 420                 425

acc agc gcg ggc cgg gcg cgc gtg ctc ccc ggg ggg acg ctg gag atc     1449
Thr Ser Ala Gly Arg Ala Arg Val Leu Pro Gly Gly Thr Leu Glu Ile
        430                 435                 440

cag gac gcg cgg ccg cag gac agc ggc acc tac acg tgc gtg gcc agc      1497
Gln Asp Ala Arg Pro Gln Asp Ser Gly Thr Tyr Thr Cys Val Ala Ser
    445                 450                 455

aac gcg ggc ggc aac gac acc tac ttc gcc acg ctg acc gtg cgc ccc      1545
Asn Ala Gly Gly Asn Asp Thr Tyr Phe Ala Thr Leu Thr Val Arg Pro
460                 465                 470                 475

gag ccg gcc gcc aac cgg acc ccg ggc gag gcc cac aac gag acg ctg      1593
Glu Pro Ala Ala Asn Arg Thr Pro Gly Glu Ala His Asn Glu Thr Leu
                480                 485                 490

gcg gcc ctg cgc gcg ccg ctc gac ctc acc acc atc ctg gtg tcc acc      1641
Ala Ala Leu Arg Ala Pro Leu Asp Leu Thr Thr Ile Leu Val Ser Thr
            495                 500                 505

gcc atg ggc tgc atc acc ttc ctg ggc gtg gtc ctc ttc tgc ttc gtg      1689
Ala Met Gly Cys Ile Thr Phe Leu Gly Val Val Leu Phe Cys Phe Val
        510                 515                 520

ctg ctg ttc gtg tgg agc cgc ggc cgc ggg cag cac aaa aac aac ttc      1737
Leu Leu Phe Val Trp Ser Arg Gly Arg Gly Gln His Lys Asn Asn Phe
    525                 530                 535

tcg gtg gag tac tcc ttc cgc aag gtg gat ggg ccg gcc gcc gcg gcg      1785
Ser Val Glu Tyr Ser Phe Arg Lys Val Asp Gly Pro Ala Ala Ala Ala
540                 545                 550                 555

ggc cag gga ggc gcg cgc aag ttc aac atg aag atg atc tgaggggtcc      1834
Gly Gln Gly Gly Ala Arg Lys Phe Asn Met Lys Met Ile
                560                 565

ccagggcgga ccctcccctc ccctcccccg cgggccggcc gctcgcgtgt ccacctatgc   1894

atttcccgga ggggaagggg acggctgcac ggcgctcccc aggcagaact tccccttttt   1954

ttgtagacgc ccaaccgcag gactgttttt catcagcatg cgtctttttt gcagtttctc   2014

aagcgttttc taaagatttc aacccgtctt cctgtccctg ctatggtggc tgagctgggg   2074

ggtgggggtg cgggctgcaa ggatgggggg atgggctccc gtctaccaac ccggggtggc   2134

tgggggcgct cagagacccc aggatcctca gggcccgcaa ttcacaccca ggaggcacaa   2194

gactgctgcc cccaagccct gggcttcc                                      2222

<210>  14

<211>  592

<212>  PRT

<213>  Homo sapiens

<400>  14; LP243(b)

Met Thr Cys Trp Leu Cys Val Leu Ser Leu Pro Leu Leu Leu Leu Pro
                -20                 -15                 -10

Ala Ala Pro Pro Pro Ala Gly Gly Cys Pro Ala Arg Cys Glu Cys Thr
            -5              -1  1               5

Val Gln Thr Arg Ala Val Ala Cys Thr Arg Arg Arg Leu Thr Ala Val
    10                  15                  20

Pro Asp Gly Ile Pro Ala Glu Thr Arg Leu Leu Glu Leu Ser Arg Asn
25                  30                  35                  40

Arg Ile Arg Cys Leu Asn Pro Gly Asp Leu Ala Ala Leu Pro Ala Leu
                45                  50                  55

Glu Glu Leu Asp Leu Ser Glu Asn Ala Ile Ala His Val Glu Pro Gly
            60                  65                  70

Ala Phe Ala Asn Leu Pro Arg Leu Arg Val Leu Arg Leu Arg Gly Asn
        75                  80                  85

Gln Leu Lys Leu Ile Pro Pro Gly Val Phe Thr Arg Leu Asp Asn Leu
    90                  95                  100

Thr Leu Leu Asp Leu Ser Glu Asn Lys Leu Val Ile Leu Leu Asp Tyr
105                 110                 115                 120

Thr Phe Gln Asp Leu His Ser Leu Arg Arg Leu Glu Val Gly Asp Asn
                125                 130                 135

Asp Leu Val Phe Val Ser Arg Arg Ala Phe Ala Gly Leu Leu Ala Leu
            140                 145                 150

Glu Glu Leu Thr Leu Glu Arg Cys Asn Leu Thr Ala Leu Ser Gly Glu
        155                 160                 165

Ser Leu Gly His Leu Arg Ser Leu Gly Ala Leu Arg Leu Arg His Leu
    170                 175                 180

Ala Ile Ala Ser Leu Glu Asp Gln Asn Phe Arg Arg Leu Pro Gly Leu
185                 190                 195                 200

Leu His Leu Glu Ile Asp Asn Trp Pro Leu Leu Glu Glu Val Ala Ala
                205                 210                 215

Gly Ser Leu Arg Gly Leu Asn Leu Thr Ser Leu Ser Val Thr His Thr
            220                 225                 230

Asn Ile Thr Ala Val Pro Ala Ala Ala Leu Arg His Gln Ala His Leu
        235                 240                 245

Thr Cys Leu Asn Leu Ser His Asn Pro Ile Ser Thr Val Pro Arg Gly
    250                 255                 260

Ser Phe Arg Asp Leu Val Arg Leu Arg Glu Leu His Leu Ala Gly Ala
265                 270                 275                 280

Leu Leu Ala Val Val Glu Pro Gln Ala Phe Leu Gly Leu Arg Gln Ile
                285                 290                 295

Arg Leu Leu Asn Leu Ser Asn Asn Leu Leu Ser Thr Leu Glu Glu Ser
            300                 305                 310

Thr Phe His Ser Val Asn Thr Leu Glu Thr Leu Arg Val Asp Gly Asn
        315                 320                 325

Pro Leu Ala Cys Asp Cys Arg Leu Leu Trp Ile Val Gln Arg Arg Lys
    330                 335                 340

Thr Leu Asn Phe Asp Gly Arg Leu Pro Ala Cys Ala Thr Pro Ala Glu
345                 350                 355                 360

Val Arg Gly Asp Ala Leu Arg Asn Leu Pro Asp Ser Val Leu Phe Glu
                365                 370                 375

Tyr Phe Val Cys Arg Lys Pro Lys Ile Arg Glu Arg Arg Leu Gln Arg
            380                 385                 390

Val Thr Ala Thr Ala Gly Glu Asp Val Arg Phe Leu Cys Arg Ala Glu
        395                 400                 405

Gly Glu Pro Ala Pro Thr Val Ala Trp Val Thr Pro Gln His Arg Pro
    410                 415                 420

Val Thr Ala Thr Ser Ala Gly Arg Ala Arg Val Leu Pro Gly Gly Thr
425                 430                 435                 440

Leu Glu Ile Gln Asp Ala Arg Pro Gln Asp Ser Gly Thr Tyr Thr Cys
                445                 450                 455

Val Ala Ser Asn Ala Gly Gly Asn Asp Thr Tyr Phe Ala Thr Leu Thr
            460                 465                 470

Val Arg Pro Glu Pro Ala Ala Asn Arg Thr Pro Gly Glu Ala His Asn
        475                 480                 485

Glu Thr Leu Ala Ala Leu Arg Ala Pro Leu Asp Leu Thr Thr Ile Leu
    490                 495                 500

Val Ser Thr Ala Met Gly Cys Ile Thr Phe Leu Gly Val Val Leu Phe
505                 510                 515                 520

Cys Phe Val Leu Leu Phe Val Trp Ser Arg Gly Arg Gly Gln His Lys
                525                 530                 535

Asn Asn Phe Ser Val Glu Tyr Ser Phe Arg Lys Val Asp Gly Pro Ala
            540                 545                 550

Ala Ala Ala Gly Gln Gly Gly Ala Arg Lys Phe Asn Met Lys Met Ile
        555                 560                 565

<210>  15

<211>  4392

<212>  DNA

<213>  Homo sapiens


<220>

<221>  CDS

<222>  (1)..(1662)

<223>


<220>

<221>  mat_peptide

<222>  (82)..()

<223>

<400>  15; LP253
atg gcc ccg tgg ctg cag ctc tgc tcc gtc ttc ttt acg gtc aac gcc      48
Met Ala Pro Trp Leu Gln Leu Cys Ser Val Phe Phe Thr Val Asn Ala
        -25                 -20                 -15

tgc ctc aac ggc tcg cag ctg gct gtg gcc gct ggc ggg tcc ggc cgc      96
Cys Leu Asn Gly Ser Gln Leu Ala Val Ala Ala Gly Gly Ser Gly Arg
    -10                 -5                  -1  1               5

gcg cgg ggc gcc gac acc tgt ggc tgg agg gga gtg ggg cca gcc agc      144
Ala Arg Gly Ala Asp Thr Cys Gly Trp Arg Gly Val Gly Pro Ala Ser
                10                  15                  20

aga aac agt ggg ctg tac aac atc acc ttc aaa tat gac aat tgt acc      192
Arg Asn Ser Gly Leu Tyr Asn Ile Thr Phe Lys Tyr Asp Asn Cys Thr
            25                  30                  35

acc tac ttg aat cca gtg ggg aag cat gtg att gct gac gcc cag aat      240
Thr Tyr Leu Asn Pro Val Gly Lys His Val Ile Ala Asp Ala Gln Asn
        40                  45                  50

atc acc atc agc cag tat gct tgc cat gac caa gtg gca gtc acc att      288
Ile Thr Ile Ser Gln Tyr Ala Cys His Asp Gln Val Ala Val Thr Ile
    55                  60                  65

ctt tgg tcc cca ggg gcc ctc ggc atc gaa ttc ctg aaa gga ttt cgg      336
Leu Trp Ser Pro Gly Ala Leu Gly Ile Glu Phe Leu Lys Gly Phe Arg
70                  75                  80                  85

gta ata ctg gag gag ctg aag tcg gag gga aga cag tgc caa caa ctg      384
Val Ile Leu Glu Glu Leu Lys Ser Glu Gly Arg Gln Cys Gln Gln Leu
                90                  95                  100

att cta aag gat ccg aag cag ctc aac agt agc ttc aaa aga act gga      432
Ile Leu Lys Asp Pro Lys Gln Leu Asn Ser Ser Phe Lys Arg Thr Gly
            105                 110                 115

atg gaa tct caa cct ttc ctg aat atg aaa ttt gaa acg gat tat ttc      480
Met Glu Ser Gln Pro Phe Leu Asn Met Lys Phe Glu Thr Asp Tyr Phe
        120                 125                 130

gta aag gtt gtc cct ttt cct tcc att aaa aac gaa agc aat tac cac      528
Val Lys Val Val Pro Phe Pro Ser Ile Lys Asn Glu Ser Asn Tyr His
    135                 140                 145

cct ttc ttc ttt aga acc cga gcc tgt gac ctg ttg tta cag ccg gac      576
Pro Phe Phe Phe Arg Thr Arg Ala Cys Asp Leu Leu Leu Gln Pro Asp
150                 155                 160                 165

aat cta gct tgt aaa ccc ttc tgg aag cct cgg aac ctg aac atc agc      624
Asn Leu Ala Cys Lys Pro Phe Trp Lys Pro Arg Asn Leu Asn Ile Ser
                170                 175                 180

cag cat ggc tcg gac atg cag gtg tcc ttc gac cat gca ccg cac aac 672
Gln His Gly Ser Asp Met Gln Val Ser Phe Asp His Ala Pro His Asn
185 190 195

ttc ggc ttc cgt ttc ttc tat ctt cac tac aag ctc aag cac gaa gga 720
Phe Gly Phe Arg Phe Phe Tyr Leu His Tyr Lys Leu Lys His Glu Gly
200 205 210

cct ttc aag cga aag acc tgt aag cag gag caa act aca gag acg acc 768
Pro Phe Lys Arg Lys Thr Cys Lys Gln Glu Gln Thr Thr Glu Thr Thr
215 220 225

agc tgc ctc ctt caa aat gtt tct cca ggg gat tat ata att gag ctg 816
Ser Cys Leu Leu Gln Asn Val Ser Pro Gly Asp Tyr Ile Ile Glu Leu
230 235 240 245

gtg gat gac act aac aca aca aga aaa gtg atg cat tat gcc tta aag 864
Val Asp Asp Thr Asn Thr Thr Arg Lys Val Met His Tyr Ala Leu Lys
250 255 260

cca gtg cac tcc ccg tgg gcc ggg ccc atc aga gcc gtg gcc atc aca 912
Pro Val His Ser Pro Trp Ala Gly Pro Ile Arg Ala Val Ala Ile Thr
265 270 275

gtg cca ctg gta gtc ata tcg gca ttc gcg acg ctc ttc act gtg atg 960
Val Pro Leu Val Val Ile Ser Ala Phe Ala Thr Leu Phe Thr Val Met
280 285 290

tgc cgc aag aag caa caa gaa aat ata tat tca cat tta gat gaa gag 1008
Cys Arg Lys Lys Gln Gln Glu Asn Ile Tyr Ser His Leu Asp Glu Glu
295 300 305

agc tct gag tct tcc aca tac act gca gca ctc cca aga gag agg ctc 1056
Ser Ser Glu Ser Ser Thr Tyr Thr Ala Ala Leu Pro Arg Glu Arg Leu
310 315 320 325

cgg ccg cgg ccg aag gtc ttt ctc tgc tat tcc agt aaa gat ggc cag 1104
Arg Pro Arg Pro Lys Val Phe Leu Cys Tyr Ser Ser Lys Asp Gly Gln
330 335 340

aat cac atg aat gtc gtc cag tgt ttc gcc tac ttc ctc cag gac ttc 1152
Asn His Met Asn Val Val Gln Cys Phe Ala Tyr Phe Leu Gln Asp Phe
345 350 355

tgt ggc tgt gag gtg gct ctg gac ctg tgg gaa gac ttc agc ctc tgt 1200
Cys Gly Cys Glu Val Ala Leu Asp Leu Trp Glu Asp Phe Ser Leu Cys
360 365 370

aga gaa ggg cag aga gaa tgg gtc atc cag aag atc cac gag tcc cag 1248
Arg Glu Gly Gln Arg Glu Trp Val Ile Gln Lys Ile His Glu Ser Gln
375 380 385

ttc atc att gtg gtt tgt tcc aaa ggt atg aag tac ttt gtg gac aag 1296
Phe Ile Ile Val Val Cys Ser Lys Gly Met Lys Tyr Phe Val Asp Lys
390 395 400 405

aag aac tac aaa cac aaa gga ggt ggc cga ggc tcg ggg aaa gga gag 1344

Lys Asn Tyr Lys His Lys Gly Gly Gly Arg Gly Ser Gly Lys Gly Glu
                410                 415                 420

ctc ttc ctg gtg gcg gtg tca gcc att gcc gaa aag ctc cgc cag gcc      1392
Leu Phe Leu Val Ala Val Ser Ala Ile Ala Glu Lys Leu Arg Gln Ala
            425                 430                 435

aag cag agt tcg tcc gcg gcg ctc agc aag ttt atc gcc gtc tac ttt      1440
Lys Gln Ser Ser Ser Ala Ala Leu Ser Lys Phe Ile Ala Val Tyr Phe
        440                 445                 450

gat tat tcc tgc gag gga gac gtc ccc ggt atc cta gac ctg agt acc      1488
Asp Tyr Ser Cys Glu Gly Asp Val Pro Gly Ile Leu Asp Leu Ser Thr
    455                 460                 465

aag tac aga ctc atg gac aat ctt tcc tca gct ctg ttc cca ctc tgc      1536
Lys Tyr Arg Leu Met Asp Asn Leu Ser Ser Ala Leu Phe Pro Leu Cys
470                 475                 480                 485

act ccc gag acc acg gcc tcc agg agc cgg ggc atg cac acg cga cag      1584
Thr Pro Glu Thr Thr Ala Ser Arg Ser Arg Gly Met His Thr Arg Gln
                490                 495                 500

ggc agg gag aag gaa cta ctt ccg gag caa gtc agg ccg gtc cct ata      1632
Gly Arg Glu Lys Glu Leu Leu Pro Glu Gln Val Arg Pro Val Pro Ile
            505                 510                 515

cgt cgc cat ttg caa cat gca cca gtt tat tgacgaggag cccgactggt      1682
Arg Arg His Leu Gln His Ala Pro Val Tyr
        520                 525

tcgaaaagca gttcgttccc ttccatcctc ctccactgcg ctaccgggag ccagtcttgg      1742

agaaatttga ttcgggcttg gttttaaatg atgtcatgtg caaaccaggg cctgagagtg      1802

acttctgcct aaaggtagag gcggctgttc ttggggcaac cggaccagcc gactcccagc      1862

acgagagtca gcatgggggc ctggaccaag acggggaggc ccggcctgcc cttgacggta      1922

gcgccgccct gcaacccctg ctgcacacgg tgaaagccgg cagcccctcg gacatgccgc      1982

gggactcagg catctatgac tcgtctgtgc cctcatccga gctgtctctg ccactgatgg      2042

aaggactctc gacggaccag acagaaacgt cttccctgac ggagagcgtg tcctcctctt      2102

caggcctggg tgaggaggaa cctcctgccc ttccttccaa gctcctctct tctgggtcat      2162

gcaaagcaga tcttggttgc cgcagctaca ctgatgaact ccacgcggtc gcccctttgt      2222

aacaaaacga aagagtctaa gcattgccac tttagctgct gcctccctct gattccccag      2282

ctcatctccc tggttgcatg gcccacttgg agctgaggtc tcatacaagg atatttggag      2342

tgaaatgctg gccagtactt gttctccctt gccccaaccc tttaccggat atcttgacaa      2402

actctccaat tttctaaaat gatatggagc tctgaaaggc atgtccataa ggtctgacaa      2462

cagcttgcca aatttggtta gtccttggat cagagcctgt tgtgggaggt agggaggaaa      2522

tatgtaaaga aaaacaggaa gatacctgca ctaatcattc agacttcatt gagctctgca 2582
aactttgcct gtttgctatt ggctaccttg atttgaaatg ctttgtgaaa aaaggcactt 2642
ttaacatcat agccacagaa atcaagtgcc agtctatctg gaatccatgt tgtattgcag 2702
ataatgttct catttatttt tgatgtagaa tttacattgc catgggtgtt aaataagctt 2762
tgagtcaaaa gtcaagaaag tgactgaata tacagtcacc ttttatgaaa tgagtctctg 2822
tgttactggg tggcatgact gattgaggtg aagctcacgg ggccaggctg accgtcttga 2882
ccgttccact tgagataggt tggtcatcgt gcagaaggcc ccaggacctc agcacacaca 2942
gcctcctctt ggtctgagta ggcatcatgt gggggccaga tctgcctgct gtttccatgg 3002
gttacattta ctgtgctgta tctcagatgt tggtgtctgg aagtttattc ttaagagact 3062
gctacccagc tggtctgtat tattggaagt tgcagttcgt gctttggttg gccttctggt 3122
ctaaagctgt gtcctgaata ttagggatca caattcactg aaatacagca gtgtgtggag 3182
gtgatggcca gttaatctgc tgaactggtt ttgactaatg acaaacctct ttttaagatg 3242
gtagaatgga ggtgatagtc acaaaagtaa atgttccatt tttatgaatg actttctaca 3302
gagtttctat ttctaaagaa aaaacaattg ttcacatccc atctgatgat tagcatgtgt 3362
gtaatgaatg ctgtcttggt ctcccctgtg gaaacccttc tccctgtgcc ttagagcagg 3422
tgtgtacatc tctcactacc tttctcatgg gtgctgttag attttggcac ccgttttctc 3482
agcattcagc ccagggaatg tggttttcac ttcttcgtca gataagacca acatgaaggg 3542
gtatgttgag aaacatcctg aggcaaggtg ggaggtggga tggggcagga ctttcccttc 3602
caagcacatg catggcaggt ggggaaaggg gggcttgcac ccctgctgga aagaaaaggt 3662
ttgtgtatat ttctgatgca aatgtcatac tcactgctct gtaaaggcag ctggcagctt 3722
tttgggaaaa gaacgtgctc gtctgttctc tggcatcaag tttcttgcag ctgctctgag 3782
ggagagacag tgagctgcaa gactgcctcc ccataacaac aggcaactca gagaagagtc 3842
attttatgtt gttcctatgg aatctggaat gagtgcagag ctcctaccca cacatgactg 3902
ccccgccatt tcatcctagg cattctgtga aggagattgg ttagtccaaa cttgctaaca 3962
tacgaaaatt cacttggaac atgatgagag atttcttatt gaggccaaga gatgtttcct 4022
gtcccagagg aaccattagg agtcgctttt agggtattca gctttgttca tgaaataagg 4082
catctctgag aaagtggccc cagggagaga atggaggact gggaggagaa gcattaactg 4142
agctccaagg gtgtgtgggc agagagcttg ctatgtgaac tcactcctta agaaaatgga 4202
agagaaaaag agagtgctag ttaaaaaatc gggatgtttt agtttggatt tagggttttg 4262

atacttatgt tgaaatacta atgtttctga tcaataaaat caaactctta atataccgag    4322

taatgaaacc atagtgtgat tgcctcagaa taaattgaga agtccaaaaa aaaaaaaaaa    4382

aaaaaaaaaa                                                            4392

<210>  16

<211>  554

<212>  PRT

<213>  Homo sapiens

<400>  16; LP253

Met Ala Pro Trp Leu Gln Leu Cys Ser Val Phe Phe Thr Val Asn Ala
        -25                 -20                 -15

Cys Leu Asn Gly Ser Gln Leu Ala Val Ala Ala Gly Gly Ser Gly Arg
    -10                 -5              -1  1               5

Ala Arg Gly Ala Asp Thr Cys Gly Trp Arg Gly Val Gly Pro Ala Ser
                10                  15                  20

Arg Asn Ser Gly Leu Tyr Asn Ile Thr Phe Lys Tyr Asp Asn Cys Thr
            25                  30                  35

Thr Tyr Leu Asn Pro Val Gly Lys His Val Ile Ala Asp Ala Gln Asn
        40                  45                  50

Ile Thr Ile Ser Gln Tyr Ala Cys His Asp Gln Val Ala Val Thr Ile
    55                  60                  65

Leu Trp Ser Pro Gly Ala Leu Gly Ile Glu Phe Leu Lys Gly Phe Arg
70                  75                  80                  85

Val Ile Leu Glu Glu Leu Lys Ser Glu Gly Arg Gln Cys Gln Gln Leu
                90                  95                  100

Ile Leu Lys Asp Pro Lys Gln Leu Asn Ser Ser Phe Lys Arg Thr Gly
            105                 110                 115

Met Glu Ser Gln Pro Phe Leu Asn Met Lys Phe Glu Thr Asp Tyr Phe
        120                 125                 130

Val Lys Val Val Pro Phe Pro Ser Ile Lys Asn Glu Ser Asn Tyr His
    135                 140                 145

Pro Phe Phe Phe Arg Thr Arg Ala Cys Asp Leu Leu Leu Gln Pro Asp
150                 155                 160                 165

Asn Leu Ala Cys Lys Pro Phe Trp Lys Pro Arg Asn Leu Asn Ile Ser
                170                 175                 180

Gln His Gly Ser Asp Met Gln Val Ser Phe Asp His Ala Pro His Asn
            185                 190                 195

Phe Gly Phe Arg Phe Phe Tyr Leu His Tyr Lys Leu Lys His Glu Gly
        200                 205                 210

Pro Phe Lys Arg Lys Thr Cys Lys Gln Glu Gln Thr Thr Glu Thr Thr
    215                 220                 225

Ser Cys Leu Leu Gln Asn Val Ser Pro Gly Asp Tyr Ile Ile Glu Leu
230                 235                 240                 245

Val Asp Asp Thr Asn Thr Thr Arg Lys Val Met His Tyr Ala Leu Lys
                250                 255                 260

Pro Val His Ser Pro Trp Ala Gly Pro Ile Arg Ala Val Ala Ile Thr
            265                 270                 275

Val Pro Leu Val Val Ile Ser Ala Phe Ala Thr Leu Phe Thr Val Met
        280                 285                 290

Cys Arg Lys Lys Gln Gln Glu Asn Ile Tyr Ser His Leu Asp Glu Glu
    295                 300                 305

Ser Ser Glu Ser Ser Thr Tyr Thr Ala Ala Leu Pro Arg Glu Arg Leu
310                 315                 320                 325

Arg Pro Arg Pro Lys Val Phe Leu Cys Tyr Ser Ser Lys Asp Gly Gln
                330                 335                 340

Asn His Met Asn Val Val Gln Cys Phe Ala Tyr Phe Leu Gln Asp Phe
            345                 350                 355

Cys Gly Cys Glu Val Ala Leu Asp Leu Trp Glu Asp Phe Ser Leu Cys
        360                 365                 370

Arg Glu Gly Gln Arg Glu Trp Val Ile Gln Lys Ile His Glu Ser Gln
    375                 380                 385

Phe Ile Ile Val Val Cys Ser Lys Gly Met Lys Tyr Phe Val Asp Lys
390                 395                 400                 405

Lys Asn Tyr Lys His Lys Gly Gly Gly Arg Gly Ser Gly Lys Gly Glu
                410                 415                 420

Leu Phe Leu Val Ala Val Ser Ala Ile Ala Glu Lys Leu Arg Gln Ala
            425                 430                 435

Lys Gln Ser Ser Ser Ala Ala Leu Ser Lys Phe Ile Ala Val Tyr Phe
        440                 445                 450

Asp Tyr Ser Cys Glu Gly Asp Val Pro Gly Ile Leu Asp Leu Ser Thr
    455                 460                 465

Lys Tyr Arg Leu Met Asp Asn Leu Ser Ser Ala Leu Phe Pro Leu Cys
470                 475                 480                 485

Thr Pro Glu Thr Thr Ala Ser Arg Ser Arg Gly Met His Thr Arg Gln
                490                 495                 500

Gly Arg Glu Lys Glu Leu Leu Pro Glu Gln Val Arg Pro Val Pro Ile
            505                 510                 515

Arg Arg His Leu Gln His Ala Pro Val Tyr
        520                 525

<210>  17

<211>  1010

<212>  DNA

<213>  Homo sapiens


<220>

<221>  CDS

<222>  (162)..(923)

<223>

<220>

<221>  mat_peptide

<222>  (279)..()

<223>

<400>  17; LP218
gccggccgcg gctggcccgg gatcagggag ccgatgtgga atttcctgcc cggctcggcg      60

cccctcctgc cgccccccgc ccggcctcgc actccgcttc cggccgctct cgctgcggcc      120

gcacccgcgc ccgtgcccgc cccgcgcctg ccccgcgcct c atg gag ggc gca ggg      176
                                              Met Glu Gly Ala Gly
                                                              -35

ccc cgg ggg gcc ggg ccg gcg cgg cgc cgg gga gcc ggg ggg ccg ccg      224
Pro Arg Gly Ala Gly Pro Ala Arg Arg Arg Gly Ala Gly Gly Pro Pro
                -30                 -25                 -20

tca ccg ctg ctg ccg tcg ctg ctg ctg ctg ctg ctg ctc tgg atg ctg      272
Ser Pro Leu Leu Pro Ser Leu Leu Leu Leu Leu Leu Leu Trp Met Leu
            -15                 -10                 -5

ccg gac acc gtg gcg cct cag gaa ctg aac cct cgc ggc cgc aac gtg      320
Pro Asp Thr Val Ala Pro Gln Glu Leu Asn Pro Arg Gly Arg Asn Val
    -1  1               5                   10

tgc cgt gct ccc ggc tcc cag gtg ccc acg tgc tgc gct ggc tgg agg      368
Cys Arg Ala Pro Gly Ser Gln Val Pro Thr Cys Cys Ala Gly Trp Arg
15                  20                  25                  30

cag caa ggg gac gag tgt ggg att gcg gtg tgc gaa ggc aac tcc acg      416
Gln Gln Gly Asp Glu Cys Gly Ile Ala Val Cys Glu Gly Asn Ser Thr
                35                  40                  45

tgc tca gag aac gag gtg tgc gtg agg cct ggc gag tgc cgc tgc cgc      464
Cys Ser Glu Asn Glu Val Cys Val Arg Pro Gly Glu Cys Arg Cys Arg
            50                  55                  60

cac ggc tac ttc ggt gcc aac tgc gac acc aag tgc ccg cgc cag ttc      512
His Gly Tyr Phe Gly Ala Asn Cys Asp Thr Lys Cys Pro Arg Gln Phe
        65                  70                  75

tgg ggc ccc gac tgc aag gag ctg tgt agc tgc cac cca cac ggg cag      560
Trp Gly Pro Asp Cys Lys Glu Leu Cys Ser Cys His Pro His Gly Gln
    80                  85                  90

tgc gag gac gtg aca ggc cag tgt act tgt cac gcg cgg cgc tgg ggc      608

Cys Glu Asp Val Thr Gly Gln Cys Thr Cys His Ala Arg Arg Trp Gly
95                  100                 105                 110

gcg cgc tgc gag cat gcg tgc cag tgc cag cac ggc acg tgc cac ccg      656
Ala Arg Cys Glu His Ala Cys Gln Cys Gln His Gly Thr Cys His Pro
                115                 120                 125

cgg agc ggc gcg tgc cgc tgt gag ccc ggc ccc gcc cct ccg ctc aga      704
Arg Ser Gly Ala Cys Arg Cys Glu Pro Gly Pro Ala Pro Pro Leu Arg
            130                 135                 140

ccc cgc ccc cgc agg gag ctt tcg ctt ggg agg aag aag gcg ccg cac      752
Pro Arg Pro Arg Arg Glu Leu Ser Leu Gly Arg Lys Lys Ala Pro His
        145                 150                 155

cga cta tgc ggg cgc ttc agt cgc atc agc atg aag ctg ccc cgg atc      800
Arg Leu Cys Gly Arg Phe Ser Arg Ile Ser Met Lys Leu Pro Arg Ile
    160                 165                 170

ccg ctc cgg agg cag aaa cta ccc aaa gtc gta ggt aag gat gac agc      848
Pro Leu Arg Arg Gln Lys Leu Pro Lys Val Val Gly Lys Asp Asp Ser
175                 180                 185                 190

gcg ggg gat ctg gga gaa aca cct ctc gac gca gga agc tgc tgg gaa      896
Ala Gly Asp Leu Gly Glu Thr Pro Leu Asp Ala Gly Ser Cys Trp Glu
                195                 200                 205

atg ggt gtg agg ggg tcc aga tct cag tgaggaaatg ggggaccttg            943
Met Gly Val Arg Gly Ser Arg Ser Gln
            210                 215

agtgttgatg tgcgtgggcc ccaggaggtg cgaccagagg gaaggtaaga ggtgtggcag   1003

ctgctga                                                             1010

<210>  18

<211>  254

<212>  PRT

<213>  Homo sapiens

<400>  18; LP218

Met Glu Gly Ala Gly Pro Arg Gly Ala Gly Pro Ala Arg Arg Arg Gly
                -35                 -30                 -25

Ala Gly Gly Pro Pro Ser Pro Leu Leu Pro Ser Leu Leu Leu Leu Leu
            -20                 -15                 -10

Leu Leu Trp Met Leu Pro Asp Thr Val Ala Pro Gln Glu Leu Asn Pro
        -5                  -1  1               5

Arg Gly Arg Asn Val Cys Arg Ala Pro Gly Ser Gln Val Pro Thr Cys
10                  15                  20                  25

Cys Ala Gly Trp Arg Gln Gln Gly Asp Glu Cys Gly Ile Ala Val Cys
                30                  35                  40

Glu Gly Asn Ser Thr Cys Ser Glu Asn Glu Val Cys Val Arg Pro Gly
            45                  50                  55

Glu Cys Arg Cys Arg His Gly Tyr Phe Gly Ala Asn Cys Asp Thr Lys
        60                  65                  70

Cys Pro Arg Gln Phe Trp Gly Pro Asp Cys Lys Glu Leu Cys Ser Cys
    75                  80                  85

His Pro His Gly Gln Cys Glu Asp Val Thr Gly Gln Cys Thr Cys His
90                  95                  100                 105

Ala Arg Arg Trp Gly Ala Arg Cys Glu His Ala Cys Gln Cys Gln His
                110                 115                 120

Gly Thr Cys His Pro Arg Ser Gly Ala Cys Arg Cys Glu Pro Gly Pro
            125                 130                 135

Ala Pro Pro Leu Arg Pro Arg Pro Arg Arg Glu Leu Ser Leu Gly Arg
        140                 145                 150

Lys Lys Ala Pro His Arg Leu Cys Gly Arg Phe Ser Arg Ile Ser Met
    155                 160                 165

Lys Leu Pro Arg Ile Pro Leu Arg Arg Gln Lys Leu Pro Lys Val Val
170                 175                 180                 185

Gly Lys Asp Asp Ser Ala Gly Asp Leu Gly Glu Thr Pro Leu Asp Ala
                190                 195                 200

Gly Ser Cys Trp Glu Met Gly Val Arg Gly Ser Arg Ser Gln
            205                 210                 215

<210>  19

<211>  2210

<212>  DNA

<213>  Homo sapiens

<220>

<221>  CDS

<222>  (39)..(2114)

<223>

<220>

<221>  mat_peptide

<222>  (93)..()

<223>

<400>  19; LP251(a)
gtctcctcgc caggacagca acctctcccc tggccctc atg ggc acc gtc agc tcc      56
                                          Met Gly Thr Val Ser Ser
                                                      -15

agg cgg tcc tgg tgg ccg ctg cca ctg ctg ctg ctg ctg ctg ctg ctc      104
Arg Arg Ser Trp Trp Pro Leu Pro Leu Leu Leu Leu Leu Leu Leu Leu
        -10                 -5                  -1  1

ctg ggt ccc gcg ggc gcc cgt gcg cag gag gac gag gac ggc gac tac      152
Leu Gly Pro Ala Gly Ala Arg Ala Gln Glu Asp Glu Asp Gly Asp Tyr
5                   10                  15                  20

gag gag ctg gtg cta gcc ttg cgt tcc gag gag gac ggc ctg gcc gaa      200
Glu Glu Leu Val Leu Ala Leu Arg Ser Glu Glu Asp Gly Leu Ala Glu
                25                  30                  35

gca ccc gag cac gga acc aca gcc acc ttc cac cgc tgc gcc aag gat      248
Ala Pro Glu His Gly Thr Thr Ala Thr Phe His Arg Cys Ala Lys Asp
            40                  45                  50

ccg tgg agg ttg cct ggc acc tac gtg gtg gtg ctg aag gag gag acc      296
Pro Trp Arg Leu Pro Gly Thr Tyr Val Val Val Leu Lys Glu Glu Thr
        55                  60                  65

cac ctc tcg cag tca gag cgc act gcc cgc cgc ctg cag gcc cag gct      344
His Leu Ser Gln Ser Glu Arg Thr Ala Arg Arg Leu Gln Ala Gln Ala
    70                  75                  80

gcc cgc cgg gga tac ctc acc aag atc ctg cat gtc ttc cat ggc ctt      392
Ala Arg Arg Gly Tyr Leu Thr Lys Ile Leu His Val Phe His Gly Leu
85                  90                  95                  100

ctt cct ggc ttc ctg gtg aag atg agt ggc gac ctg ctg gag ctg gcc 440
Leu Pro Gly Phe Leu Val Lys Met Ser Gly Asp Leu Leu Glu Leu Ala
105 110 115

ttg aag ttg ccc cat gtc gac tac atc gag gag gac tcc tct gtc ttt 488
Leu Lys Leu Pro His Val Asp Tyr Ile Glu Glu Asp Ser Ser Val Phe
120 125 130

gcc cag agc atc ccg tgg aac ctg gag cgg att acc cct cca cgg tac 536
Ala Gln Ser Ile Pro Trp Asn Leu Glu Arg Ile Thr Pro Pro Arg Tyr
135 140 145

cgg gcg gat gaa tac cag ccc ccc gac gga ggc agc ctg gtg gag gtg 584
Arg Ala Asp Glu Tyr Gln Pro Pro Asp Gly Gly Ser Leu Val Glu Val
150 155 160

tat ctc cta gac acc agc ata cag agt gac cac cgg gaa atc gag ggc 632
Tyr Leu Leu Asp Thr Ser Ile Gln Ser Asp His Arg Glu Ile Glu Gly
165 170 175 180

agg gtc atg gtc acc gac ttc gag aat gtg ccc gag gag gac ggg acc 680
Arg Val Met Val Thr Asp Phe Glu Asn Val Pro Glu Glu Asp Gly Thr
185 190 195

cgc ttc cac aga cag gcc agc aag tgt gac agt cat ggc acc cac ctg 728
Arg Phe His Arg Gln Ala Ser Lys Cys Asp Ser His Gly Thr His Leu
200 205 210

gca ggg gtg gtc agc ggc cgg gat gcc ggc gtg gcc aag ggt gcc agc 776
Ala Gly Val Val Ser Gly Arg Asp Ala Gly Val Ala Lys Gly Ala Ser
215 220 225

atg cgc agc ctg cgc gtg ctc aac tgc caa ggg aag ggc acg gtt agc 824
Met Arg Ser Leu Arg Val Leu Asn Cys Gln Gly Lys Gly Thr Val Ser
230 235 240

ggc acc ctc ata ggc ctg gag ttt att cgg aaa agc cag ctg gtc cag 872
Gly Thr Leu Ile Gly Leu Glu Phe Ile Arg Lys Ser Gln Leu Val Gln
245 250 255 260

cct gtg ggg cca ctg gtg gtg ctg ctg ccc ctg gcg ggt ggg tac agc 920
Pro Val Gly Pro Leu Val Val Leu Leu Pro Leu Ala Gly Gly Tyr Ser
265 270 275

cgc gtc ctc aac gcc gcc tgc cag cgc ctg gcg agg gct ggg gtc gtg 968
Arg Val Leu Asn Ala Ala Cys Gln Arg Leu Ala Arg Ala Gly Val Val
280 285 290

ctg gtc acc gct gcc ggc aac ttc cgg gac gat gcc tgc ctc tac tcc 1016
Leu Val Thr Ala Ala Gly Asn Phe Arg Asp Asp Ala Cys Leu Tyr Ser
295 300 305

cca gcc tca gct ccc gag gtc atc aca gtt ggg gcc acc aat gcc cag 1064
Pro Ala Ser Ala Pro Glu Val Ile Thr Val Gly Ala Thr Asn Ala Gln
310 315 320

gac cag ccg gtg acc ctg ggg act ttg ggg acc aac ttt ggc cgc tgt 1112

Asp Gln Pro Val Thr Leu Gly Thr Leu Gly Thr Asn Phe Gly Arg Cys
325                 330                 335                 340

gtg gac ctc ttt gcc cca ggg gag gac atc att ggt gcc tcc agc gac      1160
Val Asp Leu Phe Ala Pro Gly Glu Asp Ile Ile Gly Ala Ser Ser Asp
                345                 350                 355

tgc agc acc tgc ttt gtg tca cag agt ggg aca tca cag gct gct gcc      1208
Cys Ser Thr Cys Phe Val Ser Gln Ser Gly Thr Ser Gln Ala Ala Ala
            360                 365                 370

cac gtg gct ggc att gca gcc atg atg ctg tct gcc gag ccg gag ctc      1256
His Val Ala Gly Ile Ala Ala Met Met Leu Ser Ala Glu Pro Glu Leu
        375                 380                 385

acc ctg gcc gag ttg agg cag aga ctg atc cac ttc tct gcc aaa gat      1304
Thr Leu Ala Glu Leu Arg Gln Arg Leu Ile His Phe Ser Ala Lys Asp
    390                 395                 400

gtc atc aat gag gcc tgg ttc cct gag gac cag cgg gta ctg acc ccc      1352
Val Ile Asn Glu Ala Trp Phe Pro Glu Asp Gln Arg Val Leu Thr Pro
405                 410                 415                 420

aac ctg gtg gcc gcc ctg ccc ccc agc acc cat ggg gca ggt tgg cag      1400
Asn Leu Val Ala Ala Leu Pro Pro Ser Thr His Gly Ala Gly Trp Gln
                425                 430                 435

ctg ttt tgc agg act gtg tgg tca gca cac tcg ggg cct aca cgg atg      1448
Leu Phe Cys Arg Thr Val Trp Ser Ala His Ser Gly Pro Thr Arg Met
            440                 445                 450

gcc aca gcc atc gcc cgc tgc gcc cca gat gag gag ctg ctg agc tgc      1496
Ala Thr Ala Ile Ala Arg Cys Ala Pro Asp Glu Glu Leu Leu Ser Cys
        455                 460                 465

tcc agt ttc tcc agg agt ggg aag cgg cgg ggc gag cgc atg gag gcc      1544
Ser Ser Phe Ser Arg Ser Gly Lys Arg Arg Gly Glu Arg Met Glu Ala
    470                 475                 480

caa ggg ggc aag ctg gtc tgc cgg gcc cac aac gct ttt ggg ggt gag      1592
Gln Gly Gly Lys Leu Val Cys Arg Ala His Asn Ala Phe Gly Gly Glu
485                 490                 495                 500

ggt gtc tac gcc att gcc agg tgc tgc ctg cta ccc cag gcc aac tgc      1640
Gly Val Tyr Ala Ile Ala Arg Cys Cys Leu Leu Pro Gln Ala Asn Cys
                505                 510                 515

agc gtc cac aca gct cca cca gct gag gcc agc atg ggg acc cgt gtc      1688
Ser Val His Thr Ala Pro Pro Ala Glu Ala Ser Met Gly Thr Arg Val
            520                 525                 530

cac tgc cac caa cag ggc cac gtc ctc aca ggc tgc agc tcc cac tgg      1736
His Cys His Gln Gln Gly His Val Leu Thr Gly Cys Ser Ser His Trp
        535                 540                 545

gag gtg gag gac ctt ggc acc cac aag ccg cct gtg ctg agg cca cga      1784
Glu Val Glu Asp Leu Gly Thr His Lys Pro Pro Val Leu Arg Pro Arg
    550                 555                 560

ggt cag ccc aac cag tgc gtg ggc cac agg gag gcc agc atc cac gct     1832
Gly Gln Pro Asn Gln Cys Val Gly His Arg Glu Ala Ser Ile His Ala
565                 570                 575                 580

tcc tgc tgc cat gcc cca ggt ctg gaa tgc aaa gtc aag gag cat gga     1880
Ser Cys Cys His Ala Pro Gly Leu Glu Cys Lys Val Lys Glu His Gly
                585                 590                 595

atc ccg gcc cct cag gag cag gtg acc gtg gcc tgc gag gag ggc tgg     1928
Ile Pro Ala Pro Gln Glu Gln Val Thr Val Ala Cys Glu Glu Gly Trp
            600                 605                 610

acc ctg act ggc tgc agt gcc ctc cct ggg acc tcc cac gtc ctg ggg     1976
Thr Leu Thr Gly Cys Ser Ala Leu Pro Gly Thr Ser His Val Leu Gly
        615                 620                 625

gcc tac gcc gta gac aac acg tgt gta gtc agg agc cgg gac gtc agc     2024
Ala Tyr Ala Val Asp Asn Thr Cys Val Val Arg Ser Arg Asp Val Ser
    630                 635                 640

act aca ggc agc acc agc gaa gag gcc gtg aca gcc gtt gcc atc tgc     2072
Thr Thr Gly Ser Thr Ser Glu Glu Ala Val Thr Ala Val Ala Ile Cys
645                 650                 655                 660

tgc cgg agc cgg cac ctg gcg cag gcc ttc cag gag ctc cag             2114
Cys Arg Ser Arg His Leu Ala Gln Ala Phe Gln Glu Leu Gln
                665                 670

tgacagcccc atcccaggat gggtgtctgg ggagggtcaa gggctggggc tgagctttaa   2174

aatggttccg acttgtccct ctctcagccc ttcatg                             2210

<210>  20

<211>  692

<212>  PRT

<213>  Homo sapiens

<400>  20; LP251(a)

Met Gly Thr Val Ser Ser Arg Arg Ser Trp Trp Pro Leu Pro Leu Leu
            -15                 -10                 -5

Leu Leu Leu Leu Leu Leu Leu Gly Pro Ala Gly Ala Arg Ala Gln Glu
    -1  1               5                   10

Asp Glu Asp Gly Asp Tyr Glu Glu Leu Val Leu Ala Leu Arg Ser Glu
15                  20                  25                  30

Glu Asp Gly Leu Ala Glu Ala Pro Glu His Gly Thr Thr Ala Thr Phe
                35                  40                  45

His Arg Cys Ala Lys Asp Pro Trp Arg Leu Pro Gly Thr Tyr Val Val
            50                  55                  60

Val Leu Lys Glu Glu Thr His Leu Ser Gln Ser Glu Arg Thr Ala Arg
        65                  70                  75

Arg Leu Gln Ala Gln Ala Ala Arg Arg Gly Tyr Leu Thr Lys Ile Leu
    80                  85                  90

His Val Phe His Gly Leu Leu Pro Gly Phe Leu Val Lys Met Ser Gly
95                  100                 105                 110

Asp Leu Leu Glu Leu Ala Leu Lys Leu Pro His Val Asp Tyr Ile Glu
                115                 120                 125

Glu Asp Ser Ser Val Phe Ala Gln Ser Ile Pro Trp Asn Leu Glu Arg
            130                 135                 140

Ile Thr Pro Pro Arg Tyr Arg Ala Asp Glu Tyr Gln Pro Pro Asp Gly
        145                 150                 155

Gly Ser Leu Val Glu Val Tyr Leu Leu Asp Thr Ser Ile Gln Ser Asp
    160                 165                 170

His Arg Glu Ile Glu Gly Arg Val Met Val Thr Asp Phe Glu Asn Val
175                 180                 185                 190

Pro Glu Glu Asp Gly Thr Arg Phe His Arg Gln Ala Ser Lys Cys Asp
                195                 200                 205

Ser His Gly Thr His Leu Ala Gly Val Val Ser Gly Arg Asp Ala Gly
            210                 215                 220

Val Ala Lys Gly Ala Ser Met Arg Ser Leu Arg Val Leu Asn Cys Gln
        225                 230                 235

Gly Lys Gly Thr Val Ser Gly Thr Leu Ile Gly Leu Glu Phe Ile Arg
    240                 245                 250

Lys Ser Gln Leu Val Gln Pro Val Gly Pro Leu Val Val Leu Leu Pro
255                 260                 265                 270

Leu Ala Gly Gly Tyr Ser Arg Val Leu Asn Ala Ala Cys Gln Arg Leu
                275                 280                 285

Ala Arg Ala Gly Val Val Leu Val Thr Ala Ala Gly Asn Phe Arg Asp
            290                 295                 300

Asp Ala Cys Leu Tyr Ser Pro Ala Ser Ala Pro Glu Val Ile Thr Val
        305                 310                 315

Gly Ala Thr Asn Ala Gln Asp Gln Pro Val Thr Leu Gly Thr Leu Gly
    320                 325                 330

Thr Asn Phe Gly Arg Cys Val Asp Leu Phe Ala Pro Gly Glu Asp Ile
335                 340                 345                 350

Ile Gly Ala Ser Ser Asp Cys Ser Thr Cys Phe Val Ser Gln Ser Gly
                355                 360                 365

Thr Ser Gln Ala Ala Ala His Val Ala Gly Ile Ala Ala Met Met Leu
            370                 375                 380

Ser Ala Glu Pro Glu Leu Thr Leu Ala Glu Leu Arg Gln Arg Leu Ile
        385                 390                 395

His Phe Ser Ala Lys Asp Val Ile Asn Glu Ala Trp Phe Pro Glu Asp
    400                 405                 410

Gln Arg Val Leu Thr Pro Asn Leu Val Ala Ala Leu Pro Pro Ser Thr
415                 420                 425                 430

His Gly Ala Gly Trp Gln Leu Phe Cys Arg Thr Val Trp Ser Ala His
                435                 440                 445

Ser Gly Pro Thr Arg Met Ala Thr Ala Ile Ala Arg Cys Ala Pro Asp
            450                 455                 460

Glu Glu Leu Leu Ser Cys Ser Ser Phe Ser Arg Ser Gly Lys Arg Arg
        465                 470                 475

Gly Glu Arg Met Glu Ala Gln Gly Gly Lys Leu Val Cys Arg Ala His
    480                 485                 490

Asn Ala Phe Gly Gly Glu Gly Val Tyr Ala Ile Ala Arg Cys Cys Leu
495                 500                 505                 510

Leu Pro Gln Ala Asn Cys Ser Val His Thr Ala Pro Pro Ala Glu Ala
                515                 520                 525

Ser Met Gly Thr Arg Val His Cys His Gln Gln Gly His Val Leu Thr
            530                 535                 540

Gly Cys Ser Ser His Trp Glu Val Glu Asp Leu Gly Thr His Lys Pro
        545                 550                 555

Pro Val Leu Arg Pro Arg Gly Gln Pro Asn Gln Cys Val Gly His Arg
    560                 565                 570

Glu Ala Ser Ile His Ala Ser Cys Cys His Ala Pro Gly Leu Glu Cys
575                 580                 585                 590

Lys Val Lys Glu His Gly Ile Pro Ala Pro Gln Glu Gln Val Thr Val
                595                 600                 605

Ala Cys Glu Glu Gly Trp Thr Leu Thr Gly Cys Ser Ala Leu Pro Gly
            610                 615                 620

Thr Ser His Val Leu Gly Ala Tyr Ala Val Asp Asn Thr Cys Val Val
        625                 630                 635

Arg Ser Arg Asp Val Ser Thr Thr Gly Ser Thr Ser Glu Glu Ala Val
    640                 645                 650

Thr Ala Val Ala Ile Cys Cys Arg Ser Arg His Leu Ala Gln Ala Phe
655                 660                 665                 670

Gln Glu Leu Gln

<210>  21

<211>  2492

<212>  DNA

<213>  Homo sapiens

<220>

<221>  CDS

<222>  (322)..(2379)

<223>



<220>

<221>  mat_peptide

<222>  (376)..()

<223>



<220>

<221>  misc_feature

<222>  (2484)..(2484)

<223>  wherein n is A, G, C, or T



<400>  21; LP252
ggcctttcaa agtgtgcagt tgtctcctcc ctgtccagcc ccatcgtcgc ccaggaccag      60

ctgggccgcg gtctgacctg aggctgctgc tcagcgccgg ggcgctggcg ctctccattc     120

gagcaccttc cagcataccg ctcggctccg ggagccgctc tgcaaagttg ggcagctcag     180

agcgcaagct ttgcctctcg acttctccct ccttgggtcc ccggcgcccc cgcctcccac     240

gatccctttc actaggagca gccagtccca gcgggctggc aacttgcacc ccttcctagt     300

catcctccct gaaacgcgac c atg ctg tta agg ggc gtc ctc ctg gcg ttg      351
                        Met Leu Leu Arg Gly Val Leu Leu Ala Leu
                                    -15                 -10

caa gcc ctg cag ctc gcc ggt gcc ctc gac ctg ccc gct ggg tcc tgt      399
Gln Ala Leu Gln Leu Ala Gly Ala Leu Asp Leu Pro Ala Gly Ser Cys
            -5                  -1  1               5

gcc ttt gaa gag agc act tgc ggc ttt gac tcc gtg ttg gcc tct ctg      447
Ala Phe Glu Glu Ser Thr Cys Gly Phe Asp Ser Val Leu Ala Ser Leu
    10                  15                  20

ccg tgg att tta aat gag gaa ggc cat tac att tat gtg gat acc tcc      495
Pro Trp Ile Leu Asn Glu Glu Gly His Tyr Ile Tyr Val Asp Thr Ser
25                  30                  35                  40

ttt ggc aag cag ggg gag aaa gct gtg ctg cta agt cct gac tta cag      543

Phe Gly Lys Gln Gly Glu Lys Ala Val Leu Leu Ser Pro Asp Leu Gln
                45                  50                  55

gct gag gaa tgg agc tgc ctc cgt ttg gtc tac cag ata acc aca tct      591
Ala Glu Glu Trp Ser Cys Leu Arg Leu Val Tyr Gln Ile Thr Thr Ser
            60                  65                  70

tcg gag tct ctg tca gat ccc agc cag ctg aac ctc tac atg aga ttt      639
Ser Glu Ser Leu Ser Asp Pro Ser Gln Leu Asn Leu Tyr Met Arg Phe
        75                  80                  85

gaa gat gaa agc ttt gat cgc ttg ctt tgg tca gct aag gaa cct tca      687
Glu Asp Glu Ser Phe Asp Arg Leu Leu Trp Ser Ala Lys Glu Pro Ser
    90                  95                  100

gac agc tgg ctc ata gcc agc ttg gat ttg caa aac agt tcc aag aaa      735
Asp Ser Trp Leu Ile Ala Ser Leu Asp Leu Gln Asn Ser Ser Lys Lys
105                 110                 115                 120

ttc aag att tta ata gaa ggt gta cta gga cag gga aac aca gcc agc      783
Phe Lys Ile Leu Ile Glu Gly Val Leu Gly Gln Gly Asn Thr Ala Ser
                125                 130                 135

atc gca cta ttt gaa atc aag atg aca acc ggc tac tgt att gaa tgt      831
Ile Ala Leu Phe Glu Ile Lys Met Thr Thr Gly Tyr Cys Ile Glu Cys
            140                 145                 150

gac ttt gaa gaa aat cat ctc tgt ggc ttt gtg aac cgc tgg aat ccc      879
Asp Phe Glu Glu Asn His Leu Cys Gly Phe Val Asn Arg Trp Asn Pro
        155                 160                 165

aat gtg aac tgg ttt gtt gga gga gga agt att cgg aat gtc cac tcc      927
Asn Val Asn Trp Phe Val Gly Gly Gly Ser Ile Arg Asn Val His Ser
    170                 175                 180

att ctc cca cag gat cac acc ttc aag agt gaa ctg ggc cac tac atg      975
Ile Leu Pro Gln Asp His Thr Phe Lys Ser Glu Leu Gly His Tyr Met
185                 190                 195                 200

tac gtg gac tca gtt tat gtg aag cac ttc cag gag gtg gca cag ctc      1023
Tyr Val Asp Ser Val Tyr Val Lys His Phe Gln Glu Val Ala Gln Leu
                205                 210                 215

atc tcc ccg ttg acc acg gcc ccc atg gct ggc tgc ctg tca ttt tat      1071
Ile Ser Pro Leu Thr Thr Ala Pro Met Ala Gly Cys Leu Ser Phe Tyr
            220                 225                 230

tac cag atc cag cag ggg aat gac aat gtc ttt tcc ctt tac act cgg      1119
Tyr Gln Ile Gln Gln Gly Asn Asp Asn Val Phe Ser Leu Tyr Thr Arg
        235                 240                 245

gat gtg gct ggc ctt tac gag gaa atc tgg aaa gca gac agg cca ggg      1167
Asp Val Ala Gly Leu Tyr Glu Glu Ile Trp Lys Ala Asp Arg Pro Gly
    250                 255                 260

aat gct gcc tgg aac ctt gcg gag gtc gag ttc aat gct cct tac ccc      1215
Asn Ala Ala Trp Asn Leu Ala Glu Val Glu Phe Asn Ala Pro Tyr Pro
265                 270                 275                 280

atg gag gtt att ttt gaa gtt gct ttc aat ggt ccc aag gga ggt tat 1263
Met Glu Val Ile Phe Glu Val Ala Phe Asn Gly Pro Lys Gly Gly Tyr
285 290 295

gtt gcc ctg gat gat att tca ttc tct cct gtt cac tgc cag aat cag 1311
Val Ala Leu Asp Asp Ile Ser Phe Ser Pro Val His Cys Gln Asn Gln
300 305 310

aca gaa ctt ctg ttc agt gcc gtg gaa gcc agc tgc aat ttt gag caa 1359
Thr Glu Leu Leu Phe Ser Ala Val Glu Ala Ser Cys Asn Phe Glu Gln
315 320 325

gat ctc tgc aac ttt tac caa gat aaa gaa ggt cca ggt tgg acc cga 1407
Asp Leu Cys Asn Phe Tyr Gln Asp Lys Glu Gly Pro Gly Trp Thr Arg
330 335 340

gtg aaa gta aaa cca aac atg tat cgg gct gga gac cac act aca ggc 1455
Val Lys Val Lys Pro Asn Met Tyr Arg Ala Gly Asp His Thr Thr Gly
345 350 355 360

tta ggg tat tac ctg cta gcc aac aca aag ttc aca tct cag cct ggc 1503
Leu Gly Tyr Tyr Leu Leu Ala Asn Thr Lys Phe Thr Ser Gln Pro Gly
365 370 375

tac att gga agg ctc tat ggg ccc tcc cta cca gga aac ttg cag tat 1551
Tyr Ile Gly Arg Leu Tyr Gly Pro Ser Leu Pro Gly Asn Leu Gln Tyr
380 385 390

tgt ctg cgt ttt cat tat gcc atc tat gga ttt tta aaa atg agt gac 1599
Cys Leu Arg Phe His Tyr Ala Ile Tyr Gly Phe Leu Lys Met Ser Asp
395 400 405

acc cta gca gtt tac atc ttt gaa gag aac cat gtg gtt caa gag aag 1647
Thr Leu Ala Val Tyr Ile Phe Glu Glu Asn His Val Val Gln Glu Lys
410 415 420

atc tgg tct gtg ttg gag tcc cca agg ggt gtt tgg atg caa gct gaa 1695
Ile Trp Ser Val Leu Glu Ser Pro Arg Gly Val Trp Met Gln Ala Glu
425 430 435 440

atc acc ttt aag aag ccc atg cct acc aag gtg gtt ttc atg agc cta 1743
Ile Thr Phe Lys Lys Pro Met Pro Thr Lys Val Val Phe Met Ser Leu
445 450 455

tgc aaa agt ttc tgg gac tgt ggg ctt gta gcc ctg gat gac att aca 1791
Cys Lys Ser Phe Trp Asp Cys Gly Leu Val Ala Leu Asp Asp Ile Thr
460 465 470

ata caa ttg gga agc tgc tca tct tca gag aaa ctt cca cct cca cct 1839
Ile Gln Leu Gly Ser Cys Ser Ser Ser Glu Lys Leu Pro Pro Pro Pro
475 480 485

gga gag tgt act ttc gag caa gat gaa tgt aca ttt act cag gag aaa 1887
Gly Glu Cys Thr Phe Glu Gln Asp Glu Cys Thr Phe Thr Gln Glu Lys
490 495 500

aga aac cgg agc agc tgg cac agg agg agg gga gaa act ccc act tcc 1935

Arg Asn Arg Ser Ser Trp His Arg Arg Arg Gly Glu Thr Pro Thr Ser
505                 510                 515                 520

tac aca gga cca aag gga gat cac act act ggg gta ggc tac tac atg      1983
Tyr Thr Gly Pro Lys Gly Asp His Thr Thr Gly Val Gly Tyr Tyr Met
                525                 530                 535

tac att gag gcc tcc cat atg gtg tat gga caa aaa gca cgc ctc ttg      2031
Tyr Ile Glu Ala Ser His Met Val Tyr Gly Gln Lys Ala Arg Leu Leu
            540                 545                 550

tcc agg cct ctg cga gga gtc tct gga aaa cac tgc ttg acc ttt ttc      2079
Ser Arg Pro Leu Arg Gly Val Ser Gly Lys His Cys Leu Thr Phe Phe
        555                 560                 565

tac cac atg tat gga ggg ggc act ggc ctg ctg agt gtt tat ctg aaa      2127
Tyr His Met Tyr Gly Gly Gly Thr Gly Leu Leu Ser Val Tyr Leu Lys
    570                 575                 580

aag gaa gaa gac agt gaa gag tcc ctc tta tgg agg aga aga ggt gaa      2175
Lys Glu Glu Asp Ser Glu Glu Ser Leu Leu Trp Arg Arg Arg Gly Glu
585                 590                 595                 600

cag agc att tcc tgg cta cga gca ctg att gaa tac agc tgt gag agg      2223
Gln Ser Ile Ser Trp Leu Arg Ala Leu Ile Glu Tyr Ser Cys Glu Arg
                605                 610                 615

caa cac cag ata att ttt gaa gcc att cga gga gta tca ata aga agt      2271
Gln His Gln Ile Ile Phe Glu Ala Ile Arg Gly Val Ser Ile Arg Ser
            620                 625                 630

gat att gcc att gat gat gtt aaa ttt cag gca gga ccc tgt gga gaa      2319
Asp Ile Ala Ile Asp Asp Val Lys Phe Gln Ala Gly Pro Cys Gly Glu
        635                 640                 645

atg gaa gat aca act caa caa tca tca gga tat tct gag gac tta aat      2367
Met Glu Asp Thr Thr Gln Gln Ser Ser Gly Tyr Ser Glu Asp Leu Asn
    650                 655                 660

gaa att gag tat taagaaatga tctgcattgg atttactaga cgaaaaccat      2419
Glu Ile Glu Tyr
665

acctctcttc aatcaaaatg aaaacaaagc aaatgaatac tggacagtct taacaattta    2479

taagntataa aat                                                        2492


<210>  22

<211>  686

<212>  PRT

<213>  Homo sapiens

<220>

<221>  misc_feature

<222>  (2484)..(2484)

<223>  wherein n is A, G, C, or T

<400>  22; LP252

Met Leu Leu Arg Gly Val Leu Leu Ala Leu Gln Ala Leu Gln Leu Ala
            -15                 -10                 -5

Gly Ala Leu Asp Leu Pro Ala Gly Ser Cys Ala Phe Glu Glu Ser Thr
    -1  1               5                   10

Cys Gly Phe Asp Ser Val Leu Ala Ser Leu Pro Trp Ile Leu Asn Glu
15                  20                  25                  30

Glu Gly His Tyr Ile Tyr Val Asp Thr Ser Phe Gly Lys Gln Gly Glu
                35                  40                  45

Lys Ala Val Leu Leu Ser Pro Asp Leu Gln Ala Glu Glu Trp Ser Cys
            50                  55                  60

Leu Arg Leu Val Tyr Gln Ile Thr Thr Ser Ser Glu Ser Leu Ser Asp
        65                  70                  75

Pro Ser Gln Leu Asn Leu Tyr Met Arg Phe Glu Asp Glu Ser Phe Asp
    80                  85                  90

Arg Leu Leu Trp Ser Ala Lys Glu Pro Ser Asp Ser Trp Leu Ile Ala
95                  100                 105                 110

Ser Leu Asp Leu Gln Asn Ser Ser Lys Lys Phe Lys Ile Leu Ile Glu
                115                 120                 125

Gly Val Leu Gly Gln Gly Asn Thr Ala Ser Ile Ala Leu Phe Glu Ile
            130                 135                 140

Lys Met Thr Thr Gly Tyr Cys Ile Glu Cys Asp Phe Glu Glu Asn His
        145                 150                 155

Leu Cys Gly Phe Val Asn Arg Trp Asn Pro Asn Val Asn Trp Phe Val
    160                 165                 170

Gly Gly Gly Ser Ile Arg Asn Val His Ser Ile Leu Pro Gln Asp His
175                 180                 185                 190

Thr Phe Lys Ser Glu Leu Gly His Tyr Met Tyr Val Asp Ser Val Tyr
                195                 200                 205

Val Lys His Phe Gln Glu Val Ala Gln Leu Ile Ser Pro Leu Thr Thr
            210                 215                 220

Ala Pro Met Ala Gly Cys Leu Ser Phe Tyr Tyr Gln Ile Gln Gln Gly
        225                 230                 235

Asn Asp Asn Val Phe Ser Leu Tyr Thr Arg Asp Val Ala Gly Leu Tyr
    240                 245                 250

Glu Glu Ile Trp Lys Ala Asp Arg Pro Gly Asn Ala Ala Trp Asn Leu
255                 260                 265                 270

Ala Glu Val Glu Phe Asn Ala Pro Tyr Pro Met Glu Val Ile Phe Glu
                275                 280                 285

Val Ala Phe Asn Gly Pro Lys Gly Gly Tyr Val Ala Leu Asp Asp Ile
            290                 295                 300

Ser Phe Ser Pro Val His Cys Gln Asn Gln Thr Glu Leu Leu Phe Ser
        305                 310                 315

Ala Val Glu Ala Ser Cys Asn Phe Glu Gln Asp Leu Cys Asn Phe Tyr
    320                 325                 330

Gln Asp Lys Glu Gly Pro Gly Trp Thr Arg Val Lys Val Lys Pro Asn
335                 340                 345                 350


                355                 360                 365


            370                 375                 380


        385                 390                 395


    400                 405                 410


415                 420                 425                 430


                435                 440                 445


            450                 455                 460


        465                 470                 475


    480                 485                 490


495                 500                 505                 510


                515                 520                 525


            530                 535                 540


        545                 550                 555


    560                 565                 570


575                 580                 585                 590


                595                 600                 605


            610                 615                 620


        625                 630                 635


    640                 645                 650


655                 660                 665

<210>  23

<211>  1581

<212>  DNA

<213>  Homo sapiens

<220>

<221>  CDS

<222>  (136)..(1386)

<223>

<220>

<221>  mat_peptide

<222>  (196)..()

<223>

<400>  23; LP239(b)
ctcaattctt aaaaaataag tccatgaagc agaaacatca aaaagcatgg gatttggaat      60

ttgagacctg actttgaagc ccacacagaa cacagacaag tcaaccatgc tatttgggac     120

atattttgtt ccaaa atg gca tct tac ctt tat gga gta ctc ttt gct gtt      171
                 
                 -20                 -15                 -10

ggc ctc tgt gct cca atc tac tgt gtg tcc ccg gtc aat gcc ccc agt      219

            -5                  -1  1               5

gca tac ccc cgc cct tcc tcc aca aag agc acc cct gcc tca cag gtg      267

    10                  15                  20

tat tcc ctc aac acc gac ttt gcc ttc cgc cta tac cgc agg ctg gtt      315

25                  30                  35                  40

ttg gag acc ccg agt cag aac atc ttc ttc tcc cct gtg agt gtc tcc 363

45 50 55

act tcc ctg gcc atg ctc tcc ctt ggg gcc cac tca gtc acc aag acc 411

60 65 70

cag att ctc cag ggc ctg ggc ttc aac ctc aca cac aca cca gag tct 459

75 80 85

gcc atc cac cag ggc ttc cag cac ctg gtt cac tca ctg act gtt ccc 507

90 95 100

agc aaa gac ctg acc ttg aag atg gga agt gcc ctc ttc gtc aag aag 555

105 110 115 120

gag ctg cag ctg cag gca aat ttc ttg ggc aat gtc aag agg ctg tat 603

125 130 135

gaa gca gaa gtc ttt tct aca gat ttc tcc aac ccc tcc att gcc cag 651

140 145 150

gcg agg atc aac agc cat gtg aaa aag aag acc caa ggg aag gtt gta 699

155 160 165

gac ata atc caa ggc ctt gac ctt ctg acg gcc atg gtt ctg gtg aat 747

170 175 180

cac att ttc ttt aaa gcc aag tgg gag aag ccc ttt cac ctt gaa tat 795

185 190 195 200

aca aga aag aac ttc cca ttc ctg gtg ggc gag cag gtc act gtg caa 843

205 210 215

gtc ccc atg atg cac cag aaa gag cag ttc gct ttt ggg gtg gat aca 891

220 225 230

gag ctg aac tgc ttt gtg ctg cag atg gat tac aag gga gat gcc gtg 939

235 240 245

gcc ttc ttt gtc ctc cct agc aag ggc aag atg agg caa ctg gaa cag 987

250 255 260

gcc ttg tca gcc aga aca ctg ata aag tgg agc cac tca ctc cag aaa 1035


265                 270                 275                 280

agg tgg ata gag gtg ttc atc ccc aga ttt tcc att tct gcc tcc tac      1083

                285                 290                 295

aat ctg gaa acc atc ctc ccg aag atg ggc atc caa aat gcc ttt gac      1131

            300                 305                 310

aaa aat gct gat ttt tct gga att gca aag aga gac tcc ctg cag gtt      1179

        315                 320                 325

tct aaa gca acc cac aag gct gtg ctg gat gtc agt gaa gag ggc act      1227

    330                 335                 340

gag gcc aca gca gct acc acc acc aag ttc ata gtc cga tcg aag gat      1275

345                 350                 355                 360

ggt ccc tct tac ttc act gtc tcc ttc aat agg acc ttc ctg atg atg      1323

                365                 370                 375

att aca aat aaa gcc aca gac ggt att ctc ttt cta ggg aaa gtg gaa      1371

            380                 385                 390

aat ccc act aaa tcc taggtgggaa atggcctgtt aactgatggc acattgctaa      1426

        395

tgcacaagaa ataacaaacc acatcctctt tctgttctga gggtgcattt gaccccagtg   1486

ggagctggat tcgctggcag ggatgccacc ttccaaggct caatcaccaa accatcaaca   1546

gggaccccag tcacaagcca acacccatta acccc                              1581

<210>  24

<211>  417

<212>  PRT

<213>  Homo sapiens

<400>  24

<210>  25

<211>  3027

<212>  DNA

<213>  Homo sapiens

<220>

<221>  CDS

<222>  (133)..(2271)

<223>

<220>

<221>  mat_peptide

<222>  (241)..()

<223>

<400>  25; LP223(a)
tggagaactg gggaggcaga gaccccggct ggccggaggc atgtggaggg gggggcctgg      60

gcgcagggag aggcccagcg gaagccaagc caccaggccc cccagcgtcc acgcggagca     120

tgaacattga gg atg gcg cgt gcc cgc ggc tcc ccg tgc ccc ccg ctg ccg     171
              Met Ala Arg Ala Arg Gly Ser Pro Cys Pro Pro Leu Pro
                  -35                 -30                 -25

ccc ggt agg atg tcc tgg ccc cac ggg gca ttg ctc ttc ctc tgg ctc       219
Pro Gly Arg Met Ser Trp Pro His Gly Ala Leu Leu Phe Leu Trp Leu
            -20                 -15                 -10

ttc tcc cca ccc ctg ggg gcc ggt gga ggt gga gtg gcc gtg acg tct       267
Phe Ser Pro Pro Leu Gly Ala Gly Gly Gly Gly Val Ala Val Thr Ser
        -5                  -1  1               5

gcc gcc gga ggg ggc tcc ccg ccg gcc acc tcc tgc ccc gtg gcc tgc       315
Ala Ala Gly Gly Gly Ser Pro Pro Ala Thr Ser Cys Pro Val Ala Cys
10                  15                  20                  25

tcc tgc agc aac cag gcc agc cgg gtg atc tgc aca cgg aga gac ctg       363
Ser Cys Ser Asn Gln Ala Ser Arg Val Ile Cys Thr Arg Arg Asp Leu
                30                  35                  40

gcc gag gtc cca gcc agc atc ccg gtc aac acg cgg tac ctg aac ctg       411
Ala Glu Val Pro Ala Ser Ile Pro Val Asn Thr Arg Tyr Leu Asn Leu
            45                  50                  55

caa gag aac ggc atc cag gtg atc cgg acg gac acg ttc aag cac ctg       459
Gln Glu Asn Gly Ile Gln Val Ile Arg Thr Asp Thr Phe Lys His Leu
        60                  65                  70

cgg cac ctg gag att ctg cag ctg agc aag aac ctg gtg cgc aag atc       507
Arg His Leu Glu Ile Leu Gln Leu Ser Lys Asn Leu Val Arg Lys Ile
    75                  80                  85

gag gtg ggc gcc ttc aac ggg ctg ccc agc ctc aac acg ctg gag ctt       555
Glu Val Gly Ala Phe Asn Gly Leu Pro Ser Leu Asn Thr Leu Glu Leu
90                  95                  100                 105

tttt gac aac cgg ctg acc acg gtg ccc acg cag gcc ttc gag tac ctg 603
Phe Asp Asn Arg Leu Thr Thr Val Pro Thr Gln Ala Phe Glu Tyr Leu
110 115 120

tcc aag ctg cgg gag ctc tgg ctg cgg aac aac ccc atc gag agc atc 651
Ser Lys Leu Arg Glu Leu Trp Leu Arg Asn Asn Pro Ile Glu Ser Ile
125 130 135

ccc tcc tac gcc ttc aac cgc gtg ccc tcg ctg cgg cgc ctg gac ctg 699
Pro Ser Tyr Ala Phe Asn Arg Val Pro Ser Leu Arg Arg Leu Asp Leu
140 145 150

ggc gag ctc aag cgg ctg gaa tac atc tcg gag gcg gcc ttc gag ggg 747
Gly Glu Leu Lys Arg Leu Glu Tyr Ile Ser Glu Ala Ala Phe Glu Gly
155 160 165

ctg gtc aac ctg cgc tac ctc aac ctg ggc atg tgc aac ctc aag gac 795
Leu Val Asn Leu Arg Tyr Leu Asn Leu Gly Met Cys Asn Leu Lys Asp
170 175 180 185

atc ccc aac ctg acg gcc ctg gtg cgc ctg gag gag ctg gag ctg tcg 843
Ile Pro Asn Leu Thr Ala Leu Val Arg Leu Glu Glu Leu Glu Leu Ser
190 195 200

ggc aac cgg ctg gac ctg atc cgc ccg ggc tcc ttc cag ggt ctc acc 891
Gly Asn Arg Leu Asp Leu Ile Arg Pro Gly Ser Phe Gln Gly Leu Thr
205 210 215

agc ctg cgc aag ctg tgg ctc atg cac gcc cag gta gcc acc atc gag 939
Ser Leu Arg Lys Leu Trp Leu Met His Ala Gln Val Ala Thr Ile Glu
220 225 230

cgc aac gcc ttc gac gac ctc aag tcg ctg gag gag ctc aac ctg tcc 987
Arg Asn Ala Phe Asp Asp Leu Lys Ser Leu Glu Glu Leu Asn Leu Ser
235 240 245

cac aac aac ctg atg tcg ctg ccc cac gac ctc ttc acg ccc ctg cac 1035
His Asn Asn Leu Met Ser Leu Pro His Asp Leu Phe Thr Pro Leu His
250 255 260 265

cgc ctc gag cgc gtg cac ctc aac cac aac ccc tgg cat tgc aac tgc 1083
Arg Leu Glu Arg Val His Leu Asn His Asn Pro Trp His Cys Asn Cys
270 275 280

gac gtg ctc tgg ctg agc tgg tgg ctc aag gag acg gtg ccc agc aac 1131
Asp Val Leu Trp Leu Ser Trp Trp Leu Lys Glu Thr Val Pro Ser Asn
285 290 295

acg acg tgc tgc gcc cgc tgt cat gcg ccc gcc ggc ctc aag ggg cgc 1179
Thr Thr Cys Cys Ala Arg Cys His Ala Pro Ala Gly Leu Lys Gly Arg
300 305 310

tac att ggg gag ctg gac cag tcg cat ttc acc tgc tat gcg ccc gtc 1227
Tyr Ile Gly Glu Leu Asp Gln Ser His Phe Thr Cys Tyr Ala Pro Val
315 320 325

atc gtg gag ccg ccc acg gac ctc aac gtc acc gag ggc atg gct gcc 1275

Ile Val Glu Pro Pro Thr Asp Leu Asn Val Thr Glu Gly Met Ala Ala
330                 335                 340                 345

gag ctc aaa tgc cgc acg ggc acc tcc atg acc tcc gtc aac tgg ctg      1323
Glu Leu Lys Cys Arg Thr Gly Thr Ser Met Thr Ser Val Asn Trp Leu
                350                 355                 360

acg ccc aac ggc acc ctc atg acc cac ggc tcc tac cgc gtg cgc atc      1371
Thr Pro Asn Gly Thr Leu Met Thr His Gly Ser Tyr Arg Val Arg Ile
            365                 370                 375

tcc gtc ctg cat gac ggc acg ctt aac ttc acc aac gtc acc gtg cag      1419
Ser Val Leu His Asp Gly Thr Leu Asn Phe Thr Asn Val Thr Val Gln
        380                 385                 390

gac acg ggc cag tac acg tgc atg gtg acg aac tca gcc ggc aac acc      1467
Asp Thr Gly Gln Tyr Thr Cys Met Val Thr Asn Ser Ala Gly Asn Thr
    395                 400                 405

acc gcc tcg gcc acg ctc aac gtc tcg gcc gtg gac ccc gtg gcg gcc      1515
Thr Ala Ser Ala Thr Leu Asn Val Ser Ala Val Asp Pro Val Ala Ala
410                 415                 420                 425

ggg ggc acc ggc agc ggc ggg ggc ggc cct ggg ggc agt ggt ggt gtt      1563
Gly Gly Thr Gly Ser Gly Gly Gly Gly Pro Gly Gly Ser Gly Gly Val
                430                 435                 440

gga ggg ggc agt ggc ggc tac acc tac ttc acc acg gtg acc gtg gag      1611
Gly Gly Gly Ser Gly Gly Tyr Thr Tyr Phe Thr Thr Val Thr Val Glu
            445                 450                 455

acc ctg gag acg cag ccc gga gag gag gcc ctg cag ccg cgg ggg acg      1659
Thr Leu Glu Thr Gln Pro Gly Glu Glu Ala Leu Gln Pro Arg Gly Thr
        460                 465                 470

gag aag gaa ccg cca ggg ccc acg aca gac ggt gtc tgg ggt ggg ggc      1707
Glu Lys Glu Pro Pro Gly Pro Thr Thr Asp Gly Val Trp Gly Gly Gly
    475                 480                 485

cgg cct ggg gac gcg gcc ggc cct gcc tcg tct tct acc acg gca ccc      1755
Arg Pro Gly Asp Ala Ala Gly Pro Ala Ser Ser Ser Thr Thr Ala Pro
490                 495                 500                 505

gcc ccg cgc tcc tcg cgg ccc acg gag aag gcg ttc acg gtg ccc atc      1803
Ala Pro Arg Ser Ser Arg Pro Thr Glu Lys Ala Phe Thr Val Pro Ile
                510                 515                 520

acg gat gtg acg gag aac gcc ctc aag gac ctg gac gac gtc atg aag      1851
Thr Asp Val Thr Glu Asn Ala Leu Lys Asp Leu Asp Asp Val Met Lys
            525                 530                 535

acc acc aaa atc atc atc ggc tgc ttc gtg gcc atc acg ttc atg gcc      1899
Thr Thr Lys Ile Ile Ile Gly Cys Phe Val Ala Ile Thr Phe Met Ala
        540                 545                 550

gcg gtg atg ctc gtg gcc ttc tac aag ctg cgc aag cag cac cag ctc      1947
Ala Val Met Leu Val Ala Phe Tyr Lys Leu Arg Lys Gln His Gln Leu
    555                 560                 565

cac aag cac cac ggg ccc acg cgc acc gtg gag atc atc aac gtg gag     1995
His Lys His His Gly Pro Thr Arg Thr Val Glu Ile Ile Asn Val Glu
570                 575                 580                 585

gac gag ctg ccc gcc gcc tcg gcc gtg tcc gtg gcc gcc gcg gcc gcc     2043
Asp Glu Leu Pro Ala Ala Ser Ala Val Ser Val Ala Ala Ala Ala Ala
                590                 595                 600

gtg gcc agt ggg ggt ggt gtg ggc ggg gac agc cac ctg gcc ctg ccc     2091
Val Ala Ser Gly Gly Gly Val Gly Gly Asp Ser His Leu Ala Leu Pro
            605                 610                 615

gcc ctg gag cga gac cac ctc aac cac cac cac tac gtg gct gcc gcc     2139
Ala Leu Glu Arg Asp His Leu Asn His His His Tyr Val Ala Ala Ala
        620                 625                 630

ttc aag gcg cac tac agc agc aac ccc agc ggc ggg ggc tgc ggg ggc     2187
Phe Lys Ala His Tyr Ser Ser Asn Pro Ser Gly Gly Gly Cys Gly Gly
    635                 640                 645

aaa ggc ccg cct ggc ctc aac tcc atc cac gaa cct ctg ctc ttc aag     2235
Lys Gly Pro Pro Gly Leu Asn Ser Ile His Glu Pro Leu Leu Phe Lys
650                 655                 660                 665

agc ggc tcc aag gag aac gtg caa gag acg cag atc tgaggcggcg          2281
Ser Gly Ser Lys Glu Asn Val Gln Glu Thr Gln Ile
                670                 675

gggccgggcg ggcgaggggc gtggagcccc ccacccaggt cccagcccgg gcgcagcctg   2341

accgggaccc ctccctccca cagcccagcc caccttctgg gaccacgcag ggaattgggg   2401

agaggtggct tccagcccca tctggggctc ggacccccag taaggacagg gtggggctcc   2461

aggagcggag tctctgggat cctcgcctca cccccgccaa tccccggtga cgggagggga   2521

cgtgggaccc aggagaagtg gcctgagttc tccgcgtttc ctcctcgctc tcccaccaag   2581

cgttccgggc ccgccgcctt ccaggggaga gaggagcttt ctccaggggt gtcctttccc   2641

ctcggcccca aacaccttcc ttttacggtt cagtttttgc agtttgacgc ccgtccctcc   2701

ctcccactcc tccgccctca aaactgaaga gatggacacg tcgccagagc tcccgtccca   2761

gcgcccggct ctgctcccgg ccggcggtcc ctgtcgtcgg cgcggggggg agcctccacc   2821

cgccccagcc ccacctgccc cacagacact tttgatactg aagggaggtt tgcgtcaacg   2881

actacctgct ctgtaattac tttaaaaaaa aaacatggaa aaagtaaaaa aatatttttt   2941

ttggtcatga aagcacggag gagagaaaac aaagtgaatg tgggtggggg cggaaggaga   3001

ggctggagag ccagcctcgc agctcc                                        3027

<210>  26

<211>  713

<212>  PRT

<213>  Homo sapiens

<400>  26; LP223(a)

Met Ala Arg Ala Arg Gly Ser Pro Cys Pro Pro Leu Pro Pro Gly Arg
    -35                 -30                 -25

Met Ser Trp Pro His Gly Ala Leu Leu Phe Leu Trp Leu Phe Ser Pro
-20                 -15                 -10                 -5

Pro Leu Gly Ala Gly Gly Gly Gly Val Ala Val Thr Ser Ala Ala Gly
            -1  1               5                   10

Gly Gly Ser Pro Pro Ala Thr Ser Cys Pro Val Ala Cys Ser Cys Ser
        15                  20                  25

Asn Gln Ala Ser Arg Val Ile Cys Thr Arg Arg Asp Leu Ala Glu Val
    30                  35                  40

Pro Ala Ser Ile Pro Val Asn Thr Arg Tyr Leu Asn Leu Gln Glu Asn
45                  50                  55                  60

Gly Ile Gln Val Ile Arg Thr Asp Thr Phe Lys His Leu Arg His Leu
                65                  70                  75

Glu Ile Leu Gln Leu Ser Lys Asn Leu Val Arg Lys Ile Glu Val Gly
            80                  85                  90

Ala Phe Asn Gly Leu Pro Ser Leu Asn Thr Leu Glu Leu Phe Asp Asn
        95                  100                 105

Arg Leu Thr Thr Val Pro Thr Gln Ala Phe Glu Tyr Leu Ser Lys Leu
    110                 115                 120

Arg Glu Leu Trp Leu Arg Asn Asn Pro Ile Glu Ser Ile Pro Ser Tyr
125                 130                 135                 140

Ala Phe Asn Arg Val Pro Ser Leu Arg Arg Leu Asp Leu Gly Glu Leu
                145                 150                 155

Lys Arg Leu Glu Tyr Ile Ser Glu Ala Ala Phe Glu Gly Leu Val Asn
            160                 165                 170

Leu Arg Tyr Leu Asn Leu Gly Met Cys Asn Leu Lys Asp Ile Pro Asn
        175                 180                 185

Leu Thr Ala Leu Val Arg Leu Glu Glu Leu Glu Leu Ser Gly Asn Arg
    190                 195                 200

Leu Asp Leu Ile Arg Pro Gly Ser Phe Gln Gly Leu Thr Ser Leu Arg
205                 210                 215                 220

Lys Leu Trp Leu Met His Ala Gln Val Ala Thr Ile Glu Arg Asn Ala
                225                 230                 235

Phe Asp Asp Leu Lys Ser Leu Glu Glu Leu Asn Leu Ser His Asn Asn
            240                 245                 250

Leu Met Ser Leu Pro His Asp Leu Phe Thr Pro Leu His Arg Leu Glu
        255                 260                 265

Arg Val His Leu Asn His Asn Pro Trp His Cys Asn Cys Asp Val Leu
    270                 275                 280

Trp Leu Ser Trp Trp Leu Lys Glu Thr Val Pro Ser Asn Thr Thr Cys
285                 290                 295                 300

Cys Ala Arg Cys His Ala Pro Ala Gly Leu Lys Gly Arg Tyr Ile Gly
                305                 310                 315

Glu Leu Asp Gln Ser His Phe Thr Cys Tyr Ala Pro Val Ile Val Glu
            320                 325                 330

Pro Pro Thr Asp Leu Asn Val Thr Glu Gly Met Ala Ala Glu Leu Lys
        335                 340                 345

Cys Arg Thr Gly Thr Ser Met Thr Ser Val Asn Trp Leu Thr Pro Asn
    350                 355                 360

Gly Thr Leu Met Thr His Gly Ser Tyr Arg Val Arg Ile Ser Val Leu
365                 370                 375                 380

His Asp Gly Thr Leu Asn Phe Thr Asn Val Thr Val Gln Asp Thr Gly
                385                 390                 395

Gln Tyr Thr Cys Met Val Thr Asn Ser Ala Gly Asn Thr Thr Ala Ser
            400                 405                 410

Ala Thr Leu Asn Val Ser Ala Val Asp Pro Val Ala Ala Gly Gly Thr
        415                 420                 425

Gly Ser Gly Gly Gly Gly Pro Gly Gly Ser Gly Gly Val Gly Gly Gly
    430                 435                 440

Ser Gly Gly Tyr Thr Tyr Phe Thr Thr Val Thr Val Glu Thr Leu Glu
445                 450                 455                 460

Thr Gln Pro Gly Glu Glu Ala Leu Gln Pro Arg Gly Thr Glu Lys Glu
                465                 470                 475

Pro Pro Gly Pro Thr Thr Asp Gly Val Trp Gly Gly Gly Arg Pro Gly
            480                 485                 490

Asp Ala Ala Gly Pro Ala Ser Ser Ser Thr Thr Ala Pro Ala Pro Arg
        495                 500                 505

Ser Ser Arg Pro Thr Glu Lys Ala Phe Thr Val Pro Ile Thr Asp Val
    510                 515                 520

Thr Glu Asn Ala Leu Lys Asp Leu Asp Asp Val Met Lys Thr Thr Lys
525                 530                 535                 540

Ile Ile Ile Gly Cys Phe Val Ala Ile Thr Phe Met Ala Ala Val Met
                545                 550                 555

Leu Val Ala Phe Tyr Lys Leu Arg Lys Gln His Gln Leu His Lys His
            560                 565                 570

His Gly Pro Thr Arg Thr Val Glu Ile Ile Asn Val Glu Asp Glu Leu
        575                 580                 585

Pro Ala Ala Ser Ala Val Ser Val Ala Ala Ala Ala Ala Val Ala Ser
    590                 595                 600

Gly Gly Gly Val Gly Gly Asp Ser His Leu Ala Leu Pro Ala Leu Glu
605                 610                 615                 620

Arg Asp His Leu Asn His His His Tyr Val Ala Ala Ala Phe Lys Ala
                625                 630                 635

His Tyr Ser Ser Asn Pro Ser Gly Gly Gly Cys Gly Gly Lys Gly Pro
            640                 645                 650

Pro Gly Leu Asn Ser Ile His Glu Pro Leu Leu Phe Lys Ser Gly Ser
        655                 660                 665

Lys Glu Asn Val Gln Glu Thr Gln Ile
    670                 675

<210>  27

<211>  1416

<212>  DNA

<213>  Homo sapiens

<220>

<221>  CDS

<222>  (96)..(1364)

<223>

<220>

<221>  mat_peptide

<222>  (171)..()

<223>

<400>  27; LP255(a)
gtctggagat ggggggcggt ggtggccaca gcagaggctc caggaggtga ccgcctggtt      60

tccattagga agtcctggtc agcagcttgg gcgag atg gca gag tca ggg ctg      113
                                       Met Ala Glu Ser Gly Leu
                                       -25                 -20

gcc atg tgg ccg agc ctg ctg ctg ctc ctg ctg ttg ccg ggg ccc ccg      161
Ala Met Trp Pro Ser Leu Leu Leu Leu Leu Leu Leu Pro Gly Pro Pro
                -15                 -10                 -5

ccc gtc gcc ggc ttg gaa gac gct gcc ttc ccc cac ctg ggg gag agc      209

Pro Val Ala Gly Leu Glu Asp Ala Ala Phe Pro His Leu Gly Glu Ser
        -1  1               5                   10

ttg cag ccc ctg ccc cgg gcc tgt ccc ctg cgc tgc tcc tgc ccc cga     257
Leu Gln Pro Leu Pro Arg Ala Cys Pro Leu Arg Cys Ser Cys Pro Arg
    15                  20                  25

gtc gac act gtg gac tgt gat ggc ttg gac ctt cga gtg ttc ccg gac     305
Val Asp Thr Val Asp Cys Asp Gly Leu Asp Leu Arg Val Phe Pro Asp
30                  35                  40                  45

aac atc acc aga gcc gct cag cac ctc tcc ctg cag aac aac cag ctc     353
Asn Ile Thr Arg Ala Ala Gln His Leu Ser Leu Gln Asn Asn Gln Leu
                50                  55                  60

cag gaa ctc ccc tac aat gag ctg tcc cgc ctc agt ggc ctg cga acc     401
Gln Glu Leu Pro Tyr Asn Glu Leu Ser Arg Leu Ser Gly Leu Arg Thr
            65                  70                  75

ctc aac ctc cac aac aac ctc atc tcc tcc gaa ggc ctg cct gac gag     449
Leu Asn Leu His Asn Asn Leu Ile Ser Ser Glu Gly Leu Pro Asp Glu
        80                  85                  90

gcc ttc gag tcc ctc acc cag ctg cag cac ctc tgc gtg gct cac aac     497
Ala Phe Glu Ser Leu Thr Gln Leu Gln His Leu Cys Val Ala His Asn
    95                  100                 105

aag aac aat ctc atc tcc aag gtg ccc cga gga gcc ctg agc cgc cag     545
Lys Asn Asn Leu Ile Ser Lys Val Pro Arg Gly Ala Leu Ser Arg Gln
110                 115                 120                 125

act caa ctc cgt gag ctc tac ctc cag cac aac cag ctg aca gac agt     593
Thr Gln Leu Arg Glu Leu Tyr Leu Gln His Asn Gln Leu Thr Asp Ser
                130                 135                 140

ggc ctg gat gcc acc acc ttc agc aag ctg cat agc ctt gaa tac ctg     641
Gly Leu Asp Ala Thr Thr Phe Ser Lys Leu His Ser Leu Glu Tyr Leu
            145                 150                 155

gat ctc tcc cac aac cag ctg acc aca gtg ccc gcc ggc ctg ccc cgg     689
Asp Leu Ser His Asn Gln Leu Thr Thr Val Pro Ala Gly Leu Pro Arg
        160                 165                 170

acc ctg gct atc ctg cac ctg ggc cgc aac cgc atc cgg cag gtg gag     737
Thr Leu Ala Ile Leu His Leu Gly Arg Asn Arg Ile Arg Gln Val Glu
    175                 180                 185

gcg gct cgg ctg cac ggg gcg cgt ggt ctg cgc tat ttg ttg ctg cag     785
Ala Ala Arg Leu His Gly Ala Arg Gly Leu Arg Tyr Leu Leu Leu Gln
190                 195                 200                 205

cac aac cag ctg ggg agc tca ggg ctg ccc gcc ggg gct ctg cgg ccg     833
His Asn Gln Leu Gly Ser Ser Gly Leu Pro Ala Gly Ala Leu Arg Pro
                210                 215                 220

ctg cgg ggc ctg cac acg ctg cac ctc tat ggc aat ggg ctg gac cgc     881
Leu Arg Gly Leu His Thr Leu His Leu Tyr Gly Asn Gly Leu Asp Arg
            225                 230                 235

gtg cct cca gcc ctg ccc cgc cgc ctg cgt gcc ctg gtg ctg ccc cac 929
Val Pro Pro Ala Leu Pro Arg Arg Leu Arg Ala Leu Val Leu Pro His
240 245 250

aac cac gtg gcc gcg ctg ggt gcc cgt gac ctg gtc gcc aca ccg ggc 977
Asn His Val Ala Ala Leu Gly Ala Arg Asp Leu Val Ala Thr Pro Gly
255 260 265

ctg acg gag ctt aac ctg gcc tat aac cgc ctg gcc agc gcc cgt gtg 1025
Leu Thr Glu Leu Asn Leu Ala Tyr Asn Arg Leu Ala Ser Ala Arg Val
270 275 280 285

cac cac cgg gcc ttc cgc cgg ttg cgt gcc ctg cgc agc ctc gac ctg 1073
His His Arg Ala Phe Arg Arg Leu Arg Ala Leu Arg Ser Leu Asp Leu
290 295 300

gca ggg aat cag cta acc cgg ctg ccc atg ggc ctg ccc act ggc ctg 1121
Ala Gly Asn Gln Leu Thr Arg Leu Pro Met Gly Leu Pro Thr Gly Leu
305 310 315

cgc acc ctg cag ctg caa cgc aac cag ctg cgg atg ctc gag ccc gag 1169
Arg Thr Leu Gln Leu Gln Arg Asn Gln Leu Arg Met Leu Glu Pro Glu
320 325 330

cct ctg gcc ggc ctg gac caa ctg cgg gag ctc agc ctg gcg cac aac 1217
Pro Leu Ala Gly Leu Asp Gln Leu Arg Glu Leu Ser Leu Ala His Asn
335 340 345

cgg ctc cgg gtc ggc gac atc ggg cca ggc acc tgg cat gag ctc caa 1265
Arg Leu Arg Val Gly Asp Ile Gly Pro Gly Thr Trp His Glu Leu Gln
350 355 360 365

gcc ctc cag gtc agg cac agg ctg gtt agc cac act gtc ccc agg gcc 1313
Ala Leu Gln Val Arg His Arg Leu Val Ser His Thr Val Pro Arg Ala
370 375 380

cct cca tcc ccc tgc ctg ccc tgc cac gtc cca aac att cta gtt agc 1361
Pro Pro Ser Pro Cys Leu Pro Cys His Val Pro Asn Ile Leu Val Ser
385 390 395

tgg taaagcaatc agaacaagaa aatgataaga gtgggttaga aggtgatgag ga 1416
Trp

<210> 28

<211> 423

<212> PRT

<213> Homo sapiens

<400> 28; LP255(a)

Met Ala Glu Ser Gly Leu Ala Met Trp Pro Ser Leu Leu Leu Leu Leu
-25                 -20                 -15                 -10

Leu Leu Pro Gly Pro Pro Pro Val Ala Gly Leu Glu Asp Ala Ala Phe
                -5                  -1  1               5

Pro His Leu Gly Glu Ser Leu Gln Pro Leu Pro Arg Ala Cys Pro Leu
        10                  15                  20

Arg Cys Ser Cys Pro Arg Val Asp Thr Val Asp Cys Asp Gly Leu Asp
    25                  30                  35

Leu Arg Val Phe Pro Asp Asn Ile Thr Arg Ala Ala Gln His Leu Ser
40                  45                  50                  55

Leu Gln Asn Asn Gln Leu Gln Glu Leu Pro Tyr Asn Glu Leu Ser Arg
                60                  65                  70

Leu Ser Gly Leu Arg Thr Leu Asn Leu His Asn Asn Leu Ile Ser Ser
            75                  80                  85

Glu Gly Leu Pro Asp Glu Ala Phe Glu Ser Leu Thr Gln Leu Gln His
        90                  95                  100

Leu Cys Val Ala His Asn Lys Asn Asn Leu Ile Ser Lys Val Pro Arg
    105                 110                 115

Gly Ala Leu Ser Arg Gln Thr Gln Leu Arg Glu Leu Tyr Leu Gln His
120                 125                 130                 135

Asn Gln Leu Thr Asp Ser Gly Leu Asp Ala Thr Thr Phe Ser Lys Leu
                140                 145                 150

His Ser Leu Glu Tyr Leu Asp Leu Ser His Asn Gln Leu Thr Thr Val
            155                 160                 165

Pro Ala Gly Leu Pro Arg Thr Leu Ala Ile Leu His Leu Gly Arg Asn
        170                 175                 180

Arg Ile Arg Gln Val Glu Ala Ala Arg Leu His Gly Ala Arg Gly Leu
    185                 190                 195

Arg Tyr Leu Leu Leu Gln His Asn Gln Leu Gly Ser Ser Gly Leu Pro
200                 205                 210                 215

Ala Gly Ala Leu Arg Pro Leu Arg Gly Leu His Thr Leu His Leu Tyr
                220                 225                 230

Gly Asn Gly Leu Asp Arg Val Pro Pro Ala Leu Pro Arg Arg Leu Arg
            235                 240                 245

Ala Leu Val Leu Pro His Asn His Val Ala Ala Leu Gly Ala Arg Asp
        250                 255                 260

Leu Val Ala Thr Pro Gly Leu Thr Glu Leu Asn Leu Ala Tyr Asn Arg
    265                 270                 275

Leu Ala Ser Ala Arg Val His His Arg Ala Phe Arg Arg Leu Arg Ala
280                 285                 290                 295

Leu Arg Ser Leu Asp Leu Ala Gly Asn Gln Leu Thr Arg Leu Pro Met
                300                 305                 310

Gly Leu Pro Thr Gly Leu Arg Thr Leu Gln Leu Gln Arg Asn Gln Leu
            315                 320                 325

Arg Met Leu Glu Pro Glu Pro Leu Ala Gly Leu Asp Gln Leu Arg Glu
        330                 335                 340

Leu Ser Leu Ala His Asn Arg Leu Arg Val Gly Asp Ile Gly Pro Gly
    345                 350                 355

Thr Trp His Glu Leu Gln Ala Leu Gln Val Arg His Arg Leu Val Ser
360                 365                 370                 375

His Thr Val Pro Arg Ala Pro Pro Ser Pro Cys Leu Pro Cys His Val
                380                 385                 390

Pro Asn Ile Leu Val Ser Trp
            395

<210>  29

<211>  445

<212>  DNA

<213>  Homo sapiens

<220>

<221>  CDS

<222>  (25)..(330)

<223>



<220>

<221>  mat_peptide

<222>  (76)..()

<223>



<400>  29; LP244
ctgagagcag agctggccgc agcc atg acc ccg cag ctt ctc ctg gcc ctt        51
                           Met Thr Pro Gln Leu Leu Leu Ala Leu
                                   -15                 -10

gtc ctc tgg gcc agc tgc ccg ccc tgc agt gga agg aaa ggg ccc cca        99
Val Leu Trp Ala Ser Cys Pro Pro Cys Ser Gly Arg Lys Gly Pro Pro
            -5              -1  1               5

gca gct ctg aca ctg ccc cgg gtg caa tgc cga gcc tct cgg tac ccg       147
Ala Ala Leu Thr Leu Pro Arg Val Gln Cys Arg Ala Ser Arg Tyr Pro
    10                  15                  20

atc gcc gtg gat tgc tcc tgg acc ctg ccg cct gct cca aac tcc acc       195
Ile Ala Val Asp Cys Ser Trp Thr Leu Pro Pro Ala Pro Asn Ser Thr
25                  30                  35                  40

agc ccc gtg tcc ttc att gcc acg tac agg tcg gag agc ctg gaa ggg       243
Ser Pro Val Ser Phe Ile Ala Thr Tyr Arg Ser Glu Ser Leu Glu Gly
                45                  50                  55

ggc ctc agg gac act gga ctt cct gga gag ccc aga act ctg gcc ctg       291
Gly Leu Arg Asp Thr Gly Leu Pro Gly Glu Pro Arg Thr Leu Ala Leu
            60                  65                  70

aga gcc ctg ggg tca gga aaa ctg gag acc ccg aca tcc tgaaccccag        340
Arg Ala Leu Gly Ser Gly Lys Leu Glu Thr Pro Thr Ser
        75                  80                  85

gcctatggaa ttctagggcg gccccgtcca atagaaatat actttctggg ccaggaaaaa     400

aaaaaaaaaa aaaaaaaaaa aaaaaaaaaa gaaaaaaaaa aaaaa                     445


<210>  30

<211>  102

<212>  PRT

<213>  Homo sapiens

<400>  30; LP244

Met Thr Pro Gln Leu Leu Leu Ala Leu Val Leu Trp Ala Ser Cys Pro
        -15                 -10                 -5

Pro Cys Ser Gly Arg Lys Gly Pro Pro Ala Ala Leu Thr Leu Pro Arg
-1  1               5                   10                  15

Val Gln Cys Arg Ala Ser Arg Tyr Pro Ile Ala Val Asp Cys Ser Trp
                20                  25                  30

Thr Leu Pro Pro Ala Pro Asn Ser Thr Ser Pro Val Ser Phe Ile Ala
            35                  40                  45

Thr Tyr Arg Ser Glu Ser Leu Glu Gly Gly Leu Arg Asp Thr Gly Leu
        50                  55                  60

Pro Gly Glu Pro Arg Thr Leu Ala Leu Arg Ala Leu Gly Ser Gly Lys
    65                  70                  75

Leu Glu Thr Pro Thr Ser
80                  85

<210>  31

<211>  1858

<212>  DNA

<213>  Homo sapiens

<220>

<221>  CDS

<222>  (1)..(1761)

<223>

<220>

<221>  mat_peptide

<222>  (79)..()

<223>

<400>  31; LP186
atg gtc tcc cca cgg atg tcc ggg ctc ctc tcc cag act gtg atc cta      48
Met Val Ser Pro Arg Met Ser Gly Leu Leu Ser Gln Thr Val Ile Leu
    -25                 -20                 -15

gcg ctc att ttc ctc ccc cag aca cgg ccc gct ggc gtc ttc gag ctg      96
Ala Leu Ile Phe Leu Pro Gln Thr Arg Pro Ala Gly Val Phe Glu Leu
-10                 -5                  -1  1               5

cag atc cac tct ttc ggg ccg ggt cca ggc cct ggg gcc ccg cgg tcc      144
Gln Ile His Ser Phe Gly Pro Gly Pro Gly Pro Gly Ala Pro Arg Ser
            10                  15                  20

ccc tgc agc gcc cgg ctc ccc tgc cgc ctc ttc ttc aga gtc tgc ctg      192
Pro Cys Ser Ala Arg Leu Pro Cys Arg Leu Phe Phe Arg Val Cys Leu
        25                  30                  35

aag cct ggg ctc tca gag gag gcc gcc gag tcc ccg tgc gcc ctg ggc      240
Lys Pro Gly Leu Ser Glu Glu Ala Ala Glu Ser Pro Cys Ala Leu Gly
    40                  45                  50

gcg gcg ctg agt gcg cgc gga ccg gtc tac acc gag cag ccc gga gcg      288
Ala Ala Leu Ser Ala Arg Gly Pro Val Tyr Thr Glu Gln Pro Gly Ala
55                  60                  65                  70

ccc gcg cct gat ctc cca ctg ccc gac ggc ctc ttg cag gtg ccc ttc      336
Pro Ala Pro Asp Leu Pro Leu Pro Asp Gly Leu Leu Gln Val Pro Phe
                75                  80                  85

cgg gac gcc tgg cct ggc acc ttc tct ttc atc atc gaa acc tgg aga      384
Arg Asp Ala Trp Pro Gly Thr Phe Ser Phe Ile Ile Glu Thr Trp Arg
            90                  95                  100

gag gag tta gga gac cag att gga ggg ccc gcc tgg agc ctg ctg gcg      432
Glu Glu Leu Gly Asp Gln Ile Gly Gly Pro Ala Trp Ser Leu Leu Ala
        105                 110                 115

cgc gtg gct ggc agg cgg cgc ttg gca gcc gga ggc ccg tgg gcc cgg      480
Arg Val Ala Gly Arg Arg Arg Leu Ala Ala Gly Gly Pro Trp Ala Arg
    120                 125                 130

gac att cag cgc gca ggc gcc tgg gag ctg cgc tgc tcg tac cgc gcg      528
Asp Ile Gln Arg Ala Gly Ala Trp Glu Leu Arg Cys Ser Tyr Arg Ala
135                 140                 145                 150

cgc tgc gag ccg cct gcg gtc ggg acc gcg tgc acg cgc ctc tgc cgt      576
Arg Cys Glu Pro Pro Ala Val Gly Thr Ala Cys Thr Arg Leu Cys Arg
                155                 160                 165

ccg cgc agc gcc ccc tcg cgg tgc ggt ccg gga ctg cgc ccc tgc gca 624
Pro Arg Ser Ala Pro Ser Arg Cys Gly Pro Gly Leu Arg Pro Cys Ala
170 175 180

ccg ctc gag gac gaa tgt gag gcg ccg ccg gtg tgc cga gca ggc tgc 672
Pro Leu Glu Asp Glu Cys Glu Ala Pro Pro Val Cys Arg Ala Gly Cys
185 190 195

agc cct gag cat ggc ttc tgt gaa cag ccc ggt gaa tgc cga tgc cta 720
Ser Pro Glu His Gly Phe Cys Glu Gln Pro Gly Glu Cys Arg Cys Leu
200 205 210

gag ggc tgg act gga ccc ctc tgc acg gtc cct gtc tcc acc agc agc 768
Glu Gly Trp Thr Gly Pro Leu Cys Thr Val Pro Val Ser Thr Ser Ser
215 220 225 230

tgc ctc agc ccc agg ggc ccg tcc tct gct acc acc gga tgc ctt gtc 816
Cys Leu Ser Pro Arg Gly Pro Ser Ser Ala Thr Thr Gly Cys Leu Val
235 240 245

cct ggg cct ggg ccc tgt gac ggg aac ccg tgt gcc aat gga ggc agc 864
Pro Gly Pro Gly Pro Cys Asp Gly Asn Pro Cys Ala Asn Gly Gly Ser
250 255 260

tgt agt gag aca ccc agg tcc ttt gaa tgc acc tgc ccg cgt ggg ttc 912
Cys Ser Glu Thr Pro Arg Ser Phe Glu Cys Thr Cys Pro Arg Gly Phe
265 270 275

tac ggg ctg cgg tgt gag gtg agc ggg gtg aca tgt gca gat gga ccc 960
Tyr Gly Leu Arg Cys Glu Val Ser Gly Val Thr Cys Ala Asp Gly Pro
280 285 290

tgc ttc aac ggc ggc ttg tgt gtc ggg ggt gca gac cct gac tct gcc 1008
Cys Phe Asn Gly Gly Leu Cys Val Gly Gly Ala Asp Pro Asp Ser Ala
295 300 305 310

tac atc tgc cac tgc cca cct ggt ttc caa ggc tcc aac tgt gag aag 1056
Tyr Ile Cys His Cys Pro Pro Gly Phe Gln Gly Ser Asn Cys Glu Lys
315 320 325

agg gtg gac cgg tgc agc ctg cag cca tgc cgc aat ggc gga ctc tgc 1104
Arg Val Asp Arg Cys Ser Leu Gln Pro Cys Arg Asn Gly Gly Leu Cys
330 335 340

ctg gac ctg ggc cac gcc ctg cgc tgc cgc tgc cgc gcc ggc ttc gcg 1152
Leu Asp Leu Gly His Ala Leu Arg Cys Arg Cys Arg Ala Gly Phe Ala
345 350 355

ggt cct cgc tgc gag cac gac ctg gac gac tgc gcg ggc cgc gcc tgc 1200
Gly Pro Arg Cys Glu His Asp Leu Asp Asp Cys Ala Gly Arg Ala Cys
360 365 370

gct aac ggc ggc acg tgt gtg gag ggc ggc ggc gcg cac cgc tgc tcc 1248
Ala Asn Gly Gly Thr Cys Val Glu Gly Gly Gly Ala His Arg Cys Ser
375 380 385 390

tgc gcg ctg ggc ttc ggc ggc cgc gac tgc cgc gag cgc gcg gac ccg 1296

Cys Ala Leu Gly Phe Gly Gly Arg Asp Cys Arg Glu Arg Ala Asp Pro
                395                 400                 405

tgc gcc gcg cgc ccc tgt gct cac ggc ggc cgc tgc tac gcc cac ttc      1344
Cys Ala Ala Arg Pro Cys Ala His Gly Gly Arg Cys Tyr Ala His Phe
            410                 415                 420

tcc ggc ctc gtc tgc gct tgc gct ccc ggc tac atg gga gcg cgg tgt      1392
Ser Gly Leu Val Cys Ala Cys Ala Pro Gly Tyr Met Gly Ala Arg Cys
        425                 430                 435

gag ttc cca gtg cac ccc gac ggc gca agc gcc ttg ccc gcg gcc ccg      1440
Glu Phe Pro Val His Pro Asp Gly Ala Ser Ala Leu Pro Ala Ala Pro
    440                 445                 450

ccg ggc ctc agg ccc ggg gac cct cag cgc tac ctt ttg cct ccg gct      1488
Pro Gly Leu Arg Pro Gly Asp Pro Gln Arg Tyr Leu Leu Pro Pro Ala
455                 460                 465                 470

ctg gga ctg ctc gtg gcc gcg ggc gtg gcc ggc gct gcg ctc ttg ctg      1536
Leu Gly Leu Leu Val Ala Ala Gly Val Ala Gly Ala Ala Leu Leu Leu
                475                 480                 485

gtc cac gtg cgc cgc cgt ggc cac tcc cag gat gct ggg tct cgc ttg      1584
Val His Val Arg Arg Arg Gly His Ser Gln Asp Ala Gly Ser Arg Leu
            490                 495                 500

ctg gct ggg acc ccg gag ccg tca gtc cac gca ctc ccg gat gca ctc      1632
Leu Ala Gly Thr Pro Glu Pro Ser Val His Ala Leu Pro Asp Ala Leu
        505                 510                 515

aac aac cta agg acg cag gag ggt tcc ggg gat ggt ccg agc tcg tcc      1680
Asn Asn Leu Arg Thr Gln Glu Gly Ser Gly Asp Gly Pro Ser Ser Ser
    520                 525                 530

gta gat tgg aat cgc cct gaa gat gta gac cct caa ggg att tat gtc      1728
Val Asp Trp Asn Arg Pro Glu Asp Val Asp Pro Gln Gly Ile Tyr Val
535                 540                 545                 550

ata tct gct cct tcc atc tac gct cgg gag gcc tgacgcgtct cctccatccg    1781
Ile Ser Ala Pro Ser Ile Tyr Ala Arg Glu Ala
                555                 560

cacctggagt cagagcgtgg atttttgtat ttgctcggtg gtgcccagtc tctgccccag    1841

aggctttgga gttcaat                                                   1858

<210>  32

<211>  587

<212>  PRT

<213>  Homo sapiens

<400> 32; LP186

Met Val Ser Pro Arg Met Ser Gly Leu Leu Ser Gln Thr Val Ile Leu
    -25                 -20                 -15

Ala Leu Ile Phe Leu Pro Gln Thr Arg Pro Ala Gly Val Phe Glu Leu
-10                 -5              -1  1               5

Gln Ile His Ser Phe Gly Pro Gly Pro Gly Pro Gly Ala Pro Arg Ser
            10                  15                  20

Pro Cys Ser Ala Arg Leu Pro Cys Arg Leu Phe Phe Arg Val Cys Leu
        25                  30                  35

Lys Pro Gly Leu Ser Glu Glu Ala Ala Glu Ser Pro Cys Ala Leu Gly
    40                  45                  50

Ala Ala Leu Ser Ala Arg Gly Pro Val Tyr Thr Glu Gln Pro Gly Ala
55                  60                  65                  70

Pro Ala Pro Asp Leu Pro Leu Pro Asp Gly Leu Leu Gln Val Pro Phe
                75                  80                  85

Arg Asp Ala Trp Pro Gly Thr Phe Ser Phe Ile Ile Glu Thr Trp Arg
            90                  95                  100

Glu Glu Leu Gly Asp Gln Ile Gly Gly Pro Ala Trp Ser Leu Leu Ala
        105                 110                 115

Arg Val Ala Gly Arg Arg Arg Leu Ala Ala Gly Gly Pro Trp Ala Arg
    120                 125                 130

Asp Ile Gln Arg Ala Gly Ala Trp Glu Leu Arg Cys Ser Tyr Arg Ala
135                 140                 145                 150

Arg Cys Glu Pro Pro Ala Val Gly Thr Ala Cys Thr Arg Leu Cys Arg
                155                 160                 165

Pro Arg Ser Ala Pro Ser Arg Cys Gly Pro Gly Leu Arg Pro Cys Ala
            170                 175                 180

Pro Leu Glu Asp Glu Cys Glu Ala Pro Pro Val Cys Arg Ala Gly Cys
        185                 190                 195

Ser Pro Glu His Gly Phe Cys Glu Gln Pro Gly Glu Cys Arg Cys Leu
    200                 205                 210

Glu Gly Trp Thr Gly Pro Leu Cys Thr Val Pro Val Ser Thr Ser Ser
215                 220                 225                 230

Cys Leu Ser Pro Arg Gly Pro Ser Ser Ala Thr Thr Gly Cys Leu Val
                235                 240                 245

Pro Gly Pro Gly Pro Cys Asp Gly Asn Pro Cys Ala Asn Gly Gly Ser
            250                 255                 260

Cys Ser Glu Thr Pro Arg Ser Phe Glu Cys Thr Cys Pro Arg Gly Phe
        265                 270                 275

Tyr Gly Leu Arg Cys Glu Val Ser Gly Val Thr Cys Ala Asp Gly Pro
    280                 285                 290

Cys Phe Asn Gly Gly Leu Cys Val Gly Gly Ala Asp Pro Asp Ser Ala
295                 300                 305                 310

Tyr Ile Cys His Cys Pro Pro Gly Phe Gln Gly Ser Asn Cys Glu Lys
                315                 320                 325

Arg Val Asp Arg Cys Ser Leu Gln Pro Cys Arg Asn Gly Gly Leu Cys
            330                 335                 340

Leu Asp Leu Gly His Ala Leu Arg Cys Arg Cys Arg Ala Gly Phe Ala
        345                 350                 355

Gly Pro Arg Cys Glu His Asp Leu Asp Asp Cys Ala Gly Arg Ala Cys
    360                 365                 370

Ala Asn Gly Gly Thr Cys Val Glu Gly Gly Gly Ala His Arg Cys Ser
375                 380                 385                 390

Cys Ala Leu Gly Phe Gly Gly Arg Asp Cys Arg Glu Arg Ala Asp Pro
                395                 400                 405

Cys Ala Ala Arg Pro Cys Ala His Gly Gly Arg Cys Tyr Ala His Phe
            410                 415                 420

Ser Gly Leu Val Cys Ala Cys Ala Pro Gly Tyr Met Gly Ala Arg Cys
        425                 430                 435

Glu Phe Pro Val His Pro Asp Gly Ala Ser Ala Leu Pro Ala Ala Pro
    440                 445                 450

Pro Gly Leu Arg Pro Gly Asp Pro Gln Arg Tyr Leu Leu Pro Pro Ala
455                 460                 465                 470

Leu Gly Leu Leu Val Ala Ala Gly Val Ala Gly Ala Ala Leu Leu Leu
                475                 480                 485

Val His Val Arg Arg Arg Gly His Ser Gln Asp Ala Gly Ser Arg Leu
            490                 495                 500

Leu Ala Gly Thr Pro Glu Pro Ser Val His Ala Leu Pro Asp Ala Leu
        505                 510                 515

Asn Asn Leu Arg Thr Gln Glu Gly Ser Gly Asp Gly Pro Ser Ser Ser
    520                 525                 530

Val Asp Trp Asn Arg Pro Glu Asp Val Asp Pro Gln Gly Ile Tyr Val
535                 540                 545                 550

Ile Ser Ala Pro Ser Ile Tyr Ala Arg Glu Ala
                555                 560

<210>  33

<211>  1968

<212>  DNA

<213>  Homo sapiens

<220>

<221>  CDS

<222>  (25)..(1926)

<223>

<220>

<221>  mat_peptide

<222>  (79)..()

<223>

<400>  33; LP251(b)
acagcaacct ctcccctggc cctc atg ggc acc gtc agc tcc agg cgg tcc        51
                           Met Gly Thr Val Ser Ser Arg Arg Ser
                                       -15                 -10

tgg tgg ccg ctg cca ctg ctg ctg ctg ctg ctg ctg ctc ctg ggt ccc        99
Trp Trp Pro Leu Pro Leu Leu Leu Leu Leu Leu Leu Leu Leu Gly Pro
                -5                  -1  1               5

gcg ggc gcc cgt gcg cag gag gac gag gac ggc gac tac gag gag ctg       147
Ala Gly Ala Arg Ala Gln Glu Asp Glu Asp Gly Asp Tyr Glu Glu Leu
        10                  15                  20

gtg cta gcc ttg cgt tcc gag gag gac ggc ctg gcc gaa gca ccc gag       195
Val Leu Ala Leu Arg Ser Glu Glu Asp Gly Leu Ala Glu Ala Pro Glu
    25                  30                  35

cac gga acc aca gcc acc ttc cac cgc tgc gcc aag gat ccg tgg agg       243
His Gly Thr Thr Ala Thr Phe His Arg Cys Ala Lys Asp Pro Trp Arg
40                  45                  50                  55

ttg cct ggc acc tac gtg gtg gtg ctg aag gag gag acc cac ctc tcg       291
Leu Pro Gly Thr Tyr Val Val Val Leu Lys Glu Glu Thr His Leu Ser
                60                  65                  70

cag tca gag cgc act gcc cgc cgc ctg cag gcc cag gct gcc cgc cgg       339
Gln Ser Glu Arg Thr Ala Arg Arg Leu Gln Ala Gln Ala Ala Arg Arg
            75                  80                  85

gga tac ctc acc aag atc ctg cat gtc ttc cat ggc ctt ctt cct ggc       387
Gly Tyr Leu Thr Lys Ile Leu His Val Phe His Gly Leu Leu Pro Gly
        90                  95                  100

ttc ctg gtg aag atg agt ggc gac ctg ctg gag ctg gcc ttg aag ttg       435
Phe Leu Val Lys Met Ser Gly Asp Leu Leu Glu Leu Ala Leu Lys Leu
    105                 110                 115

ccc cat gtc gac tac atc gag gag gac tcc tct gtc ttt gcc cag agc       483
Pro His Val Asp Tyr Ile Glu Glu Asp Ser Ser Val Phe Ala Gln Ser
120                 125                 130                 135

atc ccg tgg aac ctg gag cgg att acc cct cca cgg tac cgg gcg gat       531
Ile Pro Trp Asn Leu Glu Arg Ile Thr Pro Pro Arg Tyr Arg Ala Asp
                140                 145                 150

gaa tac cag ccc ccc gac gga ggc agc ctg gtg gag gtg tat ctc cta       579
Glu Tyr Gln Pro Pro Asp Gly Gly Ser Leu Val Glu Val Tyr Leu Leu
            155                 160                 165

gac acc agc ata cag agt gac cac cgg gaa atc gag ggc agg gtc atg       627
Asp Thr Ser Ile Gln Ser Asp His Arg Glu Ile Glu Gly Arg Val Met
        170                 175                 180

gtc acc gac ttc gag aat gtg ccc gag gag gac ggg acc cgc ttc cac     675
Val Thr Asp Phe Glu Asn Val Pro Glu Glu Asp Gly Thr Arg Phe His
    185                 190                 195

aga cag gcc agc aag tgt gac agt cat ggc acc cac ctg gca ggg gtg     723
Arg Gln Ala Ser Lys Cys Asp Ser His Gly Thr His Leu Ala Gly Val
200                 205                 210                 215

gtc agc ggc cgg gat gcc ggc gtg gcc aag ggt gcc agc atg cgc agc     771
Val Ser Gly Arg Asp Ala Gly Val Ala Lys Gly Ala Ser Met Arg Ser
                220                 225                 230

ctg cgc gtg ctc aac tgc caa ggg aag ggc acg gtt agc ggc acc ctc     819
Leu Arg Val Leu Asn Cys Gln Gly Lys Gly Thr Val Ser Gly Thr Leu
            235                 240                 245

ata ggc ctg gag ttt att cgg aaa agc cag ctg gtc cag cct gtg ggg     867
Ile Gly Leu Glu Phe Ile Arg Lys Ser Gln Leu Val Gln Pro Val Gly
        250                 255                 260

cca ctg gtg gtg ctg ctg ccc ctg gcg ggt ggg tac agc cgc gtc ctc     915
Pro Leu Val Val Leu Leu Pro Leu Ala Gly Gly Tyr Ser Arg Val Leu
    265                 270                 275

aac gcc gcc tgc cag cgc ctg gcg agg gct ggg gtc gtg ctg gtc acc     963
Asn Ala Ala Cys Gln Arg Leu Ala Arg Ala Gly Val Val Leu Val Thr
280                 285                 290                 295

gct gcc ggc aac ttc cgg gac gat gcc tgc ctc tac tcc cca gcc tca     1011
Ala Ala Gly Asn Phe Arg Asp Asp Ala Cys Leu Tyr Ser Pro Ala Ser
                300                 305                 310

gct ccc gag gtc atc aca gtt ggg gcc acc aat gcc cag gac cag ccg     1059
Ala Pro Glu Val Ile Thr Val Gly Ala Thr Asn Ala Gln Asp Gln Pro
            315                 320                 325

gtg acc ctg ggg act ttg ggg acc aac ttt ggc cgc tgt gtg gac ctc     1107
Val Thr Leu Gly Thr Leu Gly Thr Asn Phe Gly Arg Cys Val Asp Leu
        330                 335                 340

ttt gcc cca ggg gag gac atc att ggt gcc tcc agc gac tgc agc acc     1155
Phe Ala Pro Gly Glu Asp Ile Ile Gly Ala Ser Ser Asp Cys Ser Thr
    345                 350                 355

tgc ttt gtg tca cag agt ggg aca tca cag gct gct gcc cac gtg gct     1203
Cys Phe Val Ser Gln Ser Gly Thr Ser Gln Ala Ala Ala His Val Ala
360                 365                 370                 375

ggt tgg cag ctg ttt tgc agg act gtg tgg tca gca cac tcg ggg cct     1251
Gly Trp Gln Leu Phe Cys Arg Thr Val Trp Ser Ala His Ser Gly Pro
                380                 385                 390

aca cgg atg gcc aca gcc atc gcc cgc tgc gcc cca gat gag gag ctg     1299
Thr Arg Met Ala Thr Ala Ile Ala Arg Cys Ala Pro Asp Glu Glu Leu
            395                 400                 405

ctg agc tgc tcc agt ttc tcc agg agt ggg aag cgg cgg ggc gag cgc     1347

Leu Ser Cys Ser Ser Phe Ser Arg Ser Gly Lys Arg Arg Gly Glu Arg
        410                 415                 420

atg gag gcc caa ggg ggc aag ctg gtc tgc cgg gcc cac aac gct ttt      1395
Met Glu Ala Gln Gly Gly Lys Leu Val Cys Arg Ala His Asn Ala Phe
    425                 430                 435

ggg ggt gag ggt gtc tac gcc att gcc agg tgc tgc ctg cta ccc cag      1443
Gly Gly Glu Gly Val Tyr Ala Ile Ala Arg Cys Cys Leu Leu Pro Gln
440                 445                 450                 455

gcc aac tgc agc gtc cac aca gct cca cca gct gag gcc agc atg ggg      1491
Ala Asn Cys Ser Val His Thr Ala Pro Pro Ala Glu Ala Ser Met Gly
                460                 465                 470

acc cgt gtc cac tgc cac caa cag ggc cac gtc ctc aca ggc tgc agc      1539
Thr Arg Val His Cys His Gln Gln Gly His Val Leu Thr Gly Cys Ser
            475                 480                 485

tcc cac tgg gag gtg gag gac ctt ggc acc cac aag ccg cct gtg ctg      1587
Ser His Trp Glu Val Glu Asp Leu Gly Thr His Lys Pro Pro Val Leu
        490                 495                 500

agg cca cga ggt cag ccc aac cag tgc gtg ggc cac agg gag gcc agc      1635
Arg Pro Arg Gly Gln Pro Asn Gln Cys Val Gly His Arg Glu Ala Ser
    505                 510                 515

atc cac gct tcc tgc tgc cat gcc cca ggt ctg gaa tgc aaa gtc aag      1683
Ile His Ala Ser Cys Cys His Ala Pro Gly Leu Glu Cys Lys Val Lys
520                 525                 530                 535

gag cat gga atc ccg gcc cct cag gag cag gtg acc gtg gcc tgc gag      1731
Glu His Gly Ile Pro Ala Pro Gln Glu Gln Val Thr Val Ala Cys Glu
                540                 545                 550

gag ggc tgg acc ctg act ggc tgc agt gcc ctc cct ggg acc tcc cac      1779
Glu Gly Trp Thr Leu Thr Gly Cys Ser Ala Leu Pro Gly Thr Ser His
            555                 560                 565

gtc ctg ggg gcc tac gcc gta gac aac acg tgt gta gtc agg agc cgg      1827
Val Leu Gly Ala Tyr Ala Val Asp Asn Thr Cys Val Val Arg Ser Arg
        570                 575                 580

gac gtc agc act aca ggc agc acc agc gaa ggg gcc gtg aca gcc gtt      1875
Asp Val Ser Thr Thr Gly Ser Thr Ser Glu Gly Ala Val Thr Ala Val
    585                 590                 595

gcc atc tgc tgc cgg agc cgg cac ctg gcg cag gcc tcc cag gag ctc      1923
Ala Ile Cys Cys Arg Ser Arg His Leu Ala Gln Ala Ser Gln Glu Leu
600                 605                 610                 615

cag tgacagcccc atcccaggat gggtgtctgg ggagggtcaa gg                   1968
Gln

<210>  34

<211>  634

<212>  PRT

<213>  Homo sapiens

<400>  34; LP251(b)

Met Gly Thr Val Ser Ser Arg Arg Ser Trp Trp Pro Leu Pro Leu Leu
            -15                 -10                 -5

Leu Leu Leu Leu Leu Leu Leu Gly Pro Ala Gly Ala Arg Ala Gln Glu
    -1  1               5                   10

Asp Glu Asp Gly Asp Tyr Glu Glu Leu Val Leu Ala Leu Arg Ser Glu
15                  20                  25                  30

Glu Asp Gly Leu Ala Glu Ala Pro Glu His Gly Thr Thr Ala Thr Phe
                35                  40                  45

His Arg Cys Ala Lys Asp Pro Trp Arg Leu Pro Gly Thr Tyr Val Val
            50                  55                  60

Val Leu Lys Glu Glu Thr His Leu Ser Gln Ser Glu Arg Thr Ala Arg
        65                  70                  75

Arg Leu Gln Ala Gln Ala Ala Arg Arg Gly Tyr Leu Thr Lys Ile Leu
    80                  85                  90

His Val Phe His Gly Leu Leu Pro Gly Phe Leu Val Lys Met Ser Gly
95                  100                 105                 110

Asp Leu Leu Glu Leu Ala Leu Lys Leu Pro His Val Asp Tyr Ile Glu
                115                 120                 125

Glu Asp Ser Ser Val Phe Ala Gln Ser Ile Pro Trp Asn Leu Glu Arg
            130                 135                 140

Ile Thr Pro Pro Arg Tyr Arg Ala Asp Glu Tyr Gln Pro Pro Asp Gly
        145                 150                 155

Gly Ser Leu Val Glu Val Tyr Leu Leu Asp Thr Ser Ile Gln Ser Asp
    160                 165                 170

His Arg Glu Ile Glu Gly Arg Val Met Val Thr Asp Phe Glu Asn Val
175                 180                 185                 190

Pro Glu Glu Asp Gly Thr Arg Phe His Arg Gln Ala Ser Lys Cys Asp
                195                 200                 205

Ser His Gly Thr His Leu Ala Gly Val Val Ser Gly Arg Asp Ala Gly
            210                 215                 220

Val Ala Lys Gly Ala Ser Met Arg Ser Leu Arg Val Leu Asn Cys Gln
        225                 230                 235

Gly Lys Gly Thr Val Ser Gly Thr Leu Ile Gly Leu Glu Phe Ile Arg
    240                 245                 250

Lys Ser Gln Leu Val Gln Pro Val Gly Pro Leu Val Val Leu Leu Pro
255                 260                 265                 270

Leu Ala Gly Gly Tyr Ser Arg Val Leu Asn Ala Ala Cys Gln Arg Leu
                275                 280                 285

Ala Arg Ala Gly Val Val Leu Val Thr Ala Ala Gly Asn Phe Arg Asp
            290                 295                 300

Asp Ala Cys Leu Tyr Ser Pro Ala Ser Ala Pro Glu Val Ile Thr Val
        305                 310                 315

Gly Ala Thr Asn Ala Gln Asp Gln Pro Val Thr Leu Gly Thr Leu Gly
    320                 325                 330

Thr Asn Phe Gly Arg Cys Val Asp Leu Phe Ala Pro Gly Glu Asp Ile
335                 340                 345                 350

Ile Gly Ala Ser Ser Asp Cys Ser Thr Cys Phe Val Ser Gln Ser Gly
                355                 360                 365

Thr Ser Gln Ala Ala Ala His Val Ala Gly Trp Gln Leu Phe Cys Arg
            370                 375                 380

Thr Val Trp Ser Ala His Ser Gly Pro Thr Arg Met Ala Thr Ala Ile
        385                 390                 395

Ala Arg Cys Ala Pro Asp Glu Glu Leu Leu Ser Cys Ser Ser Phe Ser
    400                 405                 410

Arg Ser Gly Lys Arg Arg Gly Glu Arg Met Glu Ala Gln Gly Gly Lys
415                 420                 425                 430

Leu Val Cys Arg Ala His Asn Ala Phe Gly Gly Glu Gly Val Tyr Ala
                435                 440                 445

Ile Ala Arg Cys Cys Leu Leu Pro Gln Ala Asn Cys Ser Val His Thr
            450                 455                 460

Ala Pro Pro Ala Glu Ala Ser Met Gly Thr Arg Val His Cys His Gln
        465                 470                 475

Gln Gly His Val Leu Thr Gly Cys Ser Ser His Trp Glu Val Glu Asp
    480                 485                 490

Leu Gly Thr His Lys Pro Pro Val Leu Arg Pro Arg Gly Gln Pro Asn
495                 500                 505                 510

Gln Cys Val Gly His Arg Glu Ala Ser Ile His Ala Ser Cys Cys His
                515                 520                 525

Ala Pro Gly Leu Glu Cys Lys Val Lys Glu His Gly Ile Pro Ala Pro
            530                 535                 540

Gln Glu Gln Val Thr Val Ala Cys Glu Glu Gly Trp Thr Leu Thr Gly
        545                 550                 555

Cys Ser Ala Leu Pro Gly Thr Ser His Val Leu Gly Ala Tyr Ala Val
    560                 565                 570

Asp Asn Thr Cys Val Val Arg Ser Arg Asp Val Ser Thr Thr Gly Ser
575                 580                 585                 590

Thr Ser Glu Gly Ala Val Thr Ala Val Ala Ile Cys Cys Arg Ser Arg
                595                 600                 605

His Leu Ala Gln Ala Ser Gln Glu Leu Gln
            610                 615

<210>  35

<211>  1507

<212>  DNA

<213>  Homo sapiens

<220>

<221>  CDS

<222>  (54)..(1454)

<223>

<220>

<221>  mat_peptide

<222>  (129)..()

<223>

<400>  35; LP255(b)
ggaggtgacc gcctggtttc cattaggaag tcctggtcag cagcttgggc gag atg        56
                                                           Met
                                                           -25

gca gag tca ggg ctg gcc atg tgg ccg agc ctg ctg ctg ctc ctg ctg       104
Ala Glu Ser Gly Leu Ala Met Trp Pro Ser Leu Leu Leu Leu Leu Leu
                -20                 -15                 -10

ttg ccg ggg ccc ccg ccc gtc gcc ggc ttg gaa gac gct gcc ttc ccc       152
Leu Pro Gly Pro Pro Pro Val Ala Gly Leu Glu Asp Ala Ala Phe Pro
            -5                  -1  1               5

cac ctg ggg gag agc ttg cag ccc ctg ccc cgg gcc tgt ccc ctg cgc       200
His Leu Gly Glu Ser Leu Gln Pro Leu Pro Arg Ala Cys Pro Leu Arg
    10                  15                  20

tgc tcc tgc ccc cga gtc gac act gtg gac tgt gat ggc ttg gac ctt       248
Cys Ser Cys Pro Arg Val Asp Thr Val Asp Cys Asp Gly Leu Asp Leu
25                  30                  35                  40

cga gtg ttc ccg gac aac atc acc aga gcc gct cag cac ctc tcc ctg       296
Arg Val Phe Pro Asp Asn Ile Thr Arg Ala Ala Gln His Leu Ser Leu
                45                  50                  55

cag aac aac cag ctc cag gaa ctc ccc tac aat gag ctg tcc cgc ctc       344
Gln Asn Asn Gln Leu Gln Glu Leu Pro Tyr Asn Glu Leu Ser Arg Leu
            60                  65                  70

agt ggc ctg cga acc ctc aac ctc cac aac aac ctc atc ttc tcc gaa       392
Ser Gly Leu Arg Thr Leu Asn Leu His Asn Asn Leu Ile Phe Ser Glu
        75                  80                  85

ggc ctg cct gac gag gcc ttc gag tcc ctc acc cag ctg cag cac ctc 440
Gly Leu Pro Asp Glu Ala Phe Glu Ser Leu Thr Gln Leu Gln His Leu
90 95 100

tgc gtg gct cac aac aag ctg agc aac gct ggc ctg ccc ccc gac gcc 488
Cys Val Ala His Asn Lys Leu Ser Asn Ala Gly Leu Pro Pro Asp Ala
105 110 115 120

ttc cgc ggc tcc gag gcc atc gcc acc ctc agc ctc tcc aac aac cag 536
Phe Arg Gly Ser Glu Ala Ile Ala Thr Leu Ser Leu Ser Asn Asn Gln
125 130 135

ctc agc tac ctg ccg ccc agc ctg ccg ccc tca ctc gag cgg ctc cac 584
Leu Ser Tyr Leu Pro Pro Ser Leu Pro Pro Ser Leu Glu Arg Leu His
140 145 150

ctg cag aac aat ctc atc tcc aag gtg ccc cga gga gcc ctg agc cgc 632
Leu Gln Asn Asn Leu Ile Ser Lys Val Pro Arg Gly Ala Leu Ser Arg
155 160 165

cag act caa ctc cgt gag ctc tac ctc cag cac aac cag ctg aca gac 680
Gln Thr Gln Leu Arg Glu Leu Tyr Leu Gln His Asn Gln Leu Thr Asp
170 175 180

agt ggc ctg gat gcc acc acc ttc agc aag ctg cat agc ctt gaa tac 728
Ser Gly Leu Asp Ala Thr Thr Phe Ser Lys Leu His Ser Leu Glu Tyr
185 190 195 200

ctg gat ctc tcc cac aac cag ctg acc aca gtg ccc gcc ggc ctg ccc 776
Leu Asp Leu Ser His Asn Gln Leu Thr Thr Val Pro Ala Gly Leu Pro
205 210 215

cgg acc ctg gct atc ctg cac ctg ggc cgc aac cgc atc cgg cag gtg 824
Arg Thr Leu Ala Ile Leu His Leu Gly Arg Asn Arg Ile Arg Gln Val
220 225 230

gag gcg gct cgg ctg cac ggg gcg cgt ggt ctg cgc tat ttg ttg ctg 872
Glu Ala Ala Arg Leu His Gly Ala Arg Gly Leu Arg Tyr Leu Leu Leu
235 240 245

cag cac aac cag ctg ggg agc tca ggg ctg ccc gcc ggg gct ctg cgg 920
Gln His Asn Gln Leu Gly Ser Ser Gly Leu Pro Ala Gly Ala Leu Arg
250 255 260

ccg ctg cgg ggc ctg cac acg ctg cac ctc tat ggc aat ggg ctg gac 968
Pro Leu Arg Gly Leu His Thr Leu His Leu Tyr Gly Asn Gly Leu Asp
265 270 275 280

cgc gtg cct cca gcc ctg ccc cgc cgc ctg cgt gcc ctg gtg ctg ccc 1016
Arg Val Pro Pro Ala Leu Pro Arg Arg Leu Arg Ala Leu Val Leu Pro
285 290 295

cac aac cac gtg gcc gcg ctg ggt gcc cgt gac ctg gtc gcc aca ccg 1064
His Asn His Val Ala Ala Leu Gly Ala Arg Asp Leu Val Ala Thr Pro
300 305 310

ggc ctg acg gag ctt aac ctg gcc tat aac cgc ctg gcc agc gcc cgt 1112

Gly Leu Thr Glu Leu Asn Leu Ala Tyr Asn Arg Leu Ala Ser Ala Arg
315 320 325

gtg cac cac cgg gcc ttc cgc cgg ttg cgt gcc ctg cgc agc ctc gac 1160
Val His His Arg Ala Phe Arg Arg Leu Arg Ala Leu Arg Ser Leu Asp
330 335 340

ctg gca ggg aat cag cta acc cgg ctg ccc atg ggc ctg ccc act ggc 1208
Leu Ala Gly Asn Gln Leu Thr Arg Leu Pro Met Gly Leu Pro Thr Gly
345 350 355 360

ctg cgc acc ctg cag ctg caa cgc aac cag ctg cgg atg ctc gag ccc 1256
Leu Arg Thr Leu Gln Leu Gln Arg Asn Gln Leu Arg Met Leu Glu Pro
365 370 375

gag cct ctg gcc ggc ctg gac caa ctg cgg gag ctc agc ctg gcg cac 1304
Glu Pro Leu Ala Gly Leu Asp Gln Leu Arg Glu Leu Ser Leu Ala His
380 385 390

aac cgg ctc cgg gtc ggc gac atc ggg cca ggc acc tgg cat gag ctc 1352
Asn Arg Leu Arg Val Gly Asp Ile Gly Pro Gly Thr Trp His Glu Leu
395 400 405

caa gcc ctc cag gtc agg cac agg ctg gtt agc cac act gtc ccc agg 1400
Gln Ala Leu Gln Val Arg His Arg Leu Val Ser His Thr Val Pro Arg
410 415 420

gcc cct cca tcc ccc tgc ctg ccc tgc cac gtc cca aac att cta gtt 1448
Ala Pro Pro Ser Pro Cys Leu Pro Cys His Val Pro Asn Ile Leu Val
425 430 435 440

agc tgg taaagcaatc agaacaagaa aatgataaga gtgggttaga aggtgataag 1504
Ser Trp

ggc 1507

<210> 36

<211> 467

<212> PRT

<213> Homo sapiens

<400> 36; LP255(b)

Met Ala Glu Ser Gly Leu Ala Met Trp Pro Ser Leu Leu Leu Leu Leu
-25 -20 -15 -10

Leu Leu Pro Gly Pro Pro Pro Val Ala Gly Leu Glu Asp Ala Ala Phe
-5 -1 1 5

Pro His Leu Gly Glu Ser Leu Gln Pro Leu Pro Arg Ala Cys Pro Leu
        10                  15                  20

Arg Cys Ser Cys Pro Arg Val Asp Thr Val Asp Cys Asp Gly Leu Asp
    25                  30                  35

Leu Arg Val Phe Pro Asp Asn Ile Thr Arg Ala Ala Gln His Leu Ser
40                  45                  50                  55

Leu Gln Asn Asn Gln Leu Gln Glu Leu Pro Tyr Asn Glu Leu Ser Arg
                60                  65                  70

Leu Ser Gly Leu Arg Thr Leu Asn Leu His Asn Asn Leu Ile Phe Ser
            75                  80                  85

Glu Gly Leu Pro Asp Glu Ala Phe Glu Ser Leu Thr Gln Leu Gln His
        90                  95                  100

Leu Cys Val Ala His Asn Lys Leu Ser Asn Ala Gly Leu Pro Pro Asp
    105                 110                 115

Ala Phe Arg Gly Ser Glu Ala Ile Ala Thr Leu Ser Leu Ser Asn Asn
120                 125                 130                 135

Gln Leu Ser Tyr Leu Pro Pro Ser Leu Pro Pro Ser Leu Glu Arg Leu
                140                 145                 150

His Leu Gln Asn Asn Leu Ile Ser Lys Val Pro Arg Gly Ala Leu Ser
            155                 160                 165

Arg Gln Thr Gln Leu Arg Glu Leu Tyr Leu Gln His Asn Gln Leu Thr
        170                 175                 180

Asp Ser Gly Leu Asp Ala Thr Thr Phe Ser Lys Leu His Ser Leu Glu
    185                 190                 195

Tyr Leu Asp Leu Ser His Asn Gln Leu Thr Thr Val Pro Ala Gly Leu
200                 205                 210                 215

Pro Arg Thr Leu Ala Ile Leu His Leu Gly Arg Asn Arg Ile Arg Gln
                220                 225                 230

Val Glu Ala Ala Arg Leu His Gly Ala Arg Gly Leu Arg Tyr Leu Leu
            235                 240                 245

Leu Gln His Asn Gln Leu Gly Ser Ser Gly Leu Pro Ala Gly Ala Leu
        250                 255                 260

Arg Pro Leu Arg Gly Leu His Thr Leu His Leu Tyr Gly Asn Gly Leu
    265                 270                 275

Asp Arg Val Pro Pro Ala Leu Pro Arg Arg Leu Arg Ala Leu Val Leu
280                 285                 290                 295

Pro His Asn His Val Ala Ala Leu Gly Ala Arg Asp Leu Val Ala Thr
                300                 305                 310

Pro Gly Leu Thr Glu Leu Asn Leu Ala Tyr Asn Arg Leu Ala Ser Ala
            315                 320                 325

Arg Val His His Arg Ala Phe Arg Arg Leu Arg Ala Leu Arg Ser Leu
        330                 335                 340

Asp Leu Ala Gly Asn Gln Leu Thr Arg Leu Pro Met Gly Leu Pro Thr
    345                 350                 355

Gly Leu Arg Thr Leu Gln Leu Gln Arg Asn Gln Leu Arg Met Leu Glu
360                 365                 370                 375

Pro Glu Pro Leu Ala Gly Leu Asp Gln Leu Arg Glu Leu Ser Leu Ala
                380                 385                 390

His Asn Arg Leu Arg Val Gly Asp Ile Gly Pro Gly Thr Trp His Glu
            395                 400                 405

Leu Gln Ala Leu Gln Val Arg His Arg Leu Val Ser His Thr Val Pro
        410                 415                 420

Arg Ala Pro Pro Ser Pro Cys Leu Pro Cys His Val Pro Asn Ile Leu
    425                 430                 435

Val Ser Trp
440

<210>  37

<211>  2397

<212>  DNA

<213>  Homo sapiens

<220>

<221>  CDS

<222>  (133)..(2100)

<223>

<220>

<221>  mat_peptide

<222>  (244)..()

<223>

<400>  37; LP223(b)
tggagaactg gggaggcaga gaccccggct ggccggaggc atgtggaggg gggggcctgg      60

gcgcaaggag aggcccagcg gaagccaagc caccaggccc cccagcgtcc acgcggagca     120

tgaacattga gg atg gcg cgt gcc cgc ggc tcc ccg tgc ccc ccg ctg ccg     171
              Met Ala Arg Ala Arg Gly Ser Pro Cys Pro Pro Leu Pro
                      -35                 -30                 -25

ccc ggt agg atg tcc tgg ccc cac ggg gca ttg ctc ttc ctc tgg ctc     219
Pro Gly Arg Met Ser Trp Pro His Gly Ala Leu Leu Phe Leu Trp Leu
                -20                 -15                 -10

ttc tcc cca ccc ctg ggg gcc ggt gga ggt gga gtg gcc gtg acg tct     267
Phe Ser Pro Pro Leu Gly Ala Gly Gly Gly Gly Val Ala Val Thr Ser
            -5                  -1  1               5

gcc gcc gga ggg ggc tcc ccg ccg gcc acc tcc tgc ccc gtg gcc tgc     315
Ala Ala Gly Gly Gly Ser Pro Pro Ala Thr Ser Cys Pro Val Ala Cys
    10                  15                  20

tcc tgc agc aac cag gcc agc cgg gtg atc tgc aca cgg aga gac ctg     363
Ser Cys Ser Asn Gln Ala Ser Arg Val Ile Cys Thr Arg Arg Asp Leu
25                  30                  35                  40

gcc gag gtc cca gcc agc atc ccg gtc aac acg cgg tac ctg aac ctg     411
Ala Glu Val Pro Ala Ser Ile Pro Val Asn Thr Arg Tyr Leu Asn Leu
                45                  50                  55

caa gag aac ggc atc cag gtg atc cgg acg gac acg ttc aag cac ctg     459
Gln Glu Asn Gly Ile Gln Val Ile Arg Thr Asp Thr Phe Lys His Leu
            60                  65                  70

cgg cac ctg gag att ctg cag ctg agc aag aac ctg gtg cgc aag atc 507
Arg His Leu Glu Ile Leu Gln Leu Ser Lys Asn Leu Val Arg Lys Ile
75 80 85

gag gtg ggc gcc ttc aac ggg ctg ccc agc ctc aac acg ctg gag ctt 555
Glu Val Gly Ala Phe Asn Gly Leu Pro Ser Leu Asn Thr Leu Glu Leu
90 95 100

ttt gac aac cgg ctg acc acg gtg ccc acg cag gcc ttc gag tac ctg 603
Phe Asp Asn Arg Leu Thr Thr Val Pro Thr Gln Ala Phe Glu Tyr Leu
105 110 115 120

tcc aag ctg cgg gag ctc tgg ctg cgg aac aac ccc atc gag agc atc 651
Ser Lys Leu Arg Glu Leu Trp Leu Arg Asn Asn Pro Ile Glu Ser Ile
125 130 135

ccc tcc tac gcc ttc aac cgc gtg ccc tcg ctg cgg cgc ctg gac ctg 699
Pro Ser Tyr Ala Phe Asn Arg Val Pro Ser Leu Arg Arg Leu Asp Leu
140 145 150

ggc gag ctc aag cgg ctg gaa tac atc tcg gag gcg gcc ttc gag ggg 747
Gly Glu Leu Lys Arg Leu Glu Tyr Ile Ser Glu Ala Ala Phe Glu Gly
155 160 165

ctg gtc aac ctg cgc tac ctc aac ctg ggc atg tgc aac ctc aag gac 795
Leu Val Asn Leu Arg Tyr Leu Asn Leu Gly Met Cys Asn Leu Lys Asp
170 175 180

atc ccc aac ctg acg gcc ctg gtg cgc ctg gag gag ctg gag ctg tcg 843
Ile Pro Asn Leu Thr Ala Leu Val Arg Leu Glu Glu Leu Glu Leu Ser
185 190 195 200

ggc aac cgg ctg gac ctg atc cgc ccg ggc tcc ttc cag ggt ctc acc 891
Gly Asn Arg Leu Asp Leu Ile Arg Pro Gly Ser Phe Gln Gly Leu Thr
205 210 215

agc ctg cgc aag ctg tgg ctc atg cac gcc cag gta gcc acc atc gag 939
Ser Leu Arg Lys Leu Trp Leu Met His Ala Gln Val Ala Thr Ile Glu
220 225 230

cgc aac gcc ttc gac gac ctc aag tcg ctg gag gag ctc aac ctg tcc 987
Arg Asn Ala Phe Asp Asp Leu Lys Ser Leu Glu Glu Leu Asn Leu Ser
235 240 245

cac aac aac ctg atg tcg ctg ccc cac gac ctc ttc acg ccc ctg cac 1035
His Asn Asn Leu Met Ser Leu Pro His Asp Leu Phe Thr Pro Leu His
250 255 260

cgc ctc gag cgc gtg cac ctc aac cac aac ccc tgg cat tgc aac tgc 1083
Arg Leu Glu Arg Val His Leu Asn His Asn Pro Trp His Cys Asn Cys
265 270 275 280

gac gtg ctc tgg ctg agc tgg tgg ctc aag gag acg gtg ccc agc aac 1131
Asp Val Leu Trp Leu Ser Trp Trp Leu Lys Glu Thr Val Pro Ser Asn
285 290 295

acg acg tgc tgc gcc cgc tgt cat gcg ccc gcc ggc ctc aag ggg cgc 1179

Thr Thr Cys Cys Ala Arg Cys His Ala Pro Ala Gly Leu Lys Gly Arg
                300                 305                 310

tac att ggg gag ctg gac cag tcg cat ttc acc tgc tat gcg ccc gtc      1227
Tyr Ile Gly Glu Leu Asp Gln Ser His Phe Thr Cys Tyr Ala Pro Val
        315                 320                 325

atc gtg gag ccg ccc acg gac ctc aac gtc acc gag ggc atg gct gcc      1275
Ile Val Glu Pro Pro Thr Asp Leu Asn Val Thr Glu Gly Met Ala Ala
    330                 335                 340

gag ctc aaa tgc cgc acg ggc acc tcc atg acc tcc gtc aac tgg ctg      1323
Glu Leu Lys Cys Arg Thr Gly Thr Ser Met Thr Ser Val Asn Trp Leu
345                 350                 355                 360

acg ccc aac ggc acc ctc atg acc cac ggc tcc tac cgc gtg cgc atc      1371
Thr Pro Asn Gly Thr Leu Met Thr His Gly Ser Tyr Arg Val Arg Ile
                365                 370                 375

tcc gtc ctg cat gac ggc acg ctt aac ttc acc aac gtc acc gtg cag      1419
Ser Val Leu His Asp Gly Thr Leu Asn Phe Thr Asn Val Thr Val Gln
            380                 385                 390

gac acg ggc cag tac acg tgc atg gtg acg aac tca gcc ggc aac acc      1467
Asp Thr Gly Gln Tyr Thr Cys Met Val Thr Asn Ser Ala Gly Asn Thr
        395                 400                 405

acc gcc tcg gcc acg ctc aac gtc tcg gcc gtg gac ccc gtg gcg gcc      1515
Thr Ala Ser Ala Thr Leu Asn Val Ser Ala Val Asp Pro Val Ala Ala
    410                 415                 420

ggg ggc acc ggc agc ggc ggg ggc ggc cct ggg ggc agt ggt ggt gtt      1563
Gly Gly Thr Gly Ser Gly Gly Gly Gly Pro Gly Gly Ser Gly Gly Val
425                 430                 435                 440

gga ggg ggc agt ggc ggc tac acc tac ttc acc acg gtg acc gtg gag      1611
Gly Gly Gly Ser Gly Gly Tyr Thr Tyr Phe Thr Thr Val Thr Val Glu
                445                 450                 455

acc ctg gag acg cag ccc gga gag gag gcc ctg cag ccg cgg ggg acg      1659
Thr Leu Glu Thr Gln Pro Gly Glu Glu Ala Leu Gln Pro Arg Gly Thr
            460                 465                 470

gag aag gaa ccg cca ggg ccc acg aca gac ggt gtc tgg ggt ggg ggc      1707
Glu Lys Glu Pro Pro Gly Pro Thr Thr Asp Gly Val Trp Gly Gly Gly
        475                 480                 485

cgg cct ggg gac gcg gcc ggc cct gcc tcg tct tct acc acg gca ccc      1755
Arg Pro Gly Asp Ala Ala Gly Pro Ala Ser Ser Ser Thr Thr Ala Pro
    490                 495                 500

gcc ccg cgc tcc tcg cgg ccc acg gag aag gcg ttc acg gtg ccc atc      1803
Ala Pro Arg Ser Ser Arg Pro Thr Glu Lys Ala Phe Thr Val Pro Ile
505                 510                 515                 520

acg gat gtg acg gag aac gcc ctc aag gac ctg gac gac gtc atg aag      1851
Thr Asp Val Thr Glu Asn Ala Leu Lys Asp Leu Asp Asp Val Met Lys
                525                 530                 535

acc acc aaa atc atc atc ggc tgc ttc gtg gcc atc acg ttc atg gcc     1899
Thr Thr Lys Ile Ile Ile Gly Cys Phe Val Ala Ile Thr Phe Met Ala
            540                 545                 550

gcg gtg atg ctc gtg gcc ttc tac aag ctg cgc aag cag cac cag ctc     1947
Ala Val Met Leu Val Ala Phe Tyr Lys Leu Arg Lys Gln His Gln Leu
        555                 560                 565

cac aag cac cac ggg ccc acg cgc acc gtg gag atc atc aac gtg gag     1995
His Lys His His Gly Pro Thr Arg Thr Val Glu Ile Ile Asn Val Glu
    570                 575                 580

gac gag ctg ccc gcc gcc tcg gcc gtg tcc gtg gcc gcc gcg gcc gcc     2043
Asp Glu Leu Pro Ala Ala Ser Ala Val Ser Val Ala Ala Ala Ala Ala
585                 590                 595                 600

gtg gcc agt ggg ggt ggt gtg ggc ggg gac agc cac ctg gcc ctg ccc     2091
Val Ala Ser Gly Gly Gly Val Gly Gly Asp Ser His Leu Ala Leu Pro
                605                 610                 615

gcc ctg gag tgagaccacc tcaaccacca ccactacgtg gctgccgcct             2140
Ala Leu Glu

tcaaggcgca ctacagcagc aaccccagcg gcgggggctg cgggggcaaa ggcccgcctg   2200

gcctcaactc catccacgaa cctctgctct tcaagagcgg ctccaaggag aacgtgcaag   2260

agacgcagat ctgaggcggc ggggccgggc gggcgagggg cgtggagccc cccacccagg   2320

tcccagcccg ggcgcagcct gaccgggacc cctccctccc acagcccagc ccaccttctg   2380

ggaccacgca gggaatt                                                  2397

<210>  38

<211>  656

<212>  PRT

<213>  Homo sapiens

<400>  38; LP223(b)

Met Ala Arg Ala Arg Gly Ser Pro Cys Pro Pro Leu Pro Pro Gly Arg
        -35                 -30                 -25

Met Ser Trp Pro His Gly Ala Leu Leu Phe Leu Trp Leu Phe Ser Pro
    -20                 -15                 -10

Pro Leu Gly Ala Gly Gly Gly Gly Val Ala Val Thr Ser Ala Ala Gly
-5                  -1  1               5                   10

Gly Gly Ser Pro Pro Ala Thr Ser Cys Pro Val Ala Cys Ser Cys Ser
            15                  20                  25

Asn Gln Ala Ser Arg Val Ile Cys Thr Arg Arg Asp Leu Ala Glu Val
        30                  35                  40

Pro Ala Ser Ile Pro Val Asn Thr Arg Tyr Leu Asn Leu Gln Glu Asn
    45                  50                  55

Gly Ile Gln Val Ile Arg Thr Asp Thr Phe Lys His Leu Arg His Leu
60                  65                  70                  75

Glu Ile Leu Gln Leu Ser Lys Asn Leu Val Arg Lys Ile Glu Val Gly
                80                  85                  90

Ala Phe Asn Gly Leu Pro Ser Leu Asn Thr Leu Glu Leu Phe Asp Asn
            95                  100                 105

Arg Leu Thr Thr Val Pro Thr Gln Ala Phe Glu Tyr Leu Ser Lys Leu
        110                 115                 120

Arg Glu Leu Trp Leu Arg Asn Asn Pro Ile Glu Ser Ile Pro Ser Tyr
    125                 130                 135

Ala Phe Asn Arg Val Pro Ser Leu Arg Arg Leu Asp Leu Gly Glu Leu
140                 145                 150                 155

Lys Arg Leu Glu Tyr Ile Ser Glu Ala Ala Phe Glu Gly Leu Val Asn
                160                 165                 170

Leu Arg Tyr Leu Asn Leu Gly Met Cys Asn Leu Lys Asp Ile Pro Asn
            175                 180                 185

Leu Thr Ala Leu Val Arg Leu Glu Glu Leu Glu Leu Ser Gly Asn Arg
        190                 195                 200

Leu Asp Leu Ile Arg Pro Gly Ser Phe Gln Gly Leu Thr Ser Leu Arg
    205                 210                 215

Lys Leu Trp Leu Met His Ala Gln Val Ala Thr Ile Glu Arg Asn Ala
220                 225                 230                 235

Phe Asp Asp Leu Lys Ser Leu Glu Glu Leu Asn Leu Ser His Asn Asn
                240                 245                 250

Leu Met Ser Leu Pro His Asp Leu Phe Thr Pro Leu His Arg Leu Glu
            255                 260                 265

Arg Val His Leu Asn His Asn Pro Trp His Cys Asn Cys Asp Val Leu
        270                 275                 280

Trp Leu Ser Trp Trp Leu Lys Glu Thr Val Pro Ser Asn Thr Thr Cys
    285                 290                 295

Cys Ala Arg Cys His Ala Pro Ala Gly Leu Lys Gly Arg Tyr Ile Gly
300                 305                 310                 315

Glu Leu Asp Gln Ser His Phe Thr Cys Tyr Ala Pro Val Ile Val Glu
                320                 325                 330

Pro Pro Thr Asp Leu Asn Val Thr Glu Gly Met Ala Ala Glu Leu Lys
            335                 340                 345

Cys Arg Thr Gly Thr Ser Met Thr Ser Val Asn Trp Leu Thr Pro Asn
        350                 355                 360

Gly Thr Leu Met Thr His Gly Ser Tyr Arg Val Arg Ile Ser Val Leu
    365                 370                 375

His Asp Gly Thr Leu Asn Phe Thr Asn Val Thr Val Gln Asp Thr Gly
380                 385                 390                 395

Gln Tyr Thr Cys Met Val Thr Asn Ser Ala Gly Asn Thr Thr Ala Ser
                400                 405                 410

Ala Thr Leu Asn Val Ser Ala Val Asp Pro Val Ala Ala Gly Gly Thr
            415                 420                 425

Gly Ser Gly Gly Gly Gly Pro Gly Gly Ser Gly Gly Val Gly Gly Gly
        430                 435                 440

Ser Gly Gly Tyr Thr Tyr Phe Thr Thr Val Thr Val Glu Thr Leu Glu
    445                 450                 455

Thr Gln Pro Gly Glu Glu Ala Leu Gln Pro Arg Gly Thr Glu Lys Glu
460                 465                 470                 475

Pro Pro Gly Pro Thr Thr Asp Gly Val Trp Gly Gly Gly Arg Pro Gly
                480                 485                 490

Asp Ala Ala Gly Pro Ala Ser Ser Ser Thr Thr Ala Pro Ala Pro Arg
            495                 500                 505

Ser Ser Arg Pro Thr Glu Lys Ala Phe Thr Val Pro Ile Thr Asp Val
        510                 515                 520

Thr Glu Asn Ala Leu Lys Asp Leu Asp Asp Val Met Lys Thr Thr Lys
    525                 530                 535

Ile Ile Ile Gly Cys Phe Val Ala Ile Thr Phe Met Ala Ala Val Met
540                 545                 550                 555

Leu Val Ala Phe Tyr Lys Leu Arg Lys Gln His Gln Leu His Lys His
                560                 565                 570

His Gly Pro Thr Arg Thr Val Glu Ile Ile Asn Val Glu Asp Glu Leu
            575                 580                 585

Pro Ala Ala Ser Ala Val Ser Val Ala Ala Ala Ala Ala Val Ala Ser
        590                 595                 600

Gly Gly Gly Val Gly Gly Asp Ser His Leu Ala Leu Pro Ala Leu Glu
    605                 610                 615

SEQUENCE LISTING

<110> Eli Lilly & Company

<120> Novel secreted proteins and their uses

<130> X-14001

<160> 38

<170> PatentIn version 3.1

<210> 1
<211> 1438
<212> DNA
<213> Homo sapiens

<220>
<221> CDS
<222> (69)..(1196)
<223>

<220>
<221> mat_peptide
<222> (132)..()
<223>

<400> 1; LP105
ctgagaccct ccttcaacct ccctagagga cagccccact ctgcctcctg ctcccccagg 60

gcagcacc atg tgg ccc ccg tgg ctc tgc tgg gca ctc tgg gtg ctg ccc 110
Met Trp Pro Pro Trp Leu Cys Trp Ala Leu Trp Val Leu Pro
-20 -15 -10

ctg gct ggc ccc ggg gcg gcc ctg acc gag gag cag ctc ctg ggc agc 158
Leu Ala Gly Pro Gly Ala Ala Leu Thr Glu Glu Gln Leu Leu Gly Ser
-5 -1 1 5

ctg ctg cgg cag ctg cag ctc agc gag gtg ccc gta ctg gac agg gcc 206
Leu Leu Arg Gln Leu Gln Leu Ser Glu Val Pro Val Leu Asp Arg Ala
10 15 20 25

gac atg gag aag ctg gtc atc ccc gcc cac gtg agg gcc cag tat gta     254
Asp Met Glu Lys Leu Val Ile Pro Ala His Val Arg Ala Gln Tyr Val
                30                  35                  40

gtc ctg ctg cgg cgc agc cac ggg gac cgc tcc cgc gga aag agg ttc     302
Val Leu Leu Arg Arg Ser His Gly Asp Arg Ser Arg Gly Lys Arg Phe
            45                  50                  55

agc cag agc ttc cga gag gtg gcc ggc agg ttc ctg gcg tcg gag gcc     350
Ser Gln Ser Phe Arg Glu Val Ala Gly Arg Phe Leu Ala Ser Glu Ala
        60                  65                  70

agc aca cac ctg ctg gtg ttc ggc atg gag cag cgg ctg ccg ccc aac     398
Ser Thr His Leu Leu Val Phe Gly Met Glu Gln Arg Leu Pro Pro Asn
    75                  80                  85

agc gag ctg gtg cag gcc gtg ctg cgg ctc ttc cag gag ccg gtc ccc     446
Ser Glu Leu Val Gln Ala Val Leu Arg Leu Phe Gln Glu Pro Val Pro
90                  95                  100                 105

aag gcc gcg ctg cac agg cac ggg cgg ctg tcc ccg cgc agc gcc cag     494
Lys Ala Ala Leu His Arg His Gly Arg Leu Ser Pro Arg Ser Ala Gln
                110                 115                 120

gcc cgg gtg gct gtc gag tgg ctg cgc gtc cgc gac gac ggc tcc aac     542
Ala Arg Val Ala Val Glu Trp Leu Arg Val Arg Asp Asp Gly Ser Asn
            125                 130                 135

cgc acc tcc ctc atc gac tcc agg ctg gtg tcc gtc cac gag agc ggc     590
Arg Thr Ser Leu Ile Asp Ser Arg Leu Val Ser Val His Glu Ser Gly
        140                 145                 150

tgg aag gcc ttc gac gtg acc gag gcc gtg aac ttc tgg cag cag ctg     638
Trp Lys Ala Phe Asp Val Thr Glu Ala Val Asn Phe Trp Gln Gln Leu
    155                 160                 165

agc cgg ccc cgg cag ccg ctg ctg cta cag gtg tcg gtg cag agg gag     686
Ser Arg Pro Arg Gln Pro Leu Leu Leu Gln Val Ser Val Gln Arg Glu
170                 175                 180                 185

cat ctg ggc ccg ctg gcg tcc ggc gcc cac aag ctg gtc cgc ttt gcc     734
His Leu Gly Pro Leu Ala Ser Gly Ala His Lys Leu Val Arg Phe Ala
                190                 195                 200

tcg cag ggg gcg cca gcc ggg ctt ggg gag ccc cag ctg gag ctg cac     782
Ser Gln Gly Ala Pro Ala Gly Leu Gly Glu Pro Gln Leu Glu Leu His
            205                 210                 215

acc ctg gac ctc agg gac tat gga gct cag ggc gac tgt gac cct gaa     830
Thr Leu Asp Leu Arg Asp Tyr Gly Ala Gln Gly Asp Cys Asp Pro Glu
        220                 225                 230

gca cca gtg acc gag ggc acc tgc tgc tgc cac cag gag atg tac act     878
Ala Pro Val Thr Glu Gly Thr Cys Cys Cys His Gln Glu Met Tyr Thr
    235                 240                 245

gac ctg cag ggg atg aag tgg gcc aag aac tgg atg ccg gag ccc ctg     926
Asp Leu Gln Gly Met Lys Trp Ala Lys Asn Trp Met Pro Glu Pro Leu
250                 255                 260                 265

ggc ttc ctg gct tac aag tgt gtg ggc acc tgc cag cag ccc ctg gag     974
Gly Phe Leu Ala Tyr Lys Cys Val Gly Thr Cys Gln Gln Pro Leu Glu
                270                 275                 280

gcc ctg gcc ttc aat tgg cca ttt ctg ggg ccg cga cac tgc atc gcc     1022
Ala Leu Ala Phe Asn Trp Pro Phe Leu Gly Pro Arg His Cys Ile Ala
            285                 290                 295

tca gag act gcc tcg ctg ccc atg atc gtc agc atc aag gag gga ggc     1070

Ser Glu Thr Ala Ser Leu Pro Met Ile Val Ser Ile Lys Glu Gly Gly
        300                 305                 310

agg acc agg ccc cag gtg gtc agc ctg cct aac atg agg gtc cac cac      1118
Arg Thr Arg Pro Gln Val Val Ser Leu Pro Asn Met Arg Val His His
    315                 320                 325

tac gcc ccg cta atg ttt gtg att tta gta gag atg agg ttt cac cat      1166
Tyr Ala Pro Leu Met Phe Val Ile Leu Val Glu Met Arg Phe His His
330                 335                 340                 345

gtt ggc cag gct ggt ctc aaa ctc ctg acc tgaggtgggc ggatcacaag        1216
Val Gly Gln Ala Gly Leu Lys Leu Leu Thr
                350                 355

gtcaggagat cgagaccatc ctggctaaca aggtgaaacc ccgtctctac taaaaataca    1276

aaaaattagc tgggtgtcgt ggcgggtgcc tgtagtctca ggtactcggg aggctgaggc    1336

aggagaatgg catgaacccg ggaggcggag gttgcagcga gccaagatca cgccactgca    1396

cttcagcctg ggcaacagag caagactcca tctcaaaaaa aa                       1438

<210>  2

<211>  376

<212>  PRT

<213>  Homo sapiens

<400>  2; LP105

Met Trp Pro Pro Trp Leu Cys Trp Ala Leu Trp Val Leu Pro Leu Ala
    -20                 -15                 -10

Gly Pro Gly Ala Ala Leu Thr Glu Glu Gln Leu Leu Gly Ser Leu Leu
-5              -1  1               5                   10

Arg Gln Leu Gln Leu Ser Glu Val Pro Val Leu Asp Arg Ala Asp Met
            15                  20                  25

Glu Lys Leu Val Ile Pro Ala His Val Arg Ala Gln Tyr Val Val Leu
        30                  35                  40

Leu Arg Arg Ser His Gly Asp Arg Ser Arg Gly Lys Arg Phe Ser Gln
    45                  50                  55

Ser Phe Arg Glu Val Ala Gly Arg Phe Leu Ala Ser Glu Ala Ser Thr
60                  65                  70                  75

His Leu Leu Val Phe Gly Met Glu Gln Arg Leu Pro Pro Asn Ser Glu
                80                  85                  90

Leu Val Gln Ala Val Leu Arg Leu Phe Gln Glu Pro Val Pro Lys Ala
            95                  100                 105

Ala Leu His Arg His Gly Arg Leu Ser Pro Arg Ser Ala Gln Ala Arg

110                 115                 120

Val Ala Val Glu Trp Leu Arg Val Arg Asp Asp Gly Ser Asn Arg Thr
    125                 130                 135

Ser Leu Ile Asp Ser Arg Leu Val Ser Val His Glu Ser Gly Trp Lys
140                 145                 150                 155

Ala Phe Asp Val Thr Glu Ala Val Asn Phe Trp Gln Gln Leu Ser Arg
                160                 165                 170

Pro Arg Gln Pro Leu Leu Leu Gln Val Ser Val Gln Arg Glu His Leu
            175                 180                 185

Gly Pro Leu Ala Ser Gly Ala His Lys Leu Val Arg Phe Ala Ser Gln
        190                 195                 200

Gly Ala Pro Ala Gly Leu Gly Glu Pro Gln Leu Glu Leu His Thr Leu
    205                 210                 215

Asp Leu Arg Asp Tyr Gly Ala Gln Gly Asp Cys Asp Pro Glu Ala Pro
220                 225                 230                 235

Val Thr Glu Gly Thr Cys Cys Cys His Gln Glu Met Tyr Thr Asp Leu
                240                 245                 250

Gln Gly Met Lys Trp Ala Lys Asn Trp Met Pro Glu Pro Leu Gly Phe
            255                 260                 265

Leu Ala Tyr Lys Cys Val Gly Thr Cys Gln Gln Pro Leu Glu Ala Leu
        270                 275                 280

Ala Phe Asn Trp Pro Phe Leu Gly Pro Arg His Cys Ile Ala Ser Glu
    285                 290                 295

Thr Ala Ser Leu Pro Met Ile Val Ser Ile Lys Glu Gly Gly Arg Thr
300                 305                 310                 315

Arg Pro Gln Val Val Ser Leu Pro Asn Met Arg Val His His Tyr Ala
                320                 325                 330

Pro Leu Met Phe Val Ile Leu Val Glu Met Arg Phe His His Val Gly
            335                 340                 345

Gln Ala Gly Leu Lys Leu Leu Thr
        350                 355

<210>  3

<211>  1041

<212>  DNA

<213>  Homo sapiens

<220>
<221>  CDS
<222>  (66)..(878)
<223>

<220>
<221>  mat_peptide
<222>  (129)..()
<223>

<400>  3; LP061
gcggccgcca gtgtgatgga tatctgcaga attcgccctt acaaccaagt gagcagcaca      60

gacta atg agt aga atc aat aag aac gtg gtt ttg gcc ctt tta acg ctg     110
      Met Ser Arg Ile Asn Lys Asn Val Val Leu Ala Leu Leu Thr Leu
          -20                 -15                 -10

aca agt tct gca ttt ctg ctg ttt cag ttg tac tac tac aag cac tat     158
Thr Ser Ser Ala Phe Leu Leu Phe Gln Leu Tyr Tyr Tyr Lys His Tyr
    -5                  -1  1               5                   10

tta tca aca aag aat gga gct ggt ttg tca aaa tcc aaa gga agc cga     206
Leu Ser Thr Lys Asn Gly Ala Gly Leu Ser Lys Ser Lys Gly Ser Arg
                15                  20                  25

att gga ttt gat agc aca cag tgg cgt gca gtt aaa aaa ttt att atg     254
Ile Gly Phe Asp Ser Thr Gln Trp Arg Ala Val Lys Lys Phe Ile Met
            30                  35                  40

tta aca tcc aac caa aat gta cca gtg ttt ctt att gat cct ttg ata     302
Leu Thr Ser Asn Gln Asn Val Pro Val Phe Leu Ile Asp Pro Leu Ile
        45                  50                  55

ctg gaa ttg att aat aag aac ttt gaa caa gtc aaa aat act tct cat     350
Leu Glu Leu Ile Asn Lys Asn Phe Glu Gln Val Lys Asn Thr Ser His
    60                  65                  70

ggc tct act tca caa tgc aag ttt ttc tgt gtt cca aga gac ttt act     398
Gly Ser Thr Ser Gln Cys Lys Phe Phe Cys Val Pro Arg Asp Phe Thr
75                  80                  85                  90

gca ttt gca ctg cag tat cac cta tgg aag aat gag gaa ggc tgg ttt     446
Ala Phe Ala Leu Gln Tyr His Leu Trp Lys Asn Glu Glu Gly Trp Phe
                95                  100                 105

cgg ata gct gag aat atg gga ttt cag tgc cta aag att gag agt aaa     494
Arg Ile Ala Glu Asn Met Gly Phe Gln Cys Leu Lys Ile Glu Ser Lys
            110                 115                 120

gat ccc cgg cta gac ggg ata gac tca ctc tct gga act gaa atc ccc     542
Asp Pro Arg Leu Asp Gly Ile Asp Ser Leu Ser Gly Thr Glu Ile Pro
        125                 130                 135

ctg cac tat atc tgc aaa ctg gcc act cat gcg atc cac ttg gta gtc     590
Leu His Tyr Ile Cys Lys Leu Ala Thr His Ala Ile His Leu Val Val

140                 145                 150

ttt cat gag agg agt ggc aac tac ctc tgg cac ggc cac ttg aga ctt      638
Phe His Glu Arg Ser Gly Asn Tyr Leu Trp His Gly His Leu Arg Leu
155                 160                 165                 170

aaa gaa cac att gac agg aaa ttt gtt ccc ttc caa aag tta cag ttt      686
Lys Glu His Ile Asp Arg Lys Phe Val Pro Phe Gln Lys Leu Gln Phe
                175                 180                 185

ggt cgt tat cca gga gct ttt gac agg cca gag tta cag caa gtt act      734
Gly Arg Tyr Pro Gly Ala Phe Asp Arg Pro Glu Leu Gln Gln Val Thr
            190                 195                 200

gtt gat gga ctg gaa gtt ctc att cca aag gat cca atg cac ttt gta      782
Val Asp Gly Leu Glu Val Leu Ile Pro Lys Asp Pro Met His Phe Val
        205                 210                 215

gaa gaa gta cca cac tct agg ttt att gag tgt agg tat aaa gaa gct      830
Glu Glu Val Pro His Ser Arg Phe Ile Glu Cys Arg Tyr Lys Glu Ala
    220                 225                 230

cga gca ttc ttt cag ata cct gtt tcc gaa gtt tac act gtg ctg gac      878
Arg Ala Phe Phe Gln Ile Pro Val Ser Glu Val Tyr Thr Val Leu Asp
235                 240                 245                 250

tgagtttgta gacatgaagg tccatgtacc ctgtgaaacc ctcgaataca ttgaagccaa      938

ctatggtaag acctggaaga ttcctgtaaa gacgtgggac tggaagcgct ctcctcccaa      998

tgtgcaaccc aatggaatct ggcctatttc tgagtgggat gag                       1041

<210>  4

<211>  271

<212>  PRT

<213>  Homo sapiens

<400>  4; LP061

Met Ser Arg Ile Asn Lys Asn Val Val Leu Ala Leu Leu Thr Leu Thr
    -20                 -15                 -10

Ser Ser Ala Phe Leu Leu Phe Gln Leu Tyr Tyr Tyr Lys His Tyr Leu
-5                  -1  1               5                   10

Ser Thr Lys Asn Gly Ala Gly Leu Ser Lys Ser Lys Gly Ser Arg Ile
            15                  20                  25

Gly Phe Asp Ser Thr Gln Trp Arg Ala Val Lys Lys Phe Ile Met Leu
        30                  35                  40

Thr Ser Asn Gln Asn Val Pro Val Phe Leu Ile Asp Pro Leu Ile Leu
    45                  50                  55

Glu Leu Ile Asn Lys Asn Phe Glu Gln Val Lys Asn Thr Ser His Gly
60                  65                  70                  75

Ser Thr Ser Gln Cys Lys Phe Phe Cys Val Pro Arg Asp Phe Thr Ala
                80                  85                  90

Phe Ala Leu Gln Tyr His Leu Trp Lys Asn Glu Glu Gly Trp Phe Arg
            95                  100                 105

Ile Ala Glu Asn Met Gly Phe Gln Cys Leu Lys Ile Glu Ser Lys Asp
        110                 115                 120

Pro Arg Leu Asp Gly Ile Asp Ser Leu Ser Gly Thr Glu Ile Pro Leu
    125                 130                 135

His Tyr Ile Cys Lys Leu Ala Thr His Ala Ile His Leu Val Val Phe
140                 145                 150                 155

His Glu Arg Ser Gly Asn Tyr Leu Trp His Gly His Leu Arg Leu Lys
                160                 165                 170

Glu His Ile Asp Arg Lys Phe Val Pro Phe Gln Lys Leu Gln Phe Gly
            175                 180                 185

Arg Tyr Pro Gly Ala Phe Asp Arg Pro Glu Leu Gln Gln Val Thr Val
        190                 195                 200

Asp Gly Leu Glu Val Leu Ile Pro Lys Asp Pro Met His Phe Val Glu
    205                 210                 215

Glu Val Pro His Ser Arg Phe Ile Glu Cys Arg Tyr Lys Glu Ala Arg
220                 225                 230                 235

Ala Phe Phe Gln Ile Pro Val Ser Glu Val Tyr Thr Val Leu Asp
                240                 245                 250

<210>  5

<211>  3664

<212>  DNA

<213>  Homo sapiens


<220>

<221>  CDS

<222>  (347)..(1981)

<223>


<220>

<221>  mutation

<222>  (383)..(385)

<223>  variant wherein nucleotides 383-385 are deleted


<220>

<221>  mat_peptide

<222>  (416)..()

<223>


<220>

<221>  sig_peptide

<222>  (1)..(23)

<223>  wherein Leu at position -11 may be deleted


<400>  5; LP224
gccggcgctg gccccctccc ccgcggggcg ggccccgcca tgcattattc attggccaga      60

gccgggctgc gggcgcgagt gctgatgtca ggcaggcgcg agtgcggcgg gcgcagcggc     120

ggcggccgcc cagcagcccg ggctcgggtg ggggggcgcc gagtgggggt ggcggccagc     180

atgctgctcg gctgcggctc ggcctcccac acccgcggct ccctagtcct cgccagcagc     240

ggcggcggca gagcagcgag cggcgcccgc gtctgcagcg gcgccgggtc cgagcgcgcg     300

gcgcggcggt gggggtcggg gcccgggcgg ggagcgggga ccgggc atg gcg ctg        355
                                                   Met Ala Leu


cgg aga ggc ggc tgc gga gcg ctc ggg ctg ctg ctg ctg ctg ctg ggc       403
Arg Arg Gly Gly Cys Gly Ala Leu Gly Leu Leu Leu Leu Leu Leu Gly
-20                 -15                 -10                 -5

gcc gcg tgc ctg ata ccg cgg agc gcg cag gtg agg cgg ctg gcg cgc       451
Ala Ala Cys Leu Ile Pro Arg Ser Ala Gln Val Arg Arg Leu Ala Arg
            -1  1               5                   10

tgc ccc gcc act tgc agc tgt acc aag gag tct atc atc tgc gtg ggc       499
Cys Pro Ala Thr Cys Ser Cys Thr Lys Glu Ser Ile Ile Cys Val Gly
        15                  20                  25

tct tcc tgg gtg ccc agg atc gtg ccg ggc gac atc agc tcc ctg agc       547
Ser Ser Trp Val Pro Arg Ile Val Pro Gly Asp Ile Ser Ser Leu Ser
    30                  35                  40

ctg gta aat ggg acg ttt tca gaa atc aag gac cga atg ttt tcc cat       595
Leu Val Asn Gly Thr Phe Ser Glu Ile Lys Asp Arg Met Phe Ser His
45                  50                  55                  60

ctg cct tct ctg cag ctg cta ttg ctg aat tct aac tca ttc acg atc       643
Leu Pro Ser Leu Gln Leu Leu Leu Leu Asn Ser Asn Ser Phe Thr Ile
                65                  70                  75

atc cgg gat gat gct ttt gct gga ctt ttt cat ctt gaa tac ctg ttc       691
Ile Arg Asp Asp Ala Phe Ala Gly Leu Phe His Leu Glu Tyr Leu Phe
            80                  85                  90

att gaa ggg aac aaa ata gaa acc att tca aga aat gcc ttt cgt ggc       739

Ile Glu Gly Asn Lys Ile Glu Thr Ile Ser Arg Asn Ala Phe Arg Gly
        95                  100                 105

ctc cgt gac ctg act cac ctt tct ttg gcc aat aac cac ata aaa gca      787
Leu Arg Asp Leu Thr His Leu Ser Leu Ala Asn Asn His Ile Lys Ala
    110                 115                 120

cta cca agg gat gtc ttc agt gat tta gac tct ctg att gaa cta gat      835
Leu Pro Arg Asp Val Phe Ser Asp Leu Asp Ser Leu Ile Glu Leu Asp
125                 130                 135                 140

ttg agg ggt aat aaa ttt gaa tgt gac tgc aaa gcc aag tgg cta tac      883
Leu Arg Gly Asn Lys Phe Glu Cys Asp Cys Lys Ala Lys Trp Leu Tyr
                145                 150                 155

ctg tgg ttg aag atg aca aat tcc acc gtt tct gat gtg ctg tgt att      931
Leu Trp Leu Lys Met Thr Asn Ser Thr Val Ser Asp Val Leu Cys Ile
            160                 165                 170

ggt cca cca gag tat cag gaa aag aag cta aat gac gtg acc agc ttt      979
Gly Pro Pro Glu Tyr Gln Glu Lys Lys Leu Asn Asp Val Thr Ser Phe
        175                 180                 185

gac tat gaa tgc aca act aca gat ttt gtt gtt cat cag act tta ccc      1027
Asp Tyr Glu Cys Thr Thr Thr Asp Phe Val Val His Gln Thr Leu Pro
    190                 195                 200

tac cag tcg gtt tca gtg gat acg ttc aac tcc aag aac gat gtg tac      1075
Tyr Gln Ser Val Ser Val Asp Thr Phe Asn Ser Lys Asn Asp Val Tyr
205                 210                 215                 220

gtg gcc atc gcg cag ccc agc atg gag aac tgc atg gtg ctg gag tgg      1123
Val Ala Ile Ala Gln Pro Ser Met Glu Asn Cys Met Val Leu Glu Trp
                225                 230                 235

gac cac att gaa atg aat ttc cgg agc tat gac aac att aca ggt cag      1171
Asp His Ile Glu Met Asn Phe Arg Ser Tyr Asp Asn Ile Thr Gly Gln
            240                 245                 250

tcc atc gtg ggc tgt aag gcc att ctc atc gat gat cag gtc ttt gtg      1219
Ser Ile Val Gly Cys Lys Ala Ile Leu Ile Asp Asp Gln Val Phe Val
        255                 260                 265

gtg gta gcc cag ctc ttc ggt ggc tct cac att tac aaa tac gac gag      1267
Val Val Ala Gln Leu Phe Gly Gly Ser His Ile Tyr Lys Tyr Asp Glu
    270                 275                 280

agt tgg acc aaa ttt gtc aaa ttc caa gac ata gag gtc tct cgc att      1315
Ser Trp Thr Lys Phe Val Lys Phe Gln Asp Ile Glu Val Ser Arg Ile
285                 290                 295                 300

tcc aag ccc aat gac atc gag ctg ttt cag atc gac gac gag acg ttc      1363
Ser Lys Pro Asn Asp Ile Glu Leu Phe Gln Ile Asp Asp Glu Thr Phe
                305                 310                 315

ttt gtc atc gca gac agc tca aag gct ggt ctg tcc aca gtt tat aaa      1411
Phe Val Ile Ala Asp Ser Ser Lys Ala Gly Leu Ser Thr Val Tyr Lys
            320                 325                 330

tgg aac agc aaa gga ttc tat tct tac cag tca ctg cac gag tgg ttc      1459
Trp Asn Ser Lys Gly Phe Tyr Ser Tyr Gln Ser Leu His Glu Trp Phe
        335                 340                 345

agg gac acg gat gcg gag ttt gtt gat atc gat gga aaa tcg cat ctc      1507
Arg Asp Thr Asp Ala Glu Phe Val Asp Ile Asp Gly Lys Ser His Leu
    350                 355                 360

atc ctg tcc agc cgc tcc cag gtc ccc atc atc ctc cag tgg aat aaa      1555
Ile Leu Ser Ser Arg Ser Gln Val Pro Ile Ile Leu Gln Trp Asn Lys

365                 370                 375                 380

agc tct aag aag ttt gtc ccc cat ggt gac atc ccc aac atg gag gac      1603
Ser Ser Lys Lys Phe Val Pro His Gly Asp Ile Pro Asn Met Glu Asp
                385                 390                 395

gta ctg gct gtg aag agc ttc cga atg caa aat acc ctc tac ctt tcc      1651
Val Leu Ala Val Lys Ser Phe Arg Met Gln Asn Thr Leu Tyr Leu Ser
            400                 405                 410

ctt acc cgc ttc atc ggg gac tcc cgg gtc atg agg tgg aac agt aag      1699
Leu Thr Arg Phe Ile Gly Asp Ser Arg Val Met Arg Trp Asn Ser Lys
        415                 420                 425

cag ttt gtg gag atc caa gct ctt cca tcc cgg ggg gcc atg acc ctg      1747
Gln Phe Val Glu Ile Gln Ala Leu Pro Ser Arg Gly Ala Met Thr Leu
    430                 435                 440

cag ccc ttt tct ttt aaa gat aat cac tac ctg gcc ctg ggg agt gac      1795
Gln Pro Phe Ser Phe Lys Asp Asn His Tyr Leu Ala Leu Gly Ser Asp
445                 450                 455                 460

tat aca ttc tct cag ata tac cag tgg gat aaa gag aag cag cta ttc      1843
Tyr Thr Phe Ser Gln Ile Tyr Gln Trp Asp Lys Glu Lys Gln Leu Phe
                465                 470                 475

aaa aag ttt aag gag att tac gtg cag gcg cct cgt tca ttc aca gct      1891
Lys Lys Phe Lys Glu Ile Tyr Val Gln Ala Pro Arg Ser Phe Thr Ala
            480                 485                 490

gtc tcc acc gac agg aga gat ttc ttt ttt gca tcc agt ttc aaa ggg      1939
Val Ser Thr Asp Arg Arg Asp Phe Phe Phe Ala Ser Ser Phe Lys Gly
        495                 500                 505

aaa aca aag att ttt gaa cat ata att gtt gac tta agt ttg              1981
Lys Thr Lys Ile Phe Glu His Ile Ile Val Asp Leu Ser Leu
    510                 515                 520

tgaaggtgtg gtgggtgaaa ctaagagaaa tgtagcatta gctctcacaa aagaggacca      2041

agaaaaatca acaaacaaat caaagccagg ctcagagctc tgaaattaaa aagcactgaa      2101

atagttagat gttttcaaac ttttagaact cacattttaa tcagggattg catttattgg      2161

ctaactgcat gacatgccca ttctaccatt ttaaaaaaaa tcttaaagcc tgtaatttct      2221

gagaaaagag tacagcattt actcttatca tctagaaatg taatatgctt cccccccgct      2281

ttttgatgag gaagaagaca attggataag atgggacagc acttataatg aaataaaaaa      2341

aaactttgag cccctctcat tccactttag caatcttttt ggtaagaact cttaaagcca      2401

aaagtctgct gaaaagattt gctgattatt agtttaaaaa tcttgtaaca ctcagcagtg      2461

ctattttgag tcatcccagt ttcctgaaag taatgcccag tcttcctgaa tcctccttaa      2521

tagcagaacc ttggtgattt tgttggctca tatgaatgct tgtcatggat atgttaacaa      2581

tttagtgttt gacattgctt cctctgccac aaagacaata ctctggtgac acatgtctag      2641

acccagcaca ggctgtaggc ccaggagtga ctcaaaggag tttttccctc tttcttacgg      2701

ttcaaaggtg accctggtgg tggccagagc agtaatgctt gtttgatgct cttcatggct      2761

catctgcttc tcagaaccca cccgttgagt ttgtgggtaa ccagcaggca ggccaaagac      2821

tggtgctttt catttcatcc tttagaggga tgaaacagtt atttccgtct gatgagcatt      2881

cggtagaatt tttgaagtga gattttatga agtcaaaggg gactttacac agatctcgac      2941

ctgctttgaa acctagaggt ggccctttga tttgtgcgtg tccttgccct ctggacaact 3001

taatatttca agtaatcgaa taccaacttc cctgccagcc cacctgcctt ccgccccgct 3061

tgtgtaacag tcctgttttg ttgagttgct gctattgcac tgccagtgca gcccacacca 3121

aatcacaacc caagatactc agataggaag actccttcct ctcccagtac tttaccaaag 3181

gaacccccgc caggacccac atggggccac gtgttggcag tggaatcagc ctgtgcaggc 3241

tggggatctc aggctgatca gtaggggcca gctttggagc cagccaagct gaatcccaca 3301

ctccaggtct gtgctcaaga gaccagatgg tgtatttcca aatgggcctc tctggtatgg 3361

gcaataggca agctcctggg gtctggttat gtggaagatt cttagtggat gttccgcctg 3421

gttagctggt tctcttcaga gaatataaag tgaatgcctt taggggtagc tctgaaagag 3481

aaacccaaca acttcattcc tagccatgaa agtagcacga tcatattgta ctgtattgtt 3541

attgtaaaat gactatttgc catgtcatga gtaggtagat gttttgccac aaatatgaat 3601

gtgtttgttg tttcctgact ttaagcaatg aagattgaga caataaatag cactcagaga 3661

atg 3664

<210> 6

<211> 545

<212> PRT

<213> Homo sapiens

<400> 6; LP224

Met Ala Leu Arg Arg Gly Gly Cys Gly Ala Leu Gly Leu Leu Leu Leu
-20 -15 -10

Leu Leu Gly Ala Ala Cys Leu Ile Pro Arg Ser Ala Gln Val Arg Arg
-5 -1 1 5

Leu Ala Arg Cys Pro Ala Thr Cys Ser Cys Thr Lys Glu Ser Ile Ile
10 15 20 25

Cys Val Gly Ser Ser Trp Val Pro Arg Ile Val Pro Gly Asp Ile Ser
30 35 40

Ser Leu Ser Leu Val Asn Gly Thr Phe Ser Glu Ile Lys Asp Arg Met
45 50 55

Phe Ser His Leu Pro Ser Leu Gln Leu Leu Leu Leu Asn Ser Asn Ser
60 65 70

Phe Thr Ile Ile Arg Asp Asp Ala Phe Ala Gly Leu Phe His Leu Glu
75 80 85

Tyr Leu Phe Ile Glu Gly Asn Lys Ile Glu Thr Ile Ser Arg Asn Ala

90                  95                  100                 105

Phe Arg Gly Leu Arg Asp Leu Thr His Leu Ser Leu Ala Asn Asn His
                110                 115                 120

Ile Lys Ala Leu Pro Arg Asp Val Phe Ser Asp Leu Asp Ser Leu Ile
            125                 130                 135

Glu Leu Asp Leu Arg Gly Asn Lys Phe Glu Cys Asp Cys Lys Ala Lys
        140                 145                 150

Trp Leu Tyr Leu Trp Leu Lys Met Thr Asn Ser Thr Val Ser Asp Val
    155                 160                 165

Leu Cys Ile Gly Pro Pro Glu Tyr Gln Glu Lys Lys Leu Asn Asp Val
170                 175                 180                 185

Thr Ser Phe Asp Tyr Glu Cys Thr Thr Thr Asp Phe Val Val His Gln
                190                 195                 200

Thr Leu Pro Tyr Gln Ser Val Ser Val Asp Thr Phe Asn Ser Lys Asn
            205                 210                 215

Asp Val Tyr Val Ala Ile Ala Gln Pro Ser Met Glu Asn Cys Met Val
        220                 225                 230

Leu Glu Trp Asp His Ile Glu Met Asn Phe Arg Ser Tyr Asp Asn Ile
    235                 240                 245

Thr Gly Gln Ser Ile Val Gly Cys Lys Ala Ile Leu Ile Asp Asp Gln
250                 255                 260                 265

Val Phe Val Val Val Ala Gln Leu Phe Gly Gly Ser His Ile Tyr Lys
                270                 275                 280

Tyr Asp Glu Ser Trp Thr Lys Phe Val Lys Phe Gln Asp Ile Glu Val
            285                 290                 295

Ser Arg Ile Ser Lys Pro Asn Asp Ile Glu Leu Phe Gln Ile Asp Asp
        300                 305                 310

Glu Thr Phe Phe Val Ile Ala Asp Ser Ser Lys Ala Gly Leu Ser Thr
    315                 320                 325

Val Tyr Lys Trp Asn Ser Lys Gly Phe Tyr Ser Tyr Gln Ser Leu His
330                 335                 340                 345

Glu Trp Phe Arg Asp Thr Asp Ala Glu Phe Val Asp Ile Asp Gly Lys
                350                 355                 360

Ser His Leu Ile Leu Ser Ser Arg Ser Gln Val Pro Ile Ile Leu Gln
            365                 370                 375

Trp Asn Lys Ser Ser Lys Lys Phe Val Pro His Gly Asp Ile Pro Asn
        380                 385                 390

Met Glu Asp Val Leu Ala Val Lys Ser Phe Arg Met Gln Asn Thr Leu
    395                 400                 405

Tyr Leu Ser Leu Thr Arg Phe Ile Gly Asp Ser Arg Val Met Arg Trp
410                 415                 420                 425

Asn Ser Lys Gln Phe Val Glu Ile Gln Ala Leu Pro Ser Arg Gly Ala
                430                 435                 440

Met Thr Leu Gln Pro Phe Ser Phe Lys Asp Asn His Tyr Leu Ala Leu
            445                 450                 455

Gly Ser Asp Tyr Thr Phe Ser Gln Ile Tyr Gln Trp Asp Lys Glu Lys
        460                 465                 470

Gln Leu Phe Lys Lys Phe Lys Glu Ile Tyr Val Gln Ala Pro Arg Ser
    475                 480                 485

Phe Thr Ala Val Ser Thr Asp Arg Arg Asp Phe Phe Phe Ala Ser Ser
490                 495                 500                 505

Phe Lys Gly Lys Thr Lys Ile Phe Glu His Ile Ile Val Asp Leu Ser
                510                 515                 520

Leu

<210>  7

<211>  898

<212>  DNA

<213>  Homo sapiens

<220>

<221>  CDS

<222>  (19)..(642)

<223>

<220>

<221>  mat_peptide

<222>  (64)..()

<223>

<400>  7; LP240
cggcgggccg ggacgggc atg gcc ctg ctg ctg tgc ctg gtg tgc ctg acg      51
                    Met Ala Leu Leu Leu Cys Leu Val Cys Leu Thr
                    -15                 -10                 -5

gcg gcg ctg gcc cac ggc tgt ctg cac tgc cac agc aac ttc tcc aag      99
Ala Ala Leu Ala His Gly Cys Leu His Cys His Ser Asn Phe Ser Lys
            -1  1               5                   10

aag ttc tcc ttc tac cgc cac cat gtg aac ttc aag tcc tgg tgg gtg      147
Lys Phe Ser Phe Tyr Arg His His Val Asn Phe Lys Ser Trp Trp Val
        15                  20                  25

ggc gac atc ccc gtg tca ggg gcg ctg ctc acc gac tgg agc gac gac      195
Gly Asp Ile Pro Val Ser Gly Ala Leu Leu Thr Asp Trp Ser Asp Asp
    30                  35                  40

acg atg aag gag ctg cac ctg gcc atc ccc gcc aag atc acc cgg gag      243
Thr Met Lys Glu Leu His Leu Ala Ile Pro Ala Lys Ile Thr Arg Glu
45                  50                  55                  60

aag ctg gac caa gtg gcg aca gca gtg tac cag atg atg gat cag ctg      291
Lys Leu Asp Gln Val Ala Thr Ala Val Tyr Gln Met Met Asp Gln Leu
                65                  70                  75

tac cag ggg aag atg tac ttc ccc ggg tat ttc ccc aac gag ctg cga      339
Tyr Gln Gly Lys Met Tyr Phe Pro Gly Tyr Phe Pro Asn Glu Leu Arg
            80                  85                  90

aac atc ttc cgg gag cag gtg cac ctc atc cag aac gcc atc atc gaa      387
Asn Ile Phe Arg Glu Gln Val His Leu Ile Gln Asn Ala Ile Ile Glu
        95                  100                 105

agc cgc atc gac tgt cag cac cgc tgt ggc atc ttc cag tac gag acc      435
Ser Arg Ile Asp Cys Gln His Arg Cys Gly Ile Phe Gln Tyr Glu Thr
    110                 115                 120

atc tcc tgc aac aac tgc aca gac tcg cac gtc gcc tgc ttt ggc tat      483
Ile Ser Cys Asn Asn Cys Thr Asp Ser His Val Ala Cys Phe Gly Tyr
125                 130                 135                 140

aac tgc gag tcc tcg gcg cag tgg aag tca gct gtc cag ggc ctc ctg      531
Asn Cys Glu Ser Ser Ala Gln Trp Lys Ser Ala Val Gln Gly Leu Leu
                145                 150                 155

aac tac ata aat aac tgg cac aac ctg gta tcg cca gcc tta agg tgt      579
Asn Tyr Ile Asn Asn Trp His Asn Leu Val Ser Pro Ala Leu Arg Cys
            160                 165                 170

ctg gag ccc cca cac ttg gcc aac ctg acc ttg gaa aat gct gct gag      627
Leu Glu Pro Pro His Leu Ala Asn Leu Thr Leu Glu Asn Ala Ala Glu
        175                 180                 185

tgt ctc aag cag cac tgacagcagc tgggcctgcc ccagggcaac gtgggggcgg      682
Cys Leu Lys Gln His
    190

agactcagct ggacagcccc tgcctgtcac tctggagctg ggctgctgct gcctcaggac      742

cccctctccg accccggaca gagctgagct ggccagggcc aggagggcgg gagggaggga      802

atgggggtgg gctgtgcgca gcatcagcgc ctgggcaggt ccgcagagct gcgggatgtg      862

attaaagtcc ctgatgtttc tcaaaaaaaa aaaaaa                                898

<210>  8

<211>  208

<212>  PRT

<213>  Homo sapiens

<400>  8; LP240

Met Ala Leu Leu Leu Cys Leu Val Cys Leu Thr Ala Ala Leu Ala His
-15                 -10                 -5                  -1  1

Gly Cys Leu His Cys His Ser Asn Phe Ser Lys Lys Phe Ser Phe Tyr
            5                   10                  15

Arg His His Val Asn Phe Lys Ser Trp Trp Val Gly Asp Ile Pro Val
        20                  25                  30

Ser Gly Ala Leu Leu Thr Asp Trp Ser Asp Asp Thr Met Lys Glu Leu
    35                  40                  45

His Leu Ala Ile Pro Ala Lys Ile Thr Arg Glu Lys Leu Asp Gln Val
50                  55                  60                  65

Ala Thr Ala Val Tyr Gln Met Met Asp Gln Leu Tyr Gln Gly Lys Met
                70                  75                  80

Tyr Phe Pro Gly Tyr Phe Pro Asn Glu Leu Arg Asn Ile Phe Arg Glu
            85                  90                  95

Gln Val His Leu Ile Gln Asn Ala Ile Ile Glu Ser Arg Ile Asp Cys
        100                 105                 110

Gln His Arg Cys Gly Ile Phe Gln Tyr Glu Thr Ile Ser Cys Asn Asn
    115                 120                 125

Cys Thr Asp Ser His Val Ala Cys Phe Gly Tyr Asn Cys Glu Ser Ser
130                 135                 140                 145

Ala Gln Trp Lys Ser Ala Val Gln Gly Leu Leu Asn Tyr Ile Asn Asn
                150                 155                 160

Trp His Asn Leu Val Ser Pro Ala Leu Arg Cys Leu Glu Pro Pro His
            165                 170                 175

Leu Ala Asn Leu Thr Leu Glu Asn Ala Ala Glu Cys Leu Lys Gln His
        180                 185                 190

<210>  9

<211>  1580

<212>  DNA

<213>  Homo sapiens

<220>
<221>  CDS
<222>  (136)..(1386)
<223>

<220>
<221>  mat_peptide
<222>  (196)..()
<223>

<400>  9; LP239(a)
ctcaattctt aaaaaataag tccatgaagc agaaacatca aaaagcatgg gatttggaat      60

ttgagacctg actttgaagc ccacacagaa cacagacaag tcaaccatgc tatttgggac     120

atattttgtt ccaaa atg gca tct tac ctt tat gga gta ctc ttt gct gtt      171
                 Met Ala Ser Tyr Leu Tyr Gly Val Leu Phe Ala Val
                 -20                 -15                 -10

ggc ctc tgt gct cca atc tac tgt gtg tcc ccg gcc aat gcc ccc agt      219
Gly Leu Cys Ala Pro Ile Tyr Cys Val Ser Pro Ala Asn Ala Pro Ser
            -5                  -1  1               5

gca tac ccc cgc cct tcc tcc aca aag agc acc cct gcc tca cag gtg      267
Ala Tyr Pro Arg Pro Ser Ser Thr Lys Ser Thr Pro Ala Ser Gln Val
    10                  15                  20

tat tcc ctc aac acc gac ttt gcc ttc cgc cta tac cgc agg ctg gtt      315
Tyr Ser Leu Asn Thr Asp Phe Ala Phe Arg Leu Tyr Arg Arg Leu Val
25                  30                  35                  40

ttg gag acc ccg agt cag aac atc ttc ttc tcc cct gtg agt gtc tcc      363
Leu Glu Thr Pro Ser Gln Asn Ile Phe Phe Ser Pro Val Ser Val Ser
                45                  50                  55

act tcc ctg gcc atg ctc tcc ctt ggg gcc cac tca gtc acc aag acc      411
Thr Ser Leu Ala Met Leu Ser Leu Gly Ala His Ser Val Thr Lys Thr
            60                  65                  70

cag att ctc cag ggc ctg ggc ttc aac ctc aca cac aca cca gag tct      459
Gln Ile Leu Gln Gly Leu Gly Phe Asn Leu Thr His Thr Pro Glu Ser
        75                  80                  85

gcc atc cac cag ggc ttc cag cac ctg gtt cac tca ctg act gtt ccc      507
Ala Ile His Gln Gly Phe Gln His Leu Val His Ser Leu Thr Val Pro
    90                  95                  100

agc aaa gac ctg acc ttg aag atg gga agt gcc ctc ttc gtc aag aag      555
Ser Lys Asp Leu Thr Leu Lys Met Gly Ser Ala Leu Phe Val Lys Lys
105                 110                 115                 120

gag ctg cag ctg cag gca aat ttc ttg ggc aat gtc aag agg ctg tat      603
Glu Leu Gln Leu Gln Ala Asn Phe Leu Gly Asn Val Lys Arg Leu Tyr
                125                 130                 135

gaa gca gaa gtc ttt tct aca gat ttc tcc aac ccc tcc att gcc cag     651
Glu Ala Glu Val Phe Ser Thr Asp Phe Ser Asn Pro Ser Ile Ala Gln
            140                 145                 150

gcg agg atc aac agc cat gtg aaa aag aag acc caa ggg aag gtt gta     699
Ala Arg Ile Asn Ser His Val Lys Lys Lys Thr Gln Gly Lys Val Val
        155                 160                 165

gac ata atc caa ggc ctt gac ctt ctg acg gcc atg gtt ctg gtg aac     747
Asp Ile Ile Gln Gly Leu Asp Leu Leu Thr Ala Met Val Leu Val Asn
    170                 175                 180

cac att ttc ttt aaa gcc aag tgg gag aag ccc ttt cac cct gaa tat     795
His Ile Phe Phe Lys Ala Lys Trp Glu Lys Pro Phe His Pro Glu Tyr
185                 190                 195                 200

aca aga aag aac ttc cca ttc ctg gtg ggc gag cag gtc act gtg cat     843
Thr Arg Lys Asn Phe Pro Phe Leu Val Gly Glu Gln Val Thr Val His
                205                 210                 215

gtc ccc atg atg cac cag aaa gag cag ttc gct ttt ggg gtg gat aca     891
Val Pro Met Met His Gln Lys Glu Gln Phe Ala Phe Gly Val Asp Thr
            220                 225                 230

gag ctg aac tgc ttt gtg ctg cag atg gat tac aag gga gat gcc gtg     939
Glu Leu Asn Cys Phe Val Leu Gln Met Asp Tyr Lys Gly Asp Ala Val
        235                 240                 245

gcc ttc ttt gtc ctc cct agc aag ggc aag atg agg caa ctg gaa cag     987
Ala Phe Phe Val Leu Pro Ser Lys Gly Lys Met Arg Gln Leu Glu Gln
    250                 255                 260

gcc ttg tca gcc aga aca ctg aga aag tgg agc cac tca ctc cag aaa     1035
Ala Leu Ser Ala Arg Thr Leu Arg Lys Trp Ser His Ser Leu Gln Lys
265                 270                 275                 280

agg tgg ata gag gtg ttc atc ccc aga ttt tcc att tct gcc tcc tac     1083
Arg Trp Ile Glu Val Phe Ile Pro Arg Phe Ser Ile Ser Ala Ser Tyr
                285                 290                 295

aat ctg gaa acc atc ctc ccg aag atg ggc atc caa aat gtc ttt gac     1131
Asn Leu Glu Thr Ile Leu Pro Lys Met Gly Ile Gln Asn Val Phe Asp
            300                 305                 310

aaa aat gct gat ttt tct gga att gca aag aga gac tcc ctg cag gtt     1179
Lys Asn Ala Asp Phe Ser Gly Ile Ala Lys Arg Asp Ser Leu Gln Val
        315                 320                 325

tct aaa gca acc cac aag gct gtg ctg gat gtc agt gaa gag ggc act     1227
Ser Lys Ala Thr His Lys Ala Val Leu Asp Val Ser Glu Glu Gly Thr
    330                 335                 340

gag gcc aca gca gct acc acc acc aag ttc ata gtc cga tcg aag gat     1275
Glu Ala Thr Ala Ala Thr Thr Thr Lys Phe Ile Val Arg Ser Lys Asp
345                 350                 355                 360

ggc ccc tct tac ttc act gtc tcc ttc aat agg acc ttc ctg atg atg     1323
Gly Pro Ser Tyr Phe Thr Val Ser Phe Asn Arg Thr Phe Leu Met Met
                365                 370                 375

att aca aat aaa gcc aca gac ggt att ctc ttt cta ggg aaa gtg gaa     1371
Ile Thr Asn Lys Ala Thr Asp Gly Ile Leu Phe Leu Gly Lys Val Glu
            380                 385                 390

aat ccc act aaa tcc taggtgggaa atggcctgtt aactgatggc acattgctaa     1426
Asn Pro Thr Lys Ser
        395

tgcacaagaa ataacaaacc acatccctct ttctgttctg agggtgcatt tgaccccagt 1486
ggagctggat tcgctggcag ggatgccact tccaaggctc aatcaccaaa ccatcaacag 1546
ggaccccagt cacaagccaa cacccattaa cccc 1580

<210> 10
<211> 417
<212> PRT
<213> Homo sapiens

<400> 10; LP239(a)
Met Ala Ser Tyr Leu Tyr Gly Val Leu Phe Ala Val Gly Leu Cys Ala
-20 -15 -10 -5
Pro Ile Tyr Cys Val Ser Pro Ala Asn Ala Pro Ser Ala Tyr Pro Arg
-1 1 5 10
Pro Ser Ser Thr Lys Ser Thr Pro Ala Ser Gln Val Tyr Ser Leu Asn
15 20 25
Thr Asp Phe Ala Phe Arg Leu Tyr Arg Arg Leu Val Leu Glu Thr Pro
30 35 40
Ser Gln Asn Ile Phe Phe Ser Pro Val Ser Val Ser Thr Ser Leu Ala
45 50 55 60
Met Leu Ser Leu Gly Ala His Ser Val Thr Lys Thr Gln Ile Leu Gln
65 70 75
Gly Leu Gly Phe Asn Leu Thr His Thr Pro Glu Ser Ala Ile His Gln
80 85 90
Gly Phe Gln His Leu Val His Ser Leu Thr Val Pro Ser Lys Asp Leu
95 100 105
Thr Leu Lys Met Gly Ser Ala Leu Phe Val Lys Lys Glu Leu Gln Leu
110 115 120
Gln Ala Asn Phe Leu Gly Asn Val Lys Arg Leu Tyr Glu Ala Glu Val
125 130 135 140
Phe Ser Thr Asp Phe Ser Asn Pro Ser Ile Ala Gln Ala Arg Ile Asn
145 150 155
Ser His Val Lys Lys Lys Thr Gln Gly Lys Val Val Asp Ile Ile Gln
160 165 170
Gly Leu Asp Leu Leu Thr Ala Met Val Leu Val Asn His Ile Phe Phe
175 180 185

Lys Ala Lys Trp Glu Lys Pro Phe His Pro Glu Tyr Thr Arg Lys Asn
    190                 195                 200

Phe Pro Phe Leu Val Gly Glu Gln Val Thr Val His Val Pro Met Met
205                 210                 215                 220

His Gln Lys Glu Gln Phe Ala Phe Gly Val Asp Thr Glu Leu Asn Cys
                225                 230                 235

Phe Val Leu Gln Met Asp Tyr Lys Gly Asp Ala Val Ala Phe Phe Val
            240                 245                 250

Leu Pro Ser Lys Gly Lys Met Arg Gln Leu Glu Gln Ala Leu Ser Ala
        255                 260                 265

Arg Thr Leu Arg Lys Trp Ser His Ser Leu Gln Lys Arg Trp Ile Glu
    270                 275                 280

Val Phe Ile Pro Arg Phe Ser Ile Ser Ala Ser Tyr Asn Leu Glu Thr
285                 290                 295                 300

Ile Leu Pro Lys Met Gly Ile Gln Asn Val Phe Asp Lys Asn Ala Asp
                305                 310                 315

Phe Ser Gly Ile Ala Lys Arg Asp Ser Leu Gln Val Ser Lys Ala Thr
            320                 325                 330

His Lys Ala Val Leu Asp Val Ser Glu Glu Gly Thr Glu Ala Thr Ala
        335                 340                 345

Ala Thr Thr Thr Lys Phe Ile Val Arg Ser Lys Asp Gly Pro Ser Tyr
    350                 355                 360

Phe Thr Val Ser Phe Asn Arg Thr Phe Leu Met Met Ile Thr Asn Lys
365                 370                 375                 380

Ala Thr Asp Gly Ile Leu Phe Leu Gly Lys Val Glu Asn Pro Thr Lys
                385                 390                 395

Ser

<210>  11

<211>  2080

<212>  DNA

<213>  Homo sapiens

<220>

<221>  CDS

<222>  (12)..(1682)

<223>



<220>

<221>  mat_peptide

<222>  (96)..()

<223>



<220>

<221>  misc_feature

<222>  (82)..(82)

<223>  wherein n is A, G, C, or T



<400>  11; LP243(a)
tcaccaccat c atg gtg tcc acc gcc atg ggc tgc atc acc ttc ctg ggc       50
             Met Val Ser Thr Ala Met Gly Cys Ile Thr Phe Leu Gly
                         -25                 -20

gtg gtc ctc ttg ctg ctt tcg tgc tgc tgt tnc gtg tgg agc cgc aac       98
Val Val Leu Leu Leu Leu Ser Cys Cys Cys Xaa Val Trp Ser Arg Asn
-15                 -10                 -5                  -1  1

cgc atc cgc tgc ctg aac ccg ggc gac ctg gcc gcg ctg ccc gcg ctg      146
Arg Ile Arg Cys Leu Asn Pro Gly Asp Leu Ala Ala Leu Pro Ala Leu
            5                   10                  15

gag gag ctg gac ctg agc gag aac gcc atc gcg cac gtg gag ccc ggc      194
Glu Glu Leu Asp Leu Ser Glu Asn Ala Ile Ala His Val Glu Pro Gly
        20                  25                  30

gcc ttc gcc aac ctg ccg cgc ctg cgc gtc ctg cgt ctc cgt ggc aac      242
Ala Phe Ala Asn Leu Pro Arg Leu Arg Val Leu Arg Leu Arg Gly Asn
    35                  40                  45

cag ctg aag ctc atc ccg ccc ggg gtc ttc acg cgc ctg gac aac ctc      290
Gln Leu Lys Leu Ile Pro Pro Gly Val Phe Thr Arg Leu Asp Asn Leu
50                  55                  60                  65

acg ctg ctg gac ctg agc gag aac aag ctg gta atc ctg ctg gac tac      338
Thr Leu Leu Asp Leu Ser Glu Asn Lys Leu Val Ile Leu Leu Asp Tyr
                70                  75                  80

act ttc cag gac ctg cac agc ctg cgc cgg ctg gaa gtg ggc gac aac      386
Thr Phe Gln Asp Leu His Ser Leu Arg Arg Leu Glu Val Gly Asp Asn
            85                  90                  95

gac ctg gta ttc gtc tcg cgc cgc gcc ttc gcg ggg ctg ctg gcc ctg      434
Asp Leu Val Phe Val Ser Arg Arg Ala Phe Ala Gly Leu Leu Ala Leu
        100                 105                 110

gag gag ctg acc ctg gag cgc tgc aac ctc acg gct ctg tcc ggg gag      482
Glu Glu Leu Thr Leu Glu Arg Cys Asn Leu Thr Ala Leu Ser Gly Glu
    115                 120                 125

tcg ctg ggc cat ctg cgc agc ctg ggc gcc ctg cgg ctg cgc cac ctg 530
Ser Leu Gly His Leu Arg Ser Leu Gly Ala Leu Arg Leu Arg His Leu
130 135 140 145

gcc atc gcc tcc ctg gag gac cag aac ttc cgc agg ctg ccc ggg ctg 578
Ala Ile Ala Ser Leu Glu Asp Gln Asn Phe Arg Arg Leu Pro Gly Leu
150 155 160

ctg cac ctg gag att gac aac tgg ccg ctg ctg gag gag gtg gcg gcg 626
Leu His Leu Glu Ile Asp Asn Trp Pro Leu Leu Glu Glu Val Ala Ala
                165                 170                 175

ggc agc ctg cgg ggc ctg aac ctg acc tcg ctg tcg gtc acc cac acc 674
Gly Ser Leu Arg Gly Leu Asn Leu Thr Ser Leu Ser Val Thr His Thr
            180                 185                 190

aac atc acc gcc gtg ccg gcc gcc gcg ctg cgg cac cag gcg cac ctc 722
Asn Ile Thr Ala Val Pro Ala Ala Ala Leu Arg His Gln Ala His Leu
        195                 200                 205

acc tgc ctc aat ctg tcg cac aac ccc atc agc acg gtg ccg cgg ggg 770
Thr Cys Leu Asn Leu Ser His Asn Pro Ile Ser Thr Val Pro Arg Gly
210 215 220 225

tcg ttc cgg gac ctg gtc cgc ctg cgc gag ctg cac ctg gcc ggg gcc 818
Ser Phe Arg Asp Leu Val Arg Leu Arg Glu Leu His Leu Ala Gly Ala
230 235 240

ctg ctg gct gtg gtg gag ccg cag gcc ttc ctg ggc ctg cgc cag atc 866
Leu Leu Ala Val Val Glu Pro Gln Ala Phe Leu Gly Leu Arg Gln Ile
                245                 250                 255

cgc ctg ctc aac ctc tcc aac aac ctg ctc tcc acg ttg gag gag agc 914
Arg Leu Leu Asn Leu Ser Asn Asn Leu Leu Ser Thr Leu Glu Glu Ser
            260                 265                 270

acc ttc cac tcg gtg aac acg cta gag acg ctg cgc gtg gac ggg aac 962
Thr Phe His Ser Val Asn Thr Leu Glu Thr Leu Arg Val Asp Gly Asn
        275                 280                 285

ccg ctg gcc tgc gac tgt cgc ctg ctg tgg atc gtg cag cgt cgc aag 1010
Pro Leu Ala Cys Asp Cys Arg Leu Leu Trp Ile Val Gln Arg Arg Lys
290 295 300 305

acc ctc aac ttc gac ggg cgg ctg ccg gcc tgc gcc acc ccg gcc gag 1058
Thr Leu Asn Phe Asp Gly Arg Leu Pro Ala Cys Ala Thr Pro Ala Glu
310 315 320

gtg cgc ggc gac gcg ctg cga aac ctg ccg gac tcc gtg ctg ttc gag 1106
Val Arg Gly Asp Ala Leu Arg Asn Leu Pro Asp Ser Val Leu Phe Glu
                325                 330                 335

tac ttc gtg tgc cgc aaa ccc aag atc cgg gag cgg cgg ctg cag cgc 1154
Tyr Phe Val Cys Arg Lys Pro Lys Ile Arg Glu Arg Arg Leu Gln Arg
            340                 345                 350

gtc acg gcc acc gcg ggc gaa gac gtc cgc ttc ctc tgc cgc gcc gag 1202
Val Thr Ala Thr Ala Gly Glu Asp Val Arg Phe Leu Cys Arg Ala Glu
        355                 360                 365

ggc gag ccg gcg ccc acc gtg gcc tgg gtg acc ccc cag cac cgg ccg 1250
Gly Glu Pro Ala Pro Thr Val Ala Trp Val Thr Pro Gln His Arg Pro
370 375 380 385

gtg acg gcc acc agc gcg ggc cgg gcg cgc gtg ctc ccc ggg ggg acg 1298
Val Thr Ala Thr Ser Ala Gly Arg Ala Arg Val Leu Pro Gly Gly Thr
390 395 400

ctg gag atc cag gac gcg cgg ccg cag gac agc ggc acc tac acg tgc     1346
Leu Glu Ile Gln Asp Ala Arg Pro Gln Asp Ser Gly Thr Tyr Thr Cys
            405                 410                 415

gtg gcc agc aac gcg ggc ggc aac gac acc tac ttc gcc acg ctg acc     1394
Val Ala Ser Asn Ala Gly Gly Asn Asp Thr Tyr Phe Ala Thr Leu Thr
        420                 425                 430

gtg cgc ccc gag ccg gcc gcc aac cgg acc ccg ggc gag gcc cac aac     1442
Val Arg Pro Glu Pro Ala Ala Asn Arg Thr Pro Gly Glu Ala His Asn
    435                 440                 445

gag acg ctg gcg gcc ctg cgc gcg ccg ctc gac ctc acc acc atc ctg     1490
Glu Thr Leu Ala Ala Leu Arg Ala Pro Leu Asp Leu Thr Thr Ile Leu
450                 455                 460                 465

gtg tcc acc gcc atg ggc tgc atc acc ttc ctg ggc gtg gtc ctc ttc     1538
Val Ser Thr Ala Met Gly Cys Ile Thr Phe Leu Gly Val Val Leu Phe
                470                 475                 480

tgc ttc gtg ctg ctg ttc gtg tgg agc cgc ggc cgc ggg cag cac aaa     1586
Cys Phe Val Leu Leu Phe Val Trp Ser Arg Gly Arg Gly Gln His Lys
            485                 490                 495

aac aac ttc tcg gtg gag tac tcc ttc cgc aag gtg gat ggg ccg gcc     1634
Asn Asn Phe Ser Val Glu Tyr Ser Phe Arg Lys Val Asp Gly Pro Ala
        500                 505                 510

gcc gcg gcg ggc cag gga ggc gcg cgc aag ttc aac atg aag atg atc     1682
Ala Ala Ala Gly Gln Gly Gly Ala Arg Lys Phe Asn Met Lys Met Ile
    515                 520                 525

tgaggggtcc ccagggcgga ccctcccctc ccctcccccg cgggccggcc gctcgcgtgt     1742

ccacctatgc atttcccgga ggggaagggg acggctgcac ggcgctcccc aggcagaact     1802

tccccttttt ttgtagacgc ccaaccgcag gactgttttt catcagcatg cgtctttttt     1862

gcagtttctc aagcgttttc taaagatttc aacccgtctt cctgtccctg ctatggtggc     1922

tgagctgggg ggtgggggtg cgggctgcaa ggatgggggg atgggctccc gtctaccaac     1982

ccggggtggc tgggggcgct cagagacccc aggatcctca gggcccgcaa ttcacaccca     2042

ggaggcacaa gactgctgcc cccaagccct gggcttcc                             2080

<210>  12

<211>  557

<212>  PRT

<213>  Homo sapiens


<220>

<221>  misc_feature

<222>  (-5)..(-5)

<223>  The 'Xaa' at location -5 stands for Tyr, Cys, Ser, or Phe.

<220>

<221>  misc_feature

<222>  (82)..(82)

<223>  wherein n is A, G, C, or T

<400>  12; LP243(a)

Met Val Ser Thr Ala Met Gly Cys Ile Thr Phe Leu Gly Val Val Leu
            -25                 -20                 -15

Leu Leu Leu Ser Cys Cys Cys Xaa Val Trp Ser Arg Asn Arg Ile Arg
        -10                 -5                  -1  1

Cys Leu Asn Pro Gly Asp Leu Ala Ala Leu Pro Ala Leu Glu Glu Leu
5                   10                  15                  20

Asp Leu Ser Glu Asn Ala Ile Ala His Val Glu Pro Gly Ala Phe Ala
                25                  30                  35

Asn Leu Pro Arg Leu Arg Val Leu Arg Leu Arg Gly Asn Gln Leu Lys
            40                  45                  50

Leu Ile Pro Pro Gly Val Phe Thr Arg Leu Asp Asn Leu Thr Leu Leu
        55                  60                  65

Asp Leu Ser Glu Asn Lys Leu Val Ile Leu Leu Asp Tyr Thr Phe Gln
    70                  75                  80

Asp Leu His Ser Leu Arg Arg Leu Glu Val Gly Asp Asn Asp Leu Val
85                  90                  95                  100

Phe Val Ser Arg Arg Ala Phe Ala Gly Leu Leu Ala Leu Glu Glu Leu
                105                 110                 115

Thr Leu Glu Arg Cys Asn Leu Thr Ala Leu Ser Gly Glu Ser Leu Gly
            120                 125                 130

His Leu Arg Ser Leu Gly Ala Leu Arg Leu Arg His Leu Ala Ile Ala
        135                 140                 145

Ser Leu Glu Asp Gln Asn Phe Arg Arg Leu Pro Gly Leu Leu His Leu
    150                 155                 160

Glu Ile Asp Asn Trp Pro Leu Leu Glu Glu Val Ala Ala Gly Ser Leu
165                 170                 175                 180

Arg Gly Leu Asn Leu Thr Ser Leu Ser Val Thr His Thr Asn Ile Thr
                185                 190                 195

Ala Val Pro Ala Ala Ala Leu Arg His Gln Ala His Leu Thr Cys Leu
            200                 205                 210

Asn Leu Ser His Asn Pro Ile Ser Thr Val Pro Arg Gly Ser Phe Arg
        215                 220                 225

Asp Leu Val Arg Leu Arg Glu Leu His Leu Ala Gly Ala Leu Leu Ala
    230                 235                 240

Val Val Glu Pro Gln Ala Phe Leu Gly Leu Arg Gln Ile Arg Leu Leu
245                 250                 255                 260

Asn Leu Ser Asn Asn Leu Leu Ser Thr Leu Glu Glu Ser Thr Phe His
                265                 270                 275

Ser Val Asn Thr Leu Glu Thr Leu Arg Val Asp Gly Asn Pro Leu Ala
            280                 285                 290

Cys Asp Cys Arg Leu Leu Trp Ile Val Gln Arg Arg Lys Thr Leu Asn
        295                 300                 305

Phe Asp Gly Arg Leu Pro Ala Cys Ala Thr Pro Ala Glu Val Arg Gly
    310                 315                 320

Asp Ala Leu Arg Asn Leu Pro Asp Ser Val Leu Phe Glu Tyr Phe Val
325                 330                 335                 340

Cys Arg Lys Pro Lys Ile Arg Glu Arg Arg Leu Gln Arg Val Thr Ala
                345                 350                 355

Thr Ala Gly Glu Asp Val Arg Phe Leu Cys Arg Ala Glu Gly Glu Pro
            360                 365                 370

Ala Pro Thr Val Ala Trp Val Thr Pro Gln His Arg Pro Val Thr Ala
        375                 380                 385

Thr Ser Ala Gly Arg Ala Arg Val Leu Pro Gly Gly Thr Leu Glu Ile
    390                 395                 400

Gln Asp Ala Arg Pro Gln Asp Ser Gly Thr Tyr Thr Cys Val Ala Ser
405                 410                 415                 420

Asn Ala Gly Gly Asn Asp Thr Tyr Phe Ala Thr Leu Thr Val Arg Pro
                425                 430                 435

Glu Pro Ala Ala Asn Arg Thr Pro Gly Glu Ala His Asn Glu Thr Leu
            440                 445                 450

Ala Ala Leu Arg Ala Pro Leu Asp Leu Thr Thr Ile Leu Val Ser Thr
        455                 460                 465

Ala Met Gly Cys Ile Thr Phe Leu Gly Val Val Leu Phe Cys Phe Val
    470                 475                 480

Leu Leu Phe Val Trp Ser Arg Gly Arg Gly Gln His Lys Asn Asn Phe
485                 490                 495                 500

Ser Val Glu Tyr Ser Phe Arg Lys Val Asp Gly Pro Ala Ala Ala Ala
                505                 510                 515

Gly Gln Gly Gly Ala Arg Lys Phe Asn Met Lys Met Ile
            520                 525

<210>  13

<211>  2222

<212>  DNA

<213>  Homo sapiens

<220>

<221>  CDS

<222>  (49)..(1824)

<223>

<220>

<221>  mat_peptide

<222>  (121)..()

<223>

<400>  13; LP243(b)
aggtgcgcag gaggatggtg gcgcggccct aggcccacgc tccgcacc atg acc tgc      57
                                                     Met Thr Cys

tgg ctg tgc gtc ctg agc ctg ccc ctg ctc ctg ctg ccc gcg gcg ccg      105
Trp Leu Cys Val Leu Ser Leu Pro Leu Leu Leu Leu Pro Ala Ala Pro
    -20                 -15                 -10

ccc ccg gct gga ggc tgc ccg gcc cgc tgc gag tgc acc gtg cag acc      153
Pro Pro Ala Gly Gly Cys Pro Ala Arg Cys Glu Cys Thr Val Gln Thr
-5                  -1  1               5                   10

cgc gcg gtg gcc tgc acg cgc cgc cgc ctg acc gcc gtg ccc gac ggc      201
Arg Ala Val Ala Cys Thr Arg Arg Arg Leu Thr Ala Val Pro Asp Gly
            15                  20                  25

atc ccg gcc gag acc cgc ctg ctg gag ctc agc cgc aac cgc atc cgc      249
Ile Pro Ala Glu Thr Arg Leu Leu Glu Leu Ser Arg Asn Arg Ile Arg
        30                  35                  40

tgc ctg aac ccg ggc gac ctg gcc gcg ctg ccc gcg ctg gag gag ctg      297
Cys Leu Asn Pro Gly Asp Leu Ala Ala Leu Pro Ala Leu Glu Glu Leu
    45                  50                  55

gac ctg agc gag aac gcc atc gcg cac gtg gag ccc ggc gcc ttc gcc      345
Asp Leu Ser Glu Asn Ala Ile Ala His Val Glu Pro Gly Ala Phe Ala
60                  65                  70                  75

aac ctg ccg cgc ctg cgc gtc ctg cgt ctc cgt ggc aac cag ctg aag      393
Asn Leu Pro Arg Leu Arg Val Leu Arg Leu Arg Gly Asn Gln Leu Lys

                    80                  85                  90

ctc atc ccg ccc ggg gtc ttc acg cgc ctg gac aac ctc acg ctg ctg      441
Leu Ile Pro Pro Gly Val Phe Thr Arg Leu Asp Asn Leu Thr Leu Leu
            95                  100                 105

gac ctg agc gag aac aag ctg gta atc ctg ctg gac tac act ttc cag      489
Asp Leu Ser Glu Asn Lys Leu Val Ile Leu Leu Asp Tyr Thr Phe Gln
        110                 115                 120

gac ctg cac agc ctg cgc cgg ctg gaa gtg ggc gac aac gac ctg gta      537
Asp Leu His Ser Leu Arg Arg Leu Glu Val Gly Asp Asn Asp Leu Val
    125                 130                 135

ttc gtc tcg cgc cgc gcc ttc gcg ggg ctg ctg gcc ctg gag gag ctg      585
Phe Val Ser Arg Arg Ala Phe Ala Gly Leu Leu Ala Leu Glu Glu Leu
140                 145                 150                 155

acc ctg gag cgc tgc aac ctc acg gct ctg tcc ggg gag tcg ctg ggc      633
Thr Leu Glu Arg Cys Asn Leu Thr Ala Leu Ser Gly Glu Ser Leu Gly
                160                 165                 170

cat ctg cgc agc ctg ggc gcc ctg cgg ctg cgc cac ctg gcc atc gcc      681
His Leu Arg Ser Leu Gly Ala Leu Arg Leu Arg His Leu Ala Ile Ala
            175                 180                 185

tcc ctg gag gac cag aac ttc cgc agg ctg ccc ggg ctg ctg cac ctg      729
Ser Leu Glu Asp Gln Asn Phe Arg Arg Leu Pro Gly Leu Leu His Leu
        190                 195                 200

gag att gac aac tgg ccg ctg ctg gag gag gtg gcg gcg ggc agc ctg      777
Glu Ile Asp Asn Trp Pro Leu Leu Glu Glu Val Ala Ala Gly Ser Leu
    205                 210                 215

cgg ggc ctg aac ctg acc tcg ctg tcg gtc acc cac acc aac atc acc      825
Arg Gly Leu Asn Leu Thr Ser Leu Ser Val Thr His Thr Asn Ile Thr
220                 225                 230                 235

gcc gtg ccg gcc gcc gcg ctg cgg cac cag gcg cac ctc acc tgc ctc      873
Ala Val Pro Ala Ala Ala Leu Arg His Gln Ala His Leu Thr Cys Leu
                240                 245                 250

aat ctg tcg cac aac ccc atc agc acg gtg ccg cgg ggg tcg ttc cgg      921
Asn Leu Ser His Asn Pro Ile Ser Thr Val Pro Arg Gly Ser Phe Arg
            255                 260                 265

gac ctg gtc cgc ctg cgc gag ctg cac ctg gcc ggg gcc ctg ctg gct      969
Asp Leu Val Arg Leu Arg Glu Leu His Leu Ala Gly Ala Leu Leu Ala
        270                 275                 280

gtg gtg gag ccg cag gcc ttc ctg ggc ctg cgc cag atc cgc ctg ctc     1017
Val Val Glu Pro Gln Ala Phe Leu Gly Leu Arg Gln Ile Arg Leu Leu
    285                 290                 295

aac ctc tcc aac aac ctg ctc tcc acg ttg gag gag agc acc ttc cac     1065
Asn Leu Ser Asn Asn Leu Leu Ser Thr Leu Glu Glu Ser Thr Phe His
300                 305                 310                 315

tcg gtg aac acg cta gag acg ctg cgc gtg gac ggg aac ccg ctg gcc     1113
Ser Val Asn Thr Leu Glu Thr Leu Arg Val Asp Gly Asn Pro Leu Ala
                320                 325                 330

tgc gac tgt cgc ctg ctg tgg atc gtg cag cgt cgc aag acc ctc aac     1161
Cys Asp Cys Arg Leu Leu Trp Ile Val Gln Arg Arg Lys Thr Leu Asn
            335                 340                 345

ttc gac ggg cgg ctg ccg gcc tgc gcc acc ccg gcc gag gtg cgc ggc     1209
Phe Asp Gly Arg Leu Pro Ala Cys Ala Thr Pro Ala Glu Val Arg Gly
        350                 355                 360

gac gcg ctg cga aac ctg ccg gac tcc gtg ctg ttc gag tac ttc gtg     1257
Asp Ala Leu Arg Asn Leu Pro Asp Ser Val Leu Phe Glu Tyr Phe Val
    365                 370                 375

tgc cgc aaa ccc aag atc cgg gag cgg cgg ctg cag cgc gtc acg gcc     1305
Cys Arg Lys Pro Lys Ile Arg Glu Arg Arg Leu Gln Arg Val Thr Ala
380                 385                 390                 395

acc gcg ggc gaa gac gtc cgc ttc ctc tgc cgc gcc gag ggc gag ccg     1353
Thr Ala Gly Glu Asp Val Arg Phe Leu Cys Arg Ala Glu Gly Glu Pro
                400                 405                 410

gcg ccc acc gtg gcc tgg gtg acc ccc cag cac cgg ccg gtg acg gcc     1401
Ala Pro Thr Val Ala Trp Val Thr Pro Gln His Arg Pro Val Thr Ala
            415                 420                 425

acc agc gcg ggc cgg gcg cgc gtg ctc ccc ggg ggg acg ctg gag atc     1449
Thr Ser Ala Gly Arg Ala Arg Val Leu Pro Gly Gly Thr Leu Glu Ile
        430                 435                 440

cag gac gcg cgg ccg cag gac agc ggc acc tac acg tgc gtg gcc agc     1497
Gln Asp Ala Arg Pro Gln Asp Ser Gly Thr Tyr Thr Cys Val Ala Ser
    445                 450                 455

aac gcg ggc ggc aac gac acc tac ttc gcc acg ctg acc gtg cgc ccc     1545
Asn Ala Gly Gly Asn Asp Thr Tyr Phe Ala Thr Leu Thr Val Arg Pro
460                 465                 470                 475

gag ccg gcc gcc aac cgg acc ccg ggc gag gcc cac aac gag acg ctg     1593
Glu Pro Ala Ala Asn Arg Thr Pro Gly Glu Ala His Asn Glu Thr Leu
                480                 485                 490

gcg gcc ctg cgc gcg ccg ctc gac ctc acc acc atc ctg gtg tcc acc     1641
Ala Ala Leu Arg Ala Pro Leu Asp Leu Thr Thr Ile Leu Val Ser Thr
            495                 500                 505

gcc atg ggc tgc atc acc ttc ctg ggc gtg gtc ctc ttc tgc ttc gtg     1689
Ala Met Gly Cys Ile Thr Phe Leu Gly Val Val Leu Phe Cys Phe Val
        510                 515                 520

ctg ctg ttc gtg tgg agc cgc ggc cgc ggg cag cac aaa aac aac ttc     1737
Leu Leu Phe Val Trp Ser Arg Gly Arg Gly Gln His Lys Asn Asn Phe
    525                 530                 535

tcg gtg gag tac tcc ttc cgc aag gtg gat ggg ccg gcc gcc gcg gcg     1785
Ser Val Glu Tyr Ser Phe Arg Lys Val Asp Gly Pro Ala Ala Ala Ala
540                 545                 550                 555

ggc cag gga ggc gcg cgc aag ttc aac atg aag atg atc tgaggggtcc     1834
Gly Gln Gly Gly Ala Arg Lys Phe Asn Met Lys Met Ile
                560                 565

ccagggcgga ccctcccctc ccctcccccg cgggccggcc gctcgcgtgt ccacctatgc     1894

atttcccgga ggggaagggg acggctgcac ggcgctcccc aggcagaact tccccttttt     1954

ttgtagacgc ccaaccgcag gactgttttt catcagcatg cgtctttttt gcagtttctc     2014

aagcgttttc taaagatttc aacccgtctt cctgtccctg ctatggtggc tgagctgggg     2074

ggtgggggtg cgggctgcaa ggatgggggg atgggctccc gtctaccaac ccggggtggc     2134

tgggggcgct cagagacccc aggatcctca gggcccgcaa ttcacaccca ggaggcacaa     2194

gactgctgcc cccaagccct gggcttcc                                        2222

<210>  14

<211>  592

<212>  PRT

<213>  Homo sapiens

<400>  14; LP243(b)

Met Thr Cys Trp Leu Cys Val Leu Ser Leu Pro Leu Leu Leu Leu Pro
                -20                 -15                 -10

Ala Ala Pro Pro Pro Ala Gly Gly Cys Pro Ala Arg Cys Glu Cys Thr
            -5                  -1  1               5

Val Gln Thr Arg Ala Val Ala Cys Thr Arg Arg Arg Leu Thr Ala Val
    10                  15                  20

Pro Asp Gly Ile Pro Ala Glu Thr Arg Leu Leu Glu Leu Ser Arg Asn
25                  30                  35                  40

Arg Ile Arg Cys Leu Asn Pro Gly Asp Leu Ala Ala Leu Pro Ala Leu
                45                  50                  55

Glu Glu Leu Asp Leu Ser Glu Asn Ala Ile Ala His Val Glu Pro Gly
            60                  65                  70

Ala Phe Ala Asn Leu Pro Arg Leu Arg Val Leu Arg Leu Arg Gly Asn
        75                  80                  85

Gln Leu Lys Leu Ile Pro Pro Gly Val Phe Thr Arg Leu Asp Asn Leu
    90                  95                  100

Thr Leu Leu Asp Leu Ser Glu Asn Lys Leu Val Ile Leu Leu Asp Tyr
105                 110                 115                 120

Thr Phe Gln Asp Leu His Ser Leu Arg Arg Leu Glu Val Gly Asp Asn
                125                 130                 135

Asp Leu Val Phe Val Ser Arg Arg Ala Phe Ala Gly Leu Leu Ala Leu
            140                 145                 150

Glu Glu Leu Thr Leu Glu Arg Cys Asn Leu Thr Ala Leu Ser Gly Glu
        155                 160                 165

Ser Leu Gly His Leu Arg Ser Leu Gly Ala Leu Arg Leu Arg His Leu
    170                 175                 180

Ala Ile Ala Ser Leu Glu Asp Gln Asn Phe Arg Arg Leu Pro Gly Leu
185                 190                 195                 200

Leu His Leu Glu Ile Asp Asn Trp Pro Leu Leu Glu Glu Val Ala Ala
                205                 210                 215

Gly Ser Leu Arg Gly Leu Asn Leu Thr Ser Leu Ser Val Thr His Thr
            220                 225                 230

Asn Ile Thr Ala Val Pro Ala Ala Ala Leu Arg His Gln Ala His Leu
        235                 240                 245

Thr Cys Leu Asn Leu Ser His Asn Pro Ile Ser Thr Val Pro Arg Gly
    250                 255                 260

Ser Phe Arg Asp Leu Val Arg Leu Arg Glu Leu His Leu Ala Gly Ala
265                 270                 275                 280

Leu Leu Ala Val Val Glu Pro Gln Ala Phe Leu Gly Leu Arg Gln Ile
                285                 290                 295

Arg Leu Leu Asn Leu Ser Asn Asn Leu Leu Ser Thr Leu Glu Glu Ser
            300                 305                 310

Thr Phe His Ser Val Asn Thr Leu Glu Thr Leu Arg Val Asp Gly Asn
        315                 320                 325

Pro Leu Ala Cys Asp Cys Arg Leu Leu Trp Ile Val Gln Arg Arg Lys
    330                 335                 340

Thr Leu Asn Phe Asp Gly Arg Leu Pro Ala Cys Ala Thr Pro Ala Glu
345                 350                 355                 360

Val Arg Gly Asp Ala Leu Arg Asn Leu Pro Asp Ser Val Leu Phe Glu
                365                 370                 375

Tyr Phe Val Cys Arg Lys Pro Lys Ile Arg Glu Arg Arg Leu Gln Arg
            380                 385                 390

Val Thr Ala Thr Ala Gly Glu Asp Val Arg Phe Leu Cys Arg Ala Glu
        395                 400                 405

Gly Glu Pro Ala Pro Thr Val Ala Trp Val Thr Pro Gln His Arg Pro
    410                 415                 420

Val Thr Ala Thr Ser Ala Gly Arg Ala Arg Val Leu Pro Gly Gly Thr
425                 430                 435                 440

Leu Glu Ile Gln Asp Ala Arg Pro Gln Asp Ser Gly Thr Tyr Thr Cys
                445                 450                 455

Val Ala Ser Asn Ala Gly Gly Asn Asp Thr Tyr Phe Ala Thr Leu Thr
            460                 465                 470

Val Arg Pro Glu Pro Ala Ala Asn Arg Thr Pro Gly Glu Ala His Asn
        475                 480                 485

Glu Thr Leu Ala Ala Leu Arg Ala Pro Leu Asp Leu Thr Thr Ile Leu
    490                 495                 500

Val Ser Thr Ala Met Gly Cys Ile Thr Phe Leu Gly Val Val Leu Phe
505                 510                 515                 520

Cys Phe Val Leu Leu Phe Val Trp Ser Arg Gly Arg Gly Gln His Lys
                525                 530                 535

Asn Asn Phe Ser Val Glu Tyr Ser Phe Arg Lys Val Asp Gly Pro Ala
            540                 545                 550

Ala Ala Ala Gly Gln Gly Gly Ala Arg Lys Phe Asn Met Lys Met Ile
        555                 560                 565

<210>  15

<211>  4392

<212>  DNA

<213>  Homo sapiens


<220>

<221>  CDS

<222>  (1)..(1662)

<223>


<220>

<221>  mat_peptide

<222>  (82)..()

<223>


<400>  15; LP253
atg gcc ccg tgg ctg cag ctc tgc tcc gtc ttc ttt acg gtc aac gcc        48
Met Ala Pro Trp Leu Gln Leu Cys Ser Val Phe Phe Thr Val Asn Ala
        -25                 -20                 -15

tgc ctc aac ggc tcg cag ctg gct gtg gcc gct ggc ggg tcc ggc cgc        96
Cys Leu Asn Gly Ser Gln Leu Ala Val Ala Ala Gly Gly Ser Gly Arg
    -10                 -5                  -1  1               5

gcg cgg ggc gcc gac acc tgt ggc tgg agg gga gtg ggg cca gcc agc       144
Ala Arg Gly Ala Asp Thr Cys Gly Trp Arg Gly Val Gly Pro Ala Ser
                10                  15                  20

aga aac agt ggg ctg tac aac atc acc ttc aaa tat gac aat tgt acc       192
Arg Asn Ser Gly Leu Tyr Asn Ile Thr Phe Lys Tyr Asp Asn Cys Thr
            25                  30                  35

acc tac ttg aat cca gtg ggg aag cat gtg att gct gac gcc cag aat       240

Thr Tyr Leu Asn Pro Val Gly Lys His Val Ile Ala Asp Ala Gln Asn
        40                  45                  50

atc acc atc agc cag tat gct tgc cat gac caa gtg gca gtc acc att        288
Ile Thr Ile Ser Gln Tyr Ala Cys His Asp Gln Val Ala Val Thr Ile
    55                  60                  65

ctt tgg tcc cca ggg gcc ctc ggc atc gaa ttc ctg aaa gga ttt cgg        336
Leu Trp Ser Pro Gly Ala Leu Gly Ile Glu Phe Leu Lys Gly Phe Arg
70                  75                  80                  85

gta ata ctg gag gag ctg aag tcg gag gga aga cag tgc caa caa ctg        384
Val Ile Leu Glu Glu Leu Lys Ser Glu Gly Arg Gln Cys Gln Gln Leu
                90                  95                  100

att cta aag gat ccg aag cag ctc aac agt agc ttc aaa aga act gga        432
Ile Leu Lys Asp Pro Lys Gln Leu Asn Ser Ser Phe Lys Arg Thr Gly
            105                 110                 115

atg gaa tct caa cct ttc ctg aat atg aaa ttt gaa acg gat tat ttc        480
Met Glu Ser Gln Pro Phe Leu Asn Met Lys Phe Glu Thr Asp Tyr Phe
        120                 125                 130

gta aag gtt gtc cct ttt cct tcc att aaa aac gaa agc aat tac cac        528
Val Lys Val Val Pro Phe Pro Ser Ile Lys Asn Glu Ser Asn Tyr His
    135                 140                 145

cct ttc ttc ttt aga acc cga gcc tgt gac ctg ttg tta cag ccg gac        576
Pro Phe Phe Phe Arg Thr Arg Ala Cys Asp Leu Leu Leu Gln Pro Asp
150                 155                 160                 165

aat cta gct tgt aaa ccc ttc tgg aag cct cgg aac ctg aac atc agc        624
Asn Leu Ala Cys Lys Pro Phe Trp Lys Pro Arg Asn Leu Asn Ile Ser
                170                 175                 180

cag cat ggc tcg gac atg cag gtg tcc ttc gac cat gca ccg cac aac        672
Gln His Gly Ser Asp Met Gln Val Ser Phe Asp His Ala Pro His Asn
            185                 190                 195

ttc ggc ttc cgt ttc ttc tat ctt cac tac aag ctc aag cac gaa gga        720
Phe Gly Phe Arg Phe Phe Tyr Leu His Tyr Lys Leu Lys His Glu Gly
        200                 205                 210

cct ttc aag cga aag acc tgt aag cag gag caa act aca gag acg acc        768
Pro Phe Lys Arg Lys Thr Cys Lys Gln Glu Gln Thr Thr Glu Thr Thr
    215                 220                 225

agc tgc ctc ctt caa aat gtt tct cca ggg gat tat ata att gag ctg        816
Ser Cys Leu Leu Gln Asn Val Ser Pro Gly Asp Tyr Ile Ile Glu Leu
230                 235                 240                 245

gtg gat gac act aac aca aca aga aaa gtg atg cat tat gcc tta aag        864
Val Asp Asp Thr Asn Thr Thr Arg Lys Val Met His Tyr Ala Leu Lys
                250                 255                 260

cca gtg cac tcc ccg tgg gcc ggg ccc atc aga gcc gtg gcc atc aca        912
Pro Val His Ser Pro Trp Ala Gly Pro Ile Arg Ala Val Ala Ile Thr
            265                 270                 275

gtg cca ctg gta gtc ata tcg gca ttc gcg acg ctc ttc act gtg atg        960
Val Pro Leu Val Val Ile Ser Ala Phe Ala Thr Leu Phe Thr Val Met
        280                 285                 290

tgc cgc aag aag caa caa gaa aat ata tat tca cat tta gat gaa gag        1008
Cys Arg Lys Lys Gln Gln Glu Asn Ile Tyr Ser His Leu Asp Glu Glu
    295                 300                 305

agc tct gag tct tcc aca tac act gca gca ctc cca aga gag agg ctc        1056
Ser Ser Glu Ser Ser Thr Tyr Thr Ala Ala Leu Pro Arg Glu Arg Leu

310                 315                 320                 325

cgg ccg cgg ccg aag gtc ttt ctc tgc tat tcc agt aaa gat ggc cag     1104
Arg Pro Arg Pro Lys Val Phe Leu Cys Tyr Ser Ser Lys Asp Gly Gln
                330                 335                 340

aat cac atg aat gtc gtc cag tgt ttc gcc tac ttc ctc cag gac ttc     1152
Asn His Met Asn Val Val Gln Cys Phe Ala Tyr Phe Leu Gln Asp Phe
            345                 350                 355

tgt ggc tgt gag gtg gct ctg gac ctg tgg gaa gac ttc agc ctc tgt     1200
Cys Gly Cys Glu Val Ala Leu Asp Leu Trp Glu Asp Phe Ser Leu Cys
        360                 365                 370

aga gaa ggg cag aga gaa tgg gtc atc cag aag atc cac gag tcc cag     1248
Arg Glu Gly Gln Arg Glu Trp Val Ile Gln Lys Ile His Glu Ser Gln
    375                 380                 385

ttc atc att gtg gtt tgt tcc aaa ggt atg aag tac ttt gtg gac aag     1296
Phe Ile Ile Val Val Cys Ser Lys Gly Met Lys Tyr Phe Val Asp Lys
390                 395                 400                 405

aag aac tac aaa cac aaa gga ggt ggc cga ggc tcg ggg aaa gga gag     1344
Lys Asn Tyr Lys His Lys Gly Gly Gly Arg Gly Ser Gly Lys Gly Glu
                410                 415                 420

ctc ttc ctg gtg gcg gtg tca gcc att gcc gaa aag ctc cgc cag gcc     1392
Leu Phe Leu Val Ala Val Ser Ala Ile Ala Glu Lys Leu Arg Gln Ala
            425                 430                 435

aag cag agt tcg tcc gcg gcg ctc agc aag ttt atc gcc gtc tac ttt     1440
Lys Gln Ser Ser Ser Ala Ala Leu Ser Lys Phe Ile Ala Val Tyr Phe
        440                 445                 450

gat tat tcc tgc gag gga gac gtc ccc ggt atc cta gac ctg agt acc     1488
Asp Tyr Ser Cys Glu Gly Asp Val Pro Gly Ile Leu Asp Leu Ser Thr
    455                 460                 465

aag tac aga ctc atg gac aat ctt tcc tca gct ctg ttc cca ctc tgc     1536
Lys Tyr Arg Leu Met Asp Asn Leu Ser Ser Ala Leu Phe Pro Leu Cys
470                 475                 480                 485

act ccc gag acc acg gcc tcc agg agc cgg ggc atg cac acg cga cag     1584
Thr Pro Glu Thr Thr Ala Ser Arg Ser Arg Gly Met His Thr Arg Gln
                490                 495                 500

ggc agg gag aag gaa cta ctt ccg gag caa gtc agg ccg gtc cct ata     1632
Gly Arg Glu Lys Glu Leu Leu Pro Glu Gln Val Arg Pro Val Pro Ile
            505                 510                 515

cgt cgc cat ttg caa cat gca cca gtt tat tgacgaggag cccgactggt     1682
Arg Arg His Leu Gln His Ala Pro Val Tyr
        520                 525

tcgaaaagca gttcgttccc ttccatcctc ctccactgcg ctaccgggag ccagtcttgg     1742

agaaatttga ttcgggcttg gttttaaatg atgtcatgtg caaaccaggg cctgagagtg     1802

acttctgcct aaaggtagag gcggctgttc ttggggcaac cggaccagcc gactcccagc     1862

acgagagtca gcatgggggc ctggaccaag acggggaggc ccggcctgcc cttgacggta     1922

gcgccgccct gcaacccctg ctgcacacgg tgaaagccgg cagcccctcg gacatgccgc     1982

gggactcagg catctatgac tcgtctgtgc cctcatccga gctgtctctg ccactgatgg     2042

aaggactctc gacggaccag acagaaacgt cttccctgac ggagagcgtg tcctcctctt     2102

caggcctggg tgaggaggaa cctcctgccc ttccttccaa gctcctctct tctgggtcat     2162

gcaaagcaga tcttggttgc cgcagctaca ctgatgaact ccacgcggtc gcccctttgt 2222
aacaaaacga aagagtctaa gcattgccac tttagctgct gcctccctct gattccccag 2282
ctcatctccc tggttgcatg gcccacttgg agctgaggtc tcatacaagg atatttggag 2342
tgaaatgctg gccagtactt gttctccctt gccccaaccc tttaccggat atcttgacaa 2402
actctccaat tttctaaaat gatatggagc tctgaaaggc atgtccataa ggtctgacaa 2462
cagcttgcca aatttggtta gtccttggat cagagcctgt tgtgggaggt agggaggaaa 2522
tatgtaaaga aaaacaggaa gatacctgca ctaatcattc agacttcatt gagctctgca 2582
aactttgcct gtttgctatt ggctaccttg atttgaaatg ctttgtgaaa aaaggcactt 2642
ttaacatcat agccacagaa atcaagtgcc agtctatctg gaatccatgt tgtattgcag 2702
ataatgttct catttatttt tgatgtagaa tttacattgc catgggtgtt aaataagctt 2762
tgagtcaaaa gtcaagaaag tgactgaata tacagtcacc ttttatgaaa tgagtctctg 2822
tgttactggg tggcatgact gattgaggtg aagctcacgg ggccaggctg accgtcttga 2882
ccgttccact tgagataggt tggtcatcgt gcagaaggcc ccaggacctc agcacacaca 2942
gcctcctctt ggtctgagta ggcatcatgt gggggccaga tctgcctgct gtttccatgg 3002
gttacattta ctgtgctgta tctcagatgt tggtgtctgg aagtttattc ttaagagact 3062
gctacccagc tggtctgtat tattggaagt tgcagttcgt gctttggttg gccttctggt 3122
ctaaagctgt gtcctgaata ttagggatca caattcactg aaatacagca gtgtgtggag 3182
gtgatggcca gttaatctgc tgaactggtt ttgactaatg acaaacctct ttttaagatg 3242
gtagaatgga ggtgatagtc acaaaagtaa atgttccatt tttatgaatg actttctaca 3302
gagtttctat ttctaaagaa aaaacaattg ttcacatccc atctgatgat tagcatgtgt 3362
gtaatgaatg ctgtcttggt ctcccctgtg gaaacccttc tccctgtgcc ttagagcagg 3422
tgtgtacatc tctcactacc tttctcatgg gtgctgttag attttggcac ccgttttctc 3482
agcattcagc ccagggaatg tggttttcac ttcttcgtca gataagacca acatgaaggg 3542
gtatgttgag aaacatcctg aggcaaggtg ggaggtggga tggggcagga ctttcccttc 3602
caagcacatg catggcaggt ggggaaaggg gggcttgcac ccctgctgga aagaaaaggt 3662
ttgtgtatat ttctgatgca aatgtcatac tcactgctct gtaaaggcag ctggcagctt 3722
tttgggaaaa gaacgtgctc gtctgttctc tggcatcaag tttcttgcag ctgctctgag 3782
ggagagacag tgagctgcaa gactgcctcc ccataacaac aggcaactca gagaagagtc 3842
attttatgtt gttcctatgg aatctggaat gagtgcagag ctcctaccca cacatgactg 3902
ccccgccatt tcatcctagg cattctgtga aggagattgg ttagtccaaa cttgctaaca 3962
tacgaaaatt cacttggaac atgatgagag atttcttatt gaggccaaga gatgtttcct 4022
gtcccagagg aaccattagg agtcgctttt agggtattca gctttgttca tgaaataagg 4082
catctctgag aaagtggccc cagggagaga atggaggact gggaggagaa gcattaactg 4142
agctccaagg gtgtgtgggc agagagcttg ctatgtgaac tcactcctta agaaaatgga 4202

agagaaaaag agagtgctag ttaaaaaatc gggatgtttt agtttggatt tagggttttg 4262

atacttatgt tgaaatacta atgtttctga tcaataaaat caaactctta atataccgag 4322

taatgaaacc atagtgtgat tgcctcagaa taaattgaga agtccaaaaa aaaaaaaaaa 4382

aaaaaaaaaa 4392

<210> 16

<211> 554

<212> PRT

<213> Homo sapiens

<400> 16; LP253

Met Ala Pro Trp Leu Gln Leu Cys Ser Val Phe Phe Thr Val Asn Ala
        -25                 -20                 -15

Cys Leu Asn Gly Ser Gln Leu Ala Val Ala Ala Gly Gly Ser Gly Arg
    -10                 -5              -1  1               5

Ala Arg Gly Ala Asp Thr Cys Gly Trp Arg Gly Val Gly Pro Ala Ser
                10                  15                  20

Arg Asn Ser Gly Leu Tyr Asn Ile Thr Phe Lys Tyr Asp Asn Cys Thr
            25                  30                  35

Thr Tyr Leu Asn Pro Val Gly Lys His Val Ile Ala Asp Ala Gln Asn
        40                  45                  50

Ile Thr Ile Ser Gln Tyr Ala Cys His Asp Gln Val Ala Val Thr Ile
    55                  60                  65

Leu Trp Ser Pro Gly Ala Leu Gly Ile Glu Phe Leu Lys Gly Phe Arg
70                  75                  80                  85

Val Ile Leu Glu Glu Leu Lys Ser Glu Gly Arg Gln Cys Gln Gln Leu
                90                  95                  100

Ile Leu Lys Asp Pro Lys Gln Leu Asn Ser Ser Phe Lys Arg Thr Gly
            105                 110                 115

Met Glu Ser Gln Pro Phe Leu Asn Met Lys Phe Glu Thr Asp Tyr Phe
        120                 125                 130

Val Lys Val Val Pro Phe Pro Ser Ile Lys Asn Glu Ser Asn Tyr His
    135                 140                 145

Pro Phe Phe Phe Arg Thr Arg Ala Cys Asp Leu Leu Leu Gln Pro Asp
150                 155                 160                 165

Asn Leu Ala Cys Lys Pro Phe Trp Lys Pro Arg Asn Leu Asn Ile Ser
                170                 175                 180

Gln His Gly Ser Asp Met Gln Val Ser Phe Asp His Ala Pro His Asn
            185                 190                 195

Phe Gly Phe Arg Phe Phe Tyr Leu His Tyr Lys Leu Lys His Glu Gly
        200                 205                 210

Pro Phe Lys Arg Lys Thr Cys Lys Gln Glu Gln Thr Thr Glu Thr Thr
    215                 220                 225

Ser Cys Leu Leu Gln Asn Val Ser Pro Gly Asp Tyr Ile Ile Glu Leu
230                 235                 240                 245

Val Asp Asp Thr Asn Thr Thr Arg Lys Val Met His Tyr Ala Leu Lys
                250                 255                 260

Pro Val His Ser Pro Trp Ala Gly Pro Ile Arg Ala Val Ala Ile Thr
            265                 270                 275

Val Pro Leu Val Val Ile Ser Ala Phe Ala Thr Leu Phe Thr Val Met
        280                 285                 290

Cys Arg Lys Lys Gln Gln Glu Asn Ile Tyr Ser His Leu Asp Glu Glu
    295                 300                 305

Ser Ser Glu Ser Ser Thr Tyr Thr Ala Ala Leu Pro Arg Glu Arg Leu
310                 315                 320                 325

Arg Pro Arg Pro Lys Val Phe Leu Cys Tyr Ser Ser Lys Asp Gly Gln
                330                 335                 340

Asn His Met Asn Val Val Gln Cys Phe Ala Tyr Phe Leu Gln Asp Phe
            345                 350                 355

Cys Gly Cys Glu Val Ala Leu Asp Leu Trp Glu Asp Phe Ser Leu Cys
        360                 365                 370

Arg Glu Gly Gln Arg Glu Trp Val Ile Gln Lys Ile His Glu Ser Gln
    375                 380                 385

Phe Ile Ile Val Val Cys Ser Lys Gly Met Lys Tyr Phe Val Asp Lys
390                 395                 400                 405

Lys Asn Tyr Lys His Lys Gly Gly Gly Arg Gly Ser Gly Lys Gly Glu
                410                 415                 420

Leu Phe Leu Val Ala Val Ser Ala Ile Ala Glu Lys Leu Arg Gln Ala
            425                 430                 435

Lys Gln Ser Ser Ser Ala Ala Leu Ser Lys Phe Ile Ala Val Tyr Phe

440                 445                 450

Asp Tyr Ser Cys Glu Gly Asp Val Pro Gly Ile Leu Asp Leu Ser Thr
    455                 460                 465

Lys Tyr Arg Leu Met Asp Asn Leu Ser Ser Ala Leu Phe Pro Leu Cys
470                 475                 480                 485

Thr Pro Glu Thr Thr Ala Ser Arg Ser Arg Gly Met His Thr Arg Gln
                490                 495                 500

Gly Arg Glu Lys Glu Leu Leu Pro Glu Gln Val Arg Pro Val Pro Ile
            505                 510                 515

Arg Arg His Leu Gln His Ala Pro Val Tyr
        520                 525

<210>  17

<211>  1010

<212>  DNA

<213>  Homo sapiens


<220>

<221>  CDS

<222>  (162)..(923)

<223>


<220>

<221>  mat_peptide

<222>  (279)..()

<223>


<400>  17; LP218
gccggccgcg gctggcccgg gatcagggag ccgatgtgga atttcctgcc cggctcggcg      60

cccctcctgc cgccccccgc ccggcctcgc actccgcttc cggccgctct cgctgcggcc     120

gcacccgcgc ccgtgcccgc cccgcgcctg ccccgcgcct c atg gag ggc gca ggg     176
                                              Met Glu Gly Ala Gly
                                                              -35

ccc cgg ggg gcc ggg ccg gcg cgg cgc cgg gga gcc ggg ggg ccg ccg       224
Pro Arg Gly Ala Gly Pro Ala Arg Arg Arg Gly Ala Gly Gly Pro Pro
                -30                 -25                 -20

tca ccg ctg ctg ccg tcg ctg ctg ctg ctg ctg ctg ctc tgg atg ctg       272
Ser Pro Leu Leu Pro Ser Leu Leu Leu Leu Leu Leu Leu Trp Met Leu
            -15                 -10                 -5

ccg gac acc gtg gcg cct cag gaa ctg aac cct cgc ggc cgc aac gtg 320
Pro Asp Thr Val Ala Pro Gln Glu Leu Asn Pro Arg Gly Arg Asn Val
-1 1 5 10

tgc cgt gct ccc ggc tcc cag gtg ccc acg tgc tgc gct ggc tgg agg 368
Cys Arg Ala Pro Gly Ser Gln Val Pro Thr Cys Cys Ala Gly Trp Arg
15 20 25 30

cag caa ggg gac gag tgt ggg att gcg gtg tgc gaa ggc aac tcc acg 416
Gln Gln Gly Asp Glu Cys Gly Ile Ala Val Cys Glu Gly Asn Ser Thr
35 40 45

tgc tca gag aac gag gtg tgc gtg agg cct ggc gag tgc cgc tgc cgc 464
Cys Ser Glu Asn Glu Val Cys Val Arg Pro Gly Glu Cys Arg Cys Arg
50 55 60

cac ggc tac ttc ggt gcc aac tgc gac acc aag tgc ccg cgc cag ttc 512
His Gly Tyr Phe Gly Ala Asn Cys Asp Thr Lys Cys Pro Arg Gln Phe
65 70 75

tgg ggc ccc gac tgc aag gag ctg tgt agc tgc cac cca cac ggg cag 560
Trp Gly Pro Asp Cys Lys Glu Leu Cys Ser Cys His Pro His Gly Gln
80 85 90

tgc gag gac gtg aca ggc cag tgt act tgt cac gcg cgg cgc tgg ggc 608
Cys Glu Asp Val Thr Gly Gln Cys Thr Cys His Ala Arg Arg Trp Gly
95 100 105 110

gcg cgc tgc gag cat gcg tgc cag tgc cag cac ggc acg tgc cac ccg 656
Ala Arg Cys Glu His Ala Cys Gln Cys Gln His Gly Thr Cys His Pro
115 120 125

cgg agc ggc gcg tgc cgc tgt gag ccc ggc ccc gcc cct ccg ctc aga 704
Arg Ser Gly Ala Cys Arg Cys Glu Pro Gly Pro Ala Pro Pro Leu Arg
130 135 140

ccc cgc ccc cgc agg gag ctt tcg ctt ggg agg aag aag gcg ccg cac 752
Pro Arg Pro Arg Arg Glu Leu Ser Leu Gly Arg Lys Lys Ala Pro His
145 150 155

cga cta tgc ggg cgc ttc agt cgc atc agc atg aag ctg ccc cgg atc 800
Arg Leu Cys Gly Arg Phe Ser Arg Ile Ser Met Lys Leu Pro Arg Ile
160 165 170

ccg ctc cgg agg cag aaa cta ccc aaa gtc gta ggt aag gat gac agc 848
Pro Leu Arg Arg Gln Lys Leu Pro Lys Val Val Gly Lys Asp Asp Ser
175 180 185 190

gcg ggg gat ctg gga gaa aca cct ctc gac gca gga agc tgc tgg gaa 896
Ala Gly Asp Leu Gly Glu Thr Pro Leu Asp Ala Gly Ser Cys Trp Glu
195 200 205

atg ggt gtg agg ggg tcc aga tct cag tgaggaaatg ggggaccttg 943
Met Gly Val Arg Gly Ser Arg Ser Gln
210 215

agtgttgatg tgcgtgggcc ccaggaggtg cgaccagagg gaaggtaaga ggtgtggcag 1003

ctgctga 1010

<210> 18

<211> 254

<212> PRT

<213> Homo sapiens

<400>  18; LP218

Met Glu Gly Ala Gly Pro Arg Gly Ala Gly Pro Ala Arg Arg Arg Gly
                -35                 -30                 -25

Ala Gly Gly Pro Pro Ser Pro Leu Leu Pro Ser Leu Leu Leu Leu Leu
            -20                 -15                 -10

Leu Leu Trp Met Leu Pro Asp Thr Val Ala Pro Gln Glu Leu Asn Pro
        -5                  -1  1               5

Arg Gly Arg Asn Val Cys Arg Ala Pro Gly Ser Gln Val Pro Thr Cys
10                  15                  20                  25

Cys Ala Gly Trp Arg Gln Gln Gly Asp Glu Cys Gly Ile Ala Val Cys
                30                  35                  40

Glu Gly Asn Ser Thr Cys Ser Glu Asn Glu Val Cys Val Arg Pro Gly
            45                  50                  55

Glu Cys Arg Cys Arg His Gly Tyr Phe Gly Ala Asn Cys Asp Thr Lys
        60                  65                  70

Cys Pro Arg Gln Phe Trp Gly Pro Asp Cys Lys Glu Leu Cys Ser Cys
    75                  80                  85

His Pro His Gly Gln Cys Glu Asp Val Thr Gly Gln Cys Thr Cys His
90                  95                  100                 105

Ala Arg Arg Trp Gly Ala Arg Cys Glu His Ala Cys Gln Cys Gln His
                110                 115                 120

Gly Thr Cys His Pro Arg Ser Gly Ala Cys Arg Cys Glu Pro Gly Pro
            125                 130                 135

Ala Pro Pro Leu Arg Pro Arg Pro Arg Arg Glu Leu Ser Leu Gly Arg
        140                 145                 150

Lys Lys Ala Pro His Arg Leu Cys Gly Arg Phe Ser Arg Ile Ser Met
    155                 160                 165

Lys Leu Pro Arg Ile Pro Leu Arg Arg Gln Lys Leu Pro Lys Val Val
170                 175                 180                 185

Gly Lys Asp Asp Ser Ala Gly Asp Leu Gly Glu Thr Pro Leu Asp Ala
                190                 195                 200

Gly Ser Cys Trp Glu Met Gly Val Arg Gly Ser Arg Ser Gln
            205                 210                 215

<210>  19

<211>  2210

<212>  DNA

<213>  Homo sapiens

<220>

<221>  CDS

<222>  (39)..(2114)

<223>

<220>

<221>  mat_peptide

<222>  (93)..()

<223>

<400>  19; LP251(a)
gtctcctcgc caggacagca acctctcccc tggccctc atg ggc acc gtc agc tcc      56
                                          Met Gly Thr Val Ser Ser
                                                      -15

agg cgg tcc tgg tgg ccg ctg cca ctg ctg ctg ctg ctg ctg ctg ctc      104
Arg Arg Ser Trp Trp Pro Leu Pro Leu Leu Leu Leu Leu Leu Leu Leu
        -10                 -5                  -1  1

ctg ggt ccc gcg ggc gcc cgt gcg cag gag gac gag gac ggc gac tac      152
Leu Gly Pro Ala Gly Ala Arg Ala Gln Glu Asp Glu Asp Gly Asp Tyr
5                   10                  15                  20

gag gag ctg gtg cta gcc ttg cgt tcc gag gag gac ggc ctg gcc gaa      200
Glu Glu Leu Val Leu Ala Leu Arg Ser Glu Glu Asp Gly Leu Ala Glu
                25                  30                  35

gca ccc gag cac gga acc aca gcc acc ttc cac cgc tgc gcc aag gat      248
Ala Pro Glu His Gly Thr Thr Ala Thr Phe His Arg Cys Ala Lys Asp
            40                  45                  50

ccg tgg agg ttg cct ggc acc tac gtg gtg gtg ctg aag gag gag acc      296
Pro Trp Arg Leu Pro Gly Thr Tyr Val Val Val Leu Lys Glu Glu Thr
        55                  60                  65

cac ctc tcg cag tca gag cgc act gcc cgc cgc ctg cag gcc cag gct      344
His Leu Ser Gln Ser Glu Arg Thr Ala Arg Arg Leu Gln Ala Gln Ala
    70                  75                  80

gcc cgc cgg gga tac ctc acc aag atc ctg cat gtc ttc cat ggc ctt      392
Ala Arg Arg Gly Tyr Leu Thr Lys Ile Leu His Val Phe His Gly Leu
85                  90                  95                  100

ctt cct ggc ttc ctg gtg aag atg agt ggc gac ctg ctg gag ctg gcc      440
Leu Pro Gly Phe Leu Val Lys Met Ser Gly Asp Leu Leu Glu Leu Ala
                105                 110                 115

ttg aag ttg ccc cat gtc gac tac atc gag gag gac tcc tct gtc ttt      488
Leu Lys Leu Pro His Val Asp Tyr Ile Glu Glu Asp Ser Ser Val Phe

120 125 130

gcc cag agc atc ccg tgg aac ctg gag cgg att acc cct cca cgg tac 536
Ala Gln Ser Ile Pro Trp Asn Leu Glu Arg Ile Thr Pro Pro Arg Tyr
135 140 145

cgg gcg gat gaa tac cag ccc ccc gac gga ggc agc ctg gtg gag gtg 584
Arg Ala Asp Glu Tyr Gln Pro Pro Asp Gly Gly Ser Leu Val Glu Val
150 155 160

tat ctc cta gac acc agc ata cag agt gac cac cgg gaa atc gag ggc 632
Tyr Leu Leu Asp Thr Ser Ile Gln Ser Asp His Arg Glu Ile Glu Gly
165 170 175 180

agg gtc atg gtc acc gac ttc gag aat gtg ccc gag gag gac ggg acc 680
Arg Val Met Val Thr Asp Phe Glu Asn Val Pro Glu Glu Asp Gly Thr
185 190 195

cgc ttc cac aga cag gcc agc aag tgt gac agt cat ggc acc cac ctg 728
Arg Phe His Arg Gln Ala Ser Lys Cys Asp Ser His Gly Thr His Leu
200 205 210

gca ggg gtg gtc agc ggc cgg gat gcc ggc gtg gcc aag ggt gcc agc 776
Ala Gly Val Val Ser Gly Arg Asp Ala Gly Val Ala Lys Gly Ala Ser
215 220 225

atg cgc agc ctg cgc gtg ctc aac tgc caa ggg aag ggc acg gtt agc 824
Met Arg Ser Leu Arg Val Leu Asn Cys Gln Gly Lys Gly Thr Val Ser
230 235 240

ggc acc ctc ata ggc ctg gag ttt att cgg aaa agc cag ctg gtc cag 872
Gly Thr Leu Ile Gly Leu Glu Phe Ile Arg Lys Ser Gln Leu Val Gln
245 250 255 260

cct gtg ggg cca ctg gtg gtg ctg ctg ccc ctg gcg ggt ggg tac agc 920
Pro Val Gly Pro Leu Val Val Leu Leu Pro Leu Ala Gly Gly Tyr Ser
265 270 275

cgc gtc ctc aac gcc gcc tgc cag cgc ctg gcg agg gct ggg gtc gtg 968
Arg Val Leu Asn Ala Ala Cys Gln Arg Leu Ala Arg Ala Gly Val Val
280 285 290

ctg gtc acc gct gcc ggc aac ttc cgg gac gat gcc tgc ctc tac tcc 1016
Leu Val Thr Ala Ala Gly Asn Phe Arg Asp Asp Ala Cys Leu Tyr Ser
295 300 305

cca gcc tca gct ccc gag gtc atc aca gtt ggg gcc acc aat gcc cag 1064
Pro Ala Ser Ala Pro Glu Val Ile Thr Val Gly Ala Thr Asn Ala Gln
310 315 320

gac cag ccg gtg acc ctg ggg act ttg ggg acc aac ttt ggc cgc tgt 1112
Asp Gln Pro Val Thr Leu Gly Thr Leu Gly Thr Asn Phe Gly Arg Cys
325 330 335 340

gtg gac ctc ttt gcc cca ggg gag gac atc att ggt gcc tcc agc gac 1160
Val Asp Leu Phe Ala Pro Gly Glu Asp Ile Ile Gly Ala Ser Ser Asp
345 350 355

tgc agc acc tgc ttt gtg tca cag agt ggg aca tca cag gct gct gcc 1208
Cys Ser Thr Cys Phe Val Ser Gln Ser Gly Thr Ser Gln Ala Ala Ala
360 365 370

cac gtg gct ggc att gca gcc atg atg ctg tct gcc gag ccg gag ctc 1256
His Val Ala Gly Ile Ala Ala Met Met Leu Ser Ala Glu Pro Glu Leu
375 380 385

acc ctg gcc gag ttg agg cag aga ctg atc cac ttc tct gcc aaa gat 1304
Thr Leu Ala Glu Leu Arg Gln Arg Leu Ile His Phe Ser Ala Lys Asp
390 395 400

gtc atc aat gag gcc tgg ttc cct gag gac cag cgg gta ctg acc ccc 1352
Val Ile Asn Glu Ala Trp Phe Pro Glu Asp Gln Arg Val Leu Thr Pro
405 410 415 420

aac ctg gtg gcc gcc ctg ccc ccc agc acc cat ggg gca ggt tgg cag 1400
Asn Leu Val Ala Ala Leu Pro Pro Ser Thr His Gly Ala Gly Trp Gln
425 430 435

ctg ttt tgc agg act gtg tgg tca gca cac tcg ggg cct aca cgg atg 1448
Leu Phe Cys Arg Thr Val Trp Ser Ala His Ser Gly Pro Thr Arg Met
440 445 450

gcc aca gcc atc gcc cgc tgc gcc cca gat gag gag ctg ctg agc tgc 1496
Ala Thr Ala Ile Ala Arg Cys Ala Pro Asp Glu Glu Leu Leu Ser Cys
455 460 465

tcc agt ttc tcc agg agt ggg aag cgg cgg ggc gag cgc atg gag gcc 1544
Ser Ser Phe Ser Arg Ser Gly Lys Arg Arg Gly Glu Arg Met Glu Ala
470 475 480

caa ggg ggc aag ctg gtc tgc cgg gcc cac aac gct ttt ggg ggt gag 1592
Gln Gly Gly Lys Leu Val Cys Arg Ala His Asn Ala Phe Gly Gly Glu
485 490 495 500

ggt gtc tac gcc att gcc agg tgc tgc ctg cta ccc cag gcc aac tgc 1640
Gly Val Tyr Ala Ile Ala Arg Cys Cys Leu Leu Pro Gln Ala Asn Cys
505 510 515

agc gtc cac aca gct cca cca gct gag gcc agc atg ggg acc cgt gtc 1688
Ser Val His Thr Ala Pro Pro Ala Glu Ala Ser Met Gly Thr Arg Val
520 525 530

cac tgc cac caa cag ggc cac gtc ctc aca ggc tgc agc tcc cac tgg 1736
His Cys His Gln Gln Gly His Val Leu Thr Gly Cys Ser Ser His Trp
535 540 545

gag gtg gag gac ctt ggc acc cac aag ccg cct gtg ctg agg cca cga 1784
Glu Val Glu Asp Leu Gly Thr His Lys Pro Pro Val Leu Arg Pro Arg
550 555 560

ggt cag ccc aac cag tgc gtg ggc cac agg gag gcc agc atc cac gct 1832
Gly Gln Pro Asn Gln Cys Val Gly His Arg Glu Ala Ser Ile His Ala
565 570 575 580

tcc tgc tgc cat gcc cca ggt ctg gaa tgc aaa gtc aag gag cat gga 1880
Ser Cys Cys His Ala Pro Gly Leu Glu Cys Lys Val Lys Glu His Gly
585 590 595

atc ccg gcc cct cag gag cag gtg acc gtg gcc tgc gag gag ggc tgg 1928
Ile Pro Ala Pro Gln Glu Gln Val Thr Val Ala Cys Glu Glu Gly Trp
600 605 610

acc ctg act ggc tgc agt gcc ctc cct ggg acc tcc cac gtc ctg ggg 1976
Thr Leu Thr Gly Cys Ser Ala Leu Pro Gly Thr Ser His Val Leu Gly
615 620 625

gcc tac gcc gta gac aac acg tgt gta gtc agg agc cgg gac gtc agc 2024
Ala Tyr Ala Val Asp Asn Thr Cys Val Val Arg Ser Arg Asp Val Ser
630 635 640

act aca ggc agc acc agc gaa gag gcc gtg aca gcc gtt gcc atc tgc 2072
Thr Thr Gly Ser Thr Ser Glu Glu Ala Val Thr Ala Val Ala Ile Cys
645 650 655 660

tgc cgg agc cgg cac ctg gcg cag gcc ttc cag gag ctc cag 2114
Cys Arg Ser Arg His Leu Ala Gln Ala Phe Gln Glu Leu Gln
665 670

tgacagcccc atcccaggat gggtgtctgg ggagggtcaa gggctggggc tgagctttaa 2174

aatggttccg acttgtccct ctctcagccc ttcatg 2210

<210> 20

<211> 692

<212> PRT

<213> Homo sapiens

<400> 20; LP251(a)

Met Gly Thr Val Ser Ser Arg Arg Ser Trp Trp Pro Leu Pro Leu Leu
-15 -10 -5

Leu Leu Leu Leu Leu Leu Leu Gly Pro Ala Gly Ala Arg Ala Gln Glu
-1 1 5 10

Asp Glu Asp Gly Asp Tyr Glu Glu Leu Val Leu Ala Leu Arg Ser Glu
15 20 25 30

Glu Asp Gly Leu Ala Glu Ala Pro Glu His Gly Thr Thr Ala Thr Phe
35 40 45

His Arg Cys Ala Lys Asp Pro Trp Arg Leu Pro Gly Thr Tyr Val Val
50 55 60

Val Leu Lys Glu Glu Thr His Leu Ser Gln Ser Glu Arg Thr Ala Arg
65 70 75

Arg Leu Gln Ala Gln Ala Ala Arg Arg Gly Tyr Leu Thr Lys Ile Leu
80 85 90

His Val Phe His Gly Leu Leu Pro Gly Phe Leu Val Lys Met Ser Gly
95 100 105 110

Asp Leu Leu Glu Leu Ala Leu Lys Leu Pro His Val Asp Tyr Ile Glu
115 120 125

Glu Asp Ser Ser Val Phe Ala Gln Ser Ile Pro Trp Asn Leu Glu Arg
130 135 140

Ile Thr Pro Pro Arg Tyr Arg Ala Asp Glu Tyr Gln Pro Pro Asp Gly
145 150 155

Gly Ser Leu Val Glu Val Tyr Leu Leu Asp Thr Ser Ile Gln Ser Asp
160 165 170

His Arg Glu Ile Glu Gly Arg Val Met Val Thr Asp Phe Glu Asn Val
175 180 185 190

Pro Glu Glu Asp Gly Thr Arg Phe His Arg Gln Ala Ser Lys Cys Asp
                195                 200                 205

Ser His Gly Thr His Leu Ala Gly Val Val Ser Gly Arg Asp Ala Gly
            210                 215                 220

Val Ala Lys Gly Ala Ser Met Arg Ser Leu Arg Val Leu Asn Cys Gln
        225                 230                 235

Gly Lys Gly Thr Val Ser Gly Thr Leu Ile Gly Leu Glu Phe Ile Arg
    240                 245                 250

Lys Ser Gln Leu Val Gln Pro Val Gly Pro Leu Val Val Leu Leu Pro
255                 260                 265                 270

Leu Ala Gly Gly Tyr Ser Arg Val Leu Asn Ala Ala Cys Gln Arg Leu
                275                 280                 285

Ala Arg Ala Gly Val Val Leu Val Thr Ala Ala Gly Asn Phe Arg Asp
            290                 295                 300

Asp Ala Cys Leu Tyr Ser Pro Ala Ser Ala Pro Glu Val Ile Thr Val
        305                 310                 315

Gly Ala Thr Asn Ala Gln Asp Gln Pro Val Thr Leu Gly Thr Leu Gly
    320                 325                 330

Thr Asn Phe Gly Arg Cys Val Asp Leu Phe Ala Pro Gly Glu Asp Ile
335                 340                 345                 350

Ile Gly Ala Ser Ser Asp Cys Ser Thr Cys Phe Val Ser Gln Ser Gly
                355                 360                 365

Thr Ser Gln Ala Ala Ala His Val Ala Gly Ile Ala Ala Met Met Leu
            370                 375                 380

Ser Ala Glu Pro Glu Leu Thr Leu Ala Glu Leu Arg Gln Arg Leu Ile
        385                 390                 395

His Phe Ser Ala Lys Asp Val Ile Asn Glu Ala Trp Phe Pro Glu Asp
    400                 405                 410

Gln Arg Val Leu Thr Pro Asn Leu Val Ala Ala Leu Pro Pro Ser Thr
415                 420                 425                 430

His Gly Ala Gly Trp Gln Leu Phe Cys Arg Thr Val Trp Ser Ala His
                435                 440                 445

Ser Gly Pro Thr Arg Met Ala Thr Ala Ile Ala Arg Cys Ala Pro Asp
            450                 455                 460

Glu Glu Leu Leu Ser Cys Ser Ser Phe Ser Arg Ser Gly Lys Arg Arg

465 470 475

Gly Glu Arg Met Glu Ala Gln Gly Gly Lys Leu Val Cys Arg Ala His
480 485 490

Asn Ala Phe Gly Gly Glu Gly Val Tyr Ala Ile Ala Arg Cys Cys Leu
495 500 505 510

Leu Pro Gln Ala Asn Cys Ser Val His Thr Ala Pro Pro Ala Glu Ala
515 520 525

Ser Met Gly Thr Arg Val His Cys His Gln Gln Gly His Val Leu Thr
530 535 540

Gly Cys Ser Ser His Trp Glu Val Glu Asp Leu Gly Thr His Lys Pro
545 550 555

Pro Val Leu Arg Pro Arg Gly Gln Pro Asn Gln Cys Val Gly His Arg
560 565 570

Glu Ala Ser Ile His Ala Ser Cys Cys His Ala Pro Gly Leu Glu Cys
575 580 585 590

Lys Val Lys Glu His Gly Ile Pro Ala Pro Gln Glu Gln Val Thr Val
595 600 605

Ala Cys Glu Glu Gly Trp Thr Leu Thr Gly Cys Ser Ala Leu Pro Gly
610 615 620

Thr Ser His Val Leu Gly Ala Tyr Ala Val Asp Asn Thr Cys Val Val
625 630 635

Arg Ser Arg Asp Val Ser Thr Thr Gly Ser Thr Ser Glu Glu Ala Val
640 645 650

Thr Ala Val Ala Ile Cys Cys Arg Ser Arg His Leu Ala Gln Ala Phe
655 660 665 670

Gln Glu Leu Gln

<210> 21

<211> 2492

<212> DNA

<213> Homo sapiens

<220>

<221> CDS

<222>  (322)..(2379)

<223>


<220>

<221>  mat_peptide

<222>  (376)..()

<223>


<220>

<221>  misc_feature

<222>  (2484)..(2484)

<223>  wherein n is A, G, C, or T


<400>  21; LP252
ggcctttcaa agtgtgcagt tgtctcctcc ctgtccagcc ccatcgtcgc ccaggaccag      60

ctgggccgcg gtctgacctg aggctgctgc tcagcgccgg ggcgctggcg ctctccattc     120

gagcaccttc cagcataccg ctcggctccg ggagccgctc tgcaaagttg ggcagctcag     180

agcgcaagct ttgcctctcg acttctccct ccttgggtcc ccggcgcccc cgcctcccac     240

gatccctttc actaggagca gccagtccca gcgggctggc aacttgcacc ccttcctagt     300

catcctccct gaaacgcgac c atg ctg tta agg ggc gtc ctc ctg gcg ttg       351
                        Met Leu Leu Arg Gly Val Leu Leu Ala Leu
                                    -15                 -10

caa gcc ctg cag ctc gcc ggt gcc ctc gac ctg ccc gct ggg tcc tgt       399
Gln Ala Leu Gln Leu Ala Gly Ala Leu Asp Leu Pro Ala Gly Ser Cys
            -5                  -1  1               5

gcc ttt gaa gag agc act tgc ggc ttt gac tcc gtg ttg gcc tct ctg       447
Ala Phe Glu Glu Ser Thr Cys Gly Phe Asp Ser Val Leu Ala Ser Leu
    10                  15                  20

ccg tgg att tta aat gag gaa ggc cat tac att tat gtg gat acc tcc       495
Pro Trp Ile Leu Asn Glu Glu Gly His Tyr Ile Tyr Val Asp Thr Ser
25                  30                  35                  40

ttt ggc aag cag ggg gag aaa gct gtg ctg cta agt cct gac tta cag       543
Phe Gly Lys Gln Gly Glu Lys Ala Val Leu Leu Ser Pro Asp Leu Gln
                45                  50                  55

gct gag gaa tgg agc tgc ctc cgt ttg gtc tac cag ata acc aca tct       591
Ala Glu Glu Trp Ser Cys Leu Arg Leu Val Tyr Gln Ile Thr Thr Ser
            60                  65                  70

tcg gag tct ctg tca gat ccc agc cag ctg aac ctc tac atg aga ttt       639
Ser Glu Ser Leu Ser Asp Pro Ser Gln Leu Asn Leu Tyr Met Arg Phe
        75                  80                  85

gaa gat gaa agc ttt gat cgc ttg ctt tgg tca gct aag gaa cct tca       687
Glu Asp Glu Ser Phe Asp Arg Leu Leu Trp Ser Ala Lys Glu Pro Ser
    90                  95                  100

gac agc tgg ctc ata gcc agc ttg gat ttg caa aac agt tcc aag aaa 735
Asp Ser Trp Leu Ile Ala Ser Leu Asp Leu Gln Asn Ser Ser Lys Lys
105 110 115 120

ttc aag att tta ata gaa ggt gta cta gga cag gga aac aca gcc agc 783
Phe Lys Ile Leu Ile Glu Gly Val Leu Gly Gln Gly Asn Thr Ala Ser
125 130 135

atc gca cta ttt gaa atc aag atg aca acc ggc tac tgt att gaa tgt 831
Ile Ala Leu Phe Glu Ile Lys Met Thr Thr Gly Tyr Cys Ile Glu Cys
140 145 150

gac ttt gaa gaa aat cat ctc tgt ggc ttt gtg aac cgc tgg aat ccc 879
Asp Phe Glu Glu Asn His Leu Cys Gly Phe Val Asn Arg Trp Asn Pro
155 160 165

aat gtg aac tgg ttt gtt gga gga gga agt att cgg aat gtc cac tcc 927
Asn Val Asn Trp Phe Val Gly Gly Gly Ser Ile Arg Asn Val His Ser
170 175 180

att ctc cca cag gat cac acc ttc aag agt gaa ctg ggc cac tac atg 975
Ile Leu Pro Gln Asp His Thr Phe Lys Ser Glu Leu Gly His Tyr Met
185 190 195 200

tac gtg gac tca gtt tat gtg aag cac ttc cag gag gtg gca cag ctc 1023
Tyr Val Asp Ser Val Tyr Val Lys His Phe Gln Glu Val Ala Gln Leu
205 210 215

atc tcc ccg ttg acc acg gcc ccc atg gct ggc tgc ctg tca ttt tat 1071
Ile Ser Pro Leu Thr Thr Ala Pro Met Ala Gly Cys Leu Ser Phe Tyr
220 225 230

tac cag atc cag cag ggg aat gac aat gtc ttt tcc ctt tac act cgg 1119
Tyr Gln Ile Gln Gln Gly Asn Asp Asn Val Phe Ser Leu Tyr Thr Arg
235 240 245

gat gtg gct ggc ctt tac gag gaa atc tgg aaa gca gac agg cca ggg 1167
Asp Val Ala Gly Leu Tyr Glu Glu Ile Trp Lys Ala Asp Arg Pro Gly
250 255 260

aat gct gcc tgg aac ctt gcg gag gtc gag ttc aat gct cct tac ccc 1215
Asn Ala Ala Trp Asn Leu Ala Glu Val Glu Phe Asn Ala Pro Tyr Pro
265 270 275 280

atg gag gtt att ttt gaa gtt gct ttc aat ggt ccc aag gga ggt tat 1263
Met Glu Val Ile Phe Glu Val Ala Phe Asn Gly Pro Lys Gly Gly Tyr
285 290 295

gtt gcc ctg gat gat att tca ttc tct cct gtt cac tgc cag aat cag 1311
Val Ala Leu Asp Asp Ile Ser Phe Ser Pro Val His Cys Gln Asn Gln
300 305 310

aca gaa ctt ctg ttc agt gcc gtg gaa gcc agc tgc aat ttt gag caa 1359
Thr Glu Leu Leu Phe Ser Ala Val Glu Ala Ser Cys Asn Phe Glu Gln
315 320 325

gat ctc tgc aac ttt tac caa gat aaa gaa ggt cca ggt tgg acc cga 1407
Asp Leu Cys Asn Phe Tyr Gln Asp Lys Glu Gly Pro Gly Trp Thr Arg
330 335 340

gtg aaa gta aaa cca aac atg tat cgg gct gga gac cac act aca ggc 1455
Val Lys Val Lys Pro Asn Met Tyr Arg Ala Gly Asp His Thr Thr Gly
345 350 355 360

tta ggg tat tac ctg cta gcc aac aca aag ttc aca tct cag cct ggc 1503
Leu Gly Tyr Tyr Leu Leu Ala Asn Thr Lys Phe Thr Ser Gln Pro Gly
365 370 375

tac att gga agg ctc tat ggg ccc tcc cta cca gga aac ttg cag tat 1551

Tyr Ile Gly Arg Leu Tyr Gly Pro Ser Leu Pro Gly Asn Leu Gln Tyr
380 385 390

tgt ctg cgt ttt cat tat gcc atc tat gga ttt tta aaa atg agt gac 1599
Cys Leu Arg Phe His Tyr Ala Ile Tyr Gly Phe Leu Lys Met Ser Asp
395 400 405

acc cta gca gtt tac atc ttt gaa gag aac cat gtg gtt caa gag aag 1647
Thr Leu Ala Val Tyr Ile Phe Glu Glu Asn His Val Val Gln Glu Lys
410 415 420

atc tgg tct gtg ttg gag tcc cca agg ggt gtt tgg atg caa gct gaa 1695
Ile Trp Ser Val Leu Glu Ser Pro Arg Gly Val Trp Met Gln Ala Glu
425 430 435 440

atc acc ttt aag aag ccc atg cct acc aag gtg gtt ttc atg agc cta 1743
Ile Thr Phe Lys Lys Pro Met Pro Thr Lys Val Val Phe Met Ser Leu
445 450 455

tgc aaa agt ttc tgg gac tgt ggg ctt gta gcc ctg gat gac att aca 1791
Cys Lys Ser Phe Trp Asp Cys Gly Leu Val Ala Leu Asp Asp Ile Thr
460 465 470

ata caa ttg gga agc tgc tca tct tca gag aaa ctt cca cct cca cct 1839
Ile Gln Leu Gly Ser Cys Ser Ser Ser Glu Lys Leu Pro Pro Pro Pro
475 480 485

gga gag tgt act ttc gag caa gat gaa tgt aca ttt act cag gag aaa 1887
Gly Glu Cys Thr Phe Glu Gln Asp Glu Cys Thr Phe Thr Gln Glu Lys
490 495 500

aga aac cgg agc agc tgg cac agg agg agg gga gaa act ccc act tcc 1935
Arg Asn Arg Ser Ser Trp His Arg Arg Arg Gly Glu Thr Pro Thr Ser
505 510 515 520

tac aca gga cca aag gga gat cac act act ggg gta ggc tac tac atg 1983
Tyr Thr Gly Pro Lys Gly Asp His Thr Thr Gly Val Gly Tyr Tyr Met
525 530 535

tac att gag gcc tcc cat atg gtg tat gga caa aaa gca cgc ctc ttg 2031
Tyr Ile Glu Ala Ser His Met Val Tyr Gly Gln Lys Ala Arg Leu Leu
540 545 550

tcc agg cct ctg cga gga gtc tct gga aaa cac tgc ttg acc ttt ttc 2079
Ser Arg Pro Leu Arg Gly Val Ser Gly Lys His Cys Leu Thr Phe Phe
555 560 565

tac cac atg tat gga ggg ggc act ggc ctg ctg agt gtt tat ctg aaa 2127
Tyr His Met Tyr Gly Gly Gly Thr Gly Leu Leu Ser Val Tyr Leu Lys
570 575 580

aag gaa gaa gac agt gaa gag tcc ctc tta tgg agg aga aga ggt gaa 2175
Lys Glu Glu Asp Ser Glu Glu Ser Leu Leu Trp Arg Arg Arg Gly Glu
585 590 595 600

cag agc att tcc tgg cta cga gca ctg att gaa tac agc tgt gag agg 2223
Gln Ser Ile Ser Trp Leu Arg Ala Leu Ile Glu Tyr Ser Cys Glu Arg
605 610 615

caa cac cag ata att ttt gaa gcc att cga gga gta tca ata aga agt 2271
Gln His Gln Ile Ile Phe Glu Ala Ile Arg Gly Val Ser Ile Arg Ser
620 625 630

gat att gcc att gat gat gtt aaa ttt cag gca gga ccc tgt gga gaa 2319
Asp Ile Ala Ile Asp Asp Val Lys Phe Gln Ala Gly Pro Cys Gly Glu
635 640 645

atg gaa gat aca act caa caa tca tca gga tat tct gag gac tta aat 2367
Met Glu Asp Thr Thr Gln Gln Ser Ser Gly Tyr Ser Glu Asp Leu Asn

650                 655                 660

gaa att gag tat taagaaatga tctgcattgg atttactaga cgaaaaccat        2419
Glu Ile Glu Tyr
665

acctctcttc aatcaaaatg aaaacaaagc aaatgaatac tggacagtct taacaattta   2479

taagntataa aat                                                      2492

<210>  22

<211>  686

<212>  PRT

<213>  Homo sapiens

<220>

<221>  misc_feature

<222>  (2484)..(2484)

<223>  wherein n is A, G, C, or T

<400>  22; LP252

Met Leu Leu Arg Gly Val Leu Leu Ala Leu Gln Ala Leu Gln Leu Ala
            -15                 -10                 -5

Gly Ala Leu Asp Leu Pro Ala Gly Ser Cys Ala Phe Glu Glu Ser Thr
    -1  1               5                   10

Cys Gly Phe Asp Ser Val Leu Ala Ser Leu Pro Trp Ile Leu Asn Glu
15                  20                  25                  30

Glu Gly His Tyr Ile Tyr Val Asp Thr Ser Phe Gly Lys Gln Gly Glu
                35                  40                  45

Lys Ala Val Leu Leu Ser Pro Asp Leu Gln Ala Glu Glu Trp Ser Cys
            50                  55                  60

Leu Arg Leu Val Tyr Gln Ile Thr Thr Ser Ser Glu Ser Leu Ser Asp
        65                  70                  75

Pro Ser Gln Leu Asn Leu Tyr Met Arg Phe Glu Asp Glu Ser Phe Asp
    80                  85                  90

Arg Leu Leu Trp Ser Ala Lys Glu Pro Ser Asp Ser Trp Leu Ile Ala
95                  100                 105                 110

Ser Leu Asp Leu Gln Asn Ser Ser Lys Lys Phe Lys Ile Leu Ile Glu
                115                 120                 125

Gly Val Leu Gly Gln Gly Asn Thr Ala Ser Ile Ala Leu Phe Glu Ile
            130                 135                 140

Lys Met Thr Thr Gly Tyr Cys Ile Glu Cys Asp Phe Glu Glu Asn His
        145                 150                 155

Leu Cys Gly Phe Val Asn Arg Trp Asn Pro Asn Val Asn Trp Phe Val
    160                 165                 170

Gly Gly Gly Ser Ile Arg Asn Val His Ser Ile Leu Pro Gln Asp His
175                 180                 185                 190

Thr Phe Lys Ser Glu Leu Gly His Tyr Met Tyr Val Asp Ser Val Tyr
                195                 200                 205

Val Lys His Phe Gln Glu Val Ala Gln Leu Ile Ser Pro Leu Thr Thr
            210                 215                 220

Ala Pro Met Ala Gly Cys Leu Ser Phe Tyr Tyr Gln Ile Gln Gln Gly
        225                 230                 235

Asn Asp Asn Val Phe Ser Leu Tyr Thr Arg Asp Val Ala Gly Leu Tyr
    240                 245                 250

Glu Glu Ile Trp Lys Ala Asp Arg Pro Gly Asn Ala Ala Trp Asn Leu
255                 260                 265                 270

Ala Glu Val Glu Phe Asn Ala Pro Tyr Pro Met Glu Val Ile Phe Glu
                275                 280                 285

Val Ala Phe Asn Gly Pro Lys Gly Gly Tyr Val Ala Leu Asp Asp Ile
            290                 295                 300

Ser Phe Ser Pro Val His Cys Gln Asn Gln Thr Glu Leu Leu Phe Ser
        305                 310                 315

Ala Val Glu Ala Ser Cys Asn Phe Glu Gln Asp Leu Cys Asn Phe Tyr
    320                 325                 330

Gln Asp Lys Glu Gly Pro Gly Trp Thr Arg Val Lys Val Lys Pro Asn
335                 340                 345                 350


                355                 360                 365


            370                 375                 380


        385                 390                 395


    400                 405                 410


415                 420                 425                 430


                435                 440                 445


            450                 455                 460


        465                 470                 475


    480                 485                 490


495                 500                 505                 510


                515                 520                 525


            530                 535                 540


        545                 550                 555


    560                 565                 570


575                 580                 585                 590


                595                 600                 605


            610                 615                 620


        625                 630                 635


    640                 645                 650


655                 660                 665

<210>  23

<211>  1581

<212>  DNA

<213>  Homo sapiens


<220>

<221>  CDS

<222>  (136)..(1386)

<223>


<220>

<221>  mat_peptide

<222>  (196)..()

<223>


<400>  23; LP239(b)
ctcaattctt aaaaaataag tccatgaagc agaaacatca aaaagcatgg gatttggaat      60

ttgagacctg actttgaagc ccacacagaa cacagacaag tcaaccatgc tatttgggac     120

atattttgtt ccaaa atg gca tct tac ctt tat gga gta ctc ttt gct gtt      171
                 
                 -20                 -15                 -10

ggc ctc tgt gct cca atc tac tgt gtg tcc ccg gtc aat gcc ccc agt      219

            -5                  -1  1               5

gca tac ccc cgc cct tcc tcc aca aag agc acc cct gcc tca cag gtg      267

    10                  15                  20

tat tcc ctc aac acc gac ttt gcc ttc cgc cta tac cgc agg ctg gtt      315

25                  30                  35                  40

ttg gag acc ccg agt cag aac atc ttc ttc tcc cct gtg agt gtc tcc      363

                45                  50                  55

act tcc ctg gcc atg ctc tcc ctt ggg gcc cac tca gtc acc aag acc      411

            60                  65                  70

cag att ctc cag ggc ctg ggc ttc aac ctc aca cac aca cca gag tct      459

        75                  80                  85

gcc atc cac cag ggc ttc cag cac ctg gtt cac tca ctg act gtt ccc      507

    90                  95                  100

agc aaa gac ctg acc ttg aag atg gga agt gcc ctc ttc gtc aag aag      555

105                 110                 115                 120

gag ctg cag ctg cag gca aat ttc ttg ggc aat gtc aag agg ctg tat      603


125 130 135

gaa gca gaa gtc ttt tct aca gat ttc tcc aac ccc tcc att gcc cag 651

140 145 150

gcg agg atc aac agc cat gtg aaa aag aag acc caa ggg aag gtt gta 699

155 160 165

gac ata atc caa ggc ctt gac ctt ctg acg gcc atg gtt ctg gtg aat 747

170 175 180

cac att ttc ttt aaa gcc aag tgg gag aag ccc ttt cac ctt gaa tat 795

185 190 195 200

aca aga aag aac ttc cca ttc ctg gtg ggc gag cag gtc act gtg caa 843

205 210 215

gtc ccc atg atg cac cag aaa gag cag ttc gct ttt ggg gtg gat aca 891

220 225 230

gag ctg aac tgc ttt gtg ctg cag atg gat tac aag gga gat gcc gtg 939

235 240 245

gcc ttc ttt gtc ctc cct agc aag ggc aag atg agg caa ctg gaa cag 987

250 255 260

gcc ttg tca gcc aga aca ctg ata aag tgg agc cac tca ctc cag aaa 1035

265 270 275 280

agg tgg ata gag gtg ttc atc ccc aga ttt tcc att tct gcc tcc tac 1083

285 290 295

aat ctg gaa acc atc ctc ccg aag atg ggc atc caa aat gcc ttt gac 1131

300 305 310

aaa aat gct gat ttt tct gga att gca aag aga gac tcc ctg cag gtt 1179

315 320 325

tct aaa gca acc cac aag gct gtg ctg gat gtc agt gaa gag ggc act 1227

330 335 340

gag gcc aca gca gct acc acc acc aag ttc ata gtc cga tcg aag gat 1275

345 350 355 360

ggt ccc tct tac ttc act gtc tcc ttc aat agg acc ttc ctg atg atg 1323

365 370 375

att aca aat aaa gcc aca gac ggt att ctc ttt cta ggg aaa gtg gaa 1371

380 385 390

aat ccc act aaa tcc taggtgggaa atggcctgtt aactgatggc acattgctaa 1426

395

tgcacaagaa ataacaaacc acatcctctt tctgttctga gggtgcattt gaccccagtg 1486
ggagctggat tcgctggcag ggatgccacc ttccaaggct caatcaccaa accatcaaca 1546
gggaccccag tcacaagcca acacccatta acccc 1581

<210> 24
<211> 417
<212> PRT
<213> Homo sapiens

<400> 24

<210>  25

<211>  3027

<212>  DNA

<213>  Homo sapiens

<220>

<221>  CDS

<222>  (133)..(2271)

<223>


<220>

<221>  mat_peptide

<222>  (241)..()

<223>


<400>  25; LP223(a)
tggagaactg gggaggcaga gaccccggct ggccggaggc atgtggaggg gggggcctgg      60

gcgcagggag aggcccagcg gaagccaagc caccaggccc cccagcgtcc acgcggagca     120

tgaacattga gg atg gcg cgt gcc cgc ggc tcc ccg tgc ccc ccg ctg ccg     171
              Met Ala Arg Ala Arg Gly Ser Pro Cys Pro Pro Leu Pro
                  -35                 -30                 -25

ccc ggt agg atg tcc tgg ccc cac ggg gca ttg ctc ttc ctc tgg ctc       219
Pro Gly Arg Met Ser Trp Pro His Gly Ala Leu Leu Phe Leu Trp Leu
            -20                 -15                 -10

ttc tcc cca ccc ctg ggg gcc ggt gga ggt gga gtg gcc gtg acg tct       267
Phe Ser Pro Pro Leu Gly Ala Gly Gly Gly Gly Val Ala Val Thr Ser
        -5                  -1  1               5

gcc gcc gga ggg ggc tcc ccg ccg gcc acc tcc tgc ccc gtg gcc tgc       315
Ala Ala Gly Gly Gly Ser Pro Pro Ala Thr Ser Cys Pro Val Ala Cys
10                  15                  20                  25

tcc tgc agc aac cag gcc agc cgg gtg atc tgc aca cgg aga gac ctg       363
Ser Cys Ser Asn Gln Ala Ser Arg Val Ile Cys Thr Arg Arg Asp Leu
                30                  35                  40

gcc gag gtc cca gcc agc atc ccg gtc aac acg cgg tac ctg aac ctg       411
Ala Glu Val Pro Ala Ser Ile Pro Val Asn Thr Arg Tyr Leu Asn Leu
            45                  50                  55

caa gag aac ggc atc cag gtg atc cgg acg gac acg ttc aag cac ctg       459
Gln Glu Asn Gly Ile Gln Val Ile Arg Thr Asp Thr Phe Lys His Leu
        60                  65                  70

cgg cac ctg gag att ctg cag ctg agc aag aac ctg gtg cgc aag atc       507
Arg His Leu Glu Ile Leu Gln Leu Ser Lys Asn Leu Val Arg Lys Ile
    75                  80                  85

gag gtg ggc gcc ttc aac ggg ctg ccc agc ctc aac acg ctg gag ctt       555
Glu Val Gly Ala Phe Asn Gly Leu Pro Ser Leu Asn Thr Leu Glu Leu
90                  95                  100                 105

ttt gac aac cgg ctg acc acg gtg ccc acg cag gcc ttc gag tac ctg       603
Phe Asp Asn Arg Leu Thr Thr Val Pro Thr Gln Ala Phe Glu Tyr Leu
                110                 115                 120

tcc aag ctg cgg gag ctc tgg ctg cgg aac aac ccc atc gag agc atc       651
Ser Lys Leu Arg Glu Leu Trp Leu Arg Asn Asn Pro Ile Glu Ser Ile
            125                 130                 135

ccc tcc tac gcc ttc aac cgc gtg ccc tcg ctg cgg cgc ctg gac ctg 699
Pro Ser Tyr Ala Phe Asn Arg Val Pro Ser Leu Arg Arg Leu Asp Leu
140 145 150

ggc gag ctc aag cgg ctg gaa tac atc tcg gag gcg gcc ttc gag ggg 747
Gly Glu Leu Lys Arg Leu Glu Tyr Ile Ser Glu Ala Ala Phe Glu Gly
155 160 165

ctg gtc aac ctg cgc tac ctc aac ctg ggc atg tgc aac ctc aag gac 795
Leu Val Asn Leu Arg Tyr Leu Asn Leu Gly Met Cys Asn Leu Lys Asp
170 175 180 185

atc ccc aac ctg acg gcc ctg gtg cgc ctg gag gag ctg gag ctg tcg 843
Ile Pro Asn Leu Thr Ala Leu Val Arg Leu Glu Glu Leu Glu Leu Ser
190 195 200

ggc aac cgg ctg gac ctg atc cgc ccg ggc tcc ttc cag ggt ctc acc 891
Gly Asn Arg Leu Asp Leu Ile Arg Pro Gly Ser Phe Gln Gly Leu Thr
205 210 215

agc ctg cgc aag ctg tgg ctc atg cac gcc cag gta gcc acc atc gag 939
Ser Leu Arg Lys Leu Trp Leu Met His Ala Gln Val Ala Thr Ile Glu
220 225 230

cgc aac gcc ttc gac gac ctc aag tcg ctg gag gag ctc aac ctg tcc 987
Arg Asn Ala Phe Asp Asp Leu Lys Ser Leu Glu Glu Leu Asn Leu Ser
235 240 245

cac aac aac ctg atg tcg ctg ccc cac gac ctc ttc acg ccc ctg cac 1035
His Asn Asn Leu Met Ser Leu Pro His Asp Leu Phe Thr Pro Leu His
250 255 260 265

cgc ctc gag cgc gtg cac ctc aac cac aac ccc tgg cat tgc aac tgc 1083
Arg Leu Glu Arg Val His Leu Asn His Asn Pro Trp His Cys Asn Cys
270 275 280

gac gtg ctc tgg ctg agc tgg tgg ctc aag gag acg gtg ccc agc aac 1131
Asp Val Leu Trp Leu Ser Trp Trp Leu Lys Glu Thr Val Pro Ser Asn
285 290 295

acg acg tgc tgc gcc cgc tgt cat gcg ccc gcc ggc ctc aag ggg cgc 1179
Thr Thr Cys Cys Ala Arg Cys His Ala Pro Ala Gly Leu Lys Gly Arg
300 305 310

tac att ggg gag ctg gac cag tcg cat ttc acc tgc tat gcg ccc gtc 1227
Tyr Ile Gly Glu Leu Asp Gln Ser His Phe Thr Cys Tyr Ala Pro Val
315 320 325

atc gtg gag ccg ccc acg gac ctc aac gtc acc gag ggc atg gct gcc 1275
Ile Val Glu Pro Pro Thr Asp Leu Asn Val Thr Glu Gly Met Ala Ala
330 335 340 345

gag ctc aaa tgc cgc acg ggc acc tcc atg acc tcc gtc aac tgg ctg 1323
Glu Leu Lys Cys Arg Thr Gly Thr Ser Met Thr Ser Val Asn Trp Leu
350 355 360

acg ccc aac ggc acc ctc atg acc cac ggc tcc tac cgc gtg cgc atc 1371
Thr Pro Asn Gly Thr Leu Met Thr His Gly Ser Tyr Arg Val Arg Ile
365 370 375

tcc gtc ctg cat gac ggc acg ctt aac ttc acc aac gtc acc gtg cag 1419
Ser Val Leu His Asp Gly Thr Leu Asn Phe Thr Asn Val Thr Val Gln
380 385 390

gac acg ggc cag tac acg tgc atg gtg acg aac tca gcc ggc aac acc 1467
Asp Thr Gly Gln Tyr Thr Cys Met Val Thr Asn Ser Ala Gly Asn Thr
395 400 405

acc gcc tcg gcc acg ctc aac gtc tcg gcc gtg gac ccc gtg gcg gcc 1515

Thr Ala Ser Ala Thr Leu Asn Val Ser Ala Val Asp Pro Val Ala Ala
410                 415                 420                 425

ggg ggc acc ggc agc ggc ggg ggc ggc cct ggg ggc agt ggt ggt gtt      1563
Gly Gly Thr Gly Ser Gly Gly Gly Gly Pro Gly Gly Ser Gly Gly Val
                430                 435                 440

gga ggg ggc agt ggc ggc tac acc tac ttc acc acg gtg acc gtg gag      1611
Gly Gly Gly Ser Gly Gly Tyr Thr Tyr Phe Thr Thr Val Thr Val Glu
            445                 450                 455

acc ctg gag acg cag ccc gga gag gag gcc ctg cag ccg cgg ggg acg      1659
Thr Leu Glu Thr Gln Pro Gly Glu Glu Ala Leu Gln Pro Arg Gly Thr
        460                 465                 470

gag aag gaa ccg cca ggg ccc acg aca gac ggt gtc tgg ggt ggg ggc      1707
Glu Lys Glu Pro Pro Gly Pro Thr Thr Asp Gly Val Trp Gly Gly Gly
    475                 480                 485

cgg cct ggg gac gcg gcc ggc cct gcc tcg tct tct acc acg gca ccc      1755
Arg Pro Gly Asp Ala Ala Gly Pro Ala Ser Ser Ser Thr Thr Ala Pro
490                 495                 500                 505

gcc ccg cgc tcc tcg cgg ccc acg gag aag gcg ttc acg gtg ccc atc      1803
Ala Pro Arg Ser Ser Arg Pro Thr Glu Lys Ala Phe Thr Val Pro Ile
                510                 515                 520

acg gat gtg acg gag aac gcc ctc aag gac ctg gac gac gtc atg aag      1851
Thr Asp Val Thr Glu Asn Ala Leu Lys Asp Leu Asp Asp Val Met Lys
            525                 530                 535

acc acc aaa atc atc atc ggc tgc ttc gtg gcc atc acg ttc atg gcc      1899
Thr Thr Lys Ile Ile Ile Gly Cys Phe Val Ala Ile Thr Phe Met Ala
        540                 545                 550

gcg gtg atg ctc gtg gcc ttc tac aag ctg cgc aag cag cac cag ctc      1947
Ala Val Met Leu Val Ala Phe Tyr Lys Leu Arg Lys Gln His Gln Leu
    555                 560                 565

cac aag cac cac ggg ccc acg cgc acc gtg gag atc atc aac gtg gag      1995
His Lys His His Gly Pro Thr Arg Thr Val Glu Ile Ile Asn Val Glu
570                 575                 580                 585

gac gag ctg ccc gcc gcc tcg gcc gtg tcc gtg gcc gcc gcg gcc gcc      2043
Asp Glu Leu Pro Ala Ala Ser Ala Val Ser Val Ala Ala Ala Ala Ala
                590                 595                 600

gtg gcc agt ggg ggt ggt gtg ggc ggg gac agc cac ctg gcc ctg ccc      2091
Val Ala Ser Gly Gly Gly Val Gly Gly Asp Ser His Leu Ala Leu Pro
            605                 610                 615

gcc ctg gag cga gac cac ctc aac cac cac cac tac gtg gct gcc gcc      2139
Ala Leu Glu Arg Asp His Leu Asn His His His Tyr Val Ala Ala Ala
        620                 625                 630

ttc aag gcg cac tac agc agc aac ccc agc ggc ggg ggc tgc ggg ggc      2187
Phe Lys Ala His Tyr Ser Ser Asn Pro Ser Gly Gly Gly Cys Gly Gly
    635                 640                 645

aaa ggc ccg cct ggc ctc aac tcc atc cac gaa cct ctg ctc ttc aag      2235
Lys Gly Pro Pro Gly Leu Asn Ser Ile His Glu Pro Leu Leu Phe Lys
650                 655                 660                 665

agc ggc tcc aag gag aac gtg caa gag acg cag atc tgaggcggcg           2281
Ser Gly Ser Lys Glu Asn Val Gln Glu Thr Gln Ile
                670                 675

gggccgggcg ggcgaggggc gtggagcccc ccacccaggt cccagcccgg gcgcagcctg    2341

accgggaccc ctccctccca cagcccagcc caccttctgg gaccacgcag ggaattgggg 2401

agaggtggct tccagcccca tctggggctc ggacccccag taaggacagg gtggggctcc 2461

aggagcggag tctctgggat cctcgcctca cccccgccaa tccccggtga cgggagggga 2521

cgtgggaccc aggagaagtg gcctgagttc tccgcgtttc ctcctcgctc tcccaccaag 2581

cgttccgggc ccgccgcctt ccaggggaga gaggagcttt ctccaggggt gtcctttccc 2641

ctcggcccca aacaccttcc ttttacggtt cagtttttgc agtttgacgc ccgtccctcc 2701

ctcccactcc tccgccctca aaactgaaga gatggacacg tcgccagagc tcccgtccca 2761

gcgcccggct ctgctcccgg ccggcggtcc ctgtcgtcgg cgcggggggg agcctccacc 2821

cgccccagcc ccacctgccc cacagacact tttgatactg aagggaggtt tgcgtcaacg 2881

actacctgct ctgtaattac tttaaaaaaa aaacatggaa aaagtaaaaa aatatttttt 2941

ttggtcatga aagcacggag gagagaaaac aaagtgaatg tgggtggggg cggaaggaga 3001

ggctggagag ccagcctcgc agctcc 3027

<210> 26

<211> 713

<212> PRT

<213> Homo sapiens

<400> 26; LP223(a)

Met Ala Arg Ala Arg Gly Ser Pro Cys Pro Pro Leu Pro Pro Gly Arg
    -35                 -30                 -25

Met Ser Trp Pro His Gly Ala Leu Leu Phe Leu Trp Leu Phe Ser Pro
-20                 -15                 -10                 -5

Pro Leu Gly Ala Gly Gly Gly Gly Val Ala Val Thr Ser Ala Ala Gly
            -1  1               5                   10

Gly Gly Ser Pro Pro Ala Thr Ser Cys Pro Val Ala Cys Ser Cys Ser
        15                  20                  25

Asn Gln Ala Ser Arg Val Ile Cys Thr Arg Arg Asp Leu Ala Glu Val
    30                  35                  40

Pro Ala Ser Ile Pro Val Asn Thr Arg Tyr Leu Asn Leu Gln Glu Asn
45                  50                  55                  60

Gly Ile Gln Val Ile Arg Thr Asp Thr Phe Lys His Leu Arg His Leu
                65                  70                  75

Glu Ile Leu Gln Leu Ser Lys Asn Leu Val Arg Lys Ile Glu Val Gly
            80                  85                  90

Ala Phe Asn Gly Leu Pro Ser Leu Asn Thr Leu Glu Leu Phe Asp Asn
        95                  100                 105

Arg Leu Thr Thr Val Pro Thr Gln Ala Phe Glu Tyr Leu Ser Lys Leu
    110                 115                 120

Arg Glu Leu Trp Leu Arg Asn Asn Pro Ile Glu Ser Ile Pro Ser Tyr
125                 130                 135                 140

Ala Phe Asn Arg Val Pro Ser Leu Arg Arg Leu Asp Leu Gly Glu Leu
                145                 150                 155

Lys Arg Leu Glu Tyr Ile Ser Glu Ala Ala Phe Glu Gly Leu Val Asn
            160                 165                 170

Leu Arg Tyr Leu Asn Leu Gly Met Cys Asn Leu Lys Asp Ile Pro Asn
        175                 180                 185

Leu Thr Ala Leu Val Arg Leu Glu Glu Leu Glu Leu Ser Gly Asn Arg
    190                 195                 200

Leu Asp Leu Ile Arg Pro Gly Ser Phe Gln Gly Leu Thr Ser Leu Arg
205                 210                 215                 220

Lys Leu Trp Leu Met His Ala Gln Val Ala Thr Ile Glu Arg Asn Ala
                225                 230                 235

Phe Asp Asp Leu Lys Ser Leu Glu Glu Leu Asn Leu Ser His Asn Asn
            240                 245                 250

Leu Met Ser Leu Pro His Asp Leu Phe Thr Pro Leu His Arg Leu Glu
        255                 260                 265

Arg Val His Leu Asn His Asn Pro Trp His Cys Asn Cys Asp Val Leu
    270                 275                 280

Trp Leu Ser Trp Trp Leu Lys Glu Thr Val Pro Ser Asn Thr Thr Cys
285                 290                 295                 300

Cys Ala Arg Cys His Ala Pro Ala Gly Leu Lys Gly Arg Tyr Ile Gly
                305                 310                 315

Glu Leu Asp Gln Ser His Phe Thr Cys Tyr Ala Pro Val Ile Val Glu
            320                 325                 330

Pro Pro Thr Asp Leu Asn Val Thr Glu Gly Met Ala Ala Glu Leu Lys
        335                 340                 345

Cys Arg Thr Gly Thr Ser Met Thr Ser Val Asn Trp Leu Thr Pro Asn
    350                 355                 360

Gly Thr Leu Met Thr His Gly Ser Tyr Arg Val Arg Ile Ser Val Leu

365                 370                 375                 380

His Asp Gly Thr Leu Asn Phe Thr Asn Val Thr Val Gln Asp Thr Gly
                385                 390                 395

Gln Tyr Thr Cys Met Val Thr Asn Ser Ala Gly Asn Thr Thr Ala Ser
            400                 405                 410

Ala Thr Leu Asn Val Ser Ala Val Asp Pro Val Ala Ala Gly Gly Thr
        415                 420                 425

Gly Ser Gly Gly Gly Gly Pro Gly Gly Ser Gly Gly Val Gly Gly Gly
    430                 435                 440

Ser Gly Gly Tyr Thr Tyr Phe Thr Thr Val Thr Val Glu Thr Leu Glu
445                 450                 455                 460

Thr Gln Pro Gly Glu Glu Ala Leu Gln Pro Arg Gly Thr Glu Lys Glu
                465                 470                 475

Pro Pro Gly Pro Thr Thr Asp Gly Val Trp Gly Gly Gly Arg Pro Gly
            480                 485                 490

Asp Ala Ala Gly Pro Ala Ser Ser Ser Thr Thr Ala Pro Ala Pro Arg
        495                 500                 505

Ser Ser Arg Pro Thr Glu Lys Ala Phe Thr Val Pro Ile Thr Asp Val
    510                 515                 520

Thr Glu Asn Ala Leu Lys Asp Leu Asp Asp Val Met Lys Thr Thr Lys
525                 530                 535                 540

Ile Ile Ile Gly Cys Phe Val Ala Ile Thr Phe Met Ala Ala Val Met
                545                 550                 555

Leu Val Ala Phe Tyr Lys Leu Arg Lys Gln His Gln Leu His Lys His
            560                 565                 570

His Gly Pro Thr Arg Thr Val Glu Ile Ile Asn Val Glu Asp Glu Leu
        575                 580                 585

Pro Ala Ala Ser Ala Val Ser Val Ala Ala Ala Ala Ala Val Ala Ser
    590                 595                 600

Gly Gly Gly Val Gly Gly Asp Ser His Leu Ala Leu Pro Ala Leu Glu
605                 610                 615                 620

Arg Asp His Leu Asn His His His Tyr Val Ala Ala Ala Phe Lys Ala
                625                 630                 635

His Tyr Ser Ser Asn Pro Ser Gly Gly Gly Cys Gly Gly Lys Gly Pro
            640                 645                 650

Pro Gly Leu Asn Ser Ile His Glu Pro Leu Leu Phe Lys Ser Gly Ser
        655                 660                 665

Lys Glu Asn Val Gln Glu Thr Gln Ile
    670                 675

<210>  27

<211>  1416

<212>  DNA

<213>  Homo sapiens


<220>

<221>  CDS

<222>  (96)..(1364)

<223>


<220>

<221>  mat_peptide

<222>  (171)..()

<223>


<400>  27; LP255(a)
gtctggagat ggggggcggt ggtggccaca gcagaggctc caggaggtga ccgcctggtt      60

tccattagga agtcctggtc agcagcttgg gcgag atg gca gag tca ggg ctg       113
                                       Met Ala Glu Ser Gly Leu
                                       -25                 -20

gcc atg tgg ccg agc ctg ctg ctg ctc ctg ctg ttg ccg ggg ccc ccg      161
Ala Met Trp Pro Ser Leu Leu Leu Leu Leu Leu Leu Pro Gly Pro Pro
                -15                 -10                 -5

ccc gtc gcc ggc ttg gaa gac gct gcc ttc ccc cac ctg ggg gag agc      209
Pro Val Ala Gly Leu Glu Asp Ala Ala Phe Pro His Leu Gly Glu Ser
        -1  1               5                   10

ttg cag ccc ctg ccc cgg gcc tgt ccc ctg cgc tgc tcc tgc ccc cga      257
Leu Gln Pro Leu Pro Arg Ala Cys Pro Leu Arg Cys Ser Cys Pro Arg
    15                  20                  25

gtc gac act gtg gac tgt gat ggc ttg gac ctt cga gtg ttc ccg gac      305
Val Asp Thr Val Asp Cys Asp Gly Leu Asp Leu Arg Val Phe Pro Asp
30                  35                  40                  45

aac atc acc aga gcc gct cag cac ctc tcc ctg cag aac aac cag ctc      353
Asn Ile Thr Arg Ala Ala Gln His Leu Ser Leu Gln Asn Asn Gln Leu
                50                  55                  60

cag gaa ctc ccc tac aat gag ctg tcc cgc ctc agt ggc ctg cga acc      401
Gln Glu Leu Pro Tyr Asn Glu Leu Ser Arg Leu Ser Gly Leu Arg Thr

65 70 75

ctc aac ctc cac aac aac ctc atc tcc tcc gaa ggc ctg cct gac gag 449
Leu Asn Leu His Asn Asn Leu Ile Ser Ser Glu Gly Leu Pro Asp Glu
80 85 90

gcc ttc gag tcc ctc acc cag ctg cag cac ctc tgc gtg gct cac aac 497
Ala Phe Glu Ser Leu Thr Gln Leu Gln His Leu Cys Val Ala His Asn
95 100 105

aag aac aat ctc atc tcc aag gtg ccc cga gga gcc ctg agc cgc cag 545
Lys Asn Asn Leu Ile Ser Lys Val Pro Arg Gly Ala Leu Ser Arg Gln
110 115 120 125

act caa ctc cgt gag ctc tac ctc cag cac aac cag ctg aca gac agt 593
Thr Gln Leu Arg Glu Leu Tyr Leu Gln His Asn Gln Leu Thr Asp Ser
130 135 140

ggc ctg gat gcc acc acc ttc agc aag ctg cat agc ctt gaa tac ctg 641
Gly Leu Asp Ala Thr Thr Phe Ser Lys Leu His Ser Leu Glu Tyr Leu
145 150 155

gat ctc tcc cac aac cag ctg acc aca gtg ccc gcc ggc ctg ccc cgg 689
Asp Leu Ser His Asn Gln Leu Thr Thr Val Pro Ala Gly Leu Pro Arg
160 165 170

acc ctg gct atc ctg cac ctg ggc cgc aac cgc atc cgg cag gtg gag 737
Thr Leu Ala Ile Leu His Leu Gly Arg Asn Arg Ile Arg Gln Val Glu
175 180 185

gcg gct cgg ctg cac ggg gcg cgt ggt ctg cgc tat ttg ttg ctg cag 785
Ala Ala Arg Leu His Gly Ala Arg Gly Leu Arg Tyr Leu Leu Leu Gln
190 195 200 205

cac aac cag ctg ggg agc tca ggg ctg ccc gcc ggg gct ctg cgg ccg 833
His Asn Gln Leu Gly Ser Ser Gly Leu Pro Ala Gly Ala Leu Arg Pro
210 215 220

ctg cgg ggc ctg cac acg ctg cac ctc tat ggc aat ggg ctg gac cgc 881
Leu Arg Gly Leu His Thr Leu His Leu Tyr Gly Asn Gly Leu Asp Arg
225 230 235

gtg cct cca gcc ctg ccc cgc cgc ctg cgt gcc ctg gtg ctg ccc cac 929
Val Pro Pro Ala Leu Pro Arg Arg Leu Arg Ala Leu Val Leu Pro His
240 245 250

aac cac gtg gcc gcg ctg ggt gcc cgt gac ctg gtc gcc aca ccg ggc 977
Asn His Val Ala Ala Leu Gly Ala Arg Asp Leu Val Ala Thr Pro Gly
255 260 265

ctg acg gag ctt aac ctg gcc tat aac cgc ctg gcc agc gcc cgt gtg 1025
Leu Thr Glu Leu Asn Leu Ala Tyr Asn Arg Leu Ala Ser Ala Arg Val
270 275 280 285

cac cac cgg gcc ttc cgc cgg ttg cgt gcc ctg cgc agc ctc gac ctg 1073
His His Arg Ala Phe Arg Arg Leu Arg Ala Leu Arg Ser Leu Asp Leu
290 295 300

gca ggg aat cag cta acc cgg ctg ccc atg ggc ctg ccc act ggc ctg 1121
Ala Gly Asn Gln Leu Thr Arg Leu Pro Met Gly Leu Pro Thr Gly Leu
305 310 315

cgc acc ctg cag ctg caa cgc aac cag ctg cgg atg ctc gag ccc gag 1169
Arg Thr Leu Gln Leu Gln Arg Asn Gln Leu Arg Met Leu Glu Pro Glu
320 325 330

cct ctg gcc ggc ctg gac caa ctg cgg gag ctc agc ctg gcg cac aac 1217
Pro Leu Ala Gly Leu Asp Gln Leu Arg Glu Leu Ser Leu Ala His Asn
335 340 345

cgg ctc cgg gtc ggc gac atc ggg cca ggc acc tgg cat gag ctc caa     1265
Arg Leu Arg Val Gly Asp Ile Gly Pro Gly Thr Trp His Glu Leu Gln
350                 355                 360                 365

gcc ctc cag gtc agg cac agg ctg gtt agc cac act gtc ccc agg gcc     1313
Ala Leu Gln Val Arg His Arg Leu Val Ser His Thr Val Pro Arg Ala
                370                 375                 380

cct cca tcc ccc tgc ctg ccc tgc cac gtc cca aac att cta gtt agc     1361
Pro Pro Ser Pro Cys Leu Pro Cys His Val Pro Asn Ile Leu Val Ser
            385                 390                 395

tgg taaagcaatc agaacaagaa aatgataaga gtgggttaga aggtgatgag ga       1416
Trp

<210>  28

<211>  423

<212>  PRT

<213>  Homo sapiens

<400>  28; LP255(a)

Met Ala Glu Ser Gly Leu Ala Met Trp Pro Ser Leu Leu Leu Leu Leu
-25                 -20                 -15                 -10

Leu Leu Pro Gly Pro Pro Pro Val Ala Gly Leu Glu Asp Ala Ala Phe
                -5                  -1  1               5

Pro His Leu Gly Glu Ser Leu Gln Pro Leu Pro Arg Ala Cys Pro Leu
        10                  15                  20

Arg Cys Ser Cys Pro Arg Val Asp Thr Val Asp Cys Asp Gly Leu Asp
    25                  30                  35

Leu Arg Val Phe Pro Asp Asn Ile Thr Arg Ala Ala Gln His Leu Ser
40                  45                  50                  55

Leu Gln Asn Asn Gln Leu Gln Glu Leu Pro Tyr Asn Glu Leu Ser Arg
                60                  65                  70

Leu Ser Gly Leu Arg Thr Leu Asn Leu His Asn Asn Leu Ile Ser Ser
            75                  80                  85

Glu Gly Leu Pro Asp Glu Ala Phe Glu Ser Leu Thr Gln Leu Gln His
        90                  95                  100

Leu Cys Val Ala His Asn Lys Asn Asn Leu Ile Ser Lys Val Pro Arg
    105                 110                 115

Gly Ala Leu Ser Arg Gln Thr Gln Leu Arg Glu Leu Tyr Leu Gln His
120                 125                 130                 135

Asn Gln Leu Thr Asp Ser Gly Leu Asp Ala Thr Thr Phe Ser Lys Leu
                140                 145                 150

His Ser Leu Glu Tyr Leu Asp Leu Ser His Asn Gln Leu Thr Thr Val
            155                 160                 165

Pro Ala Gly Leu Pro Arg Thr Leu Ala Ile Leu His Leu Gly Arg Asn
        170                 175                 180

Arg Ile Arg Gln Val Glu Ala Ala Arg Leu His Gly Ala Arg Gly Leu
    185                 190                 195

Arg Tyr Leu Leu Leu Gln His Asn Gln Leu Gly Ser Ser Gly Leu Pro
200                 205                 210                 215

Ala Gly Ala Leu Arg Pro Leu Arg Gly Leu His Thr Leu His Leu Tyr
                220                 225                 230

Gly Asn Gly Leu Asp Arg Val Pro Pro Ala Leu Pro Arg Arg Leu Arg
            235                 240                 245

Ala Leu Val Leu Pro His Asn His Val Ala Ala Leu Gly Ala Arg Asp
        250                 255                 260

Leu Val Ala Thr Pro Gly Leu Thr Glu Leu Asn Leu Ala Tyr Asn Arg
    265                 270                 275

Leu Ala Ser Ala Arg Val His His Arg Ala Phe Arg Arg Leu Arg Ala
280                 285                 290                 295

Leu Arg Ser Leu Asp Leu Ala Gly Asn Gln Leu Thr Arg Leu Pro Met
                300                 305                 310

Gly Leu Pro Thr Gly Leu Arg Thr Leu Gln Leu Gln Arg Asn Gln Leu
            315                 320                 325

Arg Met Leu Glu Pro Glu Pro Leu Ala Gly Leu Asp Gln Leu Arg Glu
        330                 335                 340

Leu Ser Leu Ala His Asn Arg Leu Arg Val Gly Asp Ile Gly Pro Gly
    345                 350                 355

Thr Trp His Glu Leu Gln Ala Leu Gln Val Arg His Arg Leu Val Ser
360                 365                 370                 375

His Thr Val Pro Arg Ala Pro Pro Ser Pro Cys Leu Pro Cys His Val
                380                 385                 390

Pro Asn Ile Leu Val Ser Trp
            395

<210>  29

<211>  445

<212>  DNA

<213>  Homo sapiens


<220>

<221>  CDS

<222>  (25)..(330)

<223>


<220>

<221>  mat_peptide

<222>  (76)..()

<223>


<400>  29; LP244
ctgagagcag agctggccgc agcc atg acc ccg cag ctt ctc ctg gcc ctt        51
                           Met Thr Pro Gln Leu Leu Leu Ala Leu
                                   -15                 -10

gtc ctc tgg gcc agc tgc ccg ccc tgc agt gga agg aaa ggg ccc cca        99
Val Leu Trp Ala Ser Cys Pro Pro Cys Ser Gly Arg Lys Gly Pro Pro
            -5                  -1  1               5

gca gct ctg aca ctg ccc cgg gtg caa tgc cga gcc tct cgg tac ccg       147
Ala Ala Leu Thr Leu Pro Arg Val Gln Cys Arg Ala Ser Arg Tyr Pro
    10                  15                  20

atc gcc gtg gat tgc tcc tgg acc ctg ccg cct gct cca aac tcc acc       195
Ile Ala Val Asp Cys Ser Trp Thr Leu Pro Pro Ala Pro Asn Ser Thr
25                  30                  35                  40

agc ccc gtg tcc ttc att gcc acg tac agg tcg gag agc ctg gaa ggg       243
Ser Pro Val Ser Phe Ile Ala Thr Tyr Arg Ser Glu Ser Leu Glu Gly
                45                  50                  55

ggc ctc agg gac act gga ctt cct gga gag ccc aga act ctg gcc ctg       291
Gly Leu Arg Asp Thr Gly Leu Pro Gly Glu Pro Arg Thr Leu Ala Leu
            60                  65                  70

aga gcc ctg ggg tca gga aaa ctg gag acc ccg aca tcc tgaaccccag        340
Arg Ala Leu Gly Ser Gly Lys Leu Glu Thr Pro Thr Ser
        75                  80                  85

gcctatggaa ttctagggcg gccccgtcca atagaaatat actttctggg ccaggaaaaa     400

aaaaaaaaaa aaaaaaaaaa aaaaaaaaaa gaaaaaaaaa aaaaa                     445


<210>  30

<211>  102

<212>  PRT

<213>  Homo sapiens

<400>  30; LP244

Met Thr Pro Gln Leu Leu Leu Ala Leu Val Leu Trp Ala Ser Cys Pro
        -15                 -10                 -5

Pro Cys Ser Gly Arg Lys Gly Pro Pro Ala Ala Leu Thr Leu Pro Arg
-1  1               5                   10                  15

Val Gln Cys Arg Ala Ser Arg Tyr Pro Ile Ala Val Asp Cys Ser Trp
                20                  25                  30

Thr Leu Pro Pro Ala Pro Asn Ser Thr Ser Pro Val Ser Phe Ile Ala
            35                  40                  45

Thr Tyr Arg Ser Glu Ser Leu Glu Gly Gly Leu Arg Asp Thr Gly Leu
        50                  55                  60

Pro Gly Glu Pro Arg Thr Leu Ala Leu Arg Ala Leu Gly Ser Gly Lys
    65                  70                  75

Leu Glu Thr Pro Thr Ser
80                  85

<210>  31

<211>  1858

<212>  DNA

<213>  Homo sapiens

<220>

<221>  CDS

<222>  (1)..(1761)

<223>

<220>

<221>  mat_peptide

<222>  (79)..()

<223>

<400>  31; LP186
atg gtc tcc cca cgg atg tcc ggg ctc ctc tcc cag act gtg atc cta        48
Met Val Ser Pro Arg Met Ser Gly Leu Leu Ser Gln Thr Val Ile Leu
    -25                 -20                 -15

gcg ctc att ttc ctc ccc cag aca cgg ccc gct ggc gtc ttc gag ctg 96
Ala Leu Ile Phe Leu Pro Gln Thr Arg Pro Ala Gly Val Phe Glu Leu
-10 -5 -1 1 5

cag atc cac tct ttc ggg ccg ggt cca ggc cct ggg gcc ccg cgg tcc 144
Gln Ile His Ser Phe Gly Pro Gly Pro Gly Pro Gly Ala Pro Arg Ser
10 15 20

ccc tgc agc gcc cgg ctc ccc tgc cgc ctc ttc ttc aga gtc tgc ctg 192
Pro Cys Ser Ala Arg Leu Pro Cys Arg Leu Phe Phe Arg Val Cys Leu
25 30 35

aag cct ggg ctc tca gag gag gcc gcc gag tcc ccg tgc gcc ctg ggc 240
Lys Pro Gly Leu Ser Glu Glu Ala Ala Glu Ser Pro Cys Ala Leu Gly
40 45 50

gcg gcg ctg agt gcg cgc gga ccg gtc tac acc gag cag ccc gga gcg 288
Ala Ala Leu Ser Ala Arg Gly Pro Val Tyr Thr Glu Gln Pro Gly Ala
55 60 65 70

ccc gcg cct gat ctc cca ctg ccc gac ggc ctc ttg cag gtg ccc ttc 336
Pro Ala Pro Asp Leu Pro Leu Pro Asp Gly Leu Leu Gln Val Pro Phe
75 80 85

cgg gac gcc tgg cct ggc acc ttc tct ttc atc atc gaa acc tgg aga 384
Arg Asp Ala Trp Pro Gly Thr Phe Ser Phe Ile Ile Glu Thr Trp Arg
90 95 100

gag gag tta gga gac cag att gga ggg ccc gcc tgg agc ctg ctg gcg 432
Glu Glu Leu Gly Asp Gln Ile Gly Gly Pro Ala Trp Ser Leu Leu Ala
105 110 115

cgc gtg gct ggc agg cgg cgc ttg gca gcc gga ggc ccg tgg gcc cgg 480
Arg Val Ala Gly Arg Arg Arg Leu Ala Ala Gly Gly Pro Trp Ala Arg
120 125 130

gac att cag cgc gca ggc gcc tgg gag ctg cgc tgc tcg tac cgc gcg 528
Asp Ile Gln Arg Ala Gly Ala Trp Glu Leu Arg Cys Ser Tyr Arg Ala
135 140 145 150

cgc tgc gag ccg cct gcg gtc ggg acc gcg tgc acg cgc ctc tgc cgt 576
Arg Cys Glu Pro Pro Ala Val Gly Thr Ala Cys Thr Arg Leu Cys Arg
155 160 165

ccg cgc agc gcc ccc tcg cgg tgc ggt ccg gga ctg cgc ccc tgc gca 624
Pro Arg Ser Ala Pro Ser Arg Cys Gly Pro Gly Leu Arg Pro Cys Ala
170 175 180

ccg ctc gag gac gaa tgt gag gcg ccg ccg gtg tgc cga gca ggc tgc 672
Pro Leu Glu Asp Glu Cys Glu Ala Pro Pro Val Cys Arg Ala Gly Cys
185 190 195

agc cct gag cat ggc ttc tgt gaa cag ccc ggt gaa tgc cga tgc cta 720
Ser Pro Glu His Gly Phe Cys Glu Gln Pro Gly Glu Cys Arg Cys Leu
200 205 210

gag ggc tgg act gga ccc ctc tgc acg gtc cct gtc tcc acc agc agc 768
Glu Gly Trp Thr Gly Pro Leu Cys Thr Val Pro Val Ser Thr Ser Ser
215 220 225 230

tgc ctc agc ccc agg ggc ccg tcc tct gct acc acc gga tgc ctt gtc 816
Cys Leu Ser Pro Arg Gly Pro Ser Ser Ala Thr Thr Gly Cys Leu Val
235 240 245

cct ggg cct ggg ccc tgt gac ggg aac ccg tgt gcc aat gga ggc agc 864
Pro Gly Pro Gly Pro Cys Asp Gly Asn Pro Cys Ala Asn Gly Gly Ser
250 255 260

tgt agt gag aca ccc agg tcc ttt gaa tgc acc tgc ccg cgt ggg ttc 912
Cys Ser Glu Thr Pro Arg Ser Phe Glu Cys Thr Cys Pro Arg Gly Phe
265 270 275

tac ggg ctg cgg tgt gag gtg agc ggg gtg aca tgt gca gat gga ccc 960
Tyr Gly Leu Arg Cys Glu Val Ser Gly Val Thr Cys Ala Asp Gly Pro
280 285 290

tgc ttc aac ggc ggc ttg tgt gtc ggg ggt gca gac cct gac tct gcc 1008
Cys Phe Asn Gly Gly Leu Cys Val Gly Gly Ala Asp Pro Asp Ser Ala
295 300 305 310

tac atc tgc cac tgc cca cct ggt ttc caa ggc tcc aac tgt gag aag 1056
Tyr Ile Cys His Cys Pro Pro Gly Phe Gln Gly Ser Asn Cys Glu Lys
315 320 325

agg gtg gac cgg tgc agc ctg cag cca tgc cgc aat ggc gga ctc tgc 1104
Arg Val Asp Arg Cys Ser Leu Gln Pro Cys Arg Asn Gly Gly Leu Cys
330 335 340

ctg gac ctg ggc cac gcc ctg cgc tgc cgc tgc cgc gcc ggc ttc gcg 1152
Leu Asp Leu Gly His Ala Leu Arg Cys Arg Cys Arg Ala Gly Phe Ala
345 350 355

ggt cct cgc tgc gag cac gac ctg gac gac tgc gcg ggc cgc gcc tgc 1200
Gly Pro Arg Cys Glu His Asp Leu Asp Asp Cys Ala Gly Arg Ala Cys
360 365 370

gct aac ggc ggc acg tgt gtg gag ggc ggc ggc gcg cac cgc tgc tcc 1248
Ala Asn Gly Gly Thr Cys Val Glu Gly Gly Gly Ala His Arg Cys Ser
375 380 385 390

tgc gcg ctg ggc ttc ggc ggc cgc gac tgc cgc gag cgc gcg gac ccg 1296
Cys Ala Leu Gly Phe Gly Gly Arg Asp Cys Arg Glu Arg Ala Asp Pro
395 400 405

tgc gcc gcg cgc ccc tgt gct cac ggc ggc cgc tgc tac gcc cac ttc 1344
Cys Ala Ala Arg Pro Cys Ala His Gly Gly Arg Cys Tyr Ala His Phe
410 415 420

tcc ggc ctc gtc tgc gct tgc gct ccc ggc tac atg gga gcg cgg tgt 1392
Ser Gly Leu Val Cys Ala Cys Ala Pro Gly Tyr Met Gly Ala Arg Cys
425 430 435

gag ttc cca gtg cac ccc gac ggc gca agc gcc ttg ccc gcg gcc ccg 1440
Glu Phe Pro Val His Pro Asp Gly Ala Ser Ala Leu Pro Ala Ala Pro
440 445 450

ccg ggc ctc agg ccc ggg gac cct cag cgc tac ctt ttg cct ccg gct 1488
Pro Gly Leu Arg Pro Gly Asp Pro Gln Arg Tyr Leu Leu Pro Pro Ala
455 460 465 470

ctg gga ctg ctc gtg gcc gcg ggc gtg gcc ggc gct gcg ctc ttg ctg 1536
Leu Gly Leu Leu Val Ala Ala Gly Val Ala Gly Ala Ala Leu Leu Leu
475 480 485

gtc cac gtg cgc cgc cgt ggc cac tcc cag gat gct ggg tct cgc ttg 1584
Val His Val Arg Arg Arg Gly His Ser Gln Asp Ala Gly Ser Arg Leu
490 495 500

ctg gct ggg acc ccg gag ccg tca gtc cac gca ctc ccg gat gca ctc 1632
Leu Ala Gly Thr Pro Glu Pro Ser Val His Ala Leu Pro Asp Ala Leu
505 510 515

aac aac cta agg acg cag gag ggt tcc ggg gat ggt ccg agc tcg tcc 1680
Asn Asn Leu Arg Thr Gln Glu Gly Ser Gly Asp Gly Pro Ser Ser Ser
520 525 530

gta gat tgg aat cgc cct gaa gat gta gac cct caa ggg att tat gtc 1728

Val Asp Trp Asn Arg Pro Glu Asp Val Asp Pro Gln Gly Ile Tyr Val
535                 540                 545                 550

ata tct gct cct tcc atc tac gct cgg gag gcc tgacgcgtct cctccatccg    1781
Ile Ser Ala Pro Ser Ile Tyr Ala Arg Glu Ala
                555                 560

cacctggagt cagagcgtgg atttttgtat ttgctcggtg gtgcccagtc tctgccccag    1841

aggctttgga gttcaat                                                   1858

<210>  32

<211>  587

<212>  PRT

<213>  Homo sapiens

<400>  32; LP186

Met Val Ser Pro Arg Met Ser Gly Leu Leu Ser Gln Thr Val Ile Leu
    -25                 -20                 -15

Ala Leu Ile Phe Leu Pro Gln Thr Arg Pro Ala Gly Val Phe Glu Leu
-10                 -5                  -1  1               5

Gln Ile His Ser Phe Gly Pro Gly Pro Gly Pro Gly Ala Pro Arg Ser
            10                  15                  20

Pro Cys Ser Ala Arg Leu Pro Cys Arg Leu Phe Phe Arg Val Cys Leu
        25                  30                  35

Lys Pro Gly Leu Ser Glu Glu Ala Ala Glu Ser Pro Cys Ala Leu Gly
    40                  45                  50

Ala Ala Leu Ser Ala Arg Gly Pro Val Tyr Thr Glu Gln Pro Gly Ala
55                  60                  65                  70

Pro Ala Pro Asp Leu Pro Leu Pro Asp Gly Leu Leu Gln Val Pro Phe
                75                  80                  85

Arg Asp Ala Trp Pro Gly Thr Phe Ser Phe Ile Ile Glu Thr Trp Arg
            90                  95                  100

Glu Glu Leu Gly Asp Gln Ile Gly Gly Pro Ala Trp Ser Leu Leu Ala
        105                 110                 115

Arg Val Ala Gly Arg Arg Arg Leu Ala Ala Gly Gly Pro Trp Ala Arg
    120                 125                 130

Asp Ile Gln Arg Ala Gly Ala Trp Glu Leu Arg Cys Ser Tyr Arg Ala
135                 140                 145                 150

Arg Cys Glu Pro Pro Ala Val Gly Thr Ala Cys Thr Arg Leu Cys Arg

155 160 165

Pro Arg Ser Ala Pro Ser Arg Cys Gly Pro Gly Leu Arg Pro Cys Ala
170 175 180

Pro Leu Glu Asp Glu Cys Glu Ala Pro Pro Val Cys Arg Ala Gly Cys
185 190 195

Ser Pro Glu His Gly Phe Cys Glu Gln Pro Gly Glu Cys Arg Cys Leu
200 205 210

Glu Gly Trp Thr Gly Pro Leu Cys Thr Val Pro Val Ser Thr Ser Ser
215 220 225 230

Cys Leu Ser Pro Arg Gly Pro Ser Ser Ala Thr Thr Gly Cys Leu Val
235 240 245

Pro Gly Pro Gly Pro Cys Asp Gly Asn Pro Cys Ala Asn Gly Gly Ser
250 255 260

Cys Ser Glu Thr Pro Arg Ser Phe Glu Cys Thr Cys Pro Arg Gly Phe
265 270 275

Tyr Gly Leu Arg Cys Glu Val Ser Gly Val Thr Cys Ala Asp Gly Pro
280 285 290

Cys Phe Asn Gly Gly Leu Cys Val Gly Gly Ala Asp Pro Asp Ser Ala
295 300 305 310

Tyr Ile Cys His Cys Pro Pro Gly Phe Gln Gly Ser Asn Cys Glu Lys
315 320 325

Arg Val Asp Arg Cys Ser Leu Gln Pro Cys Arg Asn Gly Gly Leu Cys
330 335 340

Leu Asp Leu Gly His Ala Leu Arg Cys Arg Cys Arg Ala Gly Phe Ala
345 350 355

Gly Pro Arg Cys Glu His Asp Leu Asp Asp Cys Ala Gly Arg Ala Cys
360 365 370

Ala Asn Gly Gly Thr Cys Val Glu Gly Gly Gly Ala His Arg Cys Ser
375 380 385 390

Cys Ala Leu Gly Phe Gly Gly Arg Asp Cys Arg Glu Arg Ala Asp Pro
395 400 405

Cys Ala Ala Arg Pro Cys Ala His Gly Gly Arg Cys Tyr Ala His Phe
410 415 420

Ser Gly Leu Val Cys Ala Cys Ala Pro Gly Tyr Met Gly Ala Arg Cys
425 430 435

Glu Phe Pro Val His Pro Asp Gly Ala Ser Ala Leu Pro Ala Ala Pro
    440                 445                 450

Pro Gly Leu Arg Pro Gly Asp Pro Gln Arg Tyr Leu Leu Pro Pro Ala
455                 460                 465                 470

Leu Gly Leu Leu Val Ala Ala Gly Val Ala Gly Ala Ala Leu Leu Leu
                475                 480                 485

Val His Val Arg Arg Arg Gly His Ser Gln Asp Ala Gly Ser Arg Leu
            490                 495                 500

Leu Ala Gly Thr Pro Glu Pro Ser Val His Ala Leu Pro Asp Ala Leu
        505                 510                 515

Asn Asn Leu Arg Thr Gln Glu Gly Ser Gly Asp Gly Pro Ser Ser Ser
    520                 525                 530

Val Asp Trp Asn Arg Pro Glu Asp Val Asp Pro Gln Gly Ile Tyr Val
535                 540                 545                 550

Ile Ser Ala Pro Ser Ile Tyr Ala Arg Glu Ala
                555                 560

<210>  33

<211>  1968

<212>  DNA

<213>  Homo sapiens


<220>

<221>  CDS

<222>  (25)..(1926)

<223>


<220>

<221>  mat_peptide

<222>  (79)..()

<223>


<400>  33; LP251(b)
acagcaacct ctcccctggc cctc atg ggc acc gtc agc tcc agg cgg tcc        51
                           Met Gly Thr Val Ser Ser Arg Arg Ser
                                       -15                 -10

tgg tgg ccg ctg cca ctg ctg ctg ctg ctg ctg ctg ctc ctg ggt ccc 99
Trp Trp Pro Leu Pro Leu Leu Leu Leu Leu Leu Leu Leu Leu Gly Pro
-5 -1 1 5

gcg ggc gcc cgt gcg cag gag gac gag gac ggc gac tac gag gag ctg 147
Ala Gly Ala Arg Ala Gln Glu Asp Glu Asp Gly Asp Tyr Glu Glu Leu
10 15 20

gtg cta gcc ttg cgt tcc gag gag gac ggc ctg gcc gaa gca ccc gag 195
Val Leu Ala Leu Arg Ser Glu Glu Asp Gly Leu Ala Glu Ala Pro Glu
25 30 35

cac gga acc aca gcc acc ttc cac cgc tgc gcc aag gat ccg tgg agg 243
His Gly Thr Thr Ala Thr Phe His Arg Cys Ala Lys Asp Pro Trp Arg
40 45 50 55

ttg cct ggc acc tac gtg gtg gtg ctg aag gag gag acc cac ctc tcg 291
Leu Pro Gly Thr Tyr Val Val Val Leu Lys Glu Glu Thr His Leu Ser
60 65 70

cag tca gag cgc act gcc cgc cgc ctg cag gcc cag gct gcc cgc cgg 339
Gln Ser Glu Arg Thr Ala Arg Arg Leu Gln Ala Gln Ala Ala Arg Arg
75 80 85

gga tac ctc acc aag atc ctg cat gtc ttc cat ggc ctt ctt cct ggc 387
Gly Tyr Leu Thr Lys Ile Leu His Val Phe His Gly Leu Leu Pro Gly
90 95 100

ttc ctg gtg aag atg agt ggc gac ctg ctg gag ctg gcc ttg aag ttg 435
Phe Leu Val Lys Met Ser Gly Asp Leu Leu Glu Leu Ala Leu Lys Leu
105 110 115

ccc cat gtc gac tac atc gag gag gac tcc tct gtc ttt gcc cag agc 483
Pro His Val Asp Tyr Ile Glu Glu Asp Ser Ser Val Phe Ala Gln Ser
120 125 130 135

atc ccg tgg aac ctg gag cgg att acc cct cca cgg tac cgg gcg gat 531
Ile Pro Trp Asn Leu Glu Arg Ile Thr Pro Pro Arg Tyr Arg Ala Asp
140 145 150

gaa tac cag ccc ccc gac gga ggc agc ctg gtg gag gtg tat ctc cta 579
Glu Tyr Gln Pro Pro Asp Gly Gly Ser Leu Val Glu Val Tyr Leu Leu
155 160 165

gac acc agc ata cag agt gac cac cgg gaa atc gag ggc agg gtc atg 627
Asp Thr Ser Ile Gln Ser Asp His Arg Glu Ile Glu Gly Arg Val Met
170 175 180

gtc acc gac ttc gag aat gtg ccc gag gag gac ggg acc cgc ttc cac 675
Val Thr Asp Phe Glu Asn Val Pro Glu Glu Asp Gly Thr Arg Phe His
185 190 195

aga cag gcc agc aag tgt gac agt cat ggc acc cac ctg gca ggg gtg 723
Arg Gln Ala Ser Lys Cys Asp Ser His Gly Thr His Leu Ala Gly Val
200 205 210 215

gtc agc ggc cgg gat gcc ggc gtg gcc aag ggt gcc agc atg cgc agc 771
Val Ser Gly Arg Asp Ala Gly Val Ala Lys Gly Ala Ser Met Arg Ser
220 225 230

ctg cgc gtg ctc aac tgc caa ggg aag ggc acg gtt agc ggc acc ctc 819
Leu Arg Val Leu Asn Cys Gln Gly Lys Gly Thr Val Ser Gly Thr Leu
235 240 245

ata ggc ctg gag ttt att cgg aaa agc cag ctg gtc cag cct gtg ggg 867
Ile Gly Leu Glu Phe Ile Arg Lys Ser Gln Leu Val Gln Pro Val Gly
250 255 260

cca ctg gtg gtg ctg ctg ccc ctg gcg ggt ggg tac agc cgc gtc ctc 915

Pro Leu Val Val Leu Leu Pro Leu Ala Gly Gly Tyr Ser Arg Val Leu
    265                 270                 275

aac gcc gcc tgc cag cgc ctg gcg agg gct ggg gtc gtg ctg gtc acc      963
Asn Ala Ala Cys Gln Arg Leu Ala Arg Ala Gly Val Val Leu Val Thr
280                 285                 290                 295

gct gcc ggc aac ttc cgg gac gat gcc tgc ctc tac tcc cca gcc tca     1011
Ala Ala Gly Asn Phe Arg Asp Asp Ala Cys Leu Tyr Ser Pro Ala Ser
                300                 305                 310

gct ccc gag gtc atc aca gtt ggg gcc acc aat gcc cag gac cag ccg     1059
Ala Pro Glu Val Ile Thr Val Gly Ala Thr Asn Ala Gln Asp Gln Pro
            315                 320                 325

gtg acc ctg ggg act ttg ggg acc aac ttt ggc cgc tgt gtg gac ctc     1107
Val Thr Leu Gly Thr Leu Gly Thr Asn Phe Gly Arg Cys Val Asp Leu
        330                 335                 340

ttt gcc cca ggg gag gac atc att ggt gcc tcc agc gac tgc agc acc     1155
Phe Ala Pro Gly Glu Asp Ile Ile Gly Ala Ser Ser Asp Cys Ser Thr
    345                 350                 355

tgc ttt gtg tca cag agt ggg aca tca cag gct gct gcc cac gtg gct     1203
Cys Phe Val Ser Gln Ser Gly Thr Ser Gln Ala Ala Ala His Val Ala
360                 365                 370                 375

ggt tgg cag ctg ttt tgc agg act gtg tgg tca gca cac tcg ggg cct     1251
Gly Trp Gln Leu Phe Cys Arg Thr Val Trp Ser Ala His Ser Gly Pro
                380                 385                 390

aca cgg atg gcc aca gcc atc gcc cgc tgc gcc cca gat gag gag ctg     1299
Thr Arg Met Ala Thr Ala Ile Ala Arg Cys Ala Pro Asp Glu Glu Leu
            395                 400                 405

ctg agc tgc tcc agt ttc tcc agg agt ggg aag cgg cgg ggc gag cgc     1347
Leu Ser Cys Ser Ser Phe Ser Arg Ser Gly Lys Arg Arg Gly Glu Arg
        410                 415                 420

atg gag gcc caa ggg ggc aag ctg gtc tgc cgg gcc cac aac gct ttt     1395
Met Glu Ala Gln Gly Gly Lys Leu Val Cys Arg Ala His Asn Ala Phe
    425                 430                 435

ggg ggt gag ggt gtc tac gcc att gcc agg tgc tgc ctg cta ccc cag     1443
Gly Gly Glu Gly Val Tyr Ala Ile Ala Arg Cys Cys Leu Leu Pro Gln
440                 445                 450                 455

gcc aac tgc agc gtc cac aca gct cca cca gct gag gcc agc atg ggg     1491
Ala Asn Cys Ser Val His Thr Ala Pro Pro Ala Glu Ala Ser Met Gly
                460                 465                 470

acc cgt gtc cac tgc cac caa cag ggc cac gtc ctc aca ggc tgc agc     1539
Thr Arg Val His Cys His Gln Gln Gly His Val Leu Thr Gly Cys Ser
            475                 480                 485

tcc cac tgg gag gtg gag gac ctt ggc acc cac aag ccg cct gtg ctg     1587
Ser His Trp Glu Val Glu Asp Leu Gly Thr His Lys Pro Pro Val Leu
        490                 495                 500

agg cca cga ggt cag ccc aac cag tgc gtg ggc cac agg gag gcc agc     1635
Arg Pro Arg Gly Gln Pro Asn Gln Cys Val Gly His Arg Glu Ala Ser
    505                 510                 515

atc cac gct tcc tgc tgc cat gcc cca ggt ctg gaa tgc aaa gtc aag     1683
Ile His Ala Ser Cys Cys His Ala Pro Gly Leu Glu Cys Lys Val Lys
520                 525                 530                 535

gag cat gga atc ccg gcc cct cag gag cag gtg acc gtg gcc tgc gag     1731
Glu His Gly Ile Pro Ala Pro Gln Glu Gln Val Thr Val Ala Cys Glu

540 545 550

gag ggc tgg acc ctg act ggc tgc agt gcc ctc cct ggg acc tcc cac 1779
Glu Gly Trp Thr Leu Thr Gly Cys Ser Ala Leu Pro Gly Thr Ser His
555 560 565

gtc ctg ggg gcc tac gcc gta gac aac acg tgt gta gtc agg agc cgg 1827
Val Leu Gly Ala Tyr Ala Val Asp Asn Thr Cys Val Val Arg Ser Arg
570 575 580

gac gtc agc act aca ggc agc acc agc gaa ggg gcc gtg aca gcc gtt 1875
Asp Val Ser Thr Thr Gly Ser Thr Ser Glu Gly Ala Val Thr Ala Val
585 590 595

gcc atc tgc tgc cgg agc cgg cac ctg gcg cag gcc tcc cag gag ctc 1923
Ala Ile Cys Cys Arg Ser Arg His Leu Ala Gln Ala Ser Gln Glu Leu
600 605 610 615

cag tgacagcccc atcccaggat gggtgtctgg ggagggtcaa gg 1968
Gln

<210> 34

<211> 634

<212> PRT

<213> Homo sapiens

<400> 34; LP251(b)

Met Gly Thr Val Ser Ser Arg Arg Ser Trp Trp Pro Leu Pro Leu Leu
-15 -10 -5

Leu Leu Leu Leu Leu Leu Leu Gly Pro Ala Gly Ala Arg Ala Gln Glu
-1 1 5 10

Asp Glu Asp Gly Asp Tyr Glu Glu Leu Val Leu Ala Leu Arg Ser Glu
15 20 25 30

Glu Asp Gly Leu Ala Glu Ala Pro Glu His Gly Thr Thr Ala Thr Phe
35 40 45

His Arg Cys Ala Lys Asp Pro Trp Arg Leu Pro Gly Thr Tyr Val Val
50 55 60

Val Leu Lys Glu Glu Thr His Leu Ser Gln Ser Glu Arg Thr Ala Arg
65 70 75

Arg Leu Gln Ala Gln Ala Ala Arg Arg Gly Tyr Leu Thr Lys Ile Leu
80 85 90

His Val Phe His Gly Leu Leu Pro Gly Phe Leu Val Lys Met Ser Gly
95 100 105 110

Asp Leu Leu Glu Leu Ala Leu Lys Leu Pro His Val Asp Tyr Ile Glu
115 120 125

Glu Asp Ser Ser Val Phe Ala Gln Ser Ile Pro Trp Asn Leu Glu Arg
            130                 135                 140

Ile Thr Pro Pro Arg Tyr Arg Ala Asp Glu Tyr Gln Pro Pro Asp Gly
        145                 150                 155

Gly Ser Leu Val Glu Val Tyr Leu Leu Asp Thr Ser Ile Gln Ser Asp
    160                 165                 170

His Arg Glu Ile Glu Gly Arg Val Met Val Thr Asp Phe Glu Asn Val
175                 180                 185                 190

Pro Glu Glu Asp Gly Thr Arg Phe His Arg Gln Ala Ser Lys Cys Asp
                195                 200                 205

Ser His Gly Thr His Leu Ala Gly Val Val Ser Gly Arg Asp Ala Gly
            210                 215                 220

Val Ala Lys Gly Ala Ser Met Arg Ser Leu Arg Val Leu Asn Cys Gln
        225                 230                 235

Gly Lys Gly Thr Val Ser Gly Thr Leu Ile Gly Leu Glu Phe Ile Arg
    240                 245                 250

Lys Ser Gln Leu Val Gln Pro Val Gly Pro Leu Val Val Leu Leu Pro
255                 260                 265                 270

Leu Ala Gly Gly Tyr Ser Arg Val Leu Asn Ala Ala Cys Gln Arg Leu
                275                 280                 285

Ala Arg Ala Gly Val Val Leu Val Thr Ala Ala Gly Asn Phe Arg Asp
            290                 295                 300

Asp Ala Cys Leu Tyr Ser Pro Ala Ser Ala Pro Glu Val Ile Thr Val
        305                 310                 315

Gly Ala Thr Asn Ala Gln Asp Gln Pro Val Thr Leu Gly Thr Leu Gly
    320                 325                 330

Thr Asn Phe Gly Arg Cys Val Asp Leu Phe Ala Pro Gly Glu Asp Ile
335                 340                 345                 350

Ile Gly Ala Ser Ser Asp Cys Ser Thr Cys Phe Val Ser Gln Ser Gly
                355                 360                 365

Thr Ser Gln Ala Ala Ala His Val Ala Gly Trp Gln Leu Phe Cys Arg
            370                 375                 380

Thr Val Trp Ser Ala His Ser Gly Pro Thr Arg Met Ala Thr Ala Ile
        385                 390                 395

Ala Arg Cys Ala Pro Asp Glu Glu Leu Leu Ser Cys Ser Ser Phe Ser
    400                 405                 410

Arg Ser Gly Lys Arg Arg Gly Glu Arg Met Glu Ala Gln Gly Gly Lys
415                 420                 425                 430

Leu Val Cys Arg Ala His Asn Ala Phe Gly Gly Glu Gly Val Tyr Ala
                435                 440                 445

Ile Ala Arg Cys Cys Leu Leu Pro Gln Ala Asn Cys Ser Val His Thr
            450                 455                 460

Ala Pro Pro Ala Glu Ala Ser Met Gly Thr Arg Val His Cys His Gln
        465                 470                 475

Gln Gly His Val Leu Thr Gly Cys Ser Ser His Trp Glu Val Glu Asp
    480                 485                 490

Leu Gly Thr His Lys Pro Pro Val Leu Arg Pro Arg Gly Gln Pro Asn
495                 500                 505                 510

Gln Cys Val Gly His Arg Glu Ala Ser Ile His Ala Ser Cys Cys His
                515                 520                 525

Ala Pro Gly Leu Glu Cys Lys Val Lys Glu His Gly Ile Pro Ala Pro
            530                 535                 540

Gln Glu Gln Val Thr Val Ala Cys Glu Glu Gly Trp Thr Leu Thr Gly
        545                 550                 555

Cys Ser Ala Leu Pro Gly Thr Ser His Val Leu Gly Ala Tyr Ala Val
    560                 565                 570

Asp Asn Thr Cys Val Val Arg Ser Arg Asp Val Ser Thr Thr Gly Ser
575                 580                 585                 590

Thr Ser Glu Gly Ala Val Thr Ala Val Ala Ile Cys Cys Arg Ser Arg
                595                 600                 605

His Leu Ala Gln Ala Ser Gln Glu Leu Gln
            610                 615

<210>  35

<211>  1507

<212>  DNA

<213>  Homo sapiens


<220>

<221>  CDS
<222>  (54)..(1454)
<223>

<220>
<221>  mat_peptide
<222>  (129)..()
<223>

<400>  35; LP255(b)
ggaggtgacc gcctggtttc cattaggaag tcctggtcag cagcttgggc gag atg      56
                                                           Met
                                                           -25

gca gag tca ggg ctg gcc atg tgg ccg agc ctg ctg ctg ctc ctg ctg      104
Ala Glu Ser Gly Leu Ala Met Trp Pro Ser Leu Leu Leu Leu Leu Leu
            -20                 -15                 -10

ttg ccg ggg ccc ccg ccc gtc gcc ggc ttg gaa gac gct gcc ttc ccc      152
Leu Pro Gly Pro Pro Pro Val Ala Gly Leu Glu Asp Ala Ala Phe Pro
            -5                  -1  1               5

cac ctg ggg gag agc ttg cag ccc ctg ccc cgg gcc tgt ccc ctg cgc      200
His Leu Gly Glu Ser Leu Gln Pro Leu Pro Arg Ala Cys Pro Leu Arg
    10                  15                  20

tgc tcc tgc ccc cga gtc gac act gtg gac tgt gat ggc ttg gac ctt      248
Cys Ser Cys Pro Arg Val Asp Thr Val Asp Cys Asp Gly Leu Asp Leu
25                  30                  35                  40

cga gtg ttc ccg gac aac atc acc aga gcc gct cag cac ctc tcc ctg      296
Arg Val Phe Pro Asp Asn Ile Thr Arg Ala Ala Gln His Leu Ser Leu
                45                  50                  55

cag aac aac cag ctc cag gaa ctc ccc tac aat gag ctg tcc cgc ctc      344
Gln Asn Asn Gln Leu Gln Glu Leu Pro Tyr Asn Glu Leu Ser Arg Leu
            60                  65                  70

agt ggc ctg cga acc ctc aac ctc cac aac aac ctc atc ttc tcc gaa      392
Ser Gly Leu Arg Thr Leu Asn Leu His Asn Asn Leu Ile Phe Ser Glu
        75                  80                  85

ggc ctg cct gac gag gcc ttc gag tcc ctc acc cag ctg cag cac ctc      440
Gly Leu Pro Asp Glu Ala Phe Glu Ser Leu Thr Gln Leu Gln His Leu
    90                  95                  100

tgc gtg gct cac aac aag ctg agc aac gct ggc ctg ccc ccc gac gcc      488
Cys Val Ala His Asn Lys Leu Ser Asn Ala Gly Leu Pro Pro Asp Ala
105                 110                 115                 120

ttc cgc ggc tcc gag gcc atc gcc acc ctc agc ctc tcc aac aac cag      536
Phe Arg Gly Ser Glu Ala Ile Ala Thr Leu Ser Leu Ser Asn Asn Gln
                125                 130                 135

ctc agc tac ctg ccg ccc agc ctg ccg ccc tca ctc gag cgg ctc cac      584
Leu Ser Tyr Leu Pro Pro Ser Leu Pro Pro Ser Leu Glu Arg Leu His
            140                 145                 150

ctg cag aac aat ctc atc tcc aag gtg ccc cga gga gcc ctg agc cgc      632
Leu Gln Asn Asn Leu Ile Ser Lys Val Pro Arg Gly Ala Leu Ser Arg

155 160 165

cag act caa ctc cgt gag ctc tac ctc cag cac aac cag ctg aca gac 680
Gln Thr Gln Leu Arg Glu Leu Tyr Leu Gln His Asn Gln Leu Thr Asp
170 175 180

agt ggc ctg gat gcc acc acc ttc agc aag ctg cat agc ctt gaa tac 728
Ser Gly Leu Asp Ala Thr Thr Phe Ser Lys Leu His Ser Leu Glu Tyr
185 190 195 200

ctg gat ctc tcc cac aac cag ctg acc aca gtg ccc gcc ggc ctg ccc 776
Leu Asp Leu Ser His Asn Gln Leu Thr Thr Val Pro Ala Gly Leu Pro
205 210 215

cgg acc ctg gct atc ctg cac ctg ggc cgc aac cgc atc cgg cag gtg 824
Arg Thr Leu Ala Ile Leu His Leu Gly Arg Asn Arg Ile Arg Gln Val
220 225 230

gag gcg gct cgg ctg cac ggg gcg cgt ggt ctg cgc tat ttg ttg ctg 872
Glu Ala Ala Arg Leu His Gly Ala Arg Gly Leu Arg Tyr Leu Leu Leu
235 240 245

cag cac aac cag ctg ggg agc tca ggg ctg ccc gcc ggg gct ctg cgg 920
Gln His Asn Gln Leu Gly Ser Ser Gly Leu Pro Ala Gly Ala Leu Arg
250 255 260

ccg ctg cgg ggc ctg cac acg ctg cac ctc tat ggc aat ggg ctg gac 968
Pro Leu Arg Gly Leu His Thr Leu His Leu Tyr Gly Asn Gly Leu Asp
265 270 275 280

cgc gtg cct cca gcc ctg ccc cgc cgc ctg cgt gcc ctg gtg ctg ccc 1016
Arg Val Pro Pro Ala Leu Pro Arg Arg Leu Arg Ala Leu Val Leu Pro
285 290 295

cac aac cac gtg gcc gcg ctg ggt gcc cgt gac ctg gtc gcc aca ccg 1064
His Asn His Val Ala Ala Leu Gly Ala Arg Asp Leu Val Ala Thr Pro
300 305 310

ggc ctg acg gag ctt aac ctg gcc tat aac cgc ctg gcc agc gcc cgt 1112
Gly Leu Thr Glu Leu Asn Leu Ala Tyr Asn Arg Leu Ala Ser Ala Arg
315 320 325

gtg cac cac cgg gcc ttc cgc cgg ttg cgt gcc ctg cgc agc ctc gac 1160
Val His His Arg Ala Phe Arg Arg Leu Arg Ala Leu Arg Ser Leu Asp
330 335 340

ctg gca ggg aat cag cta acc cgg ctg ccc atg ggc ctg ccc act ggc 1208
Leu Ala Gly Asn Gln Leu Thr Arg Leu Pro Met Gly Leu Pro Thr Gly
345 350 355 360

ctg cgc acc ctg cag ctg caa cgc aac cag ctg cgg atg ctc gag ccc 1256
Leu Arg Thr Leu Gln Leu Gln Arg Asn Gln Leu Arg Met Leu Glu Pro
365 370 375

gag cct ctg gcc ggc ctg gac caa ctg cgg gag ctc agc ctg gcg cac 1304
Glu Pro Leu Ala Gly Leu Asp Gln Leu Arg Glu Leu Ser Leu Ala His
380 385 390

aac cgg ctc cgg gtc ggc gac atc ggg cca ggc acc tgg cat gag ctc 1352
Asn Arg Leu Arg Val Gly Asp Ile Gly Pro Gly Thr Trp His Glu Leu
395 400 405

caa gcc ctc cag gtc agg cac agg ctg gtt agc cac act gtc ccc agg 1400
Gln Ala Leu Gln Val Arg His Arg Leu Val Ser His Thr Val Pro Arg
410 415 420

gcc cct cca tcc ccc tgc ctg ccc tgc cac gtc cca aac att cta gtt 1448
Ala Pro Pro Ser Pro Cys Leu Pro Cys His Val Pro Asn Ile Leu Val
425 430 435 440

agc tgg taaagcaatc agaacaagaa aatgataaga gtgggttaga aggtgataag      1504
Ser Trp

ggc                                                                  1507

<210>  36

<211>  467

<212>  PRT

<213>  Homo sapiens

<400>  36; LP255(b)

Met Ala Glu Ser Gly Leu Ala Met Trp Pro Ser Leu Leu Leu Leu Leu
-25                 -20                 -15                 -10

Leu Leu Pro Gly Pro Pro Pro Val Ala Gly Leu Glu Asp Ala Ala Phe
                -5                  -1  1               5

Pro His Leu Gly Glu Ser Leu Gln Pro Leu Pro Arg Ala Cys Pro Leu
        10                  15                  20

Arg Cys Ser Cys Pro Arg Val Asp Thr Val Asp Cys Asp Gly Leu Asp
    25                  30                  35

Leu Arg Val Phe Pro Asp Asn Ile Thr Arg Ala Ala Gln His Leu Ser
40                  45                  50                  55

Leu Gln Asn Asn Gln Leu Gln Glu Leu Pro Tyr Asn Glu Leu Ser Arg
                60                  65                  70

Leu Ser Gly Leu Arg Thr Leu Asn Leu His Asn Asn Leu Ile Phe Ser
            75                  80                  85

Glu Gly Leu Pro Asp Glu Ala Phe Glu Ser Leu Thr Gln Leu Gln His
        90                  95                  100

Leu Cys Val Ala His Asn Lys Leu Ser Asn Ala Gly Leu Pro Pro Asp
    105                 110                 115

Ala Phe Arg Gly Ser Glu Ala Ile Ala Thr Leu Ser Leu Ser Asn Asn
120                 125                 130                 135

Gln Leu Ser Tyr Leu Pro Pro Ser Leu Pro Pro Ser Leu Glu Arg Leu
                140                 145                 150

His Leu Gln Asn Asn Leu Ile Ser Lys Val Pro Arg Gly Ala Leu Ser
            155                 160                 165

Arg Gln Thr Gln Leu Arg Glu Leu Tyr Leu Gln His Asn Gln Leu Thr

170                 175                 180

Asp Ser Gly Leu Asp Ala Thr Thr Phe Ser Lys Leu His Ser Leu Glu
    185                 190                 195

Tyr Leu Asp Leu Ser His Asn Gln Leu Thr Thr Val Pro Ala Gly Leu
200                 205                 210                 215

Pro Arg Thr Leu Ala Ile Leu His Leu Gly Arg Asn Arg Ile Arg Gln
                220                 225                 230

Val Glu Ala Ala Arg Leu His Gly Ala Arg Gly Leu Arg Tyr Leu Leu
            235                 240                 245

Leu Gln His Asn Gln Leu Gly Ser Ser Gly Leu Pro Ala Gly Ala Leu
        250                 255                 260

Arg Pro Leu Arg Gly Leu His Thr Leu His Leu Tyr Gly Asn Gly Leu
    265                 270                 275

Asp Arg Val Pro Pro Ala Leu Pro Arg Arg Leu Arg Ala Leu Val Leu
280                 285                 290                 295

Pro His Asn His Val Ala Ala Leu Gly Ala Arg Asp Leu Val Ala Thr
                300                 305                 310

Pro Gly Leu Thr Glu Leu Asn Leu Ala Tyr Asn Arg Leu Ala Ser Ala
            315                 320                 325

Arg Val His His Arg Ala Phe Arg Arg Leu Arg Ala Leu Arg Ser Leu
        330                 335                 340

Asp Leu Ala Gly Asn Gln Leu Thr Arg Leu Pro Met Gly Leu Pro Thr
    345                 350                 355

Gly Leu Arg Thr Leu Gln Leu Gln Arg Asn Gln Leu Arg Met Leu Glu
360                 365                 370                 375

Pro Glu Pro Leu Ala Gly Leu Asp Gln Leu Arg Glu Leu Ser Leu Ala
                380                 385                 390

His Asn Arg Leu Arg Val Gly Asp Ile Gly Pro Gly Thr Trp His Glu
            395                 400                 405

Leu Gln Ala Leu Gln Val Arg His Arg Leu Val Ser His Thr Val Pro
        410                 415                 420

Arg Ala Pro Pro Ser Pro Cys Leu Pro Cys His Val Pro Asn Ile Leu
    425                 430                 435

Val Ser Trp
440

<210>  37

<211>  2397

<212>  DNA

<213>  Homo sapiens

<220>

<221>  CDS

<222>  (133)..(2100)

<223>

<220>

<221>  mat_peptide

<222>  (244)..()

<223>

<400>  37; LP223(b)
tggagaactg gggaggcaga gaccccggct ggccggaggc atgtggaggg gggggcctgg      60

gcgcaaggag aggcccagcg gaagccaagc caccaggccc cccagcgtcc acgcggagca     120

tgaacattga gg atg gcg cgt gcc cgc ggc tcc ccg tgc ccc ccg ctg ccg     171
              Met Ala Arg Ala Arg Gly Ser Pro Cys Pro Pro Leu Pro
                      -35                 -30                 -25

ccc ggt agg atg tcc tgg ccc cac ggg gca ttg ctc ttc ctc tgg ctc       219
Pro Gly Arg Met Ser Trp Pro His Gly Ala Leu Leu Phe Leu Trp Leu
                -20                 -15                 -10

ttc tcc cca ccc ctg ggg gcc ggt gga ggt gga gtg gcc gtg acg tct       267
Phe Ser Pro Pro Leu Gly Ala Gly Gly Gly Gly Val Ala Val Thr Ser
            -5                  -1  1               5

gcc gcc gga ggg ggc tcc ccg ccg gcc acc tcc tgc ccc gtg gcc tgc       315
Ala Ala Gly Gly Gly Ser Pro Pro Ala Thr Ser Cys Pro Val Ala Cys
    10                  15                  20

tcc tgc agc aac cag gcc agc cgg gtg atc tgc aca cgg aga gac ctg       363
Ser Cys Ser Asn Gln Ala Ser Arg Val Ile Cys Thr Arg Arg Asp Leu
25                  30                  35                  40

gcc gag gtc cca gcc agc atc ccg gtc aac acg cgg tac ctg aac ctg       411
Ala Glu Val Pro Ala Ser Ile Pro Val Asn Thr Arg Tyr Leu Asn Leu
                45                  50                  55

caa gag aac ggc atc cag gtg atc cgg acg gac acg ttc aag cac ctg       459
Gln Glu Asn Gly Ile Gln Val Ile Arg Thr Asp Thr Phe Lys His Leu
            60                  65                  70

cgg cac ctg gag att ctg cag ctg agc aag aac ctg gtg cgc aag atc       507
Arg His Leu Glu Ile Leu Gln Leu Ser Lys Asn Leu Val Arg Lys Ile
        75                  80                  85

gag gtg ggc gcc ttc aac ggg ctg ccc agc ctc aac acg ctg gag ctt 555
Glu Val Gly Ala Phe Asn Gly Leu Pro Ser Leu Asn Thr Leu Glu Leu
90 95 100

ttt gac aac cgg ctg acc acg gtg ccc acg cag gcc ttc gag tac ctg 603
Phe Asp Asn Arg Leu Thr Thr Val Pro Thr Gln Ala Phe Glu Tyr Leu
105 110 115 120

tcc aag ctg cgg gag ctc tgg ctg cgg aac aac ccc atc gag agc atc 651
Ser Lys Leu Arg Glu Leu Trp Leu Arg Asn Asn Pro Ile Glu Ser Ile
125 130 135

ccc tcc tac gcc ttc aac cgc gtg ccc tcg ctg cgg cgc ctg gac ctg 699
Pro Ser Tyr Ala Phe Asn Arg Val Pro Ser Leu Arg Arg Leu Asp Leu
140 145 150

ggc gag ctc aag cgg ctg gaa tac atc tcg gag gcg gcc ttc gag ggg 747
Gly Glu Leu Lys Arg Leu Glu Tyr Ile Ser Glu Ala Ala Phe Glu Gly
155 160 165

ctg gtc aac ctg cgc tac ctc aac ctg ggc atg tgc aac ctc aag gac 795
Leu Val Asn Leu Arg Tyr Leu Asn Leu Gly Met Cys Asn Leu Lys Asp
170 175 180

atc ccc aac ctg acg gcc ctg gtg cgc ctg gag gag ctg gag ctg tcg 843
Ile Pro Asn Leu Thr Ala Leu Val Arg Leu Glu Glu Leu Glu Leu Ser
185 190 195 200

ggc aac cgg ctg gac ctg atc cgc ccg ggc tcc ttc cag ggt ctc acc 891
Gly Asn Arg Leu Asp Leu Ile Arg Pro Gly Ser Phe Gln Gly Leu Thr
205 210 215

agc ctg cgc aag ctg tgg ctc atg cac gcc cag gta gcc acc atc gag 939
Ser Leu Arg Lys Leu Trp Leu Met His Ala Gln Val Ala Thr Ile Glu
220 225 230

cgc aac gcc ttc gac gac ctc aag tcg ctg gag gag ctc aac ctg tcc 987
Arg Asn Ala Phe Asp Asp Leu Lys Ser Leu Glu Glu Leu Asn Leu Ser
235 240 245

cac aac aac ctg atg tcg ctg ccc cac gac ctc ttc acg ccc ctg cac 1035
His Asn Asn Leu Met Ser Leu Pro His Asp Leu Phe Thr Pro Leu His
250 255 260

cgc ctc gag cgc gtg cac ctc aac cac aac ccc tgg cat tgc aac tgc 1083
Arg Leu Glu Arg Val His Leu Asn His Asn Pro Trp His Cys Asn Cys
265 270 275 280

gac gtg ctc tgg ctg agc tgg tgg ctc aag gag acg gtg ccc agc aac 1131
Asp Val Leu Trp Leu Ser Trp Trp Leu Lys Glu Thr Val Pro Ser Asn
285 290 295

acg acg tgc tgc gcc cgc tgt cat gcg ccc gcc ggc ctc aag ggg cgc 1179
Thr Thr Cys Cys Ala Arg Cys His Ala Pro Ala Gly Leu Lys Gly Arg
300 305 310

tac att ggg gag ctg gac cag tcg cat ttc acc tgc tat gcg ccc gtc 1227
Tyr Ile Gly Glu Leu Asp Gln Ser His Phe Thr Cys Tyr Ala Pro Val
315 320 325

atc gtg gag ccg ccc acg gac ctc aac gtc acc gag ggc atg gct gcc 1275
Ile Val Glu Pro Pro Thr Asp Leu Asn Val Thr Glu Gly Met Ala Ala
330 335 340

gag ctc aaa tgc cgc acg ggc acc tcc atg acc tcc gtc aac tgg ctg 1323
Glu Leu Lys Cys Arg Thr Gly Thr Ser Met Thr Ser Val Asn Trp Leu
345 350 355 360

acg ccc aac ggc acc ctc atg acc cac ggc tcc tac cgc gtg cgc atc 1371

Thr Pro Asn Gly Thr Leu Met Thr His Gly Ser Tyr Arg Val Arg Ile
                365                 370                 375

tcc gtc ctg cat gac ggc acg ctt aac ttc acc aac gtc acc gtg cag      1419
Ser Val Leu His Asp Gly Thr Leu Asn Phe Thr Asn Val Thr Val Gln
            380                 385                 390

gac acg ggc cag tac acg tgc atg gtg acg aac tca gcc ggc aac acc      1467
Asp Thr Gly Gln Tyr Thr Cys Met Val Thr Asn Ser Ala Gly Asn Thr
        395                 400                 405

acc gcc tcg gcc acg ctc aac gtc tcg gcc gtg gac ccc gtg gcg gcc      1515
Thr Ala Ser Ala Thr Leu Asn Val Ser Ala Val Asp Pro Val Ala Ala
    410                 415                 420

ggg ggc acc ggc agc ggc ggg ggc ggc cct ggg ggc agt ggt ggt gtt      1563
Gly Gly Thr Gly Ser Gly Gly Gly Gly Pro Gly Gly Ser Gly Gly Val
425                 430                 435                 440

gga ggg ggc agt ggc ggc tac acc tac ttc acc acg gtg acc gtg gag      1611
Gly Gly Gly Ser Gly Gly Tyr Thr Tyr Phe Thr Thr Val Thr Val Glu
                445                 450                 455

acc ctg gag acg cag ccc gga gag gag gcc ctg cag ccg cgg ggg acg      1659
Thr Leu Glu Thr Gln Pro Gly Glu Glu Ala Leu Gln Pro Arg Gly Thr
            460                 465                 470

gag aag gaa ccg cca ggg ccc acg aca gac ggt gtc tgg ggt ggg ggc      1707
Glu Lys Glu Pro Pro Gly Pro Thr Thr Asp Gly Val Trp Gly Gly Gly
        475                 480                 485

cgg cct ggg gac gcg gcc ggc cct gcc tcg tct tct acc acg gca ccc      1755
Arg Pro Gly Asp Ala Ala Gly Pro Ala Ser Ser Ser Thr Thr Ala Pro
    490                 495                 500

gcc ccg cgc tcc tcg cgg ccc acg gag aag gcg ttc acg gtg ccc atc      1803
Ala Pro Arg Ser Ser Arg Pro Thr Glu Lys Ala Phe Thr Val Pro Ile
505                 510                 515                 520

acg gat gtg acg gag aac gcc ctc aag gac ctg gac gac gtc atg aag      1851
Thr Asp Val Thr Glu Asn Ala Leu Lys Asp Leu Asp Asp Val Met Lys
                525                 530                 535

acc acc aaa atc atc atc ggc tgc ttc gtg gcc atc acg ttc atg gcc      1899
Thr Thr Lys Ile Ile Ile Gly Cys Phe Val Ala Ile Thr Phe Met Ala
            540                 545                 550

gcg gtg atg ctc gtg gcc ttc tac aag ctg cgc aag cag cac cag ctc      1947
Ala Val Met Leu Val Ala Phe Tyr Lys Leu Arg Lys Gln His Gln Leu
        555                 560                 565

cac aag cac cac ggg ccc acg cgc acc gtg gag atc atc aac gtg gag      1995
His Lys His His Gly Pro Thr Arg Thr Val Glu Ile Ile Asn Val Glu
    570                 575                 580

gac gag ctg ccc gcc gcc tcg gcc gtg tcc gtg gcc gcc gcg gcc gcc      2043
Asp Glu Leu Pro Ala Ala Ser Ala Val Ser Val Ala Ala Ala Ala Ala
585                 590                 595                 600

gtg gcc agt ggg ggt ggt gtg ggc ggg gac agc cac ctg gcc ctg ccc      2091
Val Ala Ser Gly Gly Gly Val Gly Gly Asp Ser His Leu Ala Leu Pro
                605                 610                 615

gcc ctg gag tgagaccacc tcaaccacca ccactacgtg gctgccgcct              2140
Ala Leu Glu

tcaaggcgca ctacagcagc aaccccagcg gcgggggctg cgggggcaaa ggcccgcctg    2200

gcctcaactc catccacgaa cctctgctct tcaagagcgg ctccaaggag aacgtgcaag 2260
agacgcagat ctgaggcggc ggggccgggc gggcgagggg cgtggagccc cccacccagg 2320
tcccagcccg ggcgcagcct gaccgggacc cctccctccc acagcccagc ccaccttctg 2380
ggaccacgca gggaatt 2397

<210> 38
<211> 656
<212> PRT
<213> Homo sapiens

<400> 38; LP223(b)
Met Ala Arg Ala Arg Gly Ser Pro Cys Pro Pro Leu Pro Pro Gly Arg
-35 -30 -25
Met Ser Trp Pro His Gly Ala Leu Leu Phe Leu Trp Leu Phe Ser Pro
-20 -15 -10
Pro Leu Gly Ala Gly Gly Gly Gly Val Ala Val Thr Ser Ala Ala Gly
-5 -1 1 5 10
Gly Gly Ser Pro Pro Ala Thr Ser Cys Pro Val Ala Cys Ser Cys Ser
15 20 25
Asn Gln Ala Ser Arg Val Ile Cys Thr Arg Arg Asp Leu Ala Glu Val
30 35 40
Pro Ala Ser Ile Pro Val Asn Thr Arg Tyr Leu Asn Leu Gln Glu Asn
45 50 55
Gly Ile Gln Val Ile Arg Thr Asp Thr Phe Lys His Leu Arg His Leu
60 65 70 75
Glu Ile Leu Gln Leu Ser Lys Asn Leu Val Arg Lys Ile Glu Val Gly
80 85 90
Ala Phe Asn Gly Leu Pro Ser Leu Asn Thr Leu Glu Leu Phe Asp Asn
95 100 105
Arg Leu Thr Thr Val Pro Thr Gln Ala Phe Glu Tyr Leu Ser Lys Leu
110 115 120
Arg Glu Leu Trp Leu Arg Asn Asn Pro Ile Glu Ser Ile Pro Ser Tyr
125 130 135
Ala Phe Asn Arg Val Pro Ser Leu Arg Arg Leu Asp Leu Gly Glu Leu
140 145 150 155

Lys Arg Leu Glu Tyr Ile Ser Glu Ala Ala Phe Glu Gly Leu Val Asn
                160                 165                 170

Leu Arg Tyr Leu Asn Leu Gly Met Cys Asn Leu Lys Asp Ile Pro Asn
            175                 180                 185

Leu Thr Ala Leu Val Arg Leu Glu Glu Leu Glu Leu Ser Gly Asn Arg
        190                 195                 200

Leu Asp Leu Ile Arg Pro Gly Ser Phe Gln Gly Leu Thr Ser Leu Arg
    205                 210                 215

Lys Leu Trp Leu Met His Ala Gln Val Ala Thr Ile Glu Arg Asn Ala
220                 225                 230                 235

Phe Asp Asp Leu Lys Ser Leu Glu Glu Leu Asn Leu Ser His Asn Asn
                240                 245                 250

Leu Met Ser Leu Pro His Asp Leu Phe Thr Pro Leu His Arg Leu Glu
            255                 260                 265

Arg Val His Leu Asn His Asn Pro Trp His Cys Asn Cys Asp Val Leu
        270                 275                 280

Trp Leu Ser Trp Trp Leu Lys Glu Thr Val Pro Ser Asn Thr Thr Cys
    285                 290                 295

Cys Ala Arg Cys His Ala Pro Ala Gly Leu Lys Gly Arg Tyr Ile Gly
300                 305                 310                 315

Glu Leu Asp Gln Ser His Phe Thr Cys Tyr Ala Pro Val Ile Val Glu
                320                 325                 330

Pro Pro Thr Asp Leu Asn Val Thr Glu Gly Met Ala Ala Glu Leu Lys
            335                 340                 345

Cys Arg Thr Gly Thr Ser Met Thr Ser Val Asn Trp Leu Thr Pro Asn
        350                 355                 360

Gly Thr Leu Met Thr His Gly Ser Tyr Arg Val Arg Ile Ser Val Leu
    365                 370                 375

His Asp Gly Thr Leu Asn Phe Thr Asn Val Thr Val Gln Asp Thr Gly
380                 385                 390                 395

Gln Tyr Thr Cys Met Val Thr Asn Ser Ala Gly Asn Thr Thr Ala Ser
                400                 405                 410

Ala Thr Leu Asn Val Ser Ala Val Asp Pro Val Ala Ala Gly Gly Thr
            415                 420                 425

Gly Ser Gly Gly Gly Gly Pro Gly Gly Ser Gly Gly Val Gly Gly Gly

430                 435                 440

Ser Gly Gly Tyr Thr Tyr Phe Thr Thr Val Thr Val Glu Thr Leu Glu
    445                 450                 455

Thr Gln Pro Gly Glu Glu Ala Leu Gln Pro Arg Gly Thr Glu Lys Glu
460                 465                 470                 475

Pro Pro Gly Pro Thr Thr Asp Gly Val Trp Gly Gly Gly Arg Pro Gly
                480                 485                 490

Asp Ala Ala Gly Pro Ala Ser Ser Ser Thr Thr Ala Pro Ala Pro Arg
            495                 500                 505

Ser Ser Arg Pro Thr Glu Lys Ala Phe Thr Val Pro Ile Thr Asp Val
        510                 515                 520

Thr Glu Asn Ala Leu Lys Asp Leu Asp Asp Val Met Lys Thr Thr Lys
    525                 530                 535

Ile Ile Ile Gly Cys Phe Val Ala Ile Thr Phe Met Ala Ala Val Met
540                 545                 550                 555

Leu Val Ala Phe Tyr Lys Leu Arg Lys Gln His Gln Leu His Lys His
                560                 565                 570

His Gly Pro Thr Arg Thr Val Glu Ile Ile Asn Val Glu Asp Glu Leu
            575                 580                 585

Pro Ala Ala Ser Ala Val Ser Val Ala Ala Ala Ala Ala Val Ala Ser
        590                 595                 600

Gly Gly Gly Val Gly Gly Asp Ser His Leu Ala Leu Pro Ala Leu Glu
    605                 610                 615